# EUROPEAN PATENT SPECIFICATION

(11) **EP 2 431 476 B1**
(45) Date of publication and mention of the grant of the patent: **20.08.2014**
(21) Application number: 11193520.1
(22) Date of filing: 14.02.2008
(51) Int. Cl.: C12P 13/12, C12N 15/52, C12N 15/77

(54) **Coryneform bacteria with glycine cleavage activity**
Coryneforme Bakterien mit Glycin-Spaltungsaktivität
Bactéries coryneformes dotées d'une activité de division de la glycine

(30) Priority: 19.02.2007 EP 07102652
(43) Date of publication of application: 21.03.2012
(62) Divisional of application: 08716849.8
(73) Proprietor: Evonik Degussa GmbH, 45128 Essen (DE)
(72) Inventor: Zelder, Oskar, 67346 Speyer (DE); Schröder, Hartwig, 69226 Nußloch (DE); Klopprogge, Corinna, 68239 Mannheim (DE); Herold, Andrea, 69469 Weinheim (DE); Haefner, Stefan, 67346 Speyer (DE); Patterson, Thomas A., North Attleboro Massachusetts 02760-1624 (US); Yocum, R. Rogers, Lexington Massachusetts 02420 (US); Pero, Janice G., Lexington Massachusetts 02420 (US)

(56) References cited:
- WO-A-2006/138689
- WO-A-2007/020295
- WO-A-2007/090810
- WO-A2-2009/043803
- OKAMURA-IKEDA K ET AL: "CLONING AND NUCLEOTIDE SEQUENCE OF THE GCV OPERON ENCODING THE ESCHERICHIA COLI GLYCINE-CLEAVAGE SYSTEM", EUROPEAN JOURNAL OF BIOCHEMISTRY, BLACKWELL PUBLISHING, BERLIN, DE, vol. 216, no. 2, 1 September 1993 (1993-09-01), pages 539-548, XP008012895, ISSN: 0014-2956, DOI: 10.1111/J.1432-1033.1993.TB18172.X
- KROMER ET AL: "Metabolic pathway analysis for rational design of L-methionine production by Escherichia coli and Corynebacterium glutamicum", METABOLIC ENGINEERING, ACADEMIC PRESS, US, vol. 8, no. 4, 1 July 2006 (2006-07-01), pages 353-369, XP005502384, ISSN: 1096-7176
- DATABASE UniProt [Online] 2 August 2005 (2005-08-02), "SubName: Full=Glycine cleavage system P protein; EC=1.4.4.2;", XP002668713, retrieved from EBI accession no. UNIPROT:Q4JXU6 Database accession no. Q4JXU6 -& TAUCH A ET AL: "Complete Genome Sequence and Analysis of the Multiresistant Nosocomial Pathogen Corynebacterium jeikeium K411, a Lipid-Requiring Bacterium of the Human Skin Flora", JOURNAL OF BACTERIOLOGY, WASHINGTON, DC, US, vol. 187, no. 13, 1 July 2005 (2005-07-01) , pages 4671-4682, XP007904509, ISSN: 0021-9193
- DATABASE UniProt [Online] 2 August 2005 (2005-08-02), "RecName: Full=Glycine cleavage system H protein;", XP002668714, retrieved from EBI accession no. UNIPROT:Q4JXU4 Database accession no. Q4JXU4 & TAUCH A ET AL: "Complete Genome Sequence and Analysis of the Multiresistant Nosocomial Pathogen Corynebacterium jeikeium K411, a Lipid-Requiring Bacterium of the Human Skin Flora", JOURNAL OF BACTERIOLOGY, AMERICAN SOCIETY FOR MICROBIOLOGY, WASHINGTON, DC; US, vol. 187, no. 13, 1 July 2005 (2005-07-01) , pages 4671-4682, XP007904509, ISSN: 0021-9193, DOI: 10.1128/JB.187.13.4671-4682.2005
- DATABASE UniProt [Online] 2 August 2005 (2005-08-02), "RecName: Full=Aminomethyltransferase; EC=2.1.2.10; AltName: Full=Glycine cleavage system T protein;", XP002668715, retrieved from EBI accession no. UNIPROT:Q4JXU5 Database accession no. Q4JXU5 & TAUCH A ET AL: "Complete Genome Sequence and Analysis of the Multiresistant Nosocomial Pathogen Corynebacterium jeikeium K411, a Lipid-Requiring Bacterium of the Human Skin Flora", JOURNAL OF BACTERIOLOGY, AMERICAN SOCIETY FOR MICROBIOLOGY, WASHINGTON, DC; US, vol. 187, no. 13, 1 July 2005 (2005-07-01) , pages 4671-4682, XP007904509, ISSN: 0021-9193, DOI: 10.1128/JB.187.13.4671-4682.2005
- DATABASE UniProt [Online] 2 August 2005 (2005-08-02), "RecName: Full=Lipoyl synthase; EC=2.8.1.8; AltName: Full=Lip-syn; Short=LS; AltName: Full=Lipoate synthase; AltName: Full=Lipoic acid synthase; AltName: Full=Sulfur insertion protein LipA;", XP002668716, retrieved from EBI accession no. UNIPROT:Q4JWE0 Database accession no. Q4JWE0 & TAUCH A ET AL: "Complete Genome Sequence and Analysis of the Multiresistant Nosocomial Pathogen Corynebacterium jeikeium K411, a Lipid-Requiring Bacterium of the Human Skin Flora", JOURNAL OF BACTERIOLOGY, AMERICAN SOCIETY FOR MICROBIOLOGY, WASHINGTON, DC; US, vol. 187, no. 13, 1 July 2005 (2005-07-01) , pages 4671-4682, XP007904509, ISSN: 0021-9193, DOI: 10.1128/JB.187.13.4671-4682.2005
- DATABASE UniProt [Online] 27 June 2006 (2006-06-27), "RecName: Full=Octanoyltransferase; EC=2.3.1.181; AltName: Full=Lipoate-protein ligase B; AltName: Full=Lipoyl/octanoyl transferase; AltName: Full=Octanoyl-[acyl-carrier-protein]-prote in N-octanoyltransferase;", XP002668717, retrieved from EBI accession no. UNIPROT:Q4JWD9 Database accession no. Q4JWD9 & TAUCH A ET AL: "Complete Genome Sequence and Analysis of the Multiresistant Nosocomial Pathogen Corynebacterium jeikeium K411, a Lipid-Requiring Bacterium of the Human Skin Flora", JOURNAL OF BACTERIOLOGY, AMERICAN SOCIETY FOR MICROBIOLOGY, WASHINGTON, DC; US, vol. 187, no. 13, 1 July 2005 (2005-07-01) , pages 4671-4682, XP007904509, ISSN: 0021-9193, DOI: 10.1128/JB.187.13.4671-4682.2005

## Description

### Field of the invention

The present invention relates to methods for producing L-methionine and to Coryneform bacteria which display a functional glycine cleavage system.

### Background of the invention

Currently, the worldwide annual production of methionine is about 500,000 tons. Methionine is the first limiting amino acid in livestock of poultry and feed and, due to this, mainly applied as feed supplement.

In contrast to other industrial amino acids, methionine is almost exclusively applied as a racemate of D- and L-methionine which is produced by chemical synthesis. Since animals can metabolise both stereo-isomers of methionine, direct feed of the chemically produced racemic mixture is possible (D'Mello and Lewis, Effect of Nutrition Deficiencies in Animals: Amino Acids, Rechgigl (Ed.), CRC Handbook Series in Nutrition and Food, 441-490, 1978).

However, there is still a great interest in replacing the existing chemical production by a biotechnological process producing exclusively L-methionine. This is due to the fact that at lower levels of supplementation L-methionine is a better source of sulfur amino acids than D-methionine (Katz and Baker (1975) Poult. Sci. 545: 1667-74). Moreover, the chemical process uses rather hazardous chemicals and produces substantial waste streams. All these disadvantages of chemical production could be avoided by an efficient biotechnological process.

Fermentative production of fine chemicals such as amino acids, aromatic compounds, vitamins and cofactors is today typically carried out in microorganisms such as *Corynebacterium glutamicum (C. glutamicum*), *Escherichia coli* (*E.coli*), *Saccharomyces cerevisiae* (*S. cerevisiae*), *Schizzosaccharomycs pombe* (*S. pombe*), *Pichia pastoris* (*P. pastoris), Aspergillus niger*, *Bacillus subtilis*, *Ashbya gossypii*, *Kluyveromyces lactis*, *Kluyveromyces marxianus* or *Gluconobacter oxydans*.

Amino acids such as glutamate are thus produced using fermentation methods. For these purposes, certain microorganisms such as *Escherichia coli* (*E. coli*) and *Corynebacterium glutamicum* (*C. glutamicum*) have proven to be particularly suitable. The production of amino acids by fermentation also has *inter alia* the advantage that only L-amino acids are produced and that environmentally problematic chemicals such as solvents as they are typically used in chemical synthesis are avoided.

Some attempts in the prior art to produce fine chemicals such as amino acids, lipids, vitamins or carbohydrates in microorganisms such as *E. coli* and *C. glutamicum* have tried to achieve this goal by e.g. increasing the expression of genes involved in the biosynthetic pathways of the respective fine chemicals.

Attempts to increase production of e.g. lysine by upregulating the expression of genes being involved in the biosynthetic pathway of lysine production are e.g. described in WO 02/10209, WO 2006008097, WO2005059093 or in Cremer et al. (Appl. Environ. Microbiol, (1991), 57(6), 1746-1752).

Krömer et al. (Metabolic Engineering 8 (2006) 353-369) analyses theoretical metabolic pathways for L-methionine production by *E. coli* and *C*. *glutamicum.* In order to improve the methionine production from sugar substrates by *C*. *glutamicum* the authors propose introducing the glycine cleavage system into *C*. *glutamicum.*

However, there remains a strong need to identify further targets in metabolic pathways which can be used to beneficially influence the production of methionine in microorganisms such as *C. glutamicum*.

### Object and Summary of the invention

It is one object of the present invention to provide methods for production of L-methionine in microorganisms.

It is a further object of the present invention to provide microorganisms which produce L-methionine.

These and further objects of the invention, as they will become apparent from the ensuing descriptions, are attained by the subject-matter of the independent claims.

Further embodiments of the invention are defined by the dependent claims.

The method uses a microorganism of the genus Corynebacterium. Use of the species *Corynebacterium glutamicum* is particularly preferred.

The present invention relates to methods in which a microorganism, namely a Corynebacterium, is cultivated that is derived by genetic modification from a starting organism such that the enzymatic activity of a glycine cleavage system (GCS) is increased compared to the starting organism.

This may be achieved by genetic modification of a starting organism such that the amount and/or activity of PLP-dependent glycine decarboxylase (*gcvP*, P-protein), lipoamide-containing aminomethyl transferase (*gcvH*, H-protein) and N⁵, N¹⁰-methylene-THF-synthesizing enzyme (*gcvT*, T-protein) are increased compared to the starting organism. These genetic modifications ensure that accumulated glycine is converted to NH₄⁺, CO₂ and N⁵, N¹⁰-methylene-THF. The microorganisms may be cultivated in the presence of lipoic acid and/or lipoamide.

Additionally, the microorganisms are further genetically modified such that they display an increased amount and/or biological activity of lipoic acid synthase (*lipA*), lipoyl tansferase (*lipB*), and/or lipoic acid synthetase (*lplA*).

The cultivated microorganisms may additionally or alternatively be genetically modified to display an increased amount and/or activity of an NAD⁺-dependent, FAD-requiring lipoamide dehydrogenase (*lpd*).

Further disclosed is a method which allows one to produce L-methionine by cultivating microorganisms which have been genetically modified such that the amount and/or biological activity of formate-THF-synthetase is increased and such that a functional glycine cleavage system has been established. Such microorganisms will typically display an increased amount and/or biological activity of formate-THF-synthetase, *gcvH*, *gcvP* and *gcvT* (*gcvHPT*). In one aspect, these latter microorganisms will be cultivated in the presence of lipoic acid and/or lipoamide. Alternatively, or additionally, the microorganisms may be further genetically modified to display an increased amount and/or activity of *lipA*, *lipB* and/or *lplA*. The microorganisms may also display an increased amount and/or biological activity of *lpd*.

The above-described embodiments of methods in accordance with the invention are preferably undertaken by cultivating microorganisms of the species *C. glutamicum.* The above described genetic modifications may be introduced into a *C. glutamicum* wild-type strain. In a preferred embodiments, these genetic alterations are introduced into a *C. glutamicum* strain that already is considered to be a methionine-producing strain.

The coding sequences for the above-mentioned formate-THF-synthetase, *gcvP*, *gcvT*, *gcvH*, *lplA*, *lipA* and *lipB* are preferably derived from *C. jeikeium* or *E.coli*. Sequences of *C. jeikeium* are particularly to be considered in case that the method is undertaken by cultivating *C. glutamicum* strains.

Further disclosed are microorganisms which have been derived by genetic modification from a starting microorganism to produce more N⁵, N¹⁰-methylene-THF in comparison to the starting organism. The microorganisms are selected from the genus Coryneform bacteriua with the species *C*. *glutamicum* being preferred.

In one aspect, the microorganism is derived by genetic modification from a starting organism such that the amount and/or activity of formate-THF-synthetase is increased compared to the starting organism. Further elaborations of this latter aspect comprise microorganisms with a decrease in the amount and/or activity of formate-THF-deformylase and/or with an increase in any of the activities of N⁵, N¹⁰-methenyl-THF-cyclosynthetase, N⁵, N¹⁰-methenyl-THF-reductase and/or N⁵, N¹⁰-methylene-THF-reductase.

Another aspect of the invention relates to Coryneform bacteria which are derived by genetic modification from a starting organism such that the enzymatic activity of a glycine cleavage system is increased in said organism compared to the starting organism.

In addition, the microorganism are genetically further modified to display an increased capacity for uptake of externally provided lipoic acid and/or lipoamide and/or for synthesizing endogenously lipoic acid. To this end, the amount and/or activity of *lplA*, *lipA* and/or *lipB* is increased in said microorganisms. The amount and/or activity of *lpd* may also be increased compared to the starting organism.

The microorganism may be genetically altered in order to display an increased amount and/or activity of *gcvP, gcvT* and *gcvH.*

The microorganism is preferably derived from the species of *C*. *glutamicum.* The genetic alterations can be introduced in a wild-type strain of *C. glutamicum* or in a strain which is already considered to be a methionine-producing strain.

Further disclosed herein are Coryneform bacteria in which the amount and/or biological activity of formate-THF-synthetase, *gcvP*, *gcvT* and *gcvH* are increased compared to the starting organism. In further elaborations of this aspect, the amount and/or activity of *lplA*, *lipA* and/or *lipB* can be increased compared to the starting organism. Alternatively and/or additionally, the amount and/or activity of *lpd* can be increased.

The microorganism is preferably derived from the species of *C. glutamicum.* The genetic alterations can be introduced in a wild-type strain of *C. glutamicum* or in a strain which is already considered to be a methionine-producing strain.

### Figure legends

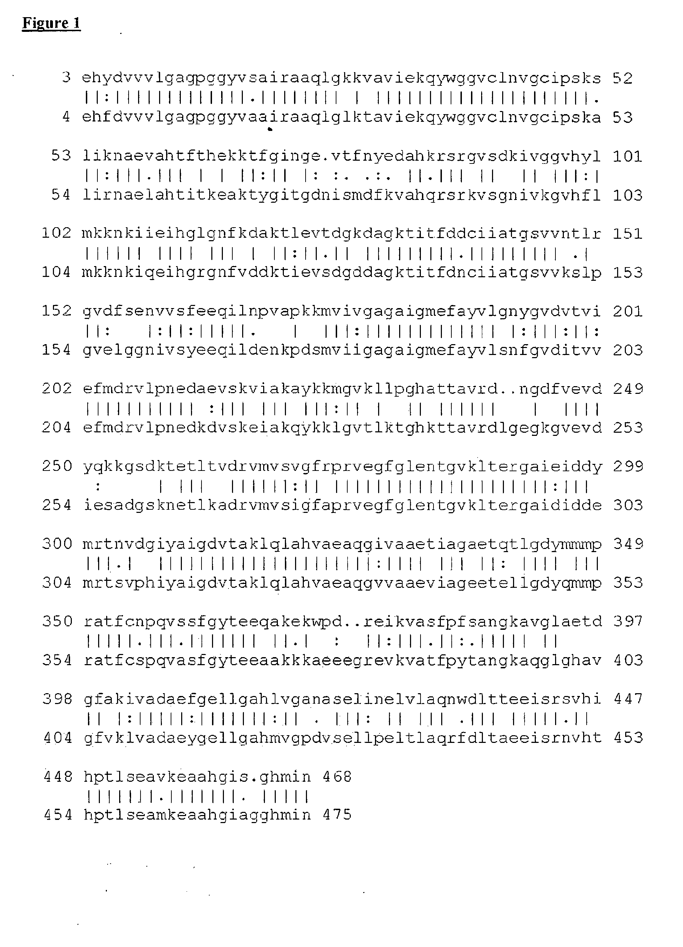

Figure 1 shows a sequence comparison of the amino acid sequence of lpd of *C. jeiekeum* and *C. glutamicum.*

### Detailed description of the invention

The present invention relates to a method of producing L-methionine, comprising the step of cultivating a genetically modified microorganism and optionally isolating methionine. The present invention also relates to a genetically modified microorganism which is capable of producing L-methionine.

The invention is based on the finding that an efficient production of L-methionine (also designated as methionine) can be achieved in microorganisms if such organisms have been genetically modified from a starting organism such that the resulting microorganism produces more N⁵, N¹⁰-methylene-tetrahydrofolate (THF) compared to the starting organism.

Before describing in detail exemplary embodiments of the present invention, the following definitions are given.

A gene name or a protein name, for example, but not limited to, formate-THF-synthetase, *gcvPTH, lipA*, *lipB, lpla, lpd* and any other gene or protein name contained herein, shall refer to either or both the gene and/or the protein or enzyme encoded by said gene, depending on the context in which the name is used.

The term "about" in the context of the present invention denotes an interval of accuracy that the person skilled in the art will understand to be common for the feature in question. The term typically indicates deviation from the indicated numerical value of +/- 10%, and preferably +/- 5%.

The term "microorganism" for the purposes of the present invention refers to prokaryotes and lower eukaryotes.

The organisms of the present invention thus comprise microorganisms as they are known in the art to be useful for production of fine chemicals such as amino acids, vitamins, emzyme cofactors etc. They are selected from the genus of Corynebacterium with a particular focus on *Corynebacterium glutamicum*.

As will be explained in detail by the following description, the present invention is primarily concerned with microorganisms that have been genetically modified in order to display an increased amount and/or activity of certain enzymes.

The terms "genetic modification" and "genetic alteration" as well as their grammatical variations within the meaning of the present invention are intended to mean that a microorganism has been modified by means of gene technology to express an altered amount of one or more proteins which can be naturally present in the respective microorganism, one or more proteins which are not naturally present in the respective microorganism, or one or more proteins with an altered activity in comparison to the proteins of the respective non-modified microorganism. A non-modified microorganism is considered to be a "starting organism", the genetic alteration of which results in a microorganism in accordance with the present invention.

The term "starting organism" therefore can refer to the wild-type of an organism. In the case of *C. glutamicum,* this may e.g. be ATCC13032. However, the term "starting organism" for the purposes of the present invention may also refer to an organism which already carries genetic alterations in comparison to the wild-type organism of the respective species, but which is then further genetically modified in order to yield an organism in accordance with the present invention.

In case of *C*. *glutamicum,* the starting organism may thus be a wild-type *C*. *glutamicum* strain such as ATCC13032. However, the starting organism may preferably also be e.g. a *C. glutamicum* strain which has already been engineered for production of methionine.

Such a methionine-producing starting organism can e.g. be derived from a wild type Coryneform bacterium and preferably from a wild type *C. glutamicum* bacterium which contains genetic alterations in at least one one of the following genes: *ask^{fbr}, hom^{fbr}* and *metH* wherein the genetic alterations lead to overexpression of any of these genes, thereby resulting in increased production of methionine relative to methionine produced in the absence of the genetic alterations. In a preferred embodiment, such a methionine producing starter organism will contain genetic alterations simulatenously in *ask^{fbr}*, *hom^{fbr}* and *metH* thereby resulting in increased production of methionine relative to methionine produced in the absence of the genetic alterations.

In these starting organisms, the endogenous copies of *ask* and *hom* are typically changed to feedback resisteant alleles which are no longer subject to feedback inhibition by lysine threonine, methionine or by a combination of these amino acids. This can be either done by mutation and selection or by defined genetic replacements of the genes by with mutatted alleles which code for proteins with reduced or diminished feedback inhibition. A *C. glutamicum* strain which includes these genetic alterations is e.g. *C. glutamicum* DSM17322. The person skilled in the art will be aware that alternative genetic alterations to those being described below for generation of *C. glutamicum* DSM17322 can be used to also achieve overexpression of *ask^{fbr}, hom^{fbr}* and *metH*.

For the purposes of the present invention, *ask^{fbr}* denotes a feedback resistant aspartate kinase. *Hom^{fbr}* denotes a feedback resistant homoserine dehydrogenase. *MetH* denotes a Vitamin B12-dependent methionine synthase.

In another preferred embodiment, a methionine-producing starting organism can be derived from a wild type Coryneform bacterium and preferably from a wild type *C. glutamicum* bacterium which contains genetic alterations in at least one one of the following genes: *ask^{fbr}*, *hom^{fbr}, metH*, *metA* (also referred to as *metX*), *metY* (also referred to as *metZ),* and *hsk^{mutated}*. wherein the genetic alterations lead to overexpression of any of these genes, thereby resulting in increased production of methionine relative to methionine produced in the absence of the genetic alterations. In a preferred embodiment, such a methionine producing starter organism will contain genetic alterations simulatenously in *ask^{fbr}, hom^{fbr}, metH*, *metA* (also referred to as *metX), metY* (also referred to as *metZ*), and *hsk^{mutated}* thereby resulting in increased production of methionine relative to methionine produced in the absence of the genetic alterations.

In these starting organisms, the endogenous copies of *ask*, *hom* and *hsk* are typically replaced by *ask^{fbr}, hom^{fbr}* and *hsk^{mutated}* as described above for *ask^{fbr}* and *hom^{fbr}*. A *C. glutamicum* strain which includes these genetic alterations is e.g. *C. glutamicum* M2014. The person skilled in the art will be aware that alternative genetic alterations to those being described below specifically for generation of *C. glutamicum* M2014 can be used to also achieve overexpression of *ask^{fbr}, hom^{fbr}, metH*, *metA* (also referred to as *metX*), *metY* (also referred to as *metZ)* and *hsk^{mutated}.*

For the purposes of the present invention, *metA* denotes a homoserine succinyltransferase e.g. from *E. coli. MetY* denotes a O-Acetylhomoserine sulfhydrylase. *Hsk^{mutated}* denotes a homoserine kinase which has been mutated to show reduced enzymatic activity. This may be achieved by exchanging threonine with serine or alanine at a position corresponding to T190 of *hsk* of *C.glutamicum* ATCC 13032 with Genbank accession no. Cgl1184. Alternatively or additionally one may replace the ATG start codon with a TTG start codon. Such mutations lead to a reduction in enzymatic activity of the resulting hsk protein compared the non-mutated *hsk* gene.

In another preferred embodiment, a methionine-producing starting organism can be derived from a wild type Coryneform bacterium and preferably from a wild type *C. glutamicum* bacterium which contains genetic alterations in at least one of the following genes: *ask^{fbr}, hom^{fbr}*, *metH*, *metA* (also referred to as *metX*), *metY* (also referred to as *metZ), hsk^{mutated}* and *metF* wherein the genetic alterations lead to overexpression of any of these genes, in combination with a genetic alterations in one of the following genes: *serA* wherein the genetic alterations decrease expression of this gene where the combination results in increased methionine production by the microorganism relative to methionine production in absence of the combination.

In these starting organisms, the endogenous copy of *ask*, *hom*, *hsk* is replaced as described above and the endogenous copy of *serA* is typically functionally disrupted. A *C. glutamicum* strain which includes these genetic alterations is e.g. *C. glutamicum* OM264C. The person skilled in the art will be aware that alternative genetic alterations to those being described below specifically for generation of *C. glutamicum* OM264C can be used to also achieve overexpression of *ask^{fbr}, hom^{fbr}, metH*, *metA* (also referred to as *metX), metY* (also referred to as *metZ*), *hsk^{mutated}* and *metF* and reduced expression of *serA*.

For the purposes of the present invention, *serA* denotes 3-phosphoglycerate dehydrogenase (see Table 1)

In another preferred embodiment, a methionine-producing starting organism can be derived from a wild type Coryneform bacterium and preferably from a wild type *C. glutamicum* bacterium which contains genetic alterations in at least one of the following genes: *ask^{fbr}, hom^{fbr}, metH*, *metA* (also referred to as *metX), metY* (also referred to as *metZ), hsk^{mutated}* and *metF* wherein the genetic alterations lead to overexpression of any of these genes, in combination with genetic alterations in at least one of the following genes : *mcbR* and *metQ* wherein the genetic alterations decrease expression of any of these genes where the combination results in increased methionine production by the microorganism relative to methionine production in absence of the combination. In a preferred embodiment, such a methionine producing starter organism will contain genetic alterations simulatenously in *ask^{fbr}, hom^{fbr}, metH*, *metA* (also referred to as *metX, metY* (also referred to as *metZ), hsk^{mutated}* and *metF* wherein the genetic alterations lead to overexpression of any of these genes, in combination with genetic alterations in *mcbR* and *metQ* wherein the genetic alterations decrease expression of any of these genes where the combination results in increased methionine production by the microorganism relative to methionine production in absence of the combination.

In these starting organisms, the endogenous copies of *ask*, *hom* and *hsk* are typically replaced as described above while the endogenous copies of *mcbR* and *metQ* are typically functionally disrupted or deleted. A *C*. *glutamicum* strain which includes these genetic alterations is e.g. *C. glutamicum* OM469. The person skilled in the art will be aware that alternative genetic alterations to those being described below specifically for generation of *C. glutamicum* OM469 can be used to also achieve overexpression of *ask^{fbr}, hom^{fbr}, metH*, *metA* (also referred to as *metX), metY* (also referred to as *metZ), hsk^{mutated}* and *metF* and reduced expression of *mcbR* and *metQ*.

For the purposes of the present invention, *metF* denotes a N5,10-methylene-tetrahydrofolate reductase (EC 1.5.1.20). *McbR* denotes a TetR-type transcriptional regulator of sulfur metabolism (Genbank accession no: AAP45010). *MetQ* denotes a D-methionine binding lipoprotein which functiony in methionine import.

In a further preferred embodiment, a methionine-producing starting organism can be derived from a wild type Coryneform bacterium and preferably from a wild type *C. glutamicum* bacterium which contains genetic alterations in at least one of the following genes: *ask^{fbr}, hom^{fbr}, metH*, *metA* (also referred to as *metX), metY* (also referred to as *metZ*), *hsk^{mutated}, metF*, *tkt*, *tal*, *zwf* and *6pgl* wherein the genetic alterations lead to overexpression of any of these genes, in combination with genetic alterations in at least one of the following genes : *mcbR, metQ* and *sda* wherein the genetic alterations decrease expression of any of these genes where the combination results in increased methionine production by the microorganism relative to methionine production in absence of the combination. In a preferred embodiment, such a methionine producing starter organism will contain genetic alterations simulatenously in *ask^{fbr}, hom^{fbr}*, *metH*, *metA* (also referred to as *metX), metY* (also referred to as *metZ*), *hsk^{mutated}, metF*, *tkt*, *tal*, *zwf* and *6pgl* wherein the genetic alterations lead to overexpression of any of these genes, in combination with genetic alterations in *mcbR*, *metQ* and *sda* wherein the genetic alterations decrease expression of any of these genes where the combination results in increased methionine production by the microorganism relative to methionine production in absence of the combination.

A *C. glutamicum* strain which includes these genetic alterations is e.g. *C. glutamicum* GK1259. The person skilled in the art will be aware that alternative genetic alterations to those being described below specifically for generation of *C. glutamicum* GK1259 can be used to also achieve overexpression of *ask^{fbr}, hom^{fbr}*, *metH*, *metA* (also referred to as *metX), metY* (al so referred to as *metZ*), *hsk^{mutated}, metF*, *tkt*, *tal*, *zwf* and *6pgl* and reduced expression of *mcbR*, *metQ* and *sda*.

For the purposes of the present invention, *tkt* denotes transketolase, *tal* denotes transaldolase, *zwf* denotes glucose-6-phosphate-dehydrogenase, *6pgl* denotes 6-phospho-glucono-lactonase and *sda* denotes serine deaminase (see Table 1). The person skilled in the art understands that for increasing the amount and/or activity of *zwf*, one will also increase the amount and/or activity of *opca* which serves as a structural scaffolding protein fo *zwf.* In GK1259, this is achieved by the use of the P_{SOD} promoter which simutenously increases transcription of the pentose phosphate operon comprising *tkt*, *tal*, *zwf* and *6pgl*.

As has been set out above, the genetically modified microorganisms of the present invention are characterized in that the amount of N⁵, N¹⁰-methylene-THF is increased.

Typically, the amount of N⁵, N¹⁰-methylene-THF will be increased in the microorganism in accordance with the present invention compared to the respective starting organism by at least about 2%, at least about 5%, at least about 10%, or at least about 20%. In other preferred embodiments, the amount of N⁵, N¹⁰-methylene-THF will be increased by at least about 30%, by at least about 50% or by at least about 75%. Even more preferred embodiments relate to microorganisms in which the amount of N⁵, N¹⁰-methylene-THF is increased by at least about factor 2, at least about factor 5 or at least about factor 10.

The methods and microorganisms in accordance with the present invention can be used to produce more methionine compared to a situation where the respective starting organism,which has not been genetically modified as outlined below, is cultivated. The microorganisms and methods of the present invention can also be used to increase the efficiency of methionine synthesis.

The term "efficiency of methionine synthesis" describes the carbon yield of methionine. This efficiency is calculated as a percentage of the energy input which entered the system in the form of a carbon substrate. Throughout the invention this value is given in percent values ((mol methionine) (mol carbon substrate (⁻¹ x 100). The term "increased efficiency of methionine synthesis" thus relates to a comparison between the starting organism and the actual Coryneform bacterium in which the amount and/or activity of at least one of the below mentioned enzymes has been increased.

Preferred carbon sources according to the present invention are sugars such as mono-, di- or polysaccharides. For example, sugars selected from the group comprising glucose, fructose, hanose, galactose, ribose, sorbose, lactose, maltose, sucrose, raffinose, starch or cellulose may serve as particularly preferred carbon sources.

The methods and Coryneform bacteria in accordance with the invention may also be used to produce more methionine compared to the starting organism.

The methods and Coryneform bacteria in accordance with the invention may also be used to produce methionine at a faster rate compared to the starting organism. If, for example, a typical production period is considered, the methods and Coryneform bacteria will allow to produce methionine at a faster rate, i.e. the same amount methionine will be produced at an earlier point in time compared to the starting organism. This particularly applies for the logarithmic growth phase.

Methods and Coryneform bacteria in accordance with the invention allow to produce at least about 3 g methionine/l culture volume if the strain is incubated in shake flask incubations. A titer of at least about 4g methionine/l culture volume, at least about 5g methionine/l culture volume or at least about 7g methionine/l culture volume can be preferred if the strain is incubated in shake flask incubations. A more preferred value amounts to at least about 10g methionine/l culture volume and even more preferably to at least about 20 g methionine/l cell mass if the strain is incubated in shake flask incubations.

Methods and Coryneform bacteria in accordance with the invention allow to produce at least about 25 g methionine/l culture volume if the strain is incubated in fermentation experiments using a stirred and carbon source fed fermentor. An titer of at least about 30g methionine/l culture volume, at least about 35g methionine/l culture volume or at least about 40g methionine/l culture volume can be preferred if the strain is incubated in fermentation experiments using a stirred and carbon source fed fermentor. A more preferred value amounts to at least about 50g methionine/l culture volume and even more preferably to at least about 60 g methionine/l cell mass if the strain is incubated in fermentation experiments using a stirred and carbon source fed fermentor.

In a preferred embodiment, the methods and microorganisms of the invention allow to increase the efficiency of methionine synthesis and/or the amount of methionine and/or the titer and/or the rate of methionine synthesis in comparison to the starting organism by at least about 2%, at least about 5%, at least about 10% or at least about 20%. In preferred embodiments the efficiency of methionine synthesis and/or the amount of methionine and/or the titer and/or the rated is increased compared to the starting organism by at least about 30%, at least about 40%, or at least about 50%. Even more preferred is an increase of at least about factor 2, at least about factor 3, at least about factor 5 and at least about factor 10.

The term "metabolite" refers to chemical compounds that are used in the metabolic pathways of organisms as precursors, intermediates and/or end products. Such metabolites may not only serve as chemical building units, but may also exert a regulatory activity on enzymes and their catalytic activity. It is known from the literature that such metabolites may inhibit or stimulate the activity of enzymes (Stryer, Biochemistry (2002) W.H. Freeman & Co., New York, New York).

The term "standard conditions" refers to the cultivation of a microorganism in a standard medium which is not enriched with respect to a particular compound. The temperature, pH and incubation time can vary, as will be described in more detail below.

The standard culture conditions for microorganisms can be taken from the literature, including textbooks such as "Sambrook & Russell, Molecular Cloning - A Laboratory Manual", Cold Spring Harbor Laboratory Press, 3rd edition (2001).

"Minimal media" are media that contain only the necessities for the growth of wild-type or mutant cells, i.e. inorganic salts, a carbon source and water. In the case of mutant cells, a minimal medium can contain one or more additives of substantially pure chemical compounds to allow growth of mutant cells that are deficient in production of such chemical(s).

In contrast, "enriched media" are designed to fulfill all growth requirements of a specific organism, i.e. in addition to the contents of the minimal media, they contain, e.g. amino acids, growth factors, enzyme co-factors, etc.

The term "increasing the amount" of at least one protein compared to a starting organism in the context of the present invention means that a starting micororganism is genetically modified to express a higher amount of e.g. one of the above-mentioned enzymes. It is to be understood that increasing the amount of e.g. one enzyme refers to a situation where the amount of functional enzyme is increased. An enzyme in the context of the present invention is considered to be functional if it is capable of catalysing the respective reaction.

There are various options to increase the amount of a protein in microorganisms such as Coryneform bacteria which are well known to the person skilled in the art. These options include increasing the copy number of the nucleic acid sequences which encode the respective protein, increasing transcription and/or translation of such nucleic acid sequences or combinations thereof. These various options will be discussed in more detail below.

The term "increasing the activity" of at least one protein refers to the situation that at least one mutation is introduced into the respective wild-type sequences of the protein which leads to production of more methionine compared to a situation where the same amount of wild-type protein is expressed. This may achieved by e.g. using enzymes which carry specific mutations that allow for an increased activity of the enzyme. Such mutations may e.g. inactivate the regions of the enzymes that are responsible for feedback inhibition. By mutating these positions by e.g. introducing non-conservative point mutations, the enzyme does not provide for feedback regulation any more and thus the activity of the enzyme is not down-regulated if e.g. more product molecules are produced. Furthermore, the activity of an enzyme can be increased by introducing mutations which increase the catalytic turnover of an enzyme. Such mutations may be either introduced into the endogenous copy of the gene encoding for the respective enzyme, or they may be provided by over-expressing a corresponding mutant from the exogenous nucleic acid sequences encoding such an enzyme. Such mutations may comprise point mutations, deletions or insertions. Point mutations may be conservative (replacement of an amino acid with an amino acid of comparable biochemical and physical-chemical properties) or non-conservative (replacement of an amino acid with another which is not comparable in terms of biochemical and physical-chemical properties). Furthermore, the deletions may comprise only two or three amino acids up to complete domains of the respective protein. To give an example, in case of transketolase of *C. glutamicum* ATCC13032 (Genbank accession no. Cgl1574), a mutation of alanine at a position corresponding to A293 to R and/or alanine at a position corresponding to A327 to T exchange leads to an enzyme with improved enzymatic activity. The person skilled in the art will be able to develop further or alternative mutations based on the information provided herein.

Thus, the term "increasing the activity" of at least one enzyme refers to the situation where mutations are introduced into the respective wild-type sequence to reduce negative regulatory mechanisms such as feedback-inhibition and/or to increase catalytic turnover of the enzyme.

An increase of the amount and/or activity of a protein such as an enzyme may be achieved by different routes, e.g. by switching off inhibitory regulatory mechanisms at the transcriptional, translational or protein level, and/or by increasing gene expression of a nucleic acid encoding for this protein in comparison with the starting organism, e.g. by inducing the endogenous gene or by introducing nucleic acid sequences coding for the protein.

Of course, the approaches of increasing the amount and/or activity of a protein such as an enzyme can be combined. Thus, it is, for example, possible to replace the endogenous copy of an enzyme of Coryneform bacteria with a mutant that encodes for the feedback-insensitive version thereof. If transcription of this mutated copy is set under the control of the strong promoter, the amount and the activity of the respective enzyme is increased. It is understood that in this case the enzyme must still be capable of catalysing the reaction in which it usually participates.

The nucleic acid sequences encoding for a protein such as an enzyme may be of endogenous or exogenous origin. Thus, one may for example increase the amount of a protein such as an enzyme by either increasing expression of nucleic acid sequences that naturally occur within the respective starting microorganism by e.g. chromosomal integration of additional nucleic acid sequences, or by using a strong promoter in front of the endogenous gene. Alternatively or additionally, one may also increase the amount of a protein such as an enzyme by expressing the nucleic acid sequence encoding for a homolog of this enzyme from another organism. Examples for this latter scenario will be put forward below.

Thus, one can e.g. increase the amount of *lpd* in *C. glutamicum* by over-expressing the respective *C. glutamicum* sequence, either from an autonomously replicating vector or from an additionally inserted chromosomal copy (see below) or one may use the corresponding enzymes from e.g. *Bacillus subtilis* or *E.coli* and over-express the enzyme by e.g. use of an autonomously replicable vector.

In some circumstances, it may be preferable to use the endogenous enzymes, as the endogenous coding sequence of e.g. *C. glutamicum* are already optimized with respect to its codon usage for expression in *C. glutamicum*.

If, in the context of the following description, it is stated that the amount and/or activity of a protein such as of a specific enzyme should be decreased in comparison to the starting organism, the above definitions apply *mutatis mutandis*.

Reduction of the amount and/or activity of a protein such as an enzyme may be achieved by partially or completely deleting the nucleic acid sequences encoding the respective protein, by inhibiting transcription by e.g. introducing weak promoters, by inhibiting translation by amending the codon usage accordingly, by introducing mutations into the nucleic acid sequences encoding the respective proteins which render the proteins non-functional and/or combinations thereof.

In the context of the following description, use will be made of the term "functional homolog". The term "functional homolog" for the purposes of the present invention relates to the fact that a certain enzymatic activity may not only be provided by a specific protein of defined amino acid sequence, but also by proteins of similar sequence from other (un)related organisms.

For example, the activity of formate-THF-synthetase can be established in *C. glutamicum* by expressing nucleic acid sequences which encode for the formate-THF-synthetase of *C. jeikeium* (SEQ ID NO.1: nucleic acid sequence, SEQ ID NO. 2: amino acid sequence, gene bank accession number (NP_939608)) or by functional homologs thereof.

Homologues of a protein from other organisms can be easily identified by the skilled person by homology analysis. This can be done by determining similarity, i.e. percent identity between amino acid or nucleic acid sequences for putative homologs and the sequences for the genes or proteins encoded by them (*e.g.*, nucleic acid sequences for formate-THF-synthetase, *gcvH*, *gcvP*, *gcvT*, *lpd*, *lplA*, *lipA*, *lipA*).

Percent identity may be determined, for example, by visual inspection or by using algorithm-based homology.

For example, in order ro determine percent identity of two amino acid sequences, the algorithm will align the sequences for optimal comparison purposes (*e.g.*, gaps can be introduced in the amino acid sequence of one protein for optimal alignment with the amino acid sequence of another protein). The amino acid residues at corresponding amino acid positions are then compared. When a position in one sequence is occupied by the same amino acid residue as the corresponding position in the other, then the molecules are identical at that position. The percent identity between the two sequences is a function of the number of identical positions shared by the sequences (*i.e.*, % identity= # of identical positions/total # of positions multiplied by 100).

Various computer programs are known in the art for these purposes. For example, percent identity of two nucleic acid or amino acid sequences can be determined by comparing sequence information using the GAP computer program described by Devereux et al. (1984) Nucl. Acids. Res., 12:387 and available from the University of Wisconsin Genetics Computer Group (UWGCG). Percent identity can also be determined by aligning two nucleic acid or amino acid sequences using the Basic Local Alignment Search Tool (BLAST^{™}) program (as described by Tatusova et al. (1999) FEMS Microbiol. Lett., 174:247.

At the filing date of this patent application, a standard software package providing the BLAST programme can be found on the BLAST website of the NCBI
(http://www.ncbi.nlm.nih.govBLAST/). For example, if one uses any of the aforementioned SEQ IDs, one can either perform a nucleic acid sequence- or amino sequence-based BLAST search and identify closely related homologs of the respective enzymes in e.g. *E.coli, S. cervisiae, Bacillus subtilis*, etc. For example, for nucleic acid sequence alignments using the BLAST^{™} program, the default settings are as follows: reward for match is 2, penalty for mismatch is -2, open gap and extension gap penalties are 5 and 2 respectively, gap.times.dropoff is 50, expect is 10, word size is 11, and filter is OFF.

Comparable sequence searches and analysis can be performed at the EMBL database (http://www.embl.org) or the Expasy homepage (http://www.expasy.org/). All of the above sequences searches are typically performed with the default parameters as they are preinstalled by the database providers at the filing date of the present application. Homology searches may also routinely be performed using software programmes such as the laser gene software of DNA Star, Inc., Madison, Winconsin, USA, which uses the CLUSTAL method (Higgins et al. (1989), Comput. Appl. Biosci., 5(2) 151).

The skilled person understands that two proteins will likely perform the same function (e.g. provide the same enzymatic activity) if they share a certain degree of identity as described above. A typical lower limit on the amino acid level is typically at least about 25% identity. On the nucleic acid level, the lower limit is typically at least 50%.

Preferred identity grades for both type of sequences are at least about 50%, at least about 60% or least about 70%. More preferred identity levels are at least about 80%, at least about 90% or at least about 95%. These identity levels are considered to be significant.

As used herein, the terms "homology" and "homologous" are not limited to designate proteins having a theoretical common genetic ancestor, but includes proteins which may be genetically unrelated that have, none the less, evolved to perform similar functions and/or have similar structures. The requirement that the homologues should be functional means that the homologues herein described encompasse proteins that have substantially the same activity as the reference protein. For proteins to have functional homology, it is not necessarily required that they have significant identity in their amino acid sequences, but, rather, proteins having functional homology are so defined by having similar or identical activities, e.g., enzymatic activities.

Preferably, an enzyme from another organism than e.g. the host Coryneform bacteria will be considered to be a functional homolog if it shows at least significant similarity, i.e. about 50% sequence identity on the amino acid level, and catalyses the same reaction as its counterpart in the Coryneform bacterium. Functional homologues which provide the same enzymatic activity and share a higher degree of identity such as at least about 60%, at least about 70%, at least about 80% or at least about 90% sequence identity on the amino acid level are further preferred functional homologues.

The person skilled in the art knows that one can also use fragments or mutated versions of the aforementioned enzymes from Corynefrom bacteria and of their functional homologues in other organisms as long as these fragments and mutated versions display the same type of functional activity. Typical functionally active fragments will display N-terminal and/or C-terminal deletions while mutated versions typically comprise deletions, insertions or point mutations.

By way of example, a sequence of *E*. *coli* will be considered to encode for a functional homolog of *C. jeikeum* formate-THF-synthetase if it displays the above-mentioned identity levels on the amino acid level to SEQ ID NO. 2 and displays the same enzymatic activity. Examples can be taken from Table 1. One can also use fragments or e.g. point mutants of these sequences as long as the resulting proteins still catalyse the same type of reaction as the full-length enzymes.

Examples of increasing the amount of an enzyme will be shown below for formate-THF-synthetase, *gcvTHP*, *lplA*, *lipA* and *lipB*. Examples for decreasing the amount of an enzyme will be provided for serine deaminase.

### Increasing the amount and/or activity of formate-THF-synthetase in microorganisms

Disclosed herein are microorganisms in which a starting organism is genetically manipulated such that the amount and/or activity of formate-THF-synthetase is increased in the resulting microorganism compared to the starting organism. Also disclosed are methods of producing methionine in microorganisms comprising the step of cultivating the aforementioned microorganism.

In microorganisms such as *E.coli* and *C. jeikeium* sequences for formate-THF-synthetase are known. In such microorganisms, increasing the amount and/or activity of formate-THF-synthetase will require raising the amount and/or activity of this enzyme above the level of the respective starting organism by e.g. over-expressing nucleic acid sequences encoding for this enzymatic activity.

In *C*. *glutamicum* a formate-THF-synthetase is not known. The following passages describe how a formate-THF-synthetase activity can be established in *C. glutamicum.* The person skilled in the art will nevertheless be aware how the amount and/or activity of a formate-THF-synthetase can be increased in other microorganisms such as *C. jeikeium* and *E.coli*.

Disclosed herein is *inter alia* a *C. glutamicum* microorganism in which the amount and/or activity of formate-THF-synthetase is increased and the use of such a microorganism for producing methionine. This can be achieved by e.g. increasing the copy number of nucleic acid sequences encoding for a formate-THF-synthetase, increasing transcription and/or translation of sequences encoding a formate-THF-synthetase or a combination thereof.

One may use a formate-THF-synthetase from *C*. *jeikeium.* The nucleic acid sequence of this formate-THF-synthetase is depicted in SEQ ID NO. 1, while the amino acid sequence is depicted in SEQ ID NO. 2. The gene bank accession number is YP_250663.1. This sequence is derived from the strain *C.jeikeium* NCTC K11915 which is also designated as K411. A formate-THF-synthetase can also be obtained from the strain strain DSMZ 7171. In this case, the nucleic acid sequence is depicted by SED ID No: 51 and the amino acid sequence is depicted by SEQ ID No. 52.

One may of course also use functional fragments of a formate-THF-synthetase as repesented by SEQ ID Nos. 1 and 2, or functional homologs thereof. Some of the homologs which can be identified by using standard homology searches are depicted in Table 1.

The copy number of nucleic acid sequences encoding formate-THF-synthetase can be increased in a microorganism and preferably in *C. glutamicum* by e.g. either expressing the sequence from autonomously replicating plasmids or by integrating additional copies of the respective nucleic acid sequences into the genome of the microorganism and preferably of *C. glutamicum*.

In case of autonomously replicable vectors , these can be stably kept within e.g. a Coryneform bacterium. Typical vectors for expressing polypeptides and enzymes such as formate-THF-synthetase in *C. glutamicum* include pCliK, pB and pEKO as described in Bott, M. and Eggeling, L., eds. Handbook of Corynebacterium glutamicum. CRC Press LLC, Boca Raton, FL; Deb, J.K. et al. (FEMS Microbiol. Lett. (1999), 175(1), 11-20), Kirchner O. et al. (J. Biotechnol. (2003), 104 (1-3), 287-299), WO2006069711 and in WO2007012078.

In another approach for increasing the copy number of nucleic acid sequences encoding a polypeptide in a Coryneform bacterium, one can integrate additional copies of nucleic acid sequences encoding such polypeptides into the chromosome of *C*. *glutamicum.* Chromosomal integration can e.g. take place at the locus where the endogenous copy of the respective polypeptide is localized. Additionally and/or alternatively, chromosomal multiplication of polypeptide encoding nucleic acid sequences can take place at other loci in the genome of a Coryneform bacterium.

In case of *C. glutamicum,* there are various methods known to the person skilled in the art for increasing the gene copy number by chromosomal integration. One such method makes e.g. use of the vector pK19 sacB and has been described in detail in the publication of Schäfer A, et al. J Bacteriol. 1994 176(23): 7309-7319. Other vectors for chromosomal integration of polypeptide-encoding nucleic acid sequences include or pCLIK int sacB as described in WO2005059093 and WO2007011845.

Another preferred approach for increasing the amount and/or activity of formate-THF-synthetase in microorganisms and particularly in *C. glutamicum* is to increase transcription of the coding sequences by use of a strong promoter.

If the activity of an endogenous formate-THF-synthetase is increased by use of a strong promoter, then the term "strong promoter" means that transcription from the newly introduced promoter is stronger than from the naturally occurring endogenous promoter.

However, in a case where formate-THF-synthetase is expressed in *C. glutamicum* which does not know this type of enzyme, a promoter can be used which is known to provide strong expression of endogenous genes of *C*. *glutamicum.*

Preferred promoters in this context are the promoters P_{SOD} (SEQ ID No. 3), P_{groES} (SEQ ID No 4), P_{EFTu} (SEQ ID No 5), phage SP01 promoter P₁₅ (SEQ ID No 42), and λP_{R} (SEQ ID No 6), also sometimes referred to as lambdaP_{R}. In *C. glutamicum* the λP_{R} promoter can be stronger than the P_{SOD} promoter. The P_{SOD} promoter can be stronger than the P_{groES} promoter, and the P_{groES} promoter can be weaker than the P_{EFTu} promoter or the P₁₅ promoter. The P_{EFTu} promoter can be stronger than the P_{SOD} promoter. However the strength of a promoter in any organism is not necessarily an inherent property of the promoter, since promoter strength can vary widely depending on the context in which the promoter is placed by the genetic engineering.

The increase of the amount and/or activity of formate-THF-synthetase in microorganisms and particularly in *C. glutamicum* will allow the microorganisms to grow on media comprising formate as the carbon source. Furthermore, the use of formate which also occurs as a metabolite during various biosynthetic pathways will also allow to increase the production of N⁵, N¹⁰-methylene-THF. An increased level of N⁵, N¹⁰-methylene-THF will lead to an increased production of methyl-THF and production of methionine.

Also disclosed herein is a method which comprises culturing the above-described microorganisms and optionally isolating methionine.

In further elaborations of the above-described disclosure in which the amount and/or activity of formate-THF-synthetase is incrased in a microorganism and preferably in *C. glutamicum*, the amount of N⁵, N¹⁰-methylene-THF can be further increased by decreasing the amount and/or activity of formyl-THF-deformylase. A nucleic acid sequence for formyl-THF-deformylase is depicted in SEQ ID NO. 7, the amino acid sequence is depicted in SEQ ID NO. 8. Table 1 provides gene bank accession numbers for this enzymatic activity.

Alternatively or additionally, the amount and/or activity of N⁵, N¹⁰-methenyl-THF-cyclosynthetase, N⁵, N¹⁰-methenyl-THF-reductase and/or N⁵, N¹⁰-methylene-THF-reductase are increased compared to the starting organism. In a preferred embodiment, the amount and/or activity of formate-THF-synthetase is increased, the amount and/or activity of formyl-THF-deformylase is decreased and the amount and/or activity of N⁵N¹⁰-methenyl-THF-cyclosynthetase, N⁵, N¹⁰-methenyl-THF-reductase and N⁵, N¹⁰-methenyl-reductase are increased compared to the starting organism. As all of these aforementioned enzymatic activities are present in microorganisms and also in *C. glutamicum* (with the exception of formate-THF-synthetase), it can be preferred to use the endogenous nucleic acid sequences for increasing and/or decreasing the amount and/or activity of the respective enzymatic activities in the microorganisms and preferably in *C. glutamicum.*

Approaches for increasing the amount and/or activity for a protein will be described in detail below. These approaches can, of course, also be applied to formate-THF-synthetase. Approaches for decreasing the amount and/or activity of a protein in a microorganism will be described below. These approaches can, of course, also be applied to the down-regulation of formyl-THF-deformylase.

One can, of course, also use functional homologs of formate-THF-synthetase of *C. jeikeium* or of the other afore-mentioned enzymes. These functional homologs will display the above-mentioned identity grades to either SEQ ID NO. 1 or SEQ ID NO. 2 and provide the same type of enzymatic activity. Accession numbers of formate-THF-synthetase from organisms other than *C. jeikeium* are provided in Table 1.

A preferred aspect relates to *C. glutamicum* microorganisms which express formate THF-synthetase and to the use of these *C. glutamicum* organisms in the production of methionine. These strains may show additionally the above mentioned genetic alterations discussed for formyl-THF-deformylase, N⁵, N¹⁰-methenyl-THF-cyclosynthetase, N⁵, N¹⁰-methenyl-THF-reductase and/or N⁵, N¹⁰-methylene-THF-reductase.

A typical *C. glutamicum* strain that can be used as a starting organism will be a wild-type strain such as ATCC13032. However, it can be preferred to use a starting organism which has already been genetically modified to ensure increased methionine production. Such an organism may display the characteristics of DSM17323 and thus display an increased amount and/or activity of *ask^{fbr}, hom^{fbr}* and *metH.* A preferred starting strain may also have the characteristics of M2014 and display an increased amount and/or activity *of ask^{fbr}, hom^{fbr}, metH, metA, metY,* and *hsk^{mutated}.* Other preferred starting organisms may have the characteristics of OM469 and display an increased amount and/or activity of *ask^{fbr}, hom^{fbr}*, *metH*, *metA*, *metY*, *hsk^{mutated}* and *metF* and display a reduced amount and/or activity *of mcbR* and *metQ.* Yet other preferred starting organisms may have the characteristics of GK1259 and display an increased amount and/or activity of *ask^{fbr}*, *hom^{fbr}, merH, metA, metY, hsk^{mutated}, tkt* (and optionally *g6pdh, zwfa and 6pgl*) and *metF* and display a reduced amount and/or activity of *mcbR*, *metQ* and *sda* or of M2616 and display an increased amount and/or activity of *ask^{fbr}, hom^{fbr}, metH*, *metA*, , *metY*, *hsk^{mutated}, tkt* (and optionally *g6pdh, zwfa and 6pgl*) and *metF* and display a reduced amount and/or activity of *mcbR*, *metQ* and *serA*.

The inventors further found that production of methionine can be further stimulated if one cultivates the above-described microorganisms which display an increased amount and/or activity of formate-THF-synthetase, in a medium containing increased amounts of formate.

This aspect, were formate is purposively added to the culture medium, is particularly preferably performed with strains of *C. glutamicum* that have been genetically modified to display the above activities of formate-THF-synthetase and the other genetic alterations. Again, it will be preferred to increase activity of formate-THF-synthetase by expressing the corresponding sequences of *C. jeikeium* in *C*. *glutamicum* or functional homologs and fragments thereof.

### Microorganisms with increased amount and/or activity of the glycine cleavage system

The present invention in one aspect relates to microorganisms and preferably *C. glutamicum* which display an increased enzymatic activity of the glycine cleavage system. The present invention also relates to methods which make use of these microorganisms for the production of methionine by cultivating said microorganisms and optionally isolating methionine.

In some industrial applications, microorganisms such as *E.coli* or *C. glutamicum*can produce glycine as by-product. The present invention makes use of this by-product by providing microorganisms that display an increased activity of the glycine cleavage system.

The glycine cleavage system of microorganisms is typically comprised of 4 to 5 subunits.

The first subunit is a PLP-dependent glycine decarboxylase (GcvP, also named simply P-protein). The second subunit is a lipoamide-containing amino methyl transferase (GcvH, also names H-protein). The third subunit is a N⁵, N¹⁰-methylene-THF synthesizing enzyme (GcvT). These three factors are sometimes also designated as gcvPTH. The fourth subunit is a NAD⁺-dependent, FAD-requiring lipoamide dehydrogenase (*lpd*, also named simply L-protein). The corresponding genes are names *gcvP*, *gcvT*, *gcvH* and *lpd*, respectively. Examples of this type of GCS are found in *E.coli* and *C. jeikeium.* The *lpd* subunit is typically also shared by at least two other multi-subunit enzymes, namely pyruvate dehydrogenase and α-ketoglutarate dehydrogenase.

If the enzymatic activity of the GCS-system is increased, glycine in excess of that required for cell mass will be preferably metabolised into NH₄⁺, CO₂ and N⁵, N¹⁰-methylene-THF, which can then be used e.g. for increased methionine synthesis. However, some microorganisms such as e.g. *C. glutamicum* lack a native GCS-system. Nevertheless, such organisms will usually have an *lpd* gene that encodes the subunit for use in the aforementioned two enzyme systems.

As will be shown below, the native Lpd protein in *C. glutamicum* is able to function together with a non-native GCS such that only the *gcvP, gcvT* and *gcvH* genes need to be installed and expressed to obtain an active GCS function in *C. glutamicum.* It can however be preferred to also over-express a non-native *lpd* gene, since this gene may be more capable of specifically and efficiently interacting with the *gcvP*, *gcvT* and *gcvH* factors.

Further, it should be noted that in some organisms the glycine cleavage system is comprised of five subunits. For example, in *Bacillus subtilis,* the P-subunit is e.g. divided into two polypeptides, sometimes named P1 and P2, which are encoded by two genes, sometimes called *gcvP1* and *gcvP2.*

The present invention, as mentioned above, relates in a preferred embodiment to microorganisms that have been genetically modified to display an increased glycine cleavage system activity. Such an increased glycine cleavage system activity can be attained by increasing the amount and/or activity of the enzymatic activities encoded by *gcvP, gcvH* and *gcvT.* It will be addressed below how an increased glycine cleavage system activity can be established in *C. glutamicum,* as this represents a preferred embodiment of the present invention.

One aspect of the present invention relates to Coryneform bacteria, and particularly *C. glutamicum,* in which the amount and/or activity of the enzymatic activities encoded by *gcvP*, *gcvH* and *gcvT* (collectively called *gcvPHT*) is increased by genetic alteration compared to the starting organism.

This can be attained by increasing the amount and/or activity of the respective enzymes of *C. jeikeium.* The nucleic acid sequence of *gcvP* is depicted in SEQ ID NO. 9, the amino acid sequence is depicted in SEQ ID NO. 10. The Genbank accession number is CAI36361.1.

The nucleic acid sequence of *gcvH* of *C. jeikeium* is depicted in SEQ ID NO. 11. The amino acid sequence is depicted in SEQ ID NO. 12. The Genbank accession number is CAI36363.1.

The nucleic acid sequence of *gcvT of C. jeikeium* is depicted in SEQ ID NO. 13. The amino acid sequence is depicted in SEQ ID NO. 14. The gene bank accession number is CAI36362.1.

For the purposes of the present invention, it can be preferred to use the above sequences which are derived from *C. jeikeium.*

The activity of a glycine cleavage system in a microorganism and particularly in *C. glutamicum* can be increased by expressing, and preferably over-expressing, the aforementioned sequences, either alone or in combination, with the latter being a particularly preferred embodiment of the present invention. Thus, the present invention particularly relates to *C*. *glutamicum* microorganisms in which the enzymatic activities of *gcvPHT* are concomitantly increased. This may be attained by over-expressing the sequences of *gcvP*, *gcvH* and *gcvT* as depicted by SEQ ID NOS. 9-14, functional fragments thereof, or functional homologs thereof. Functional homologs of the aforementioned sequences of *gcvP*, *gcvH* and *gcvT* can be easily identified through sequence homology searches in the relevant databases and will yield sequences for other organisms such as *E.coli*. Accession numbers for these enzymes from other organisms are provided in Table 1. The use of *gcvP*, *gcvH* and *gcvT* sequences of *C. jeikeium*, particularly in *C. glutamicum,* is preferred, as these genes are clustered in an operon (Tauch et al. (2005) J. Bacteriol., 187, 4671-4682). Moreover, the *lpd* genes of *C. jeikeium* and *C. glutamicum* show a high degree of sequence identity (see Fig. 1). It is reasonable to assume that the *gcvP*, *gcvH* and *gcvT* factors smoothly and efficiently interact with the *lpd* of *C. glutamicum.* The amount and/or activity of *gcvP*, *gcvH* and *gcvT* can be increased in microorganisms and preferably in *C. glutamicum* by the methods mentioned above in the context of formate-THF-synthetase. Thus, one can construct e.g. a functional unit which comprises the coding sequences of *gcvP, gcvH* and *gcvT* and increase the copy number of the nucleic acid sequence comprising this unit by using e.g. autonomously replicating plasmids or plasmids which integrate into the genome of the microorganism and preferably into the genome of *C. glutamicum.*

Alternatively or additionally, one can install a promoter in front of this operon which ensures strong transcription of the coding sequences for *gcvP*, *gcvH* and *gcvT*. Such a promoter may preferably be selected from the group of the P_{EFTu}, P_{groES,} P_{SOD}, P₁₅ and λ_{PR} promoter.

In principle, it is not necessary to increase the amount and/or activity of the *lpd* factor. This factor will typically be present in sufficiently abundant amounts by the host microorganism, which is genetically manipulated in order to increase the amount and/or activity of *gcvP, gcvH* and *gcvT* Nonetheless, in some embodiments of the invention, it can be preferred to also increase the amount and/or activity of *lpd.* To this end, the endogenous sequences of *lpd* may be over-expressed by any of the above-described methods which are put forward in some more detail below. Depending on the similarity of the *lpd* of the starting organism and the *lpd* of the from which *gcvP*, *gcvH* and *gcvT* factors are taken, it can be preferred to increase the amount and/or activity of *lpd by* increasing expression of endogenous or exogenous *lpd.* In this case, one will preferably select the *lpd* of that organism from which the other factors of the glycine cleavage system are taken.

Thus, one embodiment of the present invention relates to microorganisms which are derived from the starting organism such that the resulting microorganism displays an increased amount and/or activity of the factors *gcvP*, *gcvH* and *gcvT*. The invention relates also to methods which use these microorganisms for production of methionine by cultivating the microorganisms and optionally isolating methionine.

A preferred embodiment relates to *C. glutamicum microorganisms* which express the *gcvP*, *gcvH* and *gcvT* factors of *C. jeikeium* as depicted in SEQ ID NOS. 9-14, or functional homologs and functional fragments thereof. The present invention also relates preferably to the use of these *C. glutamicum* organisms in the production of methionine.

A typical *C*. *glutamicum* strain that can be used as a starting organism will be a wild-type strain such as ATCC13032. However, it can be preferred to use a starting organism which has already been genetically modified to ensure increased methionine production. Such an organism may display the characteristics of DSM17323 and thus display an increased amount and/or activity of *ask^{fbr}, hom^{fbr}* and *metH*. A preferred starting strain may also have the characteristics of M2014 and display an increased amount and/or activity of *ask^{fbr}, hom^{fbr}*, *metH*, *metA*, *metY*, and *hsk^{mutated}.* Other preferred starting organisms may have the characteristics of OM469 and display an increased amount and/or activity of *ask^{fbr}, hom^{fbr}*, *metH*, *metA*, , *metY*, *hsk^{mutated}* and *metF* and display a reduced amount and/or activity *of mcbR* and *metQ*.

The present inventors furthermore found that N⁵, N¹⁰-methylene-THF and methionine production can be increased in microorganisms that display an increased activity of the glycine cleavage system as described above, if the microorganisms are (i) provided with external lipoic acid and/or lipoamide and/or (ii) are further genetically modified to produce more lipoic acid and/or lipoamide than the starting organism.

Also disclosed herein methods which make use of the above-described microorganisms that display an increased amount and/or activity of the *gcvP, gcvH* and *gcvT* factors of the glycine cleavage system (and optionally of the *lpd* factor) and which are cultivated in a medium containing increased amounts of lipoic acid and/or lipoamide. For the purposes of this aspect, lipoic acid and/or lipoamide may be added to the medium up to a final concentration of at least about 0,1 mg/1, at least about 1 mg/ml and preferably at least about 10 mg/l..

It is known in the art that lipoamide, sometimes called lipoic acid amide, can substitute for lipoic acid for the lipoylation of enzymes in some organisms (Reed, L.J. et al. (1958) J. Biol. Chem. 232, 143-158). As such, feeding of lipoamide can substitute for feeding of lipoic acid in the invention disclosed herein. In other words, lipoamide, which is commercially available from the same supplier as lipoic acid in its various forms (for example, Sigma-Aldrich catalog numbers T 5875, T 5625, T 1395, T 8260) can also be used to stimulate or increase glycine cleavage activity in organisms and methods of the invention disclosed herein. Both the oxidized and the reduced forms of these two compounds can be used, as well as various salt and esters of any of these forms. Thus, the source of the lipoyl group can vary.

This aspect, where lipoic acid and/or lipoamide is purposively added to the culture medium, is particularly preferably performed with strains of *C. glutamicum* that have been genetically modified to display the activities of the *gcvP*, *gcvH* and *gcvT* factors of a glycine cleavage system. Again, it will be preferred to increase activity of the glycine cleavage system by expressing the corresponding sequences of *C. jeikeium* in *C. glutamicum* or functional homologs and fragments thereof. In a further elaboration of this aspect, invention, the amount and/or activity of the *lpd* factor may be increased by using either the endogenous *C. glutamicum* sequences or exogenous sequences (see Table 1).

Also in the case of *C. glutamicum* will the final concentration of lipoic acid and/or lipoamide in the medium be at least about 0,1 mg/1, at least about 1 mg/l and preferably at least about 10 mg/l.

The microorganisms in accordance with the present invention that display increased activity in the glycine cleavage system resulting from an increased amount and/or activity of the *gcvP, gcvH* and *gcvT* factors are further genetically modified to display an increased amount of internally synthesized lipoic acid and/or lipoamide.

There are two different pathways for making use of lipoic acid and ligating it to target proteins. In *E.coli* these two pathways are designated as the *lplA* dependent and the *lipB* dependent pathway (Morris et al. (1995) J. Bacteriol., 177, 1-10).

The *lplA* gene encodes forlipoyl synthetase (LplA protein). This enzyme activates lipoic acid with ATP and subsequently attaches lipoyl-AMP to *gcvH*.

The *lipB* dependent pathway comprises two enzymes(Morris et al. (1995) J. Bacteriol., 177, 1-10). *LipA* encodes for lipoic acid synthase (LipA protein). The *lipB* gene encodes for lipoyl transferase (LipB protein). *LipA* converts octanoyl-ACP to lipoyl-ACP. In a second step *lipB* attaches the lipoyl moiety to the lipoyl domain of *gcvH* and other lipoylated proteins.

Thus, an increase in the amount and/or activity of *lplA* allows for better incorporation of externally added lipoic acid, while an increase in the amount, type and/or activity of *lipA* and/or *lipB* increases the amount of internally synthesized lipoic acidthat becomes transferred to GcvH.

A nucleic acid sequence of *lplA* of *E. col* is depicted in SEQ ID NO. 15. The amino acid sequence is depicted in SEQ ID NO. 16. The gene bank accession number is EG11796.

The coding sequence for *lipA* of *C. jeikeium* is depicted in SEQ ID NO. 17. The amino acid sequence is depicted in SEQ ID NO. 18. The gene bank accession number is GeneID:3433570.

The nucleic acid sequence for *lipB* of *C. jeikeium* is depicted in SEQ ID NO. 19. The amino acid sequence is depicted in SEQ ID NO. 20. The gene bank accession number is GeneID:3433571.

For the purposes of the present invention, it can be preferred to use the above sequences which are derived from *C. jeikeum*.

A microorganism which displays an increased amount and/or activity of the glycine cleavage system factors *gcvP, gcvH* and *gcvT* can thus be further optimized with respect to N⁵, N¹⁰-methylene-THF and methionine synthesis by increasing the amount and/or activity of *lplA.* To this end, one may increase the expression of *lplA* by making use of SEQ ID NOS. 15, or functional homologs and fragments thereof. Such a microorganism will show a better incorporation of externally added lipoic acid and/or lipoamide and may thus particularly be suitable for those methods in accordance with the present invention in which the microorganisms are cultivated in the medium being supplemented with lipoic acid and/or lipoamide.

The present invention relates to a microorganism which, in addition to the increase in the amount and/or activity of the glycine cleavage system factors *gcvP*, *gcvH* and *gcvT* displays an increased amount and/or activity for *lipA*, *lipB* or *lipA* and *lipB.* A microorganism that in addition to an increased amount and/or activity of *gcvP, gcvH* and *gcvT* displays an increased amount and/or activity of *lipA* and *lipB* is particularly preferred. These microorganisms may show a better formation and accommodation of endogenously synthesized lipoic acid and/or lipoamide and will thus contribute to the production of N⁵, N¹⁰-methylene-THF and methionine.

Of course, these microorganisms can also be used in the methods in accordance with the present invention which pertain to the cultivation of genetically modified organisms with increased glycine cleavage system activity in medium supplemented with lipoic acid and/or lipoamide. In a further elaboration of this aspect, one may produce and use microorganisms which in addition to an increased amount and/or activity of *gcvP*, *gcvH* and *gcvT* show an increased amount and/or activity of *lplA*, *lipA* and *lipB*.

A particularly aspect again relates to *C*. *glutamicum* microorganisms which by way of genetic modification of a starting *C. glutamicum* organism display an increased amount and/or activity of the glycine cleavage system factors *gcvP*, *gcvH* and *gcvT* and which display improved accommodation of the externally provided lipoic acid and/or lipoamide and/or improved formation and accommodation of internally produced lipoic acid and/or lipoamide by being genetically modified in order to display an increased amount and/or activity of *lplA, lipA* and/or *lipB.* Preferred aspects thus relate to *C. glutamicum* microorganisms in which the amount and/or activity of *gcvP, gcvH* and *gcvT* and *lplA* is increased compared to the starting organism. In one embodiment, the *C. glutamicum* microorganism displays an increased amount and/or activity of *gcvP, gcvH* and *gcvT* and *lipA* or *lipB*. Even more preferred is a *C. glutamicum* microorganism which displays an increased amount and/or activity of *gcvP, gcvH* and *gcvT*, *lipA* and *lipB.* A *C. glutamicum* microorganism which displays an increased amount and/or activity of *gcvP, gcvH* and *gcvT*, *lpl*, *lipA* and *lipB* is particularly preferred.

It is understood by the skilled person that the *C. glutamicum* starting organism which is used for introducing the above-mentioned genetic modification may be a wild-type strain such as ATCC13032. However, it can be preferred to use a starting organism which has already been genetically modified to ensure increased methionine production. Such an organism may display the characteristics of DSM17323 and thus display an increased amount and/or activity *of ask^{fbr}, hom^{fbr}* and *metH*. A preferred starting strain may also have the characteristics of M2014 and display an increased amount and/or activity of *ask^{fbr}, hom^{fbr}, metH*, *metA*, *metY*, and *hsk^{mutated}*. Other preferred starting organisms may have the characteristics of OM469 and display an increased amount and/or activity of ask*^{fbr}*, *hom^{fbr}, metH, metA, , metY*, *hsk^{mutated}* and *metF* and display a reduced amount and/or activity *of mcbR* and *metQ*.

The person skilled in the art is furthermore clearly aware that in all of the aforementioned embodiments which relate to an increase in the cleavage glycine system and the improved uptake, formation and accommodation of lipoic acid and/or lipoamide, the starting microorganism which preferably is one of the aforementioned *C. glutamicum* strains displays further genetic modifications with respect to enzymes that are involved in the serine biosynthetic pathway as described above.

A preferred starting strain may thus have the characteristics of OM264C and display an increased amount and/or activity of *ask^{fbr}, hom^{fbr}, metH*, *metA*, , *metY*, and *hsk^{mutated}* and display a reduced amount and/or activity of *serA*. Another preferred starting strain may have the characteristics of GK1259 and display an increased amount and/or activity of ask*^{fbr}, hom^{fbr}*, *metH*, *metA*, , *metY*, *hsk^{mutated}, metF*, *tkt*, *tal*, *zwf* and *6pgl* and display a reduced amount and/or activity *of mcbR*, *metQ* and *sda.*

### Microorganisms with increased amount and/or activity of formate-THF-synthetase and of the glycine cleavage system

Further disclosed herein are microorganisms which combine the properties of the above-mentioned organisms, i.e. increasing the amount and/or activity of formate-THF-synthetase and an increased glycine cleavage system activity. It is understood that the above-described particularly preferred aspects are also to be combined.

Thus, a microorganism will be genetically modified such that it will display an increased amount and/or activity of formate-THF-synthetase, *gcvP*, *gcvH* and *gcvT*.

In a further preferred aspect, the microorganism will be further genetically modified to display an increased amount and/or activity of *lplA*.

A preferred aspect also relates to a microorganism which displays an increased activity of formate-THF-synthetase, *gcvP*, *gcvH* and *gcvT* and *lipA* or *lipB*. Even more preferred are microorganisms which display an increased amount and/or activity of a formate-THF-synthetase, *gcvP*, *gcvH* and *gcvT*, *lipA* and *lipB*.

Another preferred aspect relates to a microorganism that displays an increased amount and/or activity of formate-TBF-synthetase, *gcvP, gcoH and gcvT, lplA*, *lipA* and *lipB*.

The microorganisms may of course also display an increased amount and/or activity of *lpd.*

It is understood that the aforementioned aspects are preferentially realized in a *C. glutamicum* microorganism. Such a *C. glutamicum* strain may be a wild-type strain such as ATCC13032. However, it can be preferred to use a starting organism which has already been genetically modified to ensure increased methionine production. Such an organism may display the characteristics of DSM17323 and thus display an increased amount and/or activity of *ask^{fbr}, hom^{fbr}* and *metH*. A preferred starting strain may also have the characteristics of M2014 and display an increased amount and/or activity of *ask^{fbr}, hom^{fbr}*, *metH*, *metA*, *metY*, and *hsk^{mutated}*. Other preferred starting organisms may have the characteristics of OM469 and display an increased amount and/or activity of *ask^{fbr}, hom^{fbr}*, *metH*, *metA*, , *metY*, *hsk^{mutated}* and *metF* and display a reduced amount and/or activity *of mcbR* and *metQ* or of M2616 and display an increased amount and/or activity of *ask^{fbr}, hom^{fbr}*, *metH*, *metA*, , *metY*, *hsk^{mutated}, tkt* (and optionally *g6pdh*, *zwfa and 6pgl*) and *metF* and display a reduced amount and/or activity of *mcbR*, *metQ* and *serA.*

The person skilled in the art is furthermore clearly aware that in all of the aforementioned aspects which relate to an increase in the cleavage glycine system and the improved uptake, formation and accommodation of lipoic acid and/or lipoamide, the starting microorganism which preferably is one of the aforementioned *C. glutamicum* strains displays further genetic modifications with respect to enzymes that are involved in the serine biosynthetic pathway as described above and/or enzymes involved in the metabolisation of formyl such as formyl-THF-deformylase, N⁵, N¹⁰-methenyl-THF-cyclosynthetase, N⁵, N¹⁰-methenyl-THF-reductase and/or N⁵, N¹⁰-methylene-THF-reductase.

A preferred starting strain may thus have the characteristics of OM264C and display an increased amount and/or activity of *ask^{fbr}, hom^{fbr}*, *metH*, *metA*, , *metY*, and *hsk^{mutated}* and display a reduced amount and/or activity of *serA.* Another preferred starting strain may have the characteristics of GK1259 and display an increased amount and/or activity *of ask^{fbr}, hom^{fbr}*, *metH*, *metA*, , *metY*, *hsk^{mutated}, metF*, *tkt*, *tal*, *zwf* and *6pgl* and display a reduced amount and/or activity of *mcbR, metQ* and *sda*.

For increasing the amount and/or activity of the aforementioned enzymes, one may rely either on the endogenous nucleic acid sequences encoding for these enzymes or one may use exogenous sequences, depending on whether the respective starting microorganism provides the required activity.

In case of a *C. glutamicum* microorganism it will be preferred to use the coding sequences of *C. jeikeium* for increasing the amount and/or activity of formate-THF-synthetase, *gcvP*, *gcvH* and *gcvT*, *lplA*, *lipA*, *lipA* and/or *lpd*. The sequences for these enzymes are depicted in SEQ ID NOS. 1 and 2 and 9 to 20. The person skilled in the art is, of course, aware that one may also use sequences coding for functional homologs or fragments of the aforementioned SEQ ID numbers. Such functional homologs may include sequences from other sources such as *E.coli*. Table 1 provides an overview of possible sequences by reciting corresponding gene bank accession numbers.

The microorganisms and particularly *C*. *glutamicum* microorganisms can be used in the production of methionine by culturing them and optionally isolating methionine. As mentioned above, the modified organisms may be cultivated in a medium supplemented with increased amounts of formate and lipoic acid and/or lipoamide.

The following table provides an overview of some of the enzymes which have been discussed above in more detail. The gene bank accession numbers recited refer to the gene bank which can be found at the website http://www.ncbi.nlm.nih.gov/.

| **Enzyme** | **Gene bank accession number** | **Organism** |
|---|---|---|
| formate-THF-synthetase | NP_939G08, jk0881 DIP1253 SA1553 SAV1732 MW1675 SAR1810 SAS1658 SAOUHSC_01845 SAUSA300_1678 SAB1592c SACOL1782 LSA0947 SH1190 SP_1229 Arth_2901 SPD_1087 spr110 SE1408 SERP1295 SAK_1144 M6_Spy0916 SpyM3_0853 MGAS2096_Spy0986 SPs1053 M5005_Spy_0927 SPy1213 MGAS9429_Spy1030 M28_Spy0899 MGAS10270_Spy1041 L159505 EF1725 stu0791 MGAS10750_Spy1076 STER_0837 spyM18_1165 gbs1089 SMU.1073 str0791 LSEI_1460 SAG1055 LBA1562 lp_1779 LACR_1007 CHY_2385 LVIS_0834 BCE_2187 BCZK1914 BT9727_1938 BALH_1871 BC2101 lin1990 | *C. jeikeum* and others |
| formyl-THF-deformylase | ADD13491 NCg10371 cg10382 cg0457 Cue0400 RHA1_ro02096 nfa52050 Pfl_4436 PSEEN1152 PP_13G7 PFL_4786 PA4314 PA14_56060 HCH_04985 pSPPH_4024 PSPTO_4314 Psyr_4018 Tcr_2051 Csal_2073 Noc_1789 Daro_0056 ebA3467 Patl_0486 | *C. glutamicum* and others |
| N5-formyl-THF-cyclosynthetase | NCgl0845 cgl0881 cg1003 CE0955 DIP0861 SAV3674 SCO3183 SCE87.34 Tfu_0372 nfa49540 MSMEG_5472 Lxx05900 Mmcs_4291 Francci3_4041 ML0181 MAV_1101 RHA1_ro05631 FRAAL6398 MAP0923c Acel_0164 jk1526 aq_1731 | *C. glutamicum* and others |
| N5,N10-methenyl-THF-reductase | NCgl2091, NP_601375 NCg10G20 cg10648 cg0750 CE0659 jk1697 DIP0620 MAV_4323 MAP3463c MSMEG_1647 Rv3356c Mb3391c MT3464 ML0674 RHA1_ro06234 Tfu_2571 SCO4824 SAV3442 Mmcs_1204 Acel_0385 PPA1743 Lxx18630 Arth_1116 | *C. glatamicum* and others |
| N5,N10-methyleneTHF-reductase | NCg12091, NP_601375 | *C*. *glutamicum* |
| PLP-dependent glycine decarboxylase (gcvP) | GeneID:3433827, Q8FE66, P0A6T9, EG11810, g1789269; | *C. jeikeum, E. coli* and others |
| lipoamide- containing aminomethyltransferase (gcvH) | GeneID:3433826, CAA52144.1, P0A6T9, EG10371, g1789271 | *C*. *jeikeum, E. coli* and others |
| N5;N10-methylene-THF synthesizing enzyme (gcvT) | GeneID:3433828, EG11442, g1789272 | *C*. *jeikeum, E. coli* and others |
| lipoamide dehydrogenase (lpd) | GeneID:3433577, CAA52144, Q8FE66, P0A9P0, POA6T9, EG10545, g178G307 | *C*. *jeikeum, E. coli* and others |
| Lip A | GeneID:343570, EG11306, g1786846 | *C*. *jeikeum, E. coli* and others |
| Lip B | GeneID 343571 EG11591 g1786848 | *C. jeikeum. E. coli* and others |
| D-3-phosphoglycerate dehydrogenase (serA) | NCg11235, CE1379 DIP1104 jk1291 nfa42210 MAP3033c Mb3020c MT3074 Rv2996c ML1692 Tfu 0614 SAV2730 SCO5515, Francci3_3637, Lxx13140, CC3215, Jann 0261 CHY_2698, MMP1588, VNG2424G RSP2 CYB_1383, AGR_L_22G4, Atu3706, ZMO1685, tlr325, NP0272A Mbur_2385, Moth_0020, Adeh_1262, SMc00641, RHE CH03454, rrnAC2696, MJ1018, TTE2613, amb3193, AF0813 MK0297, DET0599, CYA 1354, Synpcc7942_1501 syc2486_c, Saro 2680 ELI_01970 MM1753, cbdb_A580, BR1685, MTH970, Mbar_A1431, SPO03355, BruAb1_1670, BAB1_1697, BMEI0349, SYNW0533, Syncc9605_2150, Ava_3759, MA0592 alr1890, Mhun_3063, Sycc9902_0527, RPB_1315, glr2139, RPD_3905, Nwi_2968, RPA4308 SYN_00123, ABC1843 Nham_1119, STH9 bl17401, sl11908, CTC00694 BH1602, GK2247, RPC_4106, SH1200 Pcar 3115 Gmet 2378 SSP1039 BLi02446, BL00647, OB2626, BG10509, Acid345_0115, Dgeo_0710, Pro1436, SAR1801, SAB1582, SAV1724, SA1545, SERP1288, SE1401, SAS1650, MW1666 SAOUHSC_01833, SAUSA300_1670, SACOL1773, ml13875, GSU1198, HH0135, WS1313, Tmden_0875, PMT1431 DR1291 PMT9312_1452, TTC0586, Msp_1145, Atlg17745, TTHA0952, PMM1354, At4g34200, RB6248, PMN2A_0926, CJE0970 Cj0891c, Pcar_0417, CMC149C, At3g19480, aq_1905, jhp0984, HP0397 PH1387 PAB0514, TK1966, C31C9.2, PF1394, Cag 1377 TM1401 Afu2g04490, CG6287-PA, rrnAC1762 AGR_pAT_578, PF0319 Atu5399 PAB2374 OB2286, Adeh_1858, BLi03698, BL03435, TK0683, PH0597, Reut_B3530, GK1954 ABC0220, MK0320, DSY0969, BP0155, Bxe_B1896, BB4474, BPP41001, STH3215, OB2844, CAC0015, RPC_3076, rrnAC2056, RPC_1162, AGR_pAT_470, Atu5328, PP3376, PAE3320 Bd1461, Pfl_2987, Rmet_4234, CNA07520, GK2965, MS1743 VV11546, LA1911, ml11021, MS0068 lp_0785, hn0070, VV2851, ebA6869 RPA2975 Tcr_0627, LIC11992, TTE1946, MA1334, LMOf2365_0095, Sde_3388, lmo0078, LmjF03.0030 SH0752, Rmet 4537, orf19.5263, VP2593 BCE1535, RPD_2906, CPE0054, OB2357, bl17965, BAS1325, BA_1955, GBAA1434, BA1434, Reut_B4747, PFL_2717, PA2263, YPTB3189, YP3611, y3301, YPO0914, GOX0218, ACL032C RSP 3407, VC2481 BT9727_1298 BCZK1299, BMEII0813, BTH_I2298, Reut_B4615, ECA3905, YPTB3910, YP3988, YPO4078, RPB_2550, BruAb2 0769 BRA0453, BAB2_0783, Pfl_2904, plu3605, PAE1038, DSY1673, Sden_3097, NTH10596, SERP1888 blr4558, Rfer_1867, | *C. glutamicum* and others |
| | YER081W, BC1415, Pcar_0629, VF2106 v4096, SPCC4G3.01, SE1879 SAR2389, BB4731, Psyc_0369, TK0551, SCO3478, Csal_1770, XCV1890, Bcep18194_A5027, PM1671, SAOUHSC_02577, SAUSA300_2254, SACOL2296 SAS2196, MW2224, BLi03415, BL02138, Mfla_0724, PSPTO5294, XOO3260, XC_1568, XCC2550, SAB2178 SAV2305, SA2098, PSPPH_4885, XCV2876, SH2023 Adeh_2960, BURPS1710b_2286, BPSL1577, Pcryo_0410, NE1688, YPTB1320, YP1303, t2980 STY3218, Mbar_A2220, Psyr_4852, HI0465, y2896, YPO1288, STM3062, SPA2933, YIL074C, SERP0516, Bxe_A1982, XAC2724, SC3003 BB1050, Afu5g05500, SSP0606, SG2009, SE0622, XCC1825, SBO_2700, PF0370, SBO_3080, SSO_3065, S3098, SF2898 UT189_C3299, c3494, ECs3784 Z4251, JW2880 b2913, SRU_0653, SAB0796, SAR0892, Bd2892, ACIAD3302, Saci_1368, SSP1845, Bcep18194_A4216, Psyr 1043, Csal_0273, PPA2251, DVU0339 PFL_5911, SDY_3169, DDB0230052, SAS0800, MW0812 IL2104, A4626, XC_2364, SAUSA300_0834, SACOL0932 SAV0930, SA0791 Bpro_1736 SMc01622 amb0136 PSPPH 1099 XOO2143, XAC1844, PAB1008, RB6394 LBA0942 MCA1407, PSPTO1215, PH0520, TM0327, SAOUHSC_00866, BG12409 Reut_A2281 ELI_06720, SMc01943 SDY_4350, TTC0431, all8087, GSU1672 Nmul_A0428, BTH_12885, BURPS1710b_1481, BPSL1250 Ta0779 DSY4020 BLi03716, BL03603, amb0195, RSP 3447, UTI89_C4093, ECs4438, Z4978, PSHAa0666, PFL_1001, SBO_3555, Rru_A2456, Dde 1681 BTH_I1700, Pfl_5387, XF2206, S4182 SF3587, c4372 Reut C5898, CPS_2082, SSO_3835, VNG0104G, TTHA0786, Pfl_2771 APE1831 SO0862, PD1255, ST1218, Moth 1954, BB1529, Csal_0096, SAV7481, Bxe_A1055, PP5155 UT189_C3212, CG1236-PA SSO0905, SAK_1826 gbs1847, SAG1806, blr3173, PA0316, ECA0078, DDB0231445, SMa2137 JW5656, b3553, GOX0065, BURPS1710b_2926, BPSL2459, BMA0513, Rmet_2446 SAOUHSC 00142, SAUSA300 0179 SACOL0162, SAS0152, SAR0178, MW0151, SAV0177 SA0171 BPP2132, RScl1034, PP1261 c3405, Dde_3689, CAC0089, SMc02849, mlr7269, PTO0372, BR2177 RSc3131, Mb0749c, MT0753, Rv0728c, DSY3442, SAB0117, Gmet_2695, Noc_2032, SC3578, BruAb1_2150, BAB1_2178, BMEI1952, BTH I1402 | |
| phosphoserine phospatase (serB) | NCg12436, cg2779 CE2417, DIP1863 jk0483, nfa42930, MAP3090c, ML1727, Mb3068c MT3127 Rv3042c, SCO1808, SAV6470, Tfu_0136, CT0173 Psyr_0557, PSPTO4957, PP4909 Sde_1075, HCH_05403, Plut_1948, PSPPH_0550, PA4960, ACIAD3567, Pfl_0506, Pcar_2283, BF2389, BF2300, RB8037, Cag_0409, PFL_0551, PG0653, BT0832, CMI086C, | *C. glutamicum* and others |
| | Csal_2542 Acid345_2803, AF2138 Lxx11750, Rmet_1368, Psyc_1857, AO0090020000345, Afu3g06550, SPBC3H7.07c, Pcryo_2146, SMU 1269 stu1519, stir1519, Reut_A1357, PBPRA0635, Mfla_1890, Rru_A0465, ACL130C, Daro_1962, VV2674, VP2431, YGR208W, Bxe_A2331, VV11730, RSc1640, blr6505, VF0509, CMQ250C, IL1876, Nwi_2345, Bcep18194_A5077, L0085 Z5989, NMB0981, SBO_4451, SSO_4538, S4691, SF4420 ECs5346 JW4351, b4388, SDY_4649, UT189_C5159, c5-473, SAK_0710, gbs0605, SAG0625, MJ1594, ECA0465 BL1792, RPB 3347, NMA1179, GOX1085, RSP 1350, Tcr 1620, SC4423, orf19.5838, NGO1468, YPTB0586, YP3740, y3738, YPO0442, STM4578, SPA4388, t4617, STY4925, RPA2029, VC234, ZMO1137, CC2097 PPA2051 ebA6034, BURPS1710b 2322 BPSL1543, BMA1313, MTH1626, CV3516 CPS_1107, RHE CH02794, AGR_C_3697, Atu2040 RPD_2096, Nmul_A0636, BTH_I2264, Msp_1096 BB3819 BPP3368, MK0121, SPO3353, BP0863, PM1657 SG0398 Mbur_0935, HI1033, NTHI1192, RPC_3257, Nham_2724, Noc 2504 mlr1449, NE0439, BR1391 BMEI0615, BAB1_1410, BruAb1_1387, plu0551, SMc01494, MMP0541, SO1223, Jann_0252, Bpro_2720, MS1758, amb3479, PSHAa0661, MA4429, MM1107, LIC11775, LA2145 Sden_1032 Mbar_A1094, Rfer_1329, MCA1267, ELI_05525, Saro_2259, WS2081, SPO2363, STM2197 NP0274A SC2213 SPA0654, t0658, STY2431 PG1170, rrnAC2717, DDB0230054 CJE0330, Cj0282c, VNG2423G, Tmden_1665, HP0652 jhp0597, CMP085C PAB1207 CMT542C, TK0052 | |
| phosphoserine aminotransferase (serC) | NCg10794, cg0948 NCg10794, CE0903, DIP0784, jk0425, nfa6550, SAV3883, MAP0823c, M2136, Tfu_0246, SCO4366, Mb0908c, MT0907, Rv0884c, Francci3_0082, Lxx17890, BL1660 PPA0483, Jann_0260, SPO3354, GSU3260, ZMO1684, Gmet_3173, Saro_2679, RSP 1351 Rru_A1104, Sde_1332, CG11899-PA, CPE0053, lp_0204, Pcar_2772, BT1153, DSY4684, amb3194, rrnAC3046, mII3876, NP0884A, Adeh_2622 BF2072, BF2018 Nham_1118, Moth_0019 PG1278 Ava_1171, RHE_CH03455, LMOf2365_2816, BT9727_3023, SMc00640, Mbur_0514, AGR_L_2260, Atu3707, all1683, BCE3285, CC3216 30.t00047, Imo2825, Nwi_2969, BruAbl_1672, BR1687 BAB1_1699, SG0990, lin2957, BMEI0347, Tbd_0949, NTHI1335, BCZK2969, PSPPH_3666, CAC0014, CMT252C GOX1446, RPC 4107 CV2301, BCI_0252, Psyr_3646, AF1417 MM2911, BC3249, BH1188, RPA4309 bll7402, DDB0230053, BAS3079, BA_3823, RPB_1314, HI1167, Nmul_A2190, STH3178 L0083, Daro_0984, Pfl_4077, PP1768, HD1382, 253.t00001, LSL_0091, ECA2594, PT00371 Mbar_A1294, BH03780, PFL_4313, PSPTO1746, | *C. glutamicum* and others |
| | GBAA1321, BA3321, Bf1383, BQ02790, Mfla_1687 y2784, YPO1389, CNL05470 RPD_3906, YPTB1414, MA2304 SRU_2207 Daro_1231, RSc0903 ELI_01955, HP0736, Rfer 1570, PA3167, plu1619, MJ0959, ST0602, BG12673, FTT0560c, MS1573, jhp0673, FTL 1018 LIC10315, LA0366, lpp1373, RB6246, BLi01082, BL05093, NE0333, F26H9.5, DP1933, Noc_0172, HCH_04982, PM0837 PMT9312_0035, ebA907 Rmet_0715, Reut_A2576, lpg1418, PBPRA2455, VF0899, str1529, Adeh_2994, BTH_I1966, lp11369,, MM0246, Mbar_A2080, Bcep18194_A4155, Csal 2167 DR1350, gbs1621 BURPS1710b_2651, BPEN_394, rrnAC1999, Mhun_2475, Cj0326, Tmden_0073, BPSL2219 Pcryo_1434, YP1204, MTH1601, GK0649 Tcr_1192, Psyc_1036, PBPRA3292, stu1529, SYN_00124, STM0977 SPA1821, MA1816, SC0931 t1957, STY0977, T1548, CJE0371 BMA1625 Dgeo_1114 XOO2388, SSO2597, BURPS1710b_2998, BMA0433, VV11425, UTI89_C0978 c1045, MK0633 HH0909 ACIAD2647 ABC1531, MCA1420 SSO_0908, S0966; SF0902 BPSL2519 WGLp-486, bbp289 SBO_2193, AO090023000099, Bxe_A0976, IL1359, SDY 2354 ECs0990 Z1253 JW0890 b0907 VV21664, Syncc9605_0014, VP2714 VP1247, XC_2645, XCC1589, SMa1495, BTH_I1634, PSHAa1422, VC1159, cbdb_A581, STH8, VVA0476, SPAC1F12.07, PAB1801, WS0024, VV2958, TTHA0582, CT0070 PMM0035, NMA1894, VV1451 VV12813, VPA0235, BU312 At4g35630, TTC1813, RHE PB00131 NMB1640, CPS 2190 XAC1648, TTC0213, Bpro 1793 Sden_0404, XF2326, At2g17630, PH1308 MMP0391, DET0600 Tbd_2509, VF0339, TTHA0173, NP2578A, VCA0604 Saci_0249, NGO1283, VC0392, SMU 1656 | |
| serine dehydratase (sdaA) | NCgl1583, NCg10939 | *C. glutamicum* |
| serine hydroxymethyltransferase (shmt) | Q93PM7, BA000035, Q8FQR1, Q6NI47, Q4JU69, Q5YQ76, Q73WG1, Q4NIE8, O53441, P59953, Q6ADF0, Q9X794, Q40XZ1, Q82JI0, O86565, Q47MD6, Q4NM56, ORF, Q4NGB0, BX251412, 053615 P66806 Q7U2X3, Q24MM6, Q2ZEP1, Q65DW5, Q426V7, Q5KUI2, Q2RFW7, Q3A934, Q3CJJ0, Q8R887, Q8Y4B2, Q4EPI3, Q71WN9, Q67N41, Q927V4, P39148, Q5HE87, Q2YUJ1, Q9K6G4, Q7SIB6, Q2FF15, Q5WB66, Q4CID1, Q2BG18, Q40L42, Q3AN03, Q8YMW8, 066776 QIYIN1, Q41G88, Q74CR5, Q3GAC7, Q3MBD8, Q2DMQ8, Q7U9J7, Q39V87, Q630T3, Q72XD7, Q2D1V8, Q6HAW9, AE017221, Q5SI56, Q72IH2, Q814V2, Q5HMB0, Q8XJ32, AE017225 Q81JY4, Q3WZQ2, CP000360, Q5NN85, Q3AW18, Q4L7Z4, Q3A4L9, Q26LA5, Q7V4U3, Q6FA66, Q2S9R4, Q3G5N8, Q2SFI7, Q3N8U1, Q6N693, Q82UP9, Q2JT50, Q31CS4, Q5P7P1, Q9HTE9, Q3KDV1, Q26XG3, Q3SGX5, Q5FNK4, Q2ILI1, S30382, Q2YD58, Q4BPZ9, Q2LQM6, Q5N2P9, | *C. glutamicum* and others |
| | Q37615, Q46HB6, P50435, Q37NB6, Q7ND67, Q72CT0, Q2WMW5, Q2CH39, Q7VDS8, Q8U7Y5, Q88AD1, O85718, Q48CP3, Q2JI36, Q8DH33, Q7V335, Q214H7, Q6MLK1, Q3QXZ6, Q2DFI0, Q9WZH9, CP000283 Q37FB0, Q3N0F7, Q4ZM83, Q44AR5, Q8EM73, Q1WTR3, Q2CP12, Q3SRV3, Q3CCS2, Q2W4T2, Q35IU4, Q21WS4, Q2CQJ5, Q4J3C4, Q49Z60, CT573326 Q4C6H0, Q31ZN2, Q607U4, P24060, Q4BQS8, Q41LQ8, Q7UQN2, Q2YN95, Q2RTB8, Q3P773, Q46RR4, Ser Q47IH1, Q3JGP5, CT573326, Q21NP8, Q3F809, Q2T437, Q3F764, Q88R12, S15203, Q4K4P6, Q5X722, Q8YGG7, Q3VCK5, Q5WYH4, CP000271, Q1UEA8, Q4LV45, Q8G1F1, Q9I138, P77962 P34895, Q62DI5, Q1QMB9, Q1V9T1, Q2BLZ4, Q30YL7, Q8XTQ1, Q92QU6, Q97GV1, Q39A26, Q45D73, AM180252, Q3WQZ9, Q9KMP4, Q2KA25, Q4LY56, Q2S4G9, Q8D7G5, Q36MR4, Q28N04, Q3K5K9, CP000254, BA000038, Q4B4P5, Q2FLH5, Q7NYI8, Q7MEH7, Q6N622, Q2RVA2, Q3XRF3, Q303B4, Q7N216, Q47WY2, Q4UQT6, Q481S6, Q4BM61, Q4BA21, Q3FFQ1, Q3HGC4, Q87I03, Q3FB08, Q5LPA8, Q88UT5, Q92XS8, Q3QH38, Q34W82, Q39J72, Q8Y1G1, CP000152, AM236080, H97501 Q391K1, Q8UG75, Q21V29, Q474L3, Q8KC36, Q3APN5, CP000124 Q3BXI8, Q62I16, Q831F9, QIQE01, Q3CX04, Q2NZ83, Q8PPE3, CP000352, Q3S0V7, Q2AFR6, Q8TK94, CP000086 Q2BI80, Q2G646, Q3J9K8, Q47XG4, Q2SYS4, Q1YWG2, Q73GC3, Q44LK7, Q33Y20, Q2NS25, Q2CGY4, Q5GTS7, Q36D93, AE008384, Q8PZQ0, Q9HVI7, Q983B6, CP000270 Q3R0R3, Q2Z5R9, Q1VX33, Q4ZNH2, Q4FUZ8, Q72PY2, Q48DU7, Q3VPD3, Q6LHN7, AE009442 Q87AS2, Q3B2I7, Q87WC1, Q7WFD2, Q4K5R9, Q1R8I4, P0A826, CT573326 Q3WKF8, Q2ZQD2, Q8EBN8, Q8XA55, Q3ZZG3, Q2J6M3, Q2DUP7, Q9XAZ1, Q3K6J0, Q3Q439, Q9A8J6, Q7W400, Q3YZ04, Q32D21, Q5F8C0, Q4AMK6, Q6D246, Q3R828, E82743, Q9PET2, Q3P6F8, Q9XAY7, Q3NK51, Q3Z9B9, Q6G3L3, Q88Q27, Q1ZIE9, Q31FS6, P56990 Q9XB01, Q3DHL3, Q6LU17, Q7W116, H82258, Q9KTG1, Q8E5C6, Q57LF7, Q3IRX5, Q3K122, Q7WPH6, AP008231, Q1YU48, Q3D8P3, Q8DPZ0, Q97R16, Q46A52, Q6F211, Q1PZE1, Q8L372, B48427, Q2KV15, Q1RGX5, Q43K52, Q3VUL2, Q31123, Q1ZPS2, Q2NAR9, Q8DU67, Q9CHW7, Q6CZV5, Q3XBK9, H84295 Q4FLT4, Q1UZA1, Q8DFC9, Q74LC1, Q488N6, Q2C6B3, Q65T08, Q1Z7P1, F75567, Q9HPY5, Q9RYB2, Q1V311, Q87RR2, Q3GI80, Q6G009, Q8ZCR1, Q5QXT4, Q5V3D7, Q2ST43, Q5E706, Q8Z2Z9, Q1XXG3, Q5PBM8, Q6MS85, Q3EFW1, Q7QM11, Q2BUE3, Q48TK6, Q5FMC0, BA000034, Q1U7W2, Q8P122, Q8K7H8, Q99ZP1, Q5M0B4, Q5XC65, Q83BT3, Q2GEI3, Q4QM19, CP000262 Q84FT0, Q5M4W1, CP000260 Q1QU94, Q4HIU1, P43844, Q40IP4, Q5NFJ3, Q2A498, Q92GH7, Q2GLH3, | |
| | 008370, A871942, Q68W07, Q4UK96, Q4HBL3, Q30P60, Q26C95, Q38WJ7, Q3YRD1, P59432, Q7P9P7, JQ1016, P57830, P24531, P34894, Q5HW65, Q2X6F1, Q2JFD4, Q2NTIT8, Q30R29, CP000238, Q6YR37, Q8A9S7, Q5LD58. Q5FG30, 051547, Q4HNY8, Q4HFT7, Q8K9P2, P57376, Q6AM21, Q3W273, Q660S1, P78011, Q6KHH3, Q.4A6A3, Q98QM2, Q492D5, Q2DZD3, Q89HS7, Q7MAR0, Q7MXW0, Q8D253, Q8EWD1, Q7NBH8, Q7VFL1, Q4QTL54, P56089, Q3W5W4, Q601P7, Q2E435, Q7VRR4, Q-4A8E1, P47634, Q4AAB2, Q9ZMP7, Q82J74, Q1VNH3, Q50LF3, Q3WZI8, Q9K4E0, Q8KJG9, Q98A81, 140886 P50434, Q9W457, Q30K91 Q30K95, Q30K92, Q30K98, Q30K94, Q30K93, Q5H888, Q29H49, Q1UKA7, Q3KLR8, Q6U9U4, Q56F03, Q268J4, Q275S8, Q41358, Q758F0, Q6CLQ5, CH476726, Q94JQ3, T05362, Q5L6P4, AJ438778, Q5B0U5, S24342, P07511, Q7SXN1, Q2KIP4, Q5E9P9, S65688 | |
| methylene tetrahydrofolate reductase (metF) | Cg12171, EG11585, g1790377 | *C glutamicum, E. coli* and others |
| cob(I)alamin dependent methionine synthase I (metH) | Cg11139, cg1701, CE1637, DIP1259, nfa31930, Rv2124c, Mb2148c, ML1307, SCO1657, Tfu_1825, SAV6667, MT2183, GOX2074 tll1027, syc0184_c, alr0308, slr0212 gll0477, SYNW1238 TTC0253 TTHA0618, PMT0729, Pro0959, PMN2A_0333, PMM0877, WS1234, BH1630, GK0716, BCE4332, ABC1869, BC4250, BCZK4005, BT9727_3995, BA_4925, GBAA4478, BA4478, BAS4156, BLi01192, BL01308, MAP1859c, BruAb1_0184, BMEI1759, BR0188, SMc03112, MCA1545, AGR_C_3907, Atu2155 DR0966 RB9857, ebA3184, VC0390, RPA3702, VV11423, VV2960, VP2717, NE1623, VF0337, LIC20085, LB108, YPTB3653, YPO3722, y0020, YP3084, CV0203, SPA4026, MS1009 SC067, SO1030, DP2202 STM4188 STY4405 t4115 PP2375, PFL_3662, Z5610 ECs4937, c4976, JW3979, b4019, SF4085, S3645, BB4456, BPP3983, BP3594, bll1418, CPS_1101, Psyr_2464 PSPT02732 R03D7 1, PSPPH_2620, PBPRA3294 Daro 0046 PA1843, ECA3987 CT1857, CAC0578, ACIAD1045 Psyc_0403, 4548, DDB0230138, BF3039, BF3199 BT0180 238505 GSU2921 STH2500 XC_2725, XCC1511, XOO2073, TTE1803, RSc0294 XAC1559,BPSL0385, DVU1585, CTC01806 CC2137 TM0268 ZMO1745, FN0163, BG13115, lin1786, SAG2048, gbs2004, LMOf2365_1702, lmo1678, SE2381 SERP0035, MW0333, SAS0333, SMU 874, SA0345, SAV0357, SACOL0429 SAR0354, SH2637 | |
| O-acetylbomoserine sulfhydrolase | NCg10625, cg0755 CE0679 DIP0630, jk1694, MAP3457, Mb3372, MT3443 Rv3340, nfa35960, Lxx18930 Tfu_2823, CAC2783 GK0284, BH2603, lmo0595, lin0604, LMOf2365_0624, ABC0432, TTE2151, BT2387, STH2782, str0987 stu0987 BF1406, SH0593, B1342, 1p_2536, L75975, OB3048, B0933, LIC11852, LA2062 | |
| | BMAA1890 BPSS0190, SMU.1173, BB1055, PP2528, PA5025, PBPRB1415 GSU1183. RPA2763, WS1015, TM0882, VP0629, BruAb1_0807. DMEI1166. BR0793. CPS 2546, XC_1090. XCC3068, plu3517, PMT0875 SYNW0851, Pro0800, CT0604, NE1697 RB8221, bll1235, Syc1143_c ACIAD3382, ebA6307 RSc1562, Daro_2851, DP2506 DR0873 MA2715, PMM0642, PMN2A_0083, IL2014, SPO1431, ECA0820 AGR_C_2311, Atu1251, mlr8465, SMc01809, CV1934, SPBC428.11 PM0738, SO1093, SAR11_1030 PFL_0498, CTC01153 BA_0514, BCE5535, BAS5258, GBAA5656, BA5656, BCZK5104, TTHA0760, TTC0408 BC5406, BT9727_5087 HH0636 YLR303W ADL031W, CJE1895, spr1095, rmAC2716, orf19.5645, Cj1727c, VNG2421G, PSPPH_1663, XOO1390, Psyr_1669 PSPT03810 MCA2488, TDE2200, FN1419, PG0343, Psyc_0792 MS1347 CC3168 Bd3795, MM3085, 389 t00003, NMB1609, SAV3305, NMA1808 GOX1671, APE1226, XAC3602, NGO1149, ZMO0676, SCO4958, lpl0921, lpg0890, lpp0951, EF0290, BPP2532, CBU205, BP3528, BLi02853, BL02018, BG12291, CG5345-PA, HP0106, ML0275, jhp0098, At3g57050, 107869, HI0086, NTHM100, SpyM3_0133, SPs0136, spyM18_0170, M6_Spy0192, SE2323, SERP0095, SPv0172,PAB0605, DDB0191318 ST0506, F228.6, PTO1102, CPE0176, PD1812 XF0864, SAR0460, SACOL0503, S0419, Ta0080 PF12,66, MW0415, SAS0418 SSO2368, PAE2420, TKT1449, 1491 TVN0174, PH1093, VF2267, Saci 0971 VV11364, CMT389C VV3008 | |
| aspartate kinase (ask) | Cg10251, NCg10247, CE0220, DIP0277 jk1998, nfa3180, Mb3736c, MT3812, Rv3709c, ML2323, MAP0311c, Tfu_0043, Francci3_0262 SCO3615, SAV4559 Lxx03450, PPA2148 CHY_1909, MCA0390, cbdb_A1731, TWT708, TW725 Gmet_1880, DET1633, GSU1799, Moth_1304, Tcr_1589 Mfla_0567, HCH_05208, PSPPH_3511, Psyr_3555, PSPTO1843, CV1018, STH1686, NMA1701, Tbd_0969, NMB1498, Pcar_1006, Daro_2515, Csal_0626, Tmden_1650, PA0904 PP4473 Sde_1300, HH0618, NGO0956, ACIAD1252, PFL_4505 ebA637 Noc_0927 WS1729 Pcryo_1639 Psyc_1461, Pfl 4274, LIC12909 LA0693, Rru_A0743, NE2132 RB8926 Cj0582, Nmul_A1941, SYN_02781, TTHA0534, CJE0685 BURPS1710b_2677, BPSL2239, BMA1652, RSc1171, TTC0166, RPA0604 BTH_11945, Bpro_2860 Rmet_1089, Reut_A1126, RPD_0099, Bxe_A1630, Bcep18194_A5380, aq_1152. RPB_0077, Rfer_1353, RPC 0514 BH3096, BLi02996, B00324, amb1612, tlr1833, jhp1150, blr0216, Dde 2048 BB1739 BPP2287, B1913, DVU1913, Nwi_0379, ZMO1653, Jann_3191, HP1229 Saro_3304, Nham_0472 CBU_1051, slr0657, SPO35, Synpcc7942_1001, BG10350, BruAb1_1850 Babe1_1874, BMEI0189, | |
| | BT9727_1658, syc0544_d. BR1871, gll1774, BC1748, mll3437, BCE1883, ELI_14545, RSP 1849, BCZK1623, BAS1676, BA_2315, GBAA1811, BA1811, Ava_3642, alr3644, PSHAa0533, AGR_L_1357, Atu4172, lin1198. BH04030, PMT9312_1740, SMc02438, CYA_1747, RHE_CH03758, 1mo1235, LMOf2365_1244. PMN2A_1246, CC0843 Pro1808, BQ03060, PMT0073, Syncc9902_0068. GOX0037, CYB 0217 | |
| homoserine dehydrogenase (hom) | Cgl1183, cg1337, NCgll136, CE1289, DIP1036, jk1352, nfa10490, SAV2918, Mb1326, MT1333, Rv1294, SCO5354, MAP2468c, ML1129, Francci_3725, Tfu_2424, Lxx06870, PPA1258, Moth_1307, BL1274, CHY 1912 DSY1363 GK2964 CAC0998 BLi03414, BL02137, BC5404, STH2739 BCZK5102, BT9727_5085, Gmet_1629, BCE5533, BB1926, BP2784, CTC02355 BG10460, BPP2479, BAS5256, BA_0512, GBAA5654, BA5654, Synpcc7942_2090, Syc2003_c, Adeh_1638, CYA_1100, Pcar_1451, Mfla_1048, Mfla_0904, TW329 TWT439 BH3422, all4120, Daro_2386, gl14295, ebA4952, Ava_0783, Syncc9605_1957 LSL_1519, OB0466, 1mo2547, PMT1143 Bro_2190, SYNW0711, LMOf2365_2520, lin2691, sl10455, CV0996 RSc1327, PMT9312_1062, ABC2942, Bcep18194_A5155, BURPS1710b 2396 BPSL1477 BMA1385, NMA1395 NMB1228, tl10277, Syncc9902_0704, GSU1693 Bxe_A2381, MCA0597 NGO0779, CYB_1425, BTH 12198 BMEI0725, Rmet_1966, Rfer_1912, SMc00293, BruAb1_1275 BAB1_1293, SYN_00890, Reut_A1993, RHE CH01878 BR1274, aq_1812, TTE2620, ACIAD0264, PFL 1103 stu0469, str0469, Pf1_1027 Psyr_1290, PMN2A_0702, MTH1232 Csal_3010, AGR C 2919 Atu1588, PSPPH_1360, PP1470, NE2369, PSPTO1480, Tcr 1251 B1964, Nmul_A1551, Saro_0019, ml10934, WS0450 spr1219, SP1361, Noc_2454, BT9727_1799, BCZK1782, BCE2051, Tbd_0843, PA3736 DET1206, amb3728, Rru_A2410, LIC10571, LA3638, SMU 965, BAS1825, BA_2468, GBAA1968, BA1968 cbdb_A1123, GOX1517, PMM1051, HCH 01779, RB8510, DVU0890, Pro1150, Nham_2309, Tmden_1904 Sde 1209 Psyc_0253, ELI_13775, RSP_0403, L0090, Dde 2731, Pcryo_0279, Nwi_1647, 1p_0571, BH10030 SPO1734, Jann 2998 b1r4362, RPA2504, EF2422, DP1732, LBA1212, RPD_2495, RPC_2816, CC1383, RPB_2966, CJE0145, Cj0149c, Acid345_1481, ZMO0483, Bpro 5333, SAK_1205, gbs1187, jhp0761, SH1579 SAG1120 HP0822 SE1009 SERP0897, SAOUHSC 01320 SAUSA300_1226, SAB1186, SACOL1362, SAS1268, SAR1338 MW1215, SAV1328 SA1164 HH1750, SSP1438, 1p_2535, TTE2152, SAR11_1025, DR1278 PFL_3809, Dgeo_0610, Mhun_2292, DSY3981, PP0664, MA2572, ABC1578, Mbar_A1898, TTHA0489 | |
| | TTC0115, MM2713, Mbur_1087, BH1737, AF0935, MK1554 MTH417, VNG2650G, Msp 0487 ABC0023. rrnAC2408, TK1627, TM0547, MJ1602 NP0302A, B1253, MMP1702, BCE2626, LmjF07.0260, BCZK2354, BT9727_2388. BAS2433, BA_3119, GBAA2608, BA2608, BC2548, Acid34_4165. CTC00886 ST1519. Saci_1636. APE1144, SSO0657, PF1104. Adeh_3931 PAB0610, PH1075. Cag_0142, PAE2868, YJR139C XOO1820, Plut 1983, XAC3038, Adeh_1400, XCV3175, PTO1417, SCOo420, SRU 0482 XC_1253, XCC2855, SO4055, CT2030, SPBC77603, AO090003000721, TVN0385 ABL080W, AO090009000136, CPS_0456, HI0089, orf192951, Sden_0616, UTI89_C4525, Afu3g11640, MS1703 SBO_3960, SSO_4114, STM4101, SC3992 t3517, STY3768, c4893 ECs4869, Z5495, JW3911, b3940, AN2882 2, ECA4251, CMN129C, NTHI0167, plu4755, ECA3891 YPTB0602 YP3723 y3718, YPO0459, PM0113 S3729, SF4018 SPA3944, Mfla_1298, PSHAa2379, PBPRA0262, XOO2242, STM0002, SC0002, SPA0002, t0002, STY0002, c0003 SRU_0691, XCC1800, PD1273, BPEN_115, SDY 3775 VC2684, SDY 0002 SBO_0001, YPTB0106, YP0118, y0303, YPO0116, UTI89_C0002, ECs0002 20002, JW0001, b0002, VV3007, VV11365, XC_2389, VP2764, XF2225, SSO_0002 S0002, SF0002 | |
| Senne deaminase (sda) | GeneID 1019614, NCg11583, EG10930, g178116 | *C. glutamicum, E coli,* and others |
| Homosenne kinase (hsk) | Cogl1184, cg0307 CE0221, DIP0279, jk1997, RHA1_r o04292, nfa3190, Mmcs_4888, MSMEG 6256, MAP0310c, MAV_034, Mb3735c, MT3811 Rv3708c, Ace1_2011, ML2322, PPA0318, L xx03460, SCO2640, SAV5397, CC3485 | *C. glutamicum* and others |
| D-methionine binding lipoprotein (metQ) | YP_224930, NP_599871, NP_737241, NP_938985, NP_938984, YP_701727,YP_251505,YP_120623, YP_062481, YP_056445, ZP_00121548, NP_696133 YP_034633¨, YP_034633, YP_081895, ZP_0039069 6 YP_016928, YP_026579, NP_842863,YP_081895 ZP_00240243, NP 976671 | *C. glutamicum* and others |
| mcbR | cg3253 CE2788 DIP2274, jk0101, nfa21280, MSME G 4517Lxx16190 SCO4454, Bcep18194_A3587 B amb_0404,Bcen2424_0499,Bcen_2606 Ava_4037, BTH_I2940, RHA1_ro02712, BMA10299_A17735, B MASAVP1_A0031, BMA2807, BURPS1710b_361 4 | *C. glutamicum* and others |
| Glucose-6-phosphate-dehydrogenase | Cgl1576, BAB98969, NCg11514, NCg11514, cg1778, CE1696,DIP1304,jk0994RHA1 ro07184,nfa3575 0,MSMEG_3101, Mmcs_2412, MAP1176c, Mb1482 c MT1494, Rv1447c SAV6313 Acel_1124,SCO193 7 MAV_3329, Lxx1590, BL0440,Arth_2094 Tfu_2 005, itte weitere angeben | Corynebacterium glutamicum and others |
| OPCA protein | Cg11577, NP_738307 1, NP_939658. 1 YP_250777 1, YP_707105. 1,YP_119788 1,ZP_01192082. 1 NP_ 335942.1 ZP_01276169.1 NP_215962.1 ZP_01684 361.1, YP_887415. 1,ZP_01130849 1 YP_062111.1, ZP_00615668.1, YP_953530.1, ZP_00995403.1 YP | Corynebactenum glutamicum and others |
| | _882512.1, NP_960109.1, YP_290062.1 YP_83157 3.1.NP_827488.1 YP_947817.1 NP_822945.1 NP_ 626203 1 NP_630735.1, CAH10103.1 ZP_0012091 0.2, NP_695642.1YP_909493.1, YP_872881.1 YP_ 923728 1.YP_056265.1 ZP_01648612. 1,ZP_01430 762 1 ZP_00569428. 1, YP_714762, 1 YP_480751, NP_301492.1, YP_642845.1 ZP_00767699.1 | |
| Transaldolase | Cgl1575,cg1776, CE1695, DIP1303, jk0993,Mmcs_ 2413 MSMEG_3102, MAP1177c, RHA1_ro07185 MAV_3328, Mb1483c, Rv 1448c MT1495, nfa35740 ,ML0582, Arth_2096, Lxx11610, SAV1767, Tfu_200 3.SCO1936, Francci3_1648 | Corynebactenum glutanucum and others |
| 6-phosphogluconolactonase | Cgl1578, NCg11516,NCg11516,cg1780, CE1698,DI P1306 Mmcs_2410, MSMEG_3099, Mb1480c, MT1 492 Rv 1445c,MAV_3331. RHA1_ro07182, nfa3577 0, MAP1174c, ML0579,jk0996,Tfu_2007 FRAAL4 578 SAV6311, SCO1939, SCC22 21 TW464 | Corynebacterium glutamicum and others |
| Transketolase | Cg11574 YP_225858, cg1774,CE1694, DIP1302,jk0 992 nfa35730, RHA1_ro07186, MSMEG_3103,MA P1178c,ML0583,MAV_3127, Mb1484c, MT1496, R v1449c,Mmcs_2414, Tfu_2002,Arth_2097, Lxx116 20 SAV1766 SCO1935, Acel_1127 | Corynebacterium glutamicum and others |

The above accession numbers are the official accession numbers of Genbank or are synonyms for accession numbers which have cross-references at Genbank. These numbers can be searched and found at http //www.ncbi nlm nih gov/

A general overview is given below how to increase and decrease the amount and/or activity of polypeptides and genes in *C. glutamicum.* Nevertheless, the person skilled in the art will be aware of other technologies and approaches for either identifying new homologs of the enzymes of Table 1 by performing appropriate database searches and/or altering the expression of these enzymes in organisms other than Coryneform bacteria.

### Increasing or introducing the amount and/or activity

With respect to increasing the amount, two basic scenarios can be differentiated. In the first scenario, the amount of the enzyme is increased by expression of an exogenous version of the respective protein. In the other scenario, expression of the endogenous protein is increased by influencing the activity of e.g. the promoter and/or enhancers element and/or other regulatory activities that regulate the activities of the respective proteins either on a transcriptional, translational or post-translational level.

Thus, the increase of the activity and the amount of a protein may be achieved via different routes, e.g. by switching off inhibitory regulatory mechanisms at the transcriptional, translational, and protein level or by increase of gene expression of a nucleic acid coding for these proteins in comparison with the starting organism, e.g. by inducing endogenous transketolase by a strong promoter and/ or by introducing nucleic acids encoding for transketolase.

In one embodiment, the increase of the amount and/or activity of the enzymes of Table 1 is achieved by introducing nucleic acids encoding the enzymes of Table 1 into microorganism such as *C. glutamicum*.

In principle, any protein of different organisms with an enzymatic activity of the proteins listed in Table 1 can be used. With genomic nucleic acid sequences of such enzymes from eukaryotic sources containing introns, already processed nucleic acid sequences like the corresponding cDNAs are to be used in the case as the host organism is not capable or cannot be made capable of splicing the corresponding mRNAs. All nucleic acids mentioned in the description can be, e.g., an RNA, DNA or cDNA sequence.

According to the present invention, increasing or introducing the amount of a protein typically comprises the following steps:
a) production of a vector comprising the following nucleic acid sequences, preferably DNA sequences, in 5'-3'-orientation:
   - a promoter sequence functional in an organism of the invention
   - operatively linked thereto a DNA sequence coding for a protein of e.g. Table 1, functional homologues, functional fragments or functional mutated versions thereof
   - optionally, a termination sequence functional in the organisms of the invention
b) transfer of the vector from step a) to an organisms of the invention such as *C. glutamicum* and, optionally, integration into the respective genomes.

As set out above, functional fragments relate to fragments of nucleic acid sequences coding for enzymes of e.g. Table 1, the expression of which still leads to proteins having the enzymatic activity substantially similar to that of the respective full length protein.

The above-mentioned method can be used for increasing the expression of DNA sequences coding for enzymes of e.g. Table 1 or functional fragments thereof. The use of such vectors comprising regulatory sequences, like promoter and termination sequences are, is known to the person skilled in the art. Furthermore, the person skilled in the art knows how a vector from step a) can be transferred to organisms such as *C*. *glutamicum* or *E. coli* and which properties a vector must have to be able to be integrated into their genomes.

If the enzyme content in an organism such as *C*. *glutamicum* is increased by transferring a nucleic acid coding for an enzyme from another organism, like e.g. *E.coli,* it is advisable to transfer the amino acid sequence encoded by the nucleic acid sequence e.g. from *E.coli* by back-translation of the polypeptide sequence according to the genetic code into a nucleic acid sequence comprising mainly those codons, which are used more often due to the organism-specific codon usage. The codon usage can be determined by means of computer evaluations of other known genes of the relevant organisms.

According to the present invention, an increase of the gene expression of a nucleic acid encoding an enzyme of Table 1 is also understood to be the manipulation of the expression of the endogenous respective endogenous enzymes of an organism, in particular of *C. glutamicum.* This can be achieved, e.g., by altering the promoter DNA sequence for genes encoding these enzymes. Such an alteration, which causes an altered, preferably increased, expression rate of these enzymes can be achieved by replacement with strong promoters and by deletion and/or insertion of DNA sequences.

An alteration of the promoter sequence of endogenous genes usually causes an alteration of the expressed amount of the gene and therefore also an alteration of the activity detectable in the cell or in the organism.

Furthermore, an altered and increased expression, respectively, of an endogenous gene can be achieved by a regulatory protein, which does not occur or has been deleted in the transformed organism, and which interacts with the promoter of these genes. Such a regulator can be a chimeric protein consisting of a DNA binding domain and a transcription activator domain, as e.g. described in WO 96/06166.

A further possibility for increasing the activity and the content of endogenous genes is to up-regulate transcription factors involved in the transcription of the endogenous genes, e.g. by means of overexpression. The measures for overexpression of transcription factors are known to the person skilled in the art.

The expression of endogenous enzymes such as those of Table 1 can e.g. be regulated via the expression of aptamers specifically binding to the promoter sequences of the genes. Depending on the aptamer binding to stimulating or repressing promoter regions, the amount of the enzymes of Table 1 can e.g. be increased.

Furthermore, an alteration of the activity of endogenous genes can be achieved by targeted mutagenesis of the endogenous gene copies.

An alteration of the endogenous genes coding for the enzymes of e.g. Table 1 can also be achieved by influencing the post-translational modifications of the enzymes. This can happen e.g. by regulating the activity of enzymes like kinases or phosphatases involved in the post-translational modification of the enzymes by means of corresponding measures like overexpression or gene silencing.

In another embodiment, an enzyme may be improved in efficiency, or its allosteric control region destroyed such that feedback inhibition of production of the compound is prevented. Similarly, a degradative enzyme may be deleted or modified by substitution, deletion, or addition such that its degradative activity is lessened for the desired enzyme of Table 1 without impairing the viability of the cell. In each case, the overall yield, rate of production or amount of methionine be increased.

These aforementioned strategies for increasing or introducing the amount and/or activity of the enzymes of Table 1 are not meant to be limiting; variations on these strategies will be readily apparent to one of ordinary skill in the art.

### Reducing the amount and or activity of enzymes

It has been set out above that it may be preferred to use a starting organism which have already been engineered for methionine production. In *C*. *glutamicum* one may, for example, downregulate the activity of *metQ.*

For reducing the amount and/or activity of enzymes, various strategies are available.

The expression of endogenous enzymes such as those of Table 1 can e.g. be regulated via the expression of aptamers specifically binding to the promoter sequences of the genes. Depending on the aptamer binding to stimulating or repressing promoter regions, the amount and thus, in this case, the activity of the enzymes of Table 1 can e.g. be reduced.

Aptamers can also be designed in a way as to specifically bind to the enzymes themselves and to reduce the activity of the enzymes by e.g. binding to the catalytic center of the respective enzymes. The expression of aptamers is usually achieved by vector-based overexpression (see above) and is, as well as the design and the selection of aptamers, well known to the person skilled in the art (Famulok et al., (1999) Curr Top Microbiol Immunol., 243,123-36).

Furthermore, a decrease of the amount and the activity of the endogenous enzymes of Table 1 can be achieved by means of various experimental measures, which are well known to the person skilled in the art. These measures are usually summarized under the term "gene silencing". For example, the expression of an endogenous gene can be silenced by transferring an above-mentioned vector, which has a DNA sequence coding for the enzyme or parts thereof in antisense order, to organisms such as *C. glutamicum.* This is based on the fact that the transcription of such a vector in the cell leads to an RNA, which can hybridize with the mRNA transcribed by the endogenous gene and therefore prevents its translation.

In principle, the antisense strategy can be coupled with a ribozyme method. Ribozymes are catalytically active RNA sequences, which, if coupled to the antisense sequences, cleave the target sequences catalytically (Tanner et al., (1999) FEMS Microbiol Rev. 23 (3), 257-75). This can enhance the efficiency of an antisense strategy.

To create a homologous recombinant microorganism, a vector is prepared which contains at least a portion of gene coding for an enzyme of Table 1 into which a deletion, addition or substitution has been introduced to thereby alter, e.g., functionally disrupt, the endogenous gene.

In one embodiment, the vector is designed such that, upon homologous recombination, the endogenous gene is functionally disrupted (i. e., no longer encodes a functional protein). Alternatively, the vector can be designed such that, upon homologous recombination, the endogenous gene is mutated or otherwise altered but still encodes functional protein, e.g., the upstream regulatory region can be altered to thereby alter the expression of the endogenous enzymes of Table 1. This approach can have the advantage that expression of an enzyme is not completely abolished, but reduced to the required minimum level. The skilled person knows which vectors can be used to replace or delete endogenous sequences. A specific description for disrupting chromosomal sequences in *C. glutamicum* is provided below.

Furthermore, gene repression is possible by reducing the amount of transcription factors.

Factors inhibiting the target protein itself can also be introduced into a cell. The protein-binding factors may e.g. be the above-mentioned aptamers (Famulok et al., (1999) Curr Top Microbiol Immunol. 243, 123-36).

As further protein-binding factors, the expression of which can cause a reduction of the amount and/or the activity of the enzymes of table 1, enzyme-specific antibodies may be considered. The production of recombinant enzyme-specific antibodies such as single chain antibodies is known in the art. The expression of antibodies is also known from the literature (Fiedler et al., (1997) Immunotechnolgy 3, 205-216; Maynard and Georgiou (2000) Annu. Rev. Biomed. Eng. 2, 339-76).

The mentioned techniques are well known to the person skilled in the art. Therefore, the skilled also knows the typical size that a nucleic acid constructs used for e.g. antisense methods must have and which complementarity, homology or identity, the respective nucleic acid sequences must have. The terms complementarity, homology, and identity are known to the person skilled in the art.

The term complementarity describes the capability of a nucleic acid molecule to hybridize with another nucleic acid molecule due to hydrogen bonds between two complementary bases. The person skilled in the art knows that two nucleic acid molecules do not have to display a complementarity of 100% in order to be able to hybridize with each other. A nucleic acid sequence, which is to hybridize with another nucleic acid sequence, is preferably at least 30%, at least 40%, at least 50%, at least 60%, preferably at least 70%, particularly preferred at least 80%, also particularly preferred at least 90%, in particular preferred at least 95% and most preferably at least 98 or 100%, respectively, complementary with said other nucleic acid sequence.

The hybridization of an antisense sequence with an endogenous mRNA sequence typically occurs *in vivo* under cellular conditions or *in vitro.* According to the present invention, hybridization is carried out *in vivo* or *in vitro* under conditions that are stringent enough to ensure a specific hybridization.

Stringent *in vitro* hybridization conditions are known to the person skilled in the art and can be taken from the literature (see e.g. Sambrook et al., Molecular Cloning, Cold Spring Harbor Press (2001)). The term "specific hybridization" refers to the case wherein a molecule preferentially binds to a certain nucleic acid sequence under stringent conditions, if this nucleic acid sequence is part of a complex mixture of e.g. DNA or RNA molecules.

The term "stringent conditions" therefore refers to conditions, under which a nucleic acid sequence preferentially binds to a target sequence, but not, or at least to a significantly reduced extent, to other sequences.

Stringent conditions are dependent on the circumstances. Longer sequences specifically hybridize at higher temperatures. In general, stringent conditions are chosen in such a way that the hybridization temperature lies about 5°C below the melting point (Tm) of the specific sequence with a defined ionic strength and a defined pH value. Tm is the temperature (with a defined pH value, a defined ionic strength and a defined nucleic acid concentration), at which 50% of the molecules, which are complementary to a target sequence, hybridize with said target sequence. Typically, stringent conditions comprise salt concentrations between 0.01 and 1.0 M sodium ions (or ions of another salt) and a pH value between 7.0 and 8.3. The temperature is at least 30°C for short molecules (e.g. for such molecules comprising between 10 and 50 nucleic acids). In addition, stringent conditions can comprise the addition of destabilizing agents like e.g. form amide. Typical hybridization and washing buffers are of the following composition.

| | |
|---|---|
| *Pre-hybridization solution:* | 0.5%SDS |
| | 5x SSC |
| | 50 mM NaPO₄, pH 6.8 |
| | 0 1% Na-pyrophosphate |
| | 5x Denhardt's reagent |
| | 100 µg/salmon sperm |
| | |
| *Hybridization solution:* | Pre-hybridization solution |
| | 1x10⁶ cpm/ml probe (5-10 min 95°C) |
| | |
| *20x SSC* | 3 M NaCl |
| | 0.3 M sodium citrate |
| | ad pH 7 with HCl |
| | |
| *50x Denhardt's reagent:* | 5 g Ficoll |
| | 5 g polyvinylpyrrolidone |
| | 5 g Bovine Serum Albumin |
| | ad 500 ml A dest |

A typical procedure for the hybridization is as follows

| | | |
|---|---|---|
| *Optional :* | wash Blot 30 min in 1x SSC/ 0 1% SDS at 65°C | |
| *Pre-hybridization :* | at least 2 h at 50-55°C | |
| *Hybridization :* | over night at 55-60°C | |
| *Washing :* | 05 min | 2x SSC/ 0.1 % SDS |

| Hybridization temperature | | |
|---|---|---|
| 30 min | 2x SSC/0.1% SDS | |

| Hybridization temperature | | |
|---|---|---|
| 30 min | 1x SSC/ 0.1% SDS | |

| Hybridization temperature | | |
|---|---|---|
| 45 min | 0.2x SSC/ 0.1% SDS | 65°C |
| 5 min | 0.1x SSC | room temperature |

For antisense purposes complementarity over sequence lengths of 100 nucleic acids, 80 nucleic acids, 60 nucleic acids, 40 nucleic acids and 20 nucleic acids may suffice. Longer nucleic acid lengths will certainly also suffice. A combined application of the above-mentioned methods is also conceivable.

If, according to the present invention, DNA sequences are used, which are operatively linked in 5'-3'-orientation to a promoter active in the organism, vectors can, in general, be constructed, which, after the transfer to the organism's cells, allow the overexpression of the coding sequence or cause the suppression or competition and blockage of endogenous nucleic acid sequences and the proteins expressed there from, respectively.

The activity of a particular enzyme may also be reduced by over-expressing a non-functional mutant thereof in the organism. Thus, a non-functional mutant which is not able to catalyze the reaction in question, but that is able to bind e.g. the substrate or co-factor, can, by way of over-expression out-compete the endogenous enzyme and therefore inhibit the reaction. Further methods in order to reduce the amount and/or activity of an enzyme in a host cell are well known to the person skilled in the art.

According to the present invention, non-functional enzymes have essentially the same nucleic acid sequences and amino acid sequences, respectively, as functional enzymes and functionally fragments thereof, but have, at some positions, point mutations, insertions or deletions of nucleic acids or amino acids, which have the effect that the non-functional enzyme are not, or only to a very limited extent, capable of catalyzing the respective reaction. These non-functional enzymes may not be intermixed with enzymes that still are capable of catalyzing the respective reaction, but which are not feedback regulated anymore. According to the present invention, the term "non-functional enzyme" does not comprise such proteins having no substantial sequence homology to the respective functional enzymes at the amino acid level and nucleic acid level, respectively. Proteins unable to catalyse the respective reactions and having no substantial sequence homology with the respective enzyme are therefore, by definition, not meant by the term "non-functional enzyme" of the present invention. Non-functional enzymes are, within the scope of the present invention, also referred to as inactivated or inactive enzymes.

Therefore, non-functional enzymes of e.g.Table 1 according to the present invention bearing the above-mentioned point mutations, insertions, and/or deletions are characterized by an substantial sequence homology to the wild type enzymes of e.g. Table 1 according to the present invention or functionally equivalent parts thereof. For determining a substantial sequence homology, the above describded identity grades are to applied.

### Vectors and Host Cells

Disclosed herein are vectors, preferably expression vectors, containing a modified nucleic acid sequences as mentioned above. As used herein, the term "vector" refers to a nucleic acid molecule capable of transporting another nucleic acid to which it has been linked.

One type of vector is a "plasmid", which refers to a circular double stranded DNA loop into which additional DNA segments can be ligated. Another type of vector is a viral vector, wherein additional DNA segments can be ligated into the viral genome.

Certain vectors are capable of autonomous replication in a host cell into which they are introduced (e.g., bacterial vectors having a bacterial origin of replication and episomal mammalian vectors). Other vectors (e. g., non-episomal mammalian vectors) are integrated into the genome of a host cell upon introduction into the host cell, and thereby are replicated along with the host genome. Moreover, certain vectors are capable of directing the expression of genes to which they are operatively linked.

Such vectors are referred to herein as "expression vectors".

In general, expression vectors of utility in recombinant DNA techniques are often in the form of plasmids. In the present specification, "plasmid" and "vector" can be used interchangeably as the plasmid is the most commonly used form of vector. However, the invention is intended to include such other forms of expression vectors, such as viral vectors (e. g., replication defective retroviruses, adenoviruses and adeno-associated viruses), which serve equivalent functions.

The recombinant expression vectors may comprise a modified nucleic acid as mentioned above in a form suitable for expression of the respective nucleic acid in a host cell, which means that the recombinant expression vectors include one or more regulatory sequences, selected on the basis of the host cells to be used for expression, which is operatively linked to the nucleic acid sequence to be expressed.

Within a recombinant expression vector, "operably linked" is intended to mean that the nucleic acid sequence of interest is linked to the regulatory sequence (s) in a manner which allows for expression of the nucleic acid sequence (e.g., in an in vitro transcription/translation system or in a host cell when the vector is introduced into the host cell). The term "regulatory sequence" is intended to include promoters, repressor binding sites, activator binding sites, enhancers and other expression control elements (e.g., terminators, polyadenylation signals, or other elements of mRNA secondary structure). Such regulatory sequences are described, for example, in Goeddel; Gene Expression Technology: Methods in Enzymology 185, Academic Press, San Diego, CA (1990). Regulatory sequences include those which direct constitutive expression of a nucleic acid sequence in many types of host cell and those which direct expression of the nucleic acid sequence only in certain host cells. Preferred regulatory sequences are, for example, promoters such as cos-, tac-, trp-, tet-, trp-, tet-, 1pp-, 1ac-, 1pp-lac-, 1acIq-, T7-, T5-, T3-, gal-, trc-, ara-, SP6-, arny, SP02,phage lambdaP_{R}, phage lambdaP_{L}, phage SP01 P₁₅, phage SP01 P₂₆, pSOD, EFTu, EFTs, GroEL, MetZ (last 5 from C. *glutamicum*), which are used preferably in bacteria. Additional regulatory sequences are, for example, promoters from yeasts and fungi, such as ADC1, MFa, AC, P-60, CYC1, GAPDH, TEF, rp28, ADH, ENO2, promoters from plants such as CaMV/35S, SSU, OCS, lib4, usp, STLS1, B33, nos or ubiquitin-or phaseolin-promoters. It is also possible to use artificial promoters. It will be appreciated by one of ordinary skill in the art that the design of the expression vector can depend on such factors as the choice of the host cell to be transformed, the level of expression of protein desired, etc. The expression vectors can be introduced into host cells to thereby produce proteins or peptides, including fusion proteins or peptides, encoded by the above-mentioned modified nucleic acid sequences.

Expression of proteins in prokaryotes is most often carried out with vectors containing constitutive or inducible promoters directing the expression of either fusion or non-fusion proteins.

Fusion vectors add a number of amino acids to a protein encoded therein, usually to the amino terminus of the recombinant protein but also to the C-terminus or fused within suitable regions in the proteins. Such fusion vectors typically serve four purposes: 1) to increase expression of recombinant protein; 2) to increase the solubility of the recombinant protein; and 3) to aid in the purification of the recombinant protein by acting as a ligand in affinity purification 4) to provide a "tag" for later detection of the protein. Often, in fusion expression vectors, a proteolytic cleavage site is introduced at the junction of the fusion moiety and the recombinant protein to enable separation of the recombinant protein from the fusion moiety subsequent to purification of the fusion protein. Such enzymes, and their cognate recognition sequences, include Factor Xa, thrombin and enterokinase.

Typical fusion expression vectors include pGEX (Pharmacia Biotech Inc; Smith, D. B. and Johnson, K. S. (1988) Gene 67: 31-40), pMAL (New England Biolabs, Beverly, MA) and pRIT5 (Pharmacia, Piscataway, NJ) which fuse glutathione S-transferase (GST), maltose E binding protein, or protein A, respectively.

Examples of suitable inducible non-fusion *E. coli* expression vectors include pTrc (Amann et al., (1988) Gene 69: 301-315), pLG338, pACYC184, pBR322,pUC18, pUC19, pKC30, pRep4,pHSl, pHS2, pPLc236, pMBL24, pLG200, pUR290,pIN-III113-B1, egtll, pBdCl, and pET 11d (Studier etal., Gene Expression Technology : Methods in Enzymology 185, Academic Press, San Diego, California (1990) 60-89; and Pouwels et al., eds. (1985) Cloning Vectors. Elsevier: New York IBSN 0 444 904018). Target gene expression from the pTrc vector relies on host RNA polymerase transcription from a hybrid trp-lac fusion promoter. Target gene expression from the pET 11d vector relies on transcription from a T7 gnlO-lac fusion promoter mediated by a coexpressed viral RNA polymerase (T7gnl). This viral polymerase is supplied by host strains BL21 (DE3) or HMS 174 (DE3) from a resident X prophage harboring a T7gnl gene under the transcriptional control of the lacUV 5 promoter. For transformation of other varieties of bacteria, appropriate vectors may be selected. For example, the plasmids pIJ101, pIJ364, pIJ702 and pIJ361 are known to be useful in transforming Streptomyces, while plasmidspUB110, pC 194 or pBD214 are suited for transformation of Bacillus species. Several plasmids of use in the transfer of genetic information into Corynebacterium include pHM1519, pBL1, pSA77 or pAJ667 (Pouwels et al., eds. (1985) Cloning Vectors. Elsevier: New York IBSN 0 444 904018).

Examples of suitable *C. glutamicnm* and *E coli* shuttle vectors are e.g. pClik5aMCS (WO2005059093) or can be found in Eikmanns et al (Gene. (1991) 102, 93-8).

Examples for suitable vectors to manipulate Corynebacteria can be found in the Handbook of Corynebacterium (edited by Eggeling and Bott, ISBN 0-8493-1821-1, 2005). One can find a list of *E. coli - C. glutamicum* shuttle vectors (table 23.1), a list of *E. coli - C. glutamicum* shuttle expression vectors (table 23.2), a list of vectors which can be used for the integration of DNA into the *C. glutamicnm* chromosome (table 23.3), a list of expression vectors for integration into the *C. glutamicum* chromosome (table 23.4.) as well as a list of vectors for site-specific integration into the *C. glutamicum* chromosome (table 23.6).

Alternatively, the protein expression vector is a yeast expression vector. Examples of vectors for expression in yeast S. cerevisiae include pYepSecl (Baldari, et al., (1987) Embo J. 6: 229-234),, 2i, pAG-1, Yep6, Yep13 , pEMBLYe23, pMFa (Kurjan and Herskowitz, (1982) Cell 30: 933-943), pJRY88 (Schultz et al., (1987) Gene 54: 113-123), and pYES2 (Invitrogen Corporation, San Diego, CA). Vectors and methods for the construction of vectors appropriate for use in other fungi, such as the filamentous fungi, include those detailed in: van den Hondel, C. A. M. J. J. & Punt,P. J. (1991) in: Applied Molecular Genetics of Fungi, J. F. Peberdy, et al., eds., p. 1-28, Cambridge University Press: Cambridge, and Pouwels et al., eds. (1985) Cloning Vectors. Elsevier: New York (IBSN 0 444 904018).

An operative link is understood to be the sequential arrangement of promoter, coding sequence, terminator and, optionally, further regulatory elements in such a way that each of the regulatory elements can fulfill its function, according to its determination, when expressing the coding sequence.

For other suitable expression systems for both prokaryotic and eukaryotic cells see chapters 16 and 17 of Sambrook, J. et al. Molecular Cloning: A Laboratory Manual. 3rd ed., Cold Spring Harbor Laboratory, Cold Spring Harbor Laboratory Press, Cold Spring Harbor, NY, 2003.

Vector DNA can be introduced into prokaryotic via conventional transformation or transfection techniques. As used herein, the terms "transformation" and "transfection", "conjugation" and "transduction" are intended to refer to a variety of art-recognized techniques for introducing foreign nucleic acid (e.g., linear DNA or RNA (e. g., a linearized vector or a gene construct alone without a vector) or nucleic acid in the form of a vector (e.g., a plasmid, phage, phasmid, phagemid, transposon or other DNA into a host cell, including calcium phosphate or calcium chloride co-precipitation, DEAE-dextran-mediated transfection, lipofection, natural competence, chemical-mediated transfer, or electroporation. Suitable methods for transforming or transfecting host cells can be found in Sambrook, et al. (Molecular Cloning : A Laboratory Manual. 3rd ed., Cold Spring Harbor Laboratory, Cold Spring Harbor Laboratory Press, Cold Spring Harbor, NY, 2003), and other laboratory manuals.

In order to identify and select these integrants, a gene that encodes a selectable marker (e.g., resistance to antibiotics) is generally introduced into the host cells along with the gene of interest. Preferred selectable markers include those which confer resistance to drugs, such as, but not limited to, G418, hygromycin , kanamycine, tetracycline, neomycineampicillin (and other pencillins), cephalosporins, fluoroquinones, naladixic a id, chloramphenicol, spectinomyin, ertythromycin, streptomycin and methotrexate.Other selectable markers include wild type genes that can complement mutated versions of the equivalent gene in a host or starting strain. For example, an essential gene for growth on a minimal medium, such as *serA*, can be mutated or deleted from the genome of a *C. glutamicum* starting or host strain of the invention as described herein above to create a serine auxotroph. Then, a vector containing a wild type or other functional copy of a *serA* gene can be used toselect for transformants or integrants. Nucleic acid encoding a selectable marker can be introduced into a host cell on the same vector as that encoding the above-mentioned modified nucleic acid sequences or can be introduced on a separate vector. Cells stably transfected with the introduced nucleic acid can be identified by drug selection (e. g., cells that have incorporated the selectable marker gene will survive, while the other cells die).

When plasmids without an origin of replication and two different marker genes are used (e.g. pClik int sacB), it is also possible to generate marker-free strains which have part of the insert inserted into the genome. This is achieved by two consecutive events of homologous recombination (see also Becker et al., Applied and Environmental Microbilogy, 71 (12), p. 8587-8596). The sequence of plasmid pClik int sacB can be found in WO2005059093; SEQ ID 24; the plasmid is called pCIS in this document.

Alternatively, recombinant microorganisms can be produced which contain selected systems which allow for regulated expression of the introduced gene. For example, inclusion of one of the above-mentioned nucleic acid sequences on a vector placing it under control of the lac operon permits expression of the gene only in the presence of IPTG. Such regulatory systems are well known in the art.

Also disclosed are organisms or host cells into which a recombinant expression vector has been introduced. The terms "host cell" and "recombinant host cell" are used interchangeably herein. It is understood that such terms refer not only to the particular subject cell but also to the progeny or potential progeny of such a cell. Because certain modifications may occur in succeeding generations due to either mutation or environmental influences, such progeny may not, in fact, be identical to the parent cell, but are still included within the scope of the term as used herein.

### Growth of E, coli and C. glutamicum - Media and Culture Conditions

The person skilled in the art is familiar with the cultivation of common microorganisms such as *C.glutamicum* and *E.coli.* Thus, a general teaching will be given below as to the cultivation of *C.glutamicum. Corresponding* information may be retrieved from standard textbooks for cultivation of *E.coli.*

*E. coli* strains are routinely grown in MB and LB broth, respectively (Follettie et al. (1993) J. Bacteriol. 175, 4096-4103).Minimal Several minimal media for bacteria, including *E. coli* and *C. glutamicum* are well knoiwn in the art. Minimal media for E. coli isinclude,but are not limited to, E medium, M9medium and modified MCGC (Yoshihama et al. (1985) J. Bacteriol. 162,591-507), respectively. Glucose may be added at a final concentration of between about 0.2% and 1%. Antibiotics may be added in the following amounts (micrograms per millilitre): ampicillin, 5 to 1000; kanamycin, 25; nalidixic acid, 25; chloramphenicol, 5 to 120, spectinomycin 50 to 100, tetracyline 5 to 120. Amino acids, vitamins, and other supplements may be added, for example, in the following amounts: methionine, 9.3 mM; arginine, 9.3 mM; histidine, 9.3 mM; thiamine, 0.05 mM. *E. coli* cells are routinely grown at 18 to 37 44° C, respectivelydepending on the particular experiment or proceddure being performed.

Genetically modified *Corynebacteria* are typically cultured in synthetic or natural growth media. A number of different growth media for *Corynebacteria* are both well-known and readily available (Lieb et al. (1989) Appl.Microbiol. Biotechnol., 32: 205-210; von der Osten et al. (1998) Biotechnology Letters, 11: 11-16; Patent DE 4,120,867; Liebl(1992) "The Genus Corynebacterium, in: The Procaryotes, Volume II, Balows, A. et al., eds. Springer-Verlag). Instructions can also be found in the Handbook of Corynebacterium (edited by Eggeling and Bott, ISBN 0-8493-1821-1, 2005).

These media consist of one or more carbon sources, nitrogen sources, inorganic salts, vitamins and trace elements. Preferred carbon sources are sugars, such as mono-, di-, or polysaccharides. For example, glucose, fructose, mannose, galactose, ribose, sorbose, ribose, lactose, maltose, sucrose, glycerol, raffinose, starch or cellulose serve as very good carbon sources.

It is also possible to supply sugar to the media via complex compounds such as molasses or other by-products from sugar refinement. It can also be advantageous to supply mixtures of different carbon sources. Other possible carbon sources are alcohols and organic acids, such as methanol, ethanol, acetic acid or lactic acid. Nitrogen sources are usually organic or inorganic nitrogen compounds, or materials which contain these compounds. Exemplary nitrogen sources include ammonia gas or ammonia salts, such as NH₄Cl or (NH₄)₂S0₄, NH₄0H, nitrates, urea, amino acids or complex nitrogen sources like corn steep liquor, soy bean flour, soy bean protein, yeast extract, meat extract and others.

The overproduction of methionine is possible using different sulfur sources. Sulfates, thiosulfates, sulfites and also more reduced sulfur sources like H₂S and sulfides and derivatives can be used. Also organic sulfur sources like methyl mercaptan, thioglycolates, thiocyanates, and thiourea, sulfur containing amino acids like cysteine and other sulfur containing compounds can be used to achieve efficient methionine production. Formate may also be possible as a supplement as are other C1 sources such as methanol or formaldehyde.

Inorganic salt compounds which may be included in the media include the chloride-, phosphorous-or sulfate-salts of calcium, magnesium, sodium, cobalt, molybdenum, potassium, manganese, zinc, copper and iron. Chelating compounds can be added to the medium to keep the metal ions in solution. Particularly useful chelating compounds include dihydroxyphenols, like catechol or protocatechuate, or organic acids, such as citric acid. It is typical for the media to also contain other growth factors, such as vitamins or growth promoters, examples of which include cyanocobalamin (or other form of vitamin B 12), biotin, riboflavin, thiamine, folic acid, nicotinic acid, pantothenate and pyridoxine. Growth factors and salts frequently originate from complex media components such as yeast extract, molasses, corn steep liquor and others. The exact composition of the media compounds depends strongly on the immediate experiment and is individually decided for each specific case. Information about media optimization is available in the textbook "Applied Microbiol. Physiology, A Practical Approach (Eds. P. M. Rhodes, P.F. Stanbury, IRL Press (1997) pp. 53-73, ISBN 0 19 963577 3). It is also possible to select growth media from commercial suppliers, like standard 1 (Merck) or BHI (grain heart infusion, DIFCO) or others.

All medium components should be sterilized, either by heat (20 minutes at 1.5 bar and121 C) or by sterile filtration. The components can either be sterilized together or, if necessary, separately.

All media components may be present at the beginning of growth, or they can optionally be added continuously or batch wise. Culture conditions are defined separately for each experiment.

The temperature should be is usually in a range between 15°C and 45°C, but the range may be higher, up to 105°C for thermophilic oraganisms. The temperature can be kept constant or can be altered during the experiment. The pH of the medium may be in the range of 5 to 8.5, preferably around 7.0, and can be maintained by the addition of buffers to the media. An exemplary buffer for this purpose is a potassium phosphate buffer. Synthetic buffers such as MOPS, HEPES, ACES and others can alternatively or simultaneously be used. It is also possible to maintain a constant culture pH through the addition of an acid or base, such as acetic acid. sulfuric acid, phosphoric acid, NaOH, KOH or NH₄OH during growth. If complex medium components such as yeast extract are utilized, the necessity for additional buffers may be reduced, due to the fact that many complex compounds have high buffer capacities. If a fermentor is utilized for culturing the microorganisms, the pH can also be controlled using gaseous ammonia.

The incubation time is usually in a range from several hours to several days. This time is selected in order to permit the maximal amount of product to accumulate in the broth. The disclosed growth experiments can be carried out in a variety of vessels, such as microtiter plates, glass tubes, glass flasks or glass or metal fermentors of different sizes. For screening a large number of clones, the microorganisms should be cultured in microtiter plates, glass tubes or shake flasks, either with or without baffles. Preferably 100 ml or 250 shake flasks are used, filled with about 10% (by volume) of the required growth medium. The flasks should be shaken on a rotary shaker (amplitudeabout 25 mm) using a speed-range of about 100-300 'rpm. Evaporation losses can be diminished by the maintenance of a humid atmosphere; alternatively, a mathematical correction for evaporation losses should be performed.

If genetically modified clones are tested, an unmodified control clone or a control clone containing the basic plasmid without any insert should also be tested. The medium is inoculated to an OD600 of 0.5-1.5 using cells grown on agar plates, such as CM plates (10g/l glucose, 2,5g/l NaCl, 2g/l urea, 10g/l polypeptone, 5g/l yeast extract, 5g/l meat extract, 22g/l (NH₄)₂SO₄, 2g/l urea, 10g/l polypeptone, 5g/l yeast extract, 5g/l meat extract, 22g/l agar, pH about 6.8 to 7.2 with 2M NaOH) that had been incubated at 30° C. Inoculation of the media is accomplished by either introduction of a saline suspension of *C. glutamicum* cells from CM plates or addition of a liquid preculture of this bacterium.

### General Methods

Protocols for general methods can be found in Handbook on Corynebacterium glutamicum, (2005) eds.: L. Eggeling, M. Bott., Boca Raton, CRC Press, at Martin et al. (Biotechnology (1987) 5, 137-146 ), Guerrero et al. (Gene (1994), 138, 35-41), Tsuchiya und Morinaga (Biotechnology (1988), 6, 428-430), Eikmanns et al. (Gene (1991), 102, 93-98), EP 0 472 869, US 4,601,893, Schwarzer and Pühler (Biotechnology (1991), 9, 84-87, Reinscheid et al. (Applied and Enviromnental Microbiology (1994), 60,126-132), LaBarre et al (Journal of Bacteriology (1993), 175, 1001-1007), WO 96/15246, Malumbres et al. (Gene (1993), 134, 15-24), inJP-A-10-229891, at Jensen und Hammer (Biotechnology and Bioengineering (1998), 58,191-195), Makrides (Microbiological Reviews (1996), 60, 512-538) and in well known textbooks of genetic and molecular biology.

### Strains, Media and Plasmids

Strains can be taken e.g for example, but not limited to, from the following list
*Corynebacterium glutamicum* ATCC 13032,
*Corynebacterium acetoglutamicum* ATCC 15806,
*Corynebacterium acetoacidophilum* ATCC 13870,
*Corynebacterium thermoaminognes* FERM BP-1539,
*Corynebacterium melassecola* ATCC 17965,
*Brevibacterium flavum* ATCC 14067,
*Brevibacterium lactofermentum* ATCC 13869, and
*Brevibacterium divaricatum ATCC 14020* or strains which have been derived therefrom such as *Coryneabacterium glutamicum* KFCC10065, DSM 17322 or
*Corynebacterium glutamicum* ATCC21608
*Corynebacterium efficiens* DSMZ44547, 44548, 44549

### Recombinant DNA technology

Protocols can be found in Sambrook, J , Fritsch, E F , and Maniatis, T , in Molecular Cloning: A Laboratory Manual, 3rd edition (2001) Cold Spring Harbor Laboratory Press, NY, Vol. 1, 2, 3, and Handbook on Corynebacterium glutamicum (2005) eds. L. Eggeling, M. Bott , Boca Raton, CRC Press

### Quantification of amino acids and methionine intermediates.

The analysis is done by HPLC (Agilent 1100, Agilent, Waldbronn, Germany) with a guard cartridge and a Synergi 4µm column (MAX-RP 80 Å, 150 * 4 6 mm) (Phenomenex, Aschaffenburg, Germany). Prior to injection the analytes are derivatized using o-phthaldialdehyde (OPA) and mercaptoethanol as reducing agent (2-MCE). Additionally sulfhydryl groups are blocked with iodoacetic acid. Separation is carried out at a flow rate of 1 ml/min using 40 mM NaH₂PO₄ (eluent A, pH=7.8, adjusted with NaOH) as polar and a methanol water mixture (100 / 1) as non-polar phase (eluent B). The following gradient is applied: Start 0% B; 39 min 39 % B; 70 min 64 % B; 100 % B for 3.5 min; 2 min 0 % B for equilibration. Derivatization at room temperature is automated as described below. Initially 0.5 µl of 0.5% 2-MCE in bicine (0.5M, pH 8.5) are mixed with 0.5 µl cell extract. Subsequently 1.5 µl of 50 mg/ml iodoacetic acid in bicine (0.5M, pH 8.5) are added, followed by addition of 2.5 µl bicine buffer (0.5M, pH 8.5). Derivatization is done by adding 0.5 µl of 10mg/ml OPA reagent dissolved in 1/45/54 v/v/v of 2-MCE/MeOH/bicine (0.5M, pH 8.5). Finally the mixture is diluted with 32 µl H₂O. Between each of the above pipetting steps there is a waiting time of 1 min. A total volume of 37 µl is then injected onto the column. Note, that the analytical results can be significantly improved, if the auto sampler needle is periodically cleaned during (e.g. within waiting time) and after sample preparation. Detection is performed by a fluorescence detector (340 nm excitation, emission 450 nm, Agilent, Waldbronn, Germany). For quantification α-amino butyric acid (ABA) is used as internal standard

### Definition of recombination protocol

In the following it will be described how a strain of *C. glutamicum* with increased efficiency of methionine production can be constructed implementing the findings of the above predictions. Before the construction of the strain is described, a definition of a recombination event/protocol is given that will be used in the following.

"Campbell in," as used herein, refers to a transformant of an original host cell in which an entire circular double stranded DNA molecule (for example a plasmid being based on pCLIK int sacB has integrated into a chromosome by a single homologous recombination event (a cross-in event), and that effectively results in the insertion of a linearized version of said circular DNA molecule into a first DNA sequence of the chromosome that is homologous to a first DNA sequence of the said circular DNA molecule. "Campbelled in" refers to the linearized DNA sequence that has been integrated into the chromosome of a "Campbell in" transformant. A "Campbell in" contains a duplication of the first homologous DNA sequence, each copy of which includes and surrounds a copy of the homologous recombination crossover point. The name comes from Professor Alan Campbell, who first proposed this kind of recombination.

"Campbell out," as used herein, refers to a cell descending from a "Campbell in" transformant, in which a second homologous recombination event (a cross out event) has occurred between a second DNA sequence that is contained on the linearized inserted DNA of the "Campbelled in" DNA, and a second DNA sequence of chromosomal origin, which is homologous to the second DNA sequence of said linearized insert, the second recombination event resulting in the deletion (jettisoning) of a portion of the integrated DNA sequence, but, importantly, also resulting in a portion (this can be as little as a single base) of the integrated Campbelled in DNA remaining in the chromosome, such that compared to the original host cell, the "Campbell out" cell contains one or more intentional changes in the chromosome (for example, a single base substitution, multiple base substitutions, insertion of a heterologous gene or DNA sequence, insertion of an additional copy or copies of a homologous gene or a modified homologous gene, or insertion of a DNA sequence comprising more than one of these aforementioned examples listed above).

A "Campbell out" cell or strain is usually, but not necessarily, obtained by a counter-selection against a gene that is contained in a portion (the portion that is desired to be jettisoned) of the "Campbelled in" DNA sequence, for example the *Bacillus subtilis sacB* gene, which is lethal when expressed in a cell that is grown in the presence of about 5% to 10% sucrose. Either with or without a counter-selection, a desired "Campbell out" cell can be obtained or identified by screening for the desired cell, using any screenable phenotype, such as, but not limited to, colony morphology, colony color, presence or absence of antibiotic resistance, presence or absence of a given DNA sequence by polymerase chain reaction, presence or absence of an auxotrophy, presence or absence of an enzyme, colony nucleic acid hybridization, antibody screening, *etc.* The term "Campbell in" and "Campbell out" can also be used as verbs in various tenses to refer to the method or process described above.

It is understood that the homologous recombination events that leads to a "Campbell in" or "Campbell out" can occur over a range of DNA bases within the homologous DNA sequence, and since the homologous sequences will be identical to each other for at least part of this range, it is not usually possible to specify exactly where the crossover event occurred. In other words, it is not possible to specify precisely which sequence was originally from the inserted DNA, and which was originally from the chromosomal DNA. Moreover, the first homologous DNA sequence and the second homologous DNA sequence are usually separated by a region of partial non-homology, and it is this region of non-homology that remains deposited in a chromosome of the "Campbell out" cell.

For practicality, in *C. glutamicum,* typical first and second homologous DNA sequence are at least about 200 base pairs in length, and can be up to several thousand base pairs in length, however, the procedure can be made to work with shorter or longer sequences. For example, a length for the first and second homologous sequences can range from about 500 to 2000 bases, and the obtaining of a "Campbell out" from a "Campbell in" is facilitated by arranging the first and second homologous sequences to be approximately the same length, preferably with a difference of less than 200 base pairs and most preferably with the shorter of the two being at least 70% of the length of the longer in base pairs. The "Campbell In and -Out-method" is described in WO2007012078

### Examples

The following experiments demonstrate how overexpression of *C.jeiekeium* formate-THF-synthetase and *gcvHTP* as well as *lipB* or *lpl* leads to increased methionine production. These examples are however in no way meant to limit the invention in any way.

### Shake flask experiments and HPLC assay

Shake flasks experiments, with the standard Molasses Medium, were performed with strains in duplicate or quadruplicate. Molasses Medium contained in one liter of medium: 40 g glucose; 60 g molasses; 20 g (NH₄)₂ SO₄; 0.4 g MgSO₄*7H₂O; 0.6 g KH₂PO₄; 10 g yeast extract (DIFCO); 5 ml of 400 mM threonine; 2 mgFeSO₄.7H₂O; 2 mg of MnSO₄.H₂O; and 50 g CaCO₃ (Riedel-de Haen), with the volume made up with ddH₂O. The pH was adjusted to 7.8 with 20% NH₄OH. 20 ml of continuously stirred medium (in order to keep CaCO₃ suspended) was added to 250 ml baffled Bellco shake flasks and the flasks were autoclaved for 20 min. Subsequent to autoclaving, 4 ml of "4B solution" was added per liter of the base medium (or 80 µl/ flask). The "4B solution" contained per liter: 0.25 g of thiamine hydrochloride (vitamin B1), 50 mg of cyanocobalamin (vitamin B 12), 25 mg biotin, 1.25 g pyridoxine hydrochloride (vitamin B6) and was buffered with 12.5 mM KPO₄ , pH 7.0 to dissolve the biotin, and was filter sterilized. Cultures were grown in baffled flasks covered with Bioshield paper secured by rubber bands for about 48 hours at about 28°C or 30°C and at 200 or 300 rpm in a New Brunswick Scientific floor shaker. Samples were typically taken at about 24 hours and/or about 48 hours. Cells were removed by centrifugation followed by dilution of the supernatant with an equal volume of 60% acetonitrile or 60% ethanol and then membrane filtration of the solution mixture using Centricon 0.45 µm spin columns. The filtrates were assayed using HPLC for the concentrations of methionine, glycine plus homoserine, O-acetylhomoserine, threonine, isoleucine, lysine, and other indicated amino acids.

For the HPLC assay, filtered supernatants were diluted 1:100 with 0.45µm filtered 1 mM Na₂EDTA and 1 µl of the solution was derivatized with OPA reagent (AGILENT) in Borate buffer (80 mM NaBO₃, 2.5 mM EDTA, pH 10.2) and injected onto a 200 x 4.1 mm Hypersil 5µ AA-ODS column run on an Agilent 1100 series HPLC equipped with a G1321A fluorescence detector (AGILENT). The excitation wavelength was 338 nm and the monitored emission wavelength was 425 nm. Amino acid standard solutions were chromatographed and used to determine the retention times and standard peak areas for the various amino acids. Chem Station, the accompanying software package provided by Agilent, was used for instrument control, data acquisition and data manipulation. The hardware was an HP Pentium 4 computer that supports Microsoft Windows NT 4.0 updated with a Microsoft Service Pack (SP6a).

### Experiment 1: Generation of the M2014 strain

*C. glutamicum* strain ATCC 13032 was transformed with DNA A (also referred to as pH273) (SEQ ID NO: 21) and "Campbelled in" to yield a "Campbell in" strain. The "Campbell in" strain was then "Campbelled out" to yield a "Campbell out" strain, M440, which contains a gene encoding a feedback resistant homoserine dehydrogenase enzyme (*hom^{fbr}*). The resultant homoserine dehydrogenase protein included an amino acid change where S393 was changed to F393 (referred to as *Hsdh* S393F).

The strain M440 was subsequently transformed with DNA B (also referred to as pH373) (SEQ ID NO: 22) to yield a "Campbell in" strain. The "Campbell in" strain were then "Campbelled out" to yield a "Campbell out" strain, M603, which contains a gene encoding a feedback resistant aspartate kinase enzyme *(Ask^{fbr}*) (encoded by *lysC*). In the resulting aspartate kinase protein, T311 was changed to I311 (referred to as LysC T311I).

It was found that the strain M603 produced about 17.4 mM lysine, while the ATCC13032 strain produced no measurable amount of lysine. Additionally, the M603 strain produced about 0.5 mM homoserine, compared to no measurable amount produced by the ATCC 13032 strain, as summarized in Table 2.

**Table 2: Amounts of homoserine, O-acetylhomoserine, methionine and lysine produced by strains ATCC13032 and M603**

| Strain | Homoserine (mM) | O-acetyl homoserine (mM) | Methionine (mM) | Lysine (mM) |
|---|---|---|---|---|
| ATCC13032 | 0.0 | 0.4 | 0.0 | 0.0 |
| M603 | 0.5 | 0.7 | 0.0 | 17.4 |

The strain M603 was transformed with DNA C (also referred to as pH304) (SEQ ID NO:23) to yield a "Campbell in" strain, which was then "Campbelled out" to yield a "Campbell out" strain, M690. The M690 strain contained a PgroES promoter upstream of the metH gene (referred to as P₄₉₇ *metH*). The sequence of the P₄₉₇ promoter is depicted in SEQ ID NO: 4. The M690 strain produced about 77.2 mM lysine and about 41.6 mM homoserine, as shown below in Table 3.

**Table 3: Amounts of homoserine, O-acetyl homoserine, methionine and lysine produced by the strains M603 and M690**

| Strain | Homoserine (mM) | O-acetyl homoserine (mM) | Methionine (mM) | Lysine (mM) |
|---|---|---|---|---|
| M603 | 0.5 | 0.7 | 0.0 | 17.4 |
| M690 | 41.6 | 0.0 | 0.0 | 77.2 |

The M690 strain was subsequently mutagenized as follows: an overnight culture of M603, grown in BHI medium (BECTON DICKINSON), was washed in 50mM citrate buffer pH 5.5, treated for 20 min at 30°C with N-methyl-N-nitrosoguanidine (10 mg/ml in 50mM citrate pH 5.5). After treatment, the cells were again washed in 50 mM citrate buffer pH 5.5 and plated on a medium containing the following ingredients: (all mentioned amounts are calculated for 500 ml medium) 109 CNH₄)₂SO₄; 0.5g KH₂PO₄; 0.5g K₂HPO₄; 0.125g MgSO₄*7H₂O; 21g MOPS; 50 mg CaCl₂; 15 mg protocatechuic acid; 0.5 mg biotin; 1 mg thiamine; and 5 g/l D,L-ethionine (SIGMA CHEMICALS, CATALOG #E5139), adjusted to pH 7.0 with KOH. In addition the medium contained 0.5 ml of a trace metal solution composed of: 10 g/l FeSO₄*7H₂O; 1 g/l MnSO₄*H₂O; 0.1 g/l ZnSO₄*7H₂O; 0.02 g/l CuSO₄; and 0.002 g/l NiCl₂*6H₂O, all dissolved in 0.1 M HCl. The final medium was sterilized by filtration and to the medium, 40 mls of sterile 50% glucose solution (40 ml) and sterile agar to a final concentration of 1.5 % were added. The final agar containing medium was poured to agar plates and was labeled as minimal-ethionine medium. The mutagenized strains were spread on the plates (minimal-ethionine) and incubated for 3-7 days at 30°C. Clones that grew on the medium were isolated and restreaked on the same minimal-ethionine medium. Several clones were selected for methionine production analysis.

Methionine production was analyzed as follows. Strains were grown on CM-agar medium for two days at 30°C, which contained: 10 g/l D-glucose, 2.5 g/l NaCl; 2 g/l urea; 10 g/l Bacto Peptone (DIFCO); 5 g/l Yeast Extract (DIFCO); 5 g/l Beef Extract (DIFCO); 22 g/l Agar (DIFCO); and which was autoclaved for 20 min at about 121°C.

After the strains were grown, cells were scraped off and resuspended in 0.15 M NaCl. For the main culture, a suspension of scraped cells was added at a starting OD of 600 nm to about 1.5 to 10 ml of Medium II (see below) together with 0.5 g solid and autoclaved CaCO₃ (RIEDEL DE HAEN) and the cells were incubated in a 100 ml shake flask without baffles for 72 h on a orbital shaking platform at about 200 rpm at 30°C. Medium II contained: 40 g/l sucrose; 60 g/l total sugar from molasses (calculated for the sugar content); 10 g/l (NH₄)₂SO₄; 0.4 g/l MgSO₄*7H₂O; 0.6 g/l KH₂PO₄; 0.3 mg/l thiamine*HCl; 1 mg/l biotin; 2 mg/l FeSO₄; and 2 mg/l MnSO₄. The medium was adjusted to pH 7.8 with NH₄OH and autoclaved at about 121°C for about 20 min). After autoclaving and cooling, vitamin B₁₂ (cyanocobalamine) (SIGMA CHEMICALS) was added from a filter sterile stock solution (200 µg/ml) to a final concentration of 100 µg/l.

Samples were taken from the medium and assayed for amino acid content. Amino acids produced, including methionine, were determined using the Agilent amino acid method on an Agilent 1100 Series LC System HPLC. (AGILENT). A pre-column derivatization of the sample with ortho-pthalaldehyde allowed the quantification of produced amino acids after separation on a Hypersil AA-column (AGILENT).

Clones that showed a methionine titer that was at least twice that in M690 were isolated. One such clone, used in further experiments, was named M1197 and was deposited on May 18, 2005, at the DSMZ strain collection as strain number DSM 17322. Amino acid production by this strain was compared to that by the strain M690, as summarized below in Table 4.

**Table 4: Amounts of homoserine, O-acetylhomoserine, methionine and lysine produced by strains M690 and M1197**

| Strain | Homoserine (mM) | O-acetyl-homoserine (mM) | Methionine (mum) | Lysine (mM) |
|---|---|---|---|---|
| M690 | 41.6 | 0.0 | 0.0 | 77.2 |
| M1179 | 26.4 | 1.9 | 0.7 | 79.2 |

The strain M1197 was transformed with DNA F (also referred to as pH399, SEQ ID NO: 24) to yield a "Campbell in" strain, which was subsequently "Campbelled out" to yield strain M1494. This strain contains a mutation in the gene for the homoserine kinase, which results in an amino acid change in the resulting homoserine kinase enzyme from T190 to A190 (referred to as HskT190A). Amino acid production by the strain M1494 was compared to the production by strain M1197, as summarized below in Table 5.

**Table 5: Amounts of homoserine, O-acetylhomoserine, methionine and lysine produced by strains M1197 and M1494**

| Strain | Homoserine (mM) | O-acetyl-homoserine (mM) | Methionine (mM) | Lysine (mM) |
|---|---|---|---|---|
| M1197 | 26.4 | 1.9 | 0.7 | 79.2 |
| M1494 | 18.3 | 0.2 | 2.5 | 50.1 |

The strain M1494 was transformed with DNA D (also referred to as pH484, SEQ ID NO:25) to yield a "Campbell in" strain, which was subsequently "Campbelled out" to yield the M1990 strain. The M1990 strain overexpresses a *metY* allele using both a groES-promoter and an EFTU (elongation factor Tu)-promoter (referred to as P₄₉₇ P₁₂₈₄ *metY*). The sequence of P₄₉₇P₁₂₈₄ promoter is set forth in SEQ ID NO:26 Amino acid production by the strain M1494 was compared to the production by strain M1990, as summarized below in Table 6.

**Table 6: Amounts of homoserine, O-acetylhomoserine, methionine and lysine produced by strains M1494 and M1990**

| Strain | Homoserine (mM) | O-acetyl-homoserine (mM) | Methionine (mM) | Lysine (mM) |
|---|---|---|---|---|
| M1494 | 18.3 | 0.2 | 2.5 | 50.1 |
| M1990 | 18.2 | 0.3 | 5.6 | 48.9 |

The strain M1990 was transformed with DNA E (also referred to as pH 491, SEQ ID NO: 27) to yield a "Campbell in" strain, which was then "Campbelled out" to yield a "Campbell out" strain M2014. The M2014 strain overexpresses a *metA* allele using a superoxide dismutase promoter (referred to as P₃₁₁₉ *metA*). The sequence of P₃₁₁₉ promoter is set forth in SEQ ID NO: 3. Amino acid production by the strain M2014 was compared to the production by strain M1990, as summarized below in Table 7

**Table 7: Amounts of homoserine, O-acetylhomoserine, methionine and lysine produced by strains M1494 and M1990**

| Strain | Homoserine (mM) | O-acetyl-homoserine (mM) | Methionine (mM) | Lysine (mM) |
|---|---|---|---|---|
| M1990 | 18.2 | 0.3 | 5.6 | 48.9 |
| M2014 | 12.3 | 1.2 | 5.7 | 49.2 |

### Experiment 2 - Deletion of mcbR from M2014

Plasmid pH429 containing an RXA00655 deletion, (SEQ ID NO:28) was used to introduce the *mcbR* deletion into *C. glutamicum* via integration and excision (see WO 2004/050694 A1).

Plasmid pH429 was transformed into the M2014 strain with selection for kanamycin resistance (Campbell in). Using sacB counter-selection, kanamycin-sensitive derivatives of the transformed strain were isolated which presumably had lost the integrated plasmid by excision (Campbell out). The transformed strain produced kanamycin-sensitive derivatives that made small colonies and larger colonies. Colonies of both sizes were screened by PCR to detect the presence of *mcbR* deletion. None of the larger colonies contained the deletion, whereas 60-70% of the smaller colonies contained the expected *mcbR* deletion.

When an original isolate was streaked for single colonies on BHI plates, a mixture of tiny and small colonies appeared. When the tiny colonies were restreaked on BHI, once again a mixture of tiny and small colonies appeared. When the small colonies were restreaked on BHI, the colony size was usually small and uniform. Two small single colony isolates, called OM403-4 and OM403-8, were selected for further study.

Shake flask experiments (Table 8) showed that OM403-8 produced at least twice the amount of methionine as the parent M2014. This strain also produced less than one-fifth the amount of lysine as M2014, suggesting a diversion of the carbon flux from aspartate semialdehyde towards homoserine. A third striking difference was a greater than 10-fold increase in the accumulation of isoleucine by OM403 relative to M2014. Cultures were grown for 48 hours in standard molasses medium.

**Table 8: Amino acid production by isolates of the OM403 strain in shake flask cultures inoculated with freshly grown cells**

| **Strain** | **Colony size** | **Deletion** Δ**mcbR** | **Met (g/l)** | **Lys (g/l)** | **Hse+Gly (g/l)** | **Ile (g/l)** |
|---|---|---|---|---|---|---|
| M2014 | Large | none | 0.2 | 2.4 | 0.3 | 0.04 |
| | | | 0.2 | 2.5 | 0.3 | 0.03 |
| | | | 0.2 | 2.4 | 0.3 | 0.03 |
| | | | 0.4 | 3.1 | 0.4 | 0.03 |
| | | | | | | |
| OM403-8 | Small | ΔRXA0655 | 1.0 | 0.3 | 0.8 | 0.8 |
| | | | 1.0 | 0.3 | 0.8 | 0.8 |
| | | | 0.9 | 0.3 | 0.8 | 0.8 |
| | | | 1.0 | 0.3 | 0.8 | 0.6 |

Also as shown in Table 9, there was a greater than 15-fold decrease in the accumulation of O-acetylhomoserine by OM403 relative to M2014. The most likely explanation for this result is that most of the O-acetylhomoserine that accumulates in M2014 is being converted to methionine, homocysteine, and isoleucine in OM403. Cultures were grown for 48 hours in standard molasses medium.

**Table 9: Amino acid production by two isolates of OM403 in shake flask cultures inoculated with freshly grown cells.**

| ***Strain*** | ***Deletion*** Δ***mcbR*** | ***Met (g*/*l)*** | ***OAc-Hse (g*/*l)*** | ***Ile (g*/*l)*** |
|---|---|---|---|---|
| *M2014* | *None* | *0.4* | *3.4* | *0.1* |
| | | *0.4* | *3.2* | *0.1* |
| *OM403-4* | Δ*XA0655* | *1.7* | *0.2* | *0.3* |
| | | *1.5* | *0.1* | *0.3* |
| *OM-403-8* | Δ*RXA0655* | *2.2* | *<0.05* | *0.6* |
| | | *2.5* | *<0.05* | *0.6* |

### Experiment 3 - Decreasing metQ expression

In order to decrease the import of methionine in OM403-8, the promoter and 5' portion of the *metQ* gene were deleted. The *metQ* gene encodes a subunit of a methionine import complex that is required for the complex to function. This was accomplished using the standard Campbelling in and Campbelling out technique with plasmid pH449 (SEQ ID NO: 29). OM403-8 and OM456-2 were assayed for methionine production in shake flask assays. The results (Table 10) show that OM456-2 produced more methionine than OM403-8. Cultures were grown for 48 hours in standard molasses medium.

**Table 10: Shake flask assays of OM456-2**

| Strain | vector | [Met] (g/l) | [Lys] (g/l) | [Gly/Hse] (g/l) | [OAcHS] (g/l) | [Ile] (g/l) |
|---|---|---|---|---|---|---|
| | | | | | | |
| OM403-8 | none | 4.0 | 0.8 | 2.2 | 0.4 | 1.9 |
| | | 3.9 | 0.6 | 2.2 | 0.4 | 1.9 |
| | | | | | | |
| OM456-2 | none | 4.2 | 0.4 | 2.3 | 0.4 | 2.3 |
| | | 4.3 | 0.5 | 2.4 | 0.4 | 2.3 |

### Experiment 4 Construction of OM469

A strain referred to as OM469 was constructed which included both deletion of *metQ* and overexpression of *metF* by replacing the *metF* promoter with the phage λP_{R} promoter in OM456-2. This was accomplished using the standard Campbelling in and Campbelling out technique with plasmid pOM427 (SEQ ID NO 30). Four isolates of OM469 were assayed for methionine production in shake flask culture assays where they all produced more methionine than OM456-2, as shown in Table 11. Cultures were grown for 48 hours in standard molasses medium containing 2 mM threonine.

**Table 11: Shake flask assays of OM469, a derivative of OM456-2 containing the phage lambda P_{R} promoter in place of the metF promoter.**

| Strain | metF promoter | MetQ | [Met] (g/l) | [Lys] (g/l) | [Gly/Hse] (g/l) | [OAcHS] (g/l) | [Ile] (g/l) |
|---|---|---|---|---|---|---|---|
| | | | | | | | |
| OM428-2 | λP_{R} | native | 4.5 | 0.5 | 2.6 | 0.4 | 2.6 |
| | | | 4.6 | 0.4 | 2.6 | 0.3 | 2.5 |
| | | | | | | | |
| OM456-2 | native | ΔmetQ | 4.2 | 0.4 | 2.4 | 0.3 | 2.5 |
| | | | 4.2 | 0.5 | 2.4 | 0.3 | 2.5 |
| | | | | | | | |
| OM469-1 | λP_{R} | ΔmetQ | 5.0 | 0.5 | 2.7 | 0.4 | 3.1 |
| -2 | | | 4.9 | 0.5 | 2.7 | 0.4 | 2.8 |
| -3 | | | 4.8 | 0.4 | 2.6 | 0.4 | 2.7 |
| -4 | | | 4.7 | 0.5 | 2.6 | 0.4 | 2.8 |

### Experiment 5 - Construction of M 2543

The strain OM469-2 was transformed by electroporation with the plasmid pCLIK5A PSOD TKT as depicted in SEQ ID NO. 31. This was accomplished using the standard Campbelling in and Campbelling out technique.

Isolates of OM 469 PSOD TKT which are labelled M2543 were assayed for methionine production in shake flask culture assays, where they produced more methionine than OM469-2. The results of strain M2543 Are shown in Table 12.

**Table 12. Shake flask assays of OM469 and M2543**

| Strain | plasmid | met genes on plasmid | [Met] (mm | [Lys] (mm) | [Gly] (mm) | [Hse] (mm) | [AHs] (mm) | [Ile] (mm) |
|---|---|---|---|---|---|---|---|---|
| | | | | | | | | |
| OM469-2 | | None | 14 | 3,4 | 16 | 1,7 | 0,3 | 11,8 |
| | | | | | | | | |
| M2543# | | None | 20,4 | 1,9 | 21,8 | 0,8 | <0,1 | 12,4 |

### Experiment 6 - Construction of GK1259

In order to decrease expression of serine deaminase (sda), a portion of the *sda* gene was deleted. This was accomplished using the standard Campbelling in and Campbelling out technique with plasmid pH626 int SacB delta sdaA (SEQ ID No. 32). To this end, strain M2543 was transformed by electroporation with the plasmid pH626 int SacB delta sdaA. The resulting strain was named GK1259.

### Experiment 7 - Construction of OM264C

Plasmid pOM253 (SEQ ID No. 33) was used to delete *serA* and substitute it with spectinomycin resistance in *C. glutamicum* strain M2014. The resulting "Campbelled out" strain, *M2014* Δ*serA*::*spec*, was named OM264C. OM264C is a serine auxotroph. Since it also lacks a functional GCS, OM264C cannot grow on a minimal medium lacking serine but containing glycine. If, however, a functional GCS system is installed in OM264C, then it will gain the ability to grow on minimal medium containing glycine.

The recipe for the minimal (chemically defined) plates was as follows:

| Stock solution name | Concentration of Stock | Volume of stock for 1 liter |
|---|---|---|
| 10 X Spizizen's Salts | See below | 100 ml |
| Glucose | 50 % w/v | 10 ml |
| 4B's Solution | See below | 4 ml |
| Threonine | 400 mM | 5 ml |
| Cysteine HCl | 4 g/l | 10 ml |
| CaCl₂·2H₂O | 5 % w/v | 5 ml |
| Sodium citrate | 1.0 M | 20 ml |
| Thymidine | 1 % w/v | 10 ml |
| Phenylalanine | 1 % w/v | 10 ml |
| Isoleucine | 1 % w/v, | 10 ml |
| Thiamine HCl | 0.1 % w/v | 5 ml |
| Methionine | 1 % w/v | 5 ml |
| Sodium succinate | 1.0 M | 3 ml |
| Potassium acetate | 5.0 M | 1.2 ml |
| Glycine (when added) | 10 % w/v | 5 ml |
| Serine (when added) | 10 % w/v | 2 ml |
| Lipoic acid (when added) | 1 g/l in 20 mM potassium phosphate, pH 7.0 | 1 ml |

All stocks were filter sterilized. For agar Petri plates, 15 g agar were suspended in 800 ml distilled water and autoclaved.
10 X Spizizen's salts
20 g Ammonium sulfate
174 g Potassium phosphate dibasic (trihydrate)
60 g Potassium phosphate monobasic (anhydrous)
10 g Sodium citrate (dihydrate)
2 g Magnesium sulfate (heptahydrate)

### Distilled water to one liter

Add 3.5 ml 0.4% FeCl₃•6H₂0 filter sterilized and 1 ml Micronutrient solution (see below). Final pH should be about 7.2. Filter sterilize.

### Micronutrient solution: amount for 1 liter

0.15 g Na₂MoO₄•2H₂O
2.5 g H₃BO₃
0.7 g CoCl₂•6H₂O
0.25 g CuSO₄•5H₂O
1.6 g MnCl•4H₂O
0.3 g ZnSO₄•7H₂O
Distilled water to one liter.
Filter sterilize.
4B's solution: amount for one liter

0.25 g Thiamine HCl (Vitamin B₁)
50 mg Cyanocobalamin (Vitamin B₁₂)
25 to 28 mg Biotin
1.25 g Pyridoxine HCl (Vitamin B₆)
Dissolve in 50 mM potassium phosphate, pH 7.0
Filter sterilize. Store in the dark.

### Experiment 8 - Construction of strains expressing the C. jeikeium gcvPTH genes in C. glutamicum.

Unlike *C. glutamicum,* a close relative named *C. jeikeium* does contain a GCS. In the *C. jeikeium* chromosome, the *gcvP*, *T*, and *H* genes are clustered together in an operon (Tauch et al., 2005, J. Bacteriol., vol 187, pp4671-4682). This cluster was cloned in four overlapping subset pieces by polymerase chain reaction (PCR) using *C. jeikeium* strain K411 chromosomal DNA as the template.

The necessary DNA was obtained by dividing the sequence into four smaller regions and obtaining four independent smaller PCR fragments. The four pieces were amplified with four sets of primers. An artificial *Xba*I site and an artificial ribosome binding site were engineered just upstream from the *gcvP* start codon in the respective sense primer, and an artificial *Bam*HI site was engineered just downstream from the *gcvH* stop codon in the respective antisense primer. This allowed the coding sequences of the gcvPTH cluster to be reconstituted and carried on an *Xba*I to *Bam*HI fragment.

The resulting *Xba*I to *Bam*HI fragment containing a reconstituted *gcvPTH* cluster was next cloned into *C. glutamicum* replicating plasmids designed to express genes from the *C. glutamicum groESL* promoter, herein named *P₄₉₇*, or a *B. subtilis* phage SPO 1 promoter, herein named *P₁₅* (SEQ ID No. 42). The resulting plasmids were named pOM615 (SEQ ID No. 34) and pOM616 (SEQ ID No. 35), respectively. The *P₄₉₇* and *P₁₅* promoters were used because they promote constitutive expression of genes situated downstream. In particular, these promoters are not significantly regulated by any glycine related metabolite, such as glycine, serine, methionine, thymidine, purine etc.

### Experiment 9 - The C. jeikeium gcvP7H genes function in C. glutamicum

Plasmids pOM615, pOM616, and empty vector pCLIK were each separately transformed into the tester strain *C*. *glutamicum* strain OM264C and the methionine producer *C. glutamicum* strain GK1259 using selection on Brain Heart Infusion (formerly Difco, now Becton Dickenson) agar plates containing kanamycin sulfate (25 mg/l). The OM264C transformants were streaked on minimal plates lacking serine but containing glycine. Lipoic acid was added to the medium to give a final concentration of 1 mg/l to ensure that a sufficient amounts of this cofactor of GcvH was present.

Both, the pOM615 and pOM616 derived strains, OM264C(pOM615) and OM264C(pOM616) as weel as GK1259(pOM615) and GK1259(pOM616) grew , while the pCLIK transformant(OM264C(pCLIK) did not.

### Experiment 10 - The lipBA genes from C. jeikeium function in C. glutamicum.

*C. glutamicum* is a lipoic acid prototroph, and *C. glutamicum* presumably has a lipoyl synthetase, since pyruvate dehydrogenase and α-ketoglutarate dehydrogenase are active.

In *E. coli,* there are two different pathways for attachment (ligation) of lipoic acid to target proteins, the LipB dependent pathway for endogenously synthesized lipoic acid, and the LplA pathway for fed lipoic acid (Morris et al., 1995, J. Bacteriol. Vol 177, pp 1-10).

By sequence comparison, *C. glutamicum* seems to have good homologs for both LipB and LplA. Thus, the *lipB* gene and its native promoter, together with the *lipA* gene, was cloned by PCR from *C. jeikeium,* strain K411 chromosomal DNA as a template. The resulting blunt PCR fragment was ligated into the unique SwaI site of pOM615 or pOM616 to give pOM620AF (SEQ ID No 36) and pOM621AR (SEQ ID No 37), respectively.

Plasmids pOM620AF and pOM621AR were each transformed into strain OM264C resulting in OM264C(pOM620AF) and OM264C(pOM621AR) respectively and into methionine producing strain GK1259 resulting in GK1259(pOM620AF) and GK1259(pOM621AR), respectively. The OM264C transformants were streaked on the minimal glycine plates described above (but without lipoic acid), and both transformants grew, while OM264C(pCLIK) did not, demonstrating that the *C. jeikeium* LipB pathway could lipoylate the *C. jeikeium* GcvH protein when the two were expressed together in *C*. *glutamicum.*

The GK1259 transformants of pOM615, pOM616, pOM620AF, pOM621AR, and pCLIK were tested for methionine and glycine production in shake flasks using molasses medium, without lipoic acid or with lipoic acid added to a final concentration of about 10 mg/ml. The results are shown in Tables 13 and 14 below.

**Table 13: Methionine and glycine production by GK1259 transformed with various plasmids designed to express C. jeikeium gcvPTH with lipBA and grown in shake flasks in molasses medium.**

| Plasmid | Promoter for *gcvPTH* | *lipBA* | Lipoic acid | Glycine g/l | Methionine g/l |
|---|---|---|---|---|---|
| pCLIK | - | - | - | 2.5 | 4.3 |
| pOM615 | *P₄₉₇* | - | + | 0.1 | 4.7 |
| pOM620AF | *P₄₉₇* | + | - | 0.0 | 5.0 |

**Table 14: Methionine and glycine production by OP264C transformed with various plasmids designed to express C. jeikeium gcvPTH with lipBA and grown in shake flasks in molasses medium.**

| Plasmid | Promoter for *gcvPTH* | *lipBA* | added lipoic acid | Glycine* g/l | Methionine* g/l |
|---|---|---|---|---|---|
| pCLIK | - | - | - | 2.1 | 3.8 |
| pOM616 | *P₁₅* | - | + | 0.0 | 4.1 |
| POM621AR | *P₁₅* | + | - | 0.0 | 3.9 |

| | | | | | |
|---|---|---|---|---|---|
| * Methionine and glycine titers are averages of duplicate samples, except for the pOM616 plus lipoic acid sample, which is a single sample. | | | | | |

From these results, it is clear that the excess glycine by-product is consumed and the methionine titer is improved by expressing the *C. jeikeium gcvPTH* operon. Further improvement is achieved by expressing the *C. jeikeium gcvPTH* operon and the *C. jeikeium lipBA* operon from the same plasmid.

### Experiment 11 - Expression of gcvPTH and lipBA from integrated cassettes.

In the examples given above, the *gcv* and *lip* genes were installed on plasmids that replicate in the *C*. *gllftamiclfm.* However, these genes can also be installed through an integrating vector. For example, the *C. jeikeium gcvPTH* operon expressed from promoter *P₁₅* has been ligated into an integrating vector to give pOM627 (SEQ ID No. 38), which is designed to integrate at the *bioB* locus of *C. glutamicum.*

pOM627 can be "Campbelled in" and "Campbelled out" of strains OM469 and GK1259. As in the above example, one observes improved methionine production and reduced glycine accumulation as is shown in Table 15.

**Table 15: Methionine and glycine production by transformants of OM469 and GK1259 using pOM627 designed to express C. jeikeium gcvPTH from P₁₅ after integration at bioB, and grown in shake flasks in molasses medium with or without 10 mg/l lipoic acid added.**

| Strain | Lipoic acid | Glycine g/l | Methionine g/l |
|---|---|---|---|
| OM469(pOM627)K | - | 2.7 | 3.8 |
| OM469(pOM627)K | + | 2.0 | 4.0 |
| GK1259(pOM627)K | - | 2.8 | 3.9 |
| GK1259(pOM627)K | + | 2.0 | 4.1 |

The effect from the integrating plasmid pOM627 on glycine and methionine production can be improved by installing multiple copies or by increasing the promoter strength.

The *C. jeikeium lipBA* operon can also be added (either on replicating or integrating vectors). An example of an integrating plasmid that expresses *lipBA* from promoter *P₄₉₇* after integrating at the *bioAD* locus of *C. glutamicum* is pOM180 (SEQ ID No 39).

### Experiment 12 - The E. coli GCS system also functions in C. glutamicum

The *E. coli lpd* gene was amplified by PCR and installed in an integrating plasmid to give pOM331 (SEQ ID No 40). The integration site is a gene herein named *metE2*, a gene that is homologous to a portion of *metE*, but which does not appear to be essential for growth or methionine production in *C*. *glutamicum.*

In pOM331, the *E. coli lpd* gene is expressed from the *P₄₉₇* promoter. pOM331 was transformed into and Campbelled out of OM264Cto give new strain OM197, which was confirmed by an appropriate diagnostic PCR to contain the integrated *P₄₉₇ lpd* cassette. In addition, the *E. coli gcvTHP* operon was amplified by PCR and ligated just downstream from the *P₄₉₇* promoter in a replicating vector to give pOM344 (SEQ ID No. 41). pOM344 and pCLIK were each separately transformed into strain OM197, and the resulting strains were streaked on minimal glycine plates containing glycine and lipoic acid, but lacking serine (see above).

After several days at 30° C, OM197/pOM344 had grown, but OM197/pCLIK had not, demonstrating that the *E*. *coli* GCS system was functioning in *C*. *glutamicum.*

In the above examples, it is shown that genes encoding GCS subunits P, T and H, and genes encoding enzymes that catalyze lipoic acid ligation or synthesis, cloned from either of two "donor" organisms (which are relatively unrelated to each other) are each capable of functioning to give measurable GCS activity in *C. glutamicum,* either by showing complementation of a serine auxotrophy on plates containing glycine but no serine or by showing a decrease in glycine production by a methionine producing strain compared to the glycine production of the relevant parent or precursor strain transformed with an empty vector as a control.

By extension, it seems reasonable to assume that one skilled in the art will be able to follow the examples disclosed herein and reconstitute other GCS systems in *C. glutamicum* and that GCS activity can be established and/or increased in other microorganisms by cloning the relevant genes from the same (i.e. *E. coli* and *C. jeikeium*) or other donor organisms. An improvement may also be achieved by feeding lipoic acid e.g. at about 0.1 to 10 mg/l.

### Experiment 13 - Construction of M2616

*C: glutamicum* M2616 was constructed. This strain which shows deleted serA activity allows for testing formate THF synthetase function.

Plasmid pHF96 (SEQ ID 43) is an integrating plasmid designed to create a deletion-substitution in the serA gene of *C. glutamicum* strains related to C. glutamicum ATCC 13032. Plasmid pHF96 int sacB delta serA was used to delete sera in M2543. The plasmid was transformed in to *C. glutamicum* M2543 cby electroporation and kanamycin resistant clones were isolated. After determining these kanamycin-resistant as successful "Campbelled in" strains by PCR the strains grown overnight in liquid CM medium and were plated on sucrose (10% concentration) containing CM Medium. The resulting "Campbelled out" strain, M2543 delta serA, was named M2616. As expected, M2616 is a serine auxotroph when assayed on minimal medium. Also as expected, since it lacks a functional formate THF synthetase, M2616 cannot grow on a minimal medium lacking serine but containing glycine and formate (see Example for recipe for minimal (chemically defined) medium). If a functional formate THF synthetase system is installed in M2616, then it will gain the ability to grow on minimal medium containing glycine.

### Experiment 14 - Cloning of two formyl- THF synthetase genes from two sources of Corynebacterium jeikeium

Unlike *C. glutamicum,* a close relative named Corynebacterium j eikeium does contain a formyl-THF synthetase protein with the accession number NP_939608 and a coresponding gene with the accession number GeneID: 2649808.

Two sources of templates were utilized for the cloning of the formyl-THF synthetase from Corynebacterium jeikeium. DNA derived from the strain of the NCTC National Collection of Type Cultures London, UK, number 11915 wit the strain designation K411 and from the DSMZ strain 7171 were used. Chromosomal DNA was prepared using the Quiagen DNA Kit as described by the manufacturer. Oligonucleotides HS1304 (SEQ ID No: 44) and HS1305 (SEQ ID No. 45) were used to amplify the genomic sequences of the formate-THF synthetase gene from the two sources of genomic DNA (NCTC 11915 and DZMZ 7171). Pwo polymerase from Roche Mannheim was used at the following conditions: Annealing at 52°C for 30" and elongation at 72°C for 120" yielded a PCR fragment of about 1700Bp.

In addition primers HS1302 (SEQ ID No. 46) and HS1303 (SEQ ID No. 47)were used to amplify the promotor expression unit with the sequence as described from chromosomal DNA derived from the strain ATCC13032. Pwo polymerase from Roche Mannheim was used at the following conditions: Annealing at 53°C for 30" and elongation at 72°C for 30" yielded a PCR fragment of about 200Bp.

Both fragments were added in a third PCR in which the primers HS13032 and HS1305 were added. The third PCR was performed with limiting amounts of the PCR fragments I and II at sufficient amounts of the end to end primers and was a fused using the end-to end primers. HS1302+HS1305 were used to amplify a fusion construct of the PCR fragments resulting from primers HS1302+HS1303 and HS1304+HS1305. Pwo polymerase from Roche Mannheim was used at the following conditions: Annealing at 55°C for 30" and elongation at 72°C for 120" yielded a PCR fragment of about 1900Bp.

The fragment was purified with the GFX PCR purification kit and was digested using the restriction enzymes MluI and XbaI. Positive plasmids containing the insert of the formyl-THF synthetase gene were sequenced.

The gene derived from C jeikeium NCTC K11915 was sequenced. Sequencing of this gene revealed the sequence to be as expected. The gene derived from C jeikeium DSMZ 7171 was sequenced. Sequencing of this gene revealed a sequence as described in the plasmid sequence pH657.

Plasmid pH655 (SEQ ID No. 48) comprising formate-THF-synthetase from NCTC K11915 and pH657 (SEQ ID No. 49) comprising formate-THF-synthetase from DSMZ7171 were transformed into the strain M2616 lacking the functional serA gene by electroporation.

The resulting strains M2616(pH655) and M2616(pH657) as well as the strain lacking a plasmid were streaked on minimal medium containing 10mM threonine, 20mM Na-formate and 20mM glycine +- 10mM serine. Strains containing the plasmids pH655 and pH657 but not pCLIK5a formed colonies on minimal medium containing formate and glycine, while even after prolonged incubation the strain M2616 did not produce colonies on this medium lacking serine. On the same minimal medium with added threonine, Na-formate and glycine but with added serine (20mM) all strains formed colonies including M2616.

This result showed the successful functional expression of the formate THF synthetase derived from Corynebacterium jeikeium NCTC and DSMZ 7171 in Corynebacterium glutamicum to provide a strain which utilizes formate in the synthesis of serine.

*Experiment 15 - Construction of a methionine overproducing strain which expresses formate-THF synthetase genes.*

Plasmids pH655 and pH657 were transformed into the strain GK1259 by electroporation. The resulting strains GK1259(pH655) and GK1259(pH657) as well as the strain containing no plasmid were incubated in shake flask assays as previoulsy described. In addition 20mM formate was added to the growth medium. It was observed that expression of the formate THF synthetase gene improved the methionine productivity of the strain over the strain lacking the formate THF synthetase gene. The data is found in table 16.

**Table 16: Shake flask assays of GK1259, and GK1259(pH655) overexpressing formate-THF synthetase**

| *Strain* | *Plasmid* | *Gene overexpressed* | *[Met] (mM*/*l)* |
|---|---|---|---|
| *GK1259* | *None* | *none* | *23,3* |
| *GK1259* | *pH655* | *Formate THF synthetase* | *24,6* |

### Experiment 16 - Deletion of formyl-THF-deformylase in strain M2543.

It was detected by sequence comparisons that *C. glutamicum* contains a gene, which has been annotated as a formyl-THF deformylase (Accession Ncgl0371). This gene has been annotated as coding for an enzyme with the enzymatic activity of formyl-THF deformylase, cleaving formate from the metabolite formyl-tetrahydrofolate (Annual review of plant physiology and plant molecular biology (2001) 52. 119-137).

Knockout of the formyl-THF deformylase (accession number Ncgl0371) was performed by cloning a DNA fragment in which the upstream region of the gene coding for N_{Cgl0371} (approximately 500Bp long) and its downstream region (approximately 500Bp long) of the same gene were amplified, fused by fusion-PCR and cloned into a vector resulting in pH670 int sacB delta deformylase (SEQ ID No. 50). The plasmid is transformed into the strain M2543 to yield first recombinants ("Campbell in step"). After successful screening of correct first recombinants by PCR the strain is grown overnight in liquid culture and is plated on growth medium that contains 10% sucrose. This treatment ("Campbell-out-step") leads to a strain called GK1546 in which the kanamycin resistance marker and the levan sucrose gene encoded on the plasmid pH670 are successfully crossed-out of the chromosome and subsequently lost from the chromosome and the cell. The successive strains from the Campbell out step are identified as deletions of the formyl-THF deformylase by PCR screening utilizing primers which code for sequences within the 5' and 3' regions of the described formyl-THF deformylase. Positive clones show a PCR Fragment, which is approximately 900bp shorter than the PCR product from a formyl-THF deformylase wildtype strain. The resulting strain was named GK1546.

### Experiment 17 - Construction of a strain deleted for formyl-THF-deformylase and overexpressing formate-THF-synthetase

The strain GK1546 is transformed with the plasmids pH655 and pH657. Resulting strains GK1546(pH655) and GK1546(pH657) show significantly improved growth behaviour when they are grown on a minimal medium containing formate, glycine and threonine but no serine over the strain M2543.

### Experiment 18 - Expression of functional formate-THF-synthetase for the production of methionine

Strain M2616 was transformed with pH655 (formate-THF-synthetase NCTC 11915) and pH657 (formate-THF-synthetase DSMZ 7171). The resulting strains M2616(pH655) and M2616(pH657) were analyzed in shake flask assays as described previously.

The medium was supplied with 20mM glycine as well as 20mM Na-formate in addition to the normal composition described above. Shake flasks were incubated at 30°C with a shaking rate of 200RPM. After 48h methionine was determined. It was found that the parental strain M2616 without the formate-THF synthetase expressing plasmid did not grow to measurable OD and it did not utilize the given carbon source. Supernatants were assayed for formate in the supernatant by HPLC. Strains containing the plasmids pH655 and pH657 show an utilisation of all formate added to the medium. In addition it is observed that strains grown on formate, glycine and that expressing the formate THF-synthatse gene produced measurable amounts of methionine while the strain M2616 did not produce methionine at all. The results are shown in table 17.

**Table 17: methionine production in M2616 and plasmid transformants of M2616**

| *Strain* | *plasmid* | *Gene overexpressed* | *[Met](mM*/*l)* |
|---|---|---|---|
| *M2616* | *None* | *none* | *0* |
| *M2616* | *pH655* | *formate-THF-synthetase NCTC* | *4,1* |
| *M2616* | *pH657* | *formate THF-synthetase DSMZ* | *5,5* |

In another experiment 20mM serine was added to the culture medium described in Experiment 18. M2616 and M2616 expressing the formate-THF-synthetase DSMZ 7171 gene were grown in the presence of serine and assayed for methionine produced. The resulting methionine titers in the case of overexpression of the were found to be higher in the case of the strains M2616, that expressed the formate-THF-synthetase DSMZ gene, compared to the strain which does not contain a formate-THF-synthetase gene. The data is found in table 18.

**Table 18 overproduction of methionine in strains overexpressing formate-THF synthetase**

| *Strain* | *Serine added* | *Gene overexpressed* | *[Met] (mM*/*l)* |
|---|---|---|---|
| *M2616* | + | *none* | *6,6* |
| *M2616 pH657* | + | *formate-THF-synthetase DSMZ* | *8,9* |

## Claims

1. Method for production of L-methionine in a Corynebacterium, selected from the group consisting of the species Corynebacterium glutamicum, Corynebacterium acetoglutamicum, Corynebacterium acetoacidophilum, Corynebacterium thermoaminogenes, Corynebacterium melassecola and Corynebacterium effiziens, wherein the Corynebacterium is derived by genetic modification from a methionine producing starting organism being a wild type Corynebacterium or a Corynebacterium which already carries genetic alterations in comparison to the wild-type Corynebacterium, such that the enzymatic activity of a glycine cleavage system (GCS) is increased in said microorganism compared to the starting organism and wherein the amount and/or activity of lipoic acid synthase *(lipA),* lipoyl tansferase *(lipB)* or *lipA* and *lipB* is increased in said Corynebacterium compared to the starting organism..

2. Method according to claim 1,
wherein the Corynebacterium is derived by genetic modification from a starting organism such that the amount and/or activity of
PLP-dependent glycine decarboxylase (*gcvP*),
N⁵,N¹⁰-methylene-THF-synthesizing enzyme (*gcvT*) and
lipoamide-containing aminomethyl transferase (*gcvH*)
are increased in said microorganism compared to the starting organism.

3. Method according to claim 1 or 2,
wherein the coding sequences for *gcvP, gcvT, gcvH, lipA* and *lipB* are from *C. jeikeium.*

4. Method according to any of claims 1 to 3,
wherein the coding sequences for *gcvP*, *gcvT*, *gcvH*, *lipA* and *lipB* are from or *E. coli.*

5. Corynebacterium selected from the group consisting of the species Corynebacterium glutamicum, Corynebacterium acetoglutamicum, Corynebacterium acetoacidophilum, Corynebacterium thermoaminogenes, Corynebacterium melassecola and Corynebacterium effiziens, wherein said Corynebacterium is derived by genetic modification from a methionine producing starting organism being a wild type Corynebacterium or a Corynebacterium which already carries genetic alterations in comparison to the wild-type Corynebacterium, such that the enzymatic activity of a glycine cleavage system (GCS) is increased in said Corynebacterium compared to the starting organism and wherein the amount and/or activity of *lipA, lipB* or *lipA* and *lipB* is increased in said Corynebacterium compared to the starting organism.

6. Corynebacterium according to claim 5,
wherein the Corynebacterium is a strain of *C. glutamicum.*

7. Corynebacterium according to claims 5 or 6,
wherein the Corynebacterium is derived by genetic modification from a starting organism such that the amount and/or activity of *gcvP, gcvT* and *gcvH* are increased in said Corynebacterium compared to the starting organism.

8. Corynebacterium according to claim 7,
wherein the coding sequences for *gcvP*, *gcvT*, *gcvH*, *lipA* and *lipB* are from *C. jeikeium.*

9. Corynebacterium according to claim 7, wherein the coding sequences for *gcvP, gcvT, gcvH, lipA* and *lipB* are from *E. coli.*

## Patentansprüche

1. Verfahren für die Produktion von L-Methionin in einem Corynebacterium, ausgewählt aus der Gruppe bestehend aus den Arten Corynebacterium glutamicum, Corynebacterium acetoglutamicum, Corynebacterium acetoacidophilum, Corynebacterium thermoaminogenes, Corynebacterium melassecola und Corynebacterium effiziens, wobei das Corynebacterium durch solch eine genetische Modifikation von einem methioninproduzierenden Ausgangsorganismus abgeleitet ist, bei dem es sich um ein Wildtyp-Corynebacterium oder ein Corynebacterium, das bereits genetische Veränderungen im Vergleich zu dem Wildtyp-Corynebacterium trägt, handelt, dass die enzymatische Aktivität eines Glycinspaltungssystems (GCS) in diesem Mikroorganismus im Vergleich zu dem Ausgangsorganismus erhöht ist und wobei die Menge und/oder Aktivität der Liponsäuresynthase (*lipA*)*,* Lipoyltransferase (*lipB*) oder *lipA* und *lipB* in diesem Corynebacterium im Vergleich zu dem Ausgangsorganismus erhöht ist.

2. Verfahren nach Anspruch 1,
wobei das Corynebacterium durch solch eine genetische Modifikation von einem Ausgangsorganismus abgeleitet ist, dass die Menge und/oder Aktivität der PLP-abhängigen Glycindecarboxylase (*gcvP*),
des N⁵, N¹⁰-methylen-THF-synthetisierenden Enzyms (*gcvT*) und
der lipoamidhaltigen Aminomethyltransferase (*gcvH*) in diesem Mikroorganismus im Vergleich zu dem Ausgangsorganismus erhöht sind.

3. Verfahren nach Anspruch 1 oder 2, wobei die Codiersequenzen für *gcvP, gcvT, gcvH, lipA* und *lipB* aus *C. jeikeium* stammen.

4. Verfahren nach einem der Ansprüche 1 bis 3, wobei die Codiersequenzen für *gcvP, gcvT, gcvH, lipA* und *lipB* von *E. coli* stammen.

5. Corynebacterium, ausgewählt aus der Gruppe bestehend aus den Arten Corynebacterium glutamicum, Corynebacterium acetoglutamicum, Corynebacterium acetoacidophilum, Corynebacterium thermoaminogenes, Corynebacterium melassecola und Corynebacterium effiziens, wobei das Corynebacterium durch solch eine genetische Modifikation von einem methioninproduzierenden Ausgangsorganismus abgeleitet ist, bei dem es sich um ein Wildtyp-Corynebacterium oder ein Corynebacterium, das bereits genetische Veränderungen im Vergleich zu dem Wildtyp-Corynebacterium trägt, handelt, dass die enzymatische Aktivität eines Glycinspaltungssystems (GCS) in diesem Corynebacterium im Vergleich zu dem Ausgangsorganismus erhöht ist und wobei die Menge und/oder Aktivität von *lipA, lipB* oder *lipA* und *lipB* in diesem Corynebacterium im Vergleich zu dem Ausgangsorganismus erhöht ist.

6. Corynebacterium nach Anspruch 5,
wobei das Corynebacterium ein Stamm von *C. glutamicum* ist.

7. Corynebacterium nach den Ansprüchen 5 oder 6,
wobei das Corynebacterium durch solch eine genetische Modifikation von einem Ausgangsorganismus abgeleitet ist, dass die Menge und/oder Aktivität von *gcvP, gcvT* und *gcvH* in dem Corynebacterium im Vergleich zu dem Ausgangsorganismus erhöht sind.

8. Corynebacterium nach Anspruch 7,
wobei die Codiersequenzen für *gcvP, gcvT, gcvH, lipA* und *lipB* aus *C. jeikeium* stammen.

9. Corynebacterium nach Anspruch 7, wobei die Codiersequenzen für *gcvP, gcvT, gcvH, lipA* und *lipB* aus *E. coli* stammen.

## Revendications

1. Procédé de production de L-méthionine dans un Corynebacterium, choisi dans le groupe constitué des espèces Corynebacterium glutamicum, Corynebacterium acetoglutamicum, Corynebacterium acetoacidophilum, Corynebacterium thermoaminogenes, Corynebacterium melassecola et Corynebacterium effiziens, le Corynebacterium étant dérivé par modification génétique d'un organisme de départ producteur de méthionine étant un Corynebacterium de type sauvage ou un Corynebacterium qui comporte déjà des modifications génétiques par rapport au Corynebacterium de type sauvage de sorte que l'activité enzymatique d'un système de clivage de glycine (GCS) soit augmenté dans ledit micro-organisme par rapport à l'organisme de départ et dans lequel la quantité et/ou l'activité acide lipoïque synthase (*lipA*), lipoyle transférase (*lipB*) ou *lipA* et *lipB* est augmentée dans ledit Corynebacterium par rapport à l'organisme de départ.

2. Procédé selon la revendication 1,
dans lequel le Corynebacterium est dérivé par modification génétique à partir d'un organisme de départ de sorte que la quantité et/ou activité de glycine décarboxylase PLP-dépendante (*gcvP*),
enzyme de synthèse de N⁵, N¹⁰-méthylène-THF (*gcvT*) et aminométhyle contenant lipoamide transférase (*gcvH*) soient augmentées dans ledit micro-organisme par rapport à l'organisme de départ.

3. Procédé selon la revendication 1 ou 2,
dans lequel les séquences codantes pour *gcvP, gcvT, gcvH, lipA* et *lipB* sont de *C. jeikeium.*

4. Procédé selon l'une quelconque des revendications 1 à 3,
dans lequel les séquences codantes pour *gcvP, gcvT, gcvH, lipA* et *lipB* sont de *E. coli.*

5. Corynebacterium choisi dans le groupe constitué des espèces Corynebacterium glutamicum, Corynebacterium acetoglutamicum, Corynebacterium acetoacidophilum, Corynebacterium thermoaminogenes, Corynebacterium melassecola et Corynebacterium effiziens, ledit Corynebacterium étant dérivé par modification génétique d'un organisme de départ producteur de méthionine étant un Corynebacterium de type sauvage ou un Corynebacterium qui comporte déjà des modifications génétiques par rapport au Corynebacterium de type sauvage, de sorte que l'activité enzymatique d'un système de clivage de glycine (GCS) soit augmentée dans ledit Corynebacterium par rapport à l'organisme de départ et dans lequel la quantité et/ou l'activité de *lipA, lipB* ou *lipA* et *lipB* est augmentée dans ledit Corynebacterium par rapport à l'organisme de départ.

6. Corynebacterium selon la revendication 5,
dans lequel le Corynebacterium est une souche de *C. glutamicum.*

7. Corynebacterium selon les revendications 5 ou 6,
dans lequel le Corynebacterium est dérivé par modification génétique d'un organisme de départ de sorte que la quantité et/ou l'activité de *gcvP, gcvT* et *gcvH* soient augmentées dans ledit Corynebacterium par rapport à l'organisme de départ.

8. Corynebacterium selon la revendication 7,
dans lequel les séquences codant pour *gcvP, gcvT, gcvH, lipA* et *lipB* sont de *C. jeikeium.*

9. Corynebacterium selon la revendication 7, dans lequel les séquences codantes pour *gcvP, gcvT, gcvH, lipA* et *lipB* sont de *E. coli.*


### SEQUENCE LISTING

<110> Evonik Degussa GmbH
<120> Coryneform bacteria with glycine cleavage activity
<130> 2007P00927WE01
<160> 52
<170> PatentIn version 3.3
<210> 1
   <211> 1689
   <212> DNA
   <213> Corynebacterium jeikeium
<400> 1
   atgactaact cttctgcaac cagcaatccc caacccagcg acgtcgaaat cgcacaggcc 60
   cacaccctgg agcccatcac cacgatcgcg gagcgcgccg gcatcccgga ggccgctttg 120
   attccctacg gccgtacaaa ggccaagatc gacgttcccg ctctgcgggc ggagcgtgag 180
   ggcgtcaata aaaagggcaa gctcgtgctc gtgaccgcaa tgagcccgac ccccgcgggt 240
   gaaggcaagt ccacggtgct gatcggcttg gcggacgcgg tgcgcacggc agggcgccag 300
   acgatggtgg cgatccgcga gccgtcccag gggccagtca tgggcatcaa gggcggcgct 360
   gctggcggcg gttatgcgca gatcgtgccg atggaagaca tcaacctgca cttcacaggc 420
   gacatgcacg ccatcaccgc cgcgacgaac actctggcgg cgatggtgga caaccacgtg 480
   cagcacggca atgctctggg catcgacccg cggcgggtga cgtggcgacg ctgccttgac 540
   gtcaacgacc gctcgctgcg ccatgtagtc acaggcctag gcggtcctgg tcagggcacg 600
   ccccgcgagg gcggattcga tatcacggcg gccagcgaga tcatggctat cctctgtctg 660
   gccaccgacc tggaggatct aaagaagcgc atcgggcgca tcgtggtggg ccagacctac 720
   gaccgtcgac cagtcaccgc gggcgacctg aagtgcgcgg gcgctatcac cgccctgctg 780
   cgcgacgcga tcaacccgaa cctggtgcag accctgggcg gcactcccgc cctggtgcac 840
   ggcgggcctt tcgccaacat cgcgcacggc tgtaactctc tgatcgcaac caccacggcg 900
   ctggatctat cagaagttgt gctgaccgag gctggtttcg gcagcgacct gggtgcggag 960
   aagttcttcg acatcaagtc ccgcgctggc gacctcgacg tcgccgcaac ggttgtggtg 1020
   gccaccatcc gttcgctgaa gcacaacggc gattccgtgt tgaaggccgg tctggccaac 1080
   ctggagcgcc acgtcagtaa catccgcaag ttcggcgtgg aacccgtggt ggcgctgaac 1140
   ctgtttagct ccgataccgc cgcggagcgc tctatggtcg ccgattgggg cgagcagttc 1200
   ggtgtgcgcg tggtggagtg cagcgtgtgg gctgagggcg gtgccggcgc ggcggatctg 1260
   gccactgcgg tgctggaggt cgttgatggt gtttctgatg aggacgcctc cagctccagc 1320
   caccagatct atcagcccgt cgatggcgtg gaagccaccc tgcacacact tgccacggaa 1380
   atctacggcg cggcggatgt gcagttcggc ccgcaggctc tgaaggacct ggcgtttctg 1440
   aaggacaacg gctgggacaa gctgccagtg tgcgtgtcga agacacagta ctccttcagc 1500
   gatgacccca gcgcgctggg cgcgccgagc ggccacaccc tgcatgtccg cgagctggtg 1560
   ccacgcatcg gcgccggatt cgtggtggct ctcaccggcg acgtgatgac tctgccgggt 1620
   ctgccgaaga aacccgcagc cgagcgtatt gatgtgaacg cgcagggggt tatctcgggg 1680
   ctgttctaa 1689
<210> 2
   <211> 562
   <212> PRT
   <213> Corynebacterium jeikeium
<400> 2
   Met Thr Asn Ser Ser Ala Thr Ser Asn Pro Gln Pro Ser Asp Val Glu
   1               5                   10                  15
   Ile Ala Gln Ala His Thr Leu Glu Pro Ile Thr Thr Ile Ala Glu Arg
               20                  25                  30
   Ala Gly Ile Pro Glu Ala Ala Leu Ile Pro Tyr Gly Arg Thr Lys Ala
           35                  40                  45
   Lys Ile Asp Val Pro Ala Leu Arg Ala Glu Arg Glu Gly Val Asn Lys
       50                  55                  60
   Lys Gly Lys Leu Val Leu Val Thr Ala Met Ser Pro Thr Pro Ala Gly
   65                  70                  75                  80
   Glu Gly Lys Ser Thr Val Leu Ile Gly Leu Ala Asp Ala Val Arg Thr
                   85                  90                  95
   Ala Gly Arg Gln Thr Met Val Ala Ile Arg Glu Pro Ser Gln Gly Pro
               100                 105                 110
   Val Met Gly Ile Lys Gly Gly Ala Ala Gly Gly Gly Tyr Ala Gln Ile
           115                 120                 125
   Val Pro Met Glu Asp Ile Asn Leu His Phe Thr Gly Asp Met His Ala
       130                 135                 140
   Ile Thr Ala Ala Thr Asn Thr Leu Ala Ala Met Val Asp Asn His Val
   145                 150                 155                 160
   Gln His Gly Asn Ala Leu Gly Ile Asp Pro Arg Arg Val Thr Trp Arg
                   165                 170                 175
   Arg Cys Leu Asp Val Asn Asp Arg Ser Leu Arg His Val Val Thr Gly
               180                 185                 190
   Leu Gly Gly Pro Gly Gln Gly Thr Pro Arg Glu Gly Gly Phe Asp Ile
           195                 200                 205
   Thr Ala Ala Ser Glu Ile Met Ala Ile Leu Cys Leu Ala Thr Asp Leu
       210                 215                 220
   Glu Asp Leu Lys Lys Arg Ile Gly Arg Ile Val Val Gly Gln Thr Tyr
   225                 230                 235                 240
   Asp Arg Arg Pro Val Thr Ala Gly Asp Leu Lys Cys Ala Gly Ala Ile
                   245                 250                 255
   Thr Ala Leu Leu Arg Asp Ala Ile Asn Pro Asn Leu Val Gln Thr Leu
               260                 265                 270
   Gly Gly Thr Pro Ala Leu Val His Gly Gly Pro Phe Ala Asn Ile Ala
           275                 280                 285
   His Gly Cys Asn Ser Leu Ile Ala Thr Thr Thr Ala Leu Asp Leu Ser
       290                 295                 300
   Glu Val Val Leu Thr Glu Ala Gly Phe Gly Ser Asp Leu Gly Ala Glu
   305                 310                 315                 320
   Lys Phe Phe Asp Ile Lys Ser Arg Ala Gly Asp Leu Asp Val Ala Ala
                   325                 330                 335
   Thr Val Val Val Ala Thr Ile Arg Ser Leu Lys His Asn Gly Asp Ser
               340                 345                 350
   Val Leu Lys Ala Gly Leu Ala Asn Leu Glu Arg His Val Ser Asn Ile
           355                 360                 365
   Arg Lys Phe Gly Val Glu Pro Val Val Ala Leu Asn Leu Phe Ser Ser
       370                 375                 380
   Asp Thr Ala Ala Glu Arg Ser Met Val Ala Asp Trp Gly Glu Gln Phe
   385                 390                 395                 400
   Gly Val Arg Val Val Glu Cys Ser Val Trp Ala Glu Gly Gly Ala Gly
                   405                 410                 415
   Ala Ala Asp Leu Ala Thr Ala Val Leu Glu Val Val Asp Gly Val Ser
               420                 425                 430
   Asp Glu Asp Ala Ser Ser Ser Ser His Gln Ile Tyr Gln Pro Val Asp
           435                 440                 445
   Gly Val Glu Ala Thr Leu His Thr Leu Ala Thr Glu Ile Tyr Gly Ala
   450 455 460
   Ala Asp Val Gln Phe Gly Pro Gln Ala Leu Lys Asp Leu Ala Phe Leu
   465 470 475 480
   Lys Asp Asn Gly Trp Asp Lys Leu Pro Val Cys Val Ser Lys Thr Gln
   485 490 495
   Tyr Ser Phe Ser Asp Asp Pro Ser Ala Leu Gly Ala Pro Ser Gly His
   500 505 510
   Thr Leu His Val Arg Glu Leu Val Pro Arg Ile Gly Ala Gly Phe Val
   515 520 525
   Val Ala Leu Thr Gly Asp Val Met Thr Leu Pro Gly Leu Pro Lys Lys
   530 535 540
   Pro Ala Ala Glu Arg Ile Asp Val Asn Ala Gln Gly Val Ile Ser Gly
   545 550 555 560
   Leu Phe
<210> 3
   <211> 192
   <212> DNA
   <213> artificial
<220>
   <223> promotor P3119 = PSOD
<400> 3
   gagctgccaa ttattccggg cttgtgaccc gctacccgat aaataggtcg gctgaaaaat 60
   ttcgttgcaa tatcaacaaa aaggcctatc attgggaggt gtcgcaccaa gtacttttgc 120
   gaagcgccat ctgacggatt ttcaaaagat gtatatgctc ggtgcggaaa cctacgaaag 180
   gattttttac cc 192
<210> 4
   <211> 184
   <212> DNA
   <213> artificial
<220>
   <223> promotor P497 = PgroES
<400> 4
   ggtcgagcgg cttaaagttt ggctgccatg tgaattttta gcaccctcaa cagttgagtg      60
   ctggcactct cgggggtaga gtgccaaata ggttgtttga cacacagttg ttcacccgcg     120
   acgacggctg tgctggaaac ccacaaccgg cacacacaaa atttttctca tggagggatt     180
   catc                                                                  184
<210> 5
   <211> 199
   <212> DNA
   <213> artificial
<220>
   <223> promotor P1284 = PEFTU
<400> 5
   ggccgttacc ctgcgaatgt ccacagggta gctggtagtt tgaaaatcaa cgccgttgcc      60
   cttaggattc agtaactggc acattttgta atgcgctaga tctgtgtgct cagtcttcca     120
   ggctgcttat cacagtgaaa gcaaaaccaa ttcgtggctg cgaaagtcgt agccaccacg     180
   aagtccagga ggacataca                                                  199
<210> 6
   <211> 114
   <212> DNA
   <213> artificial
<220>
   <223> promotor ... = IpR
<400> 6
   gtcgactcat acgttaaatc tatcaccgca agggataaat atctaacacc gtgcgtgttg      60
   actattttac ctctggcggt gataatggtt gcatgtacta aggaggatta atta           114
<210> 7
   <211> 915
   <212> DNA
   <213> Corynebacterium glutamicum
<400> 7
   atgaccccga gttctcctga agttcgtaat cgtccgagcg ctgcgcctga agagcgtcag      60
   tttgtgctca cttttggctg ccctgactcc actggaattg tggcgaagtt gtcgtcgttc     120
   ctagctgagc gtgggggttg gattactgag gctggatatt tcacggatcc tgattcgaat     180
   tggttcttta ctcgtcaggc gattcgcgct gagtcgattg ataccacgat tgagcagttg     240
   cgggaggagt tcgctccgct tgcggaggag ttcggcccga gggctaagtg gagtttcact     300
   gacactgcgc aggtgaagaa ggctgtgttg ttggtgtcta aggagggcca ctgcttgcac     360
   gatttgttag gtcgtgtggc tgagaatgat tatccgatgg aagttgttgc ggttgtgggt     420
   aaccatgaga acttgcgtta tattgcggag aaccataatg ttccgttttt ccatgtgccg     480
   tttcctaagg atgcggttgg taagcggaag gcgtttgacc aggtcgctga gattgtgaat     540
   ggttatgatc cggatgcgat tgttttggct cgttttatgc agattttgcc gccggatttg     600
   tgtgagatgt gggctggtcg tgtgttgaat attcatcaca gtttcttgcc gtcgtttatg     660
   ggtgcgcgcc cgtatcatca ggcgtatagc cgtggtgtga agttgattgg tgcgacctgc     720
   cattatgcga ctggggatct ggatgatggt ccgatcattg agcaggatgt tattcgtgtg     780
   acgcataagg atacgccgac tgagatgcag cgtttgggcc gcgatgcgga gaagcaggtg     840
   ctggctcgcg gtttgcgttt ccacttggag gaccgggtgc tggtttacgg taaccgcacg     900
   gttgtctttg attaa                                                      915
<210> 8
   <211> 304
   <212> PRT
   <213> Corynebacterium glutamicum
<400> 8
   Met Thr Pro Ser Ser Pro Glu Val Arg Asn Arg Pro Ser Ala Ala Pro
   1               5                   10                  15
   Glu Glu Arg Gln Phe Val Leu Thr Phe Gly Cys Pro Asp Ser Thr Gly
               20                  25                  30
   Ile Val Ala Lys Leu Ser Ser Phe Leu Ala Glu Arg Gly Gly Trp Ile
           35                  40                  45
   Thr Glu Ala Gly Tyr Phe Thr Asp Pro Asp Ser Asn Trp Phe Phe Thr
       50                  55                  60
   Arg Gln Ala Ile Arg Ala Glu Ser Ile Asp Thr Thr Ile Glu Gln Leu
   65                  70                  75                  80
   Arg Glu Glu Phe Ala Pro Leu Ala Glu Glu Phe Gly Pro Arg Ala Lys
                   85                  90                  95
   Trp Ser Phe Thr Asp Thr Ala Gln Val Lys Lys Ala Val Leu Leu Val
               100                 105                 110
   Ser Lys Glu Gly His Cys Leu His Asp Leu Leu Gly Arg Val Ala Glu
           115                 120                 125
   Asn Asp Tyr Pro Met Glu Val Val Ala Val Val Gly Asn His Glu Asn
       130                 135                 140
   Leu Arg Tyr Ile Ala Glu Asn His Asn Val Pro Phe Phe His Val Pro
   145                 150                 155                 160
   Phe Pro Lys Asp Ala Val Gly Lys Arg Lys Ala Phe Asp Gln Val Ala
                   165                 170                 175
   Glu Ile Val Asn Gly Tyr Asp Pro Asp Ala Ile Val Leu Ala Arg Phe
               180                 185                 190
   Met Gln Ile Leu Pro Pro Asp Leu Cys Glu Met Trp Ala Gly Arg Val
           195                 200                 205
   Leu Asn Ile His His Ser Phe Leu Pro Ser Phe Met Gly Ala Arg Pro
       210                 215                 220
   Tyr His Gln Ala Tyr Ser Arg Gly Val Lys Leu Ile Gly Ala Thr Cys
   225                 230                 235                 240
   His Tyr Ala Thr Gly Asp Leu Asp Asp Gly Pro Ile Ile Glu Gln Asp
                   245                 250                 255
   Val Ile Arg Val Thr His Lys Asp Thr Pro Thr Glu Met Gln Arg Leu
               260                 265                 270
   Gly Arg Asp Ala Glu Lys Gln Val Leu Ala Arg Gly Leu Arg Phe His
           275                 280                 285
   Leu Glu Asp Arg Val Leu Val Tyr Gly Asn Arg Thr Val Val Phe Asp
       290                 295                 300
<210> 9
   <211> 2952
   <212> DNA
   <213> Corynebacterium jeikeium
<400> 9
   atgtcttctg cagctactcg ccgtaattca gcccccttcg ttcagcgcca catcggccca 60
   aaccaggccg atacccagga gatcctcgat tacctgggct atgaatcttc cgccgcgctg 120
   gccgacgatg ccctcccgaa gtcgatccgc caggcaggcc cgatcggcct gccggaggca 180
   ctggatgaga cggacaccct ggccgccctg cgtgcttacg ctgacaagaa cgtgcagaag 240
   cagcagctga tcggcaacgg ttacttcgac acgatcactc cggccgtgat tcgccgcaac 300
   gtggtggaga acccgggctg gtacaccgcc tacaccccct accagccgga aatctcccag 360
   gggcgcctcg aggccctgct gaacttccag acgatggtgc aggacctgac cggcctgcca 420
   gtggccggtg cttcgctgtt agacgaagcc accgcagtgg ccgaggccgt gcagctgatg 480
   gctcgcggca atgcgaaggc tgccaagaag ggcggcgtgg tgctgctgga ttcctccctg 540
   caccagcagt ccatcaccgt aaccctggcg cgcgctgagg ctgcgggtat cccggtggag 600
   gtcgtggacc tggacggcga agatgctacc gccgcgtttg agggccgcga gaacctcgtc 660
   ggcgtggtgc tttccaaccc cggctccacc ggccgcgtcc gcgacctgtc cggtctgatc 720
   tctgcggcga aggagaccgg cgctctggtg acggtcgctt gtgacctgct ggctcaggtt 780
   ctggtgacct ccccgggctc ccaaggtgcc gacattgctg tcggctccgc ccagcgcttc 840
   ggcgtgccgc tattcttcgg tggcccgcac gcgggcttca tctcctgcac cgaggctctg 900
   cagcgtaagc tgccgggccg catcgtgggc gtgtccgtgg atgccgaggg caccccggcc 960
   taccgcttgg ctctgcagac ccgtgagcag cacattcgcc gcgacaaggc caccagtaac 1020
   atctgtaccg ctcaggctct gctggccgtc gttgccggtt tctacgcggt ctggcacggc 1080
   ccagccggcc tgcgcgccat cgccgagggg gtgcacgccc gcgcgaccgc cctggccgtt 1140
   gccctgtccg aggctggcct gacgctggcg cacgatacct tcttcgacac cgtcaccgtt 1200
   gacgtgtctg gttcttctct tggtgacgcc cccacggctc tgcgcgccgc agccgaggca 1260
   ggctacaacc tgcgccaggt taacgattcc ttcgtcggca tctctgtcgg cgagtccacc 1320
   acggacgagg acattgccaa gctgatcgag gtgctgggct cccgcaccgg cgaggtcaac 1380
   tccgcgagct tcgacgtcac cgccggcccg ctgggcgagg ccggcgtgct gcgcgcggag 1440
   gacgaggaga ttctgaccca cccgatcttc accgccatca cttccgagac ccagatgatg 1500
   cgctacatgc gcaagctggc cgaccgcgac ctggcgctgg atcgtacgat gatcccgctg 1560
   ggctcctgca ccatgaagct gaacgcggcc gtctccatgg agccgatcac ctggcctggc 1620
   ttcgcaggca tccacccgca cgtcccggcc gagcaggcgc agggctggct ggagctcatc 1680
   gaggacctgg aggagcgcct ggcgaagatc accggctacg ccaaggtttc cgtccagccg 1740
   aacgcgggct cccagggcga gttcgccggc ctgctggcga tccaccgcta ccaccagtcc 1800
   cgcggcgacg atcagcgtga catcgttctg atcccggcct ccgcgcacgg caccaacgct 1860
   gcctctgcag cgctggcggg cctgaaggtc gtggctgtga agaatgccga agacggctcc 1920
   atcgacgtgc cggacctgga ggccaagctg gagaagtacg gcgagcagac cgccgccatc 1980
   atgctgacct acccctccac ccacggcgtg ttcgaggagc aggtgcgcga cgtctgccag 2040
   aaggttcacg acgctggcgg ccaggtgtac gtcgacggcg ctaacctgaa cgccctggtc 2100
   ggcctggccc agccgggcga gttcggcggc gacgtatccc acctgaacct gcacaagacc 2160
   ttcaccatcc cgcacggtgg tggcggcccg ggcgttggcc cggtgtgcgt ggcagagcac 2220
   ctgatcccgt tcctgcccac cgacccgaac gccgacgtta tcgagggcga tgctgccctg 2280
   cagtccggcc agccggtctc cggcgcgcag tacggctccg ctggcgtgct gccgatcacc 2340
   tggtcctaca tcgcacagat gggcgacgaa ggcctgactg aggcctcccg catggccctg 2400
   gtgaacgcca actacgtttc ccgcaagctg gaggactact acccgacgct gtacaagggc 2460
   gacaccggcc tggtcgccca cgagtgcatc ctggacctgc gtgaactcac gaaggcctcc 2520
   ggcatcaccg ccgaggacgt atccaagcgc ctgatggact tcggcttcca cgccccgacc 2580
   ttggccttcc cggtcgccgg caccctgatg atggagccca ccgagtcgga ggacaaggaa 2640
   gagctggacc gcttcatcga agcgatgatc accatccacg gggagatcca ggaagttatc 2700
   gacggcaagg tcaccgccga acagtccgtt ctgcgccacg cgccgtttac cgcctattct 2760
   gttgtgcggg acgacttcga ggaagctgta tccggtggtc acttcagccg tgccaaggcc 2820
   gcatacccgg tggctagcct gcgacacacc aagtacttca cccccgtgcg tcgtatcgac 2880
   aacgcctatg gcgaccgcaa tctcgtgtgc acctgcccgc ccctggaaga cttcgcaatt 2940
   aacgaggact ag 2952
<210> 10
   <211> 983
   <212> PRT
   <213> Corynebacterium jeikeium
<400> 10
   Met Ser Ser Ala Ala Thr Arg Arg Asn Ser Ala Pro Phe Val Gln Arg
   1 5 10 15
   His Ile Gly Pro Asn Gln Ala Asp Thr Gln Glu Ile Leu Asp Tyr Leu
   20 25 30
   Gly Tyr Glu Ser Ser Ala Ala Leu Ala Asp Asp Ala Leu Pro Lys Ser
   35 40 45
   Ile Arg Gln Ala Gly Pro Ile Gly Leu Pro Glu Ala Leu Asp Glu Thr
   50 55 60
   Asp Thr Leu Ala Ala Leu Arg Ala Tyr Ala Asp Lys Asn Val Gln Lys
   65 70 75 80
   Gln Gln Leu Ile Gly Asn Gly Tyr Phe Asp Thr Ile Thr Pro Ala Val
   85 90 95
   Ile Arg Arg Asn Val Val Glu Asn Pro Gly Trp Tyr Thr Ala Tyr Thr
   100 105 110
   Pro Tyr Gln Pro Glu Ile Ser Gln Gly Arg Leu Glu Ala Leu Leu Asn
   115 120 125
   Phe Gln Thr Met Val Gln Asp Leu Thr Gly Leu Pro Val Ala Gly Ala
   130 135 140
   Ser Leu Leu Asp Glu Ala Thr Ala Val Ala Glu Ala Val Gln Leu Met
   145 150 155 160
   Ala Arg Gly Asn Ala Lys Ala Ala Lys Lys Gly Gly Val Val Leu Leu
   165 170 175
   Asp Ser Ser Leu His Gln Gln Ser Ile Thr Val Thr Leu Ala Arg Ala
   180 185 190
   Glu Ala Ala Gly Ile Pro Val Glu Val Val Asp Leu Asp Gly Glu Asp
   195 200 205
   Ala Thr Ala Ala Phe Glu Gly Arg Glu Asn Leu Val Gly Val Val Leu
   210 215 220
   Ser Asn Pro Gly Ser Thr Gly Arg Val Arg Asp Leu Ser Gly Leu Ile
   225 230 235 240
   Ser Ala Ala Lys Glu Thr Gly Ala Leu Val Thr Val Ala Cys Asp Leu
   245 250 255
   Leu Ala Gln Val Leu Val Thr Ser Pro Gly Ser Gln Gly Ala Asp Ile
   260 265 270
   Ala Val Gly Ser Ala Gln Arg Phe Gly Val Pro Leu Phe Phe Gly Gly
   275 280 285
   Pro His Ala Gly Phe Ile Ser Cys Thr Glu Ala Leu Gln Arg Lys Leu
   290 295 300
   Pro Gly Arg Ile Val Gly Val Ser Val Asp Ala Glu Gly Thr Pro Ala
   305 310 315 320
   Tyr Arg Leu Ala Leu Gln Thr Arg Glu Gln His Ile Arg Arg Asp Lys
   325 330 335
   Ala Thr Ser Asn Ile Cys Thr Ala Gln Ala Leu Leu Ala Val Val Ala
   340 345 350
   Gly Phe Tyr Ala Val Trp His Gly Pro Ala Gly Leu Arg Ala Ile Ala
   355 360 365
   Glu Gly Val His Ala Arg Ala Thr Ala Leu Ala Val Ala Leu Ser Glu
   370 375 380
   Ala Gly Leu Thr Leu Ala His Asp Thr Phe Phe Asp Thr Val Thr Val
   385 390 395 400
   Asp Val Ser Gly Ser Ser Leu Gly Asp Ala Pro Thr Ala Leu Arg Ala
   405 410 415
   Ala Ala Glu Ala Gly Tyr Asn Leu Arg Gln Val Asn Asp Ser Phe Val
   420 425 430
   Gly Ile Ser Val Gly Glu Ser Thr Thr Asp Glu Asp Ile Ala Lys Leu
   435 440 445
   Ile Glu Val Leu Gly Ser Arg Thr Gly Glu Val Asn Ser Ala Ser Phe
   450 455 460
   Asp Val Thr Ala Gly Pro Leu Gly Glu Ala Gly Val Leu Arg Ala Glu
   465 470 475 480
   Asp Glu Glu Ile Leu Thr His Pro Ile Phe Thr Ala Ile Thr Ser Glu
   485 490 495
   Thr Gln Met Met Arg Tyr Met Arg Lys Leu Ala Asp Arg Asp Leu Ala
   500 505 510
   Leu Asp Arg Thr Met Ile Pro Leu Gly Ser Cys Thr Met Lys Leu Asn
   515 520 525
   Ala Ala Val Ser Met Glu Pro Ile Thr Trp Pro Gly Phe Ala Gly Ile
   530 535 540
   His Pro His Val Pro Ala Glu Gln Ala Gln Gly Trp Leu Glu Leu Ile
   545 550 555 560
   Glu Asp Leu Glu Glu Arg Leu Ala Lys Ile Thr Gly Tyr Ala Lys Val
   565 570 575
   Ser Val Gln Pro Asn Ala Gly Ser Gln Gly Glu Phe Ala Gly Leu Leu
   580 585 590
   Ala Ile His Arg Tyr His Gln Ser Arg Gly Asp Asp Gln Arg Asp Ile
   595 600 605
   Val Leu Ile Pro Ala Ser Ala His Gly Thr Asn Ala Ala Ser Ala Ala
   610 615 620
   Leu Ala Gly Leu Lys Val Val Ala Val Lys Asn Ala Glu Asp Gly Ser
   625 630 635 640
   Ile Asp Val Pro Asp Leu Glu Ala Lys Leu Glu Lys Tyr Gly Glu Gln
   645 650 655
   Thr Ala Ala Ile Met Leu Thr Tyr Pro Ser Thr His Gly Val Phe Glu
   660 665 670
   Glu Gln Val Arg Asp Val Cys Gln Lys Val His Asp Ala Gly Gly Gln
   675 680 685
   Val Tyr Val Asp Gly Ala Asn Leu Asn Ala Leu Val Gly Leu Ala Gln
   690 695 700
   Pro Gly Glu Phe Gly Gly Asp Val Ser His Leu Asn Leu His Lys Thr
   705 710 715 720
   Phe Thr Ile Pro His Gly Gly Gly Gly Pro Gly Val Gly Pro Val Cys
   725 730 735
   Val Ala Glu His Leu Ile Pro Phe Leu Pro Thr Asp Pro Asn Ala Asp
   740 745 750
   Val Ile Glu Gly Asp Ala Ala Leu Gln Ser Gly Gln Pro Val Ser Gly
   755 760 765
   Ala Gln Tyr Gly Ser Ala Gly Val Leu Pro Ile Thr Trp Ser Tyr Ile
   770 775 780
   Ala Gln Met Gly Asp Glu Gly Leu Thr Glu Ala Ser Arg Met Ala Leu
   785 790 795 800
   Val Asn Ala Asn Tyr Val Ser Arg Lys Leu Glu Asp Tyr Tyr Pro Thr
   805 810 815
   Leu Tyr Lys Gly Asp Thr Gly Leu Val Ala His Glu Cys Ile Leu Asp
   820 825 830
   Leu Arg Glu Leu Thr Lys Ala Ser Gly Ile Thr Ala Glu Asp Val Ser
   835 840 845
   Lys Arg Leu Met Asp Phe Gly Phe His Ala Pro Thr Leu Ala Phe Pro
   850 855 860
   Val Ala Gly Thr Leu Met Met Glu Pro Thr Glu Ser Glu Asp Lys Glu
   865 870 875 880
   Glu Leu Asp Arg Phe Ile Glu Ala Met Ile Thr Ile His Gly Glu Ile
   885 890 895
   Gln Glu Val Ile Asp Gly Lys Val Thr Ala Glu Gln Ser Val Leu Arg
   900 905 910
   His Ala Pro Phe Thr Ala Tyr Ser Val Val Arg Asp Asp Phe Glu Glu
   915 920 925
   Ala Val Ser Gly Gly His Phe Ser Arg Ala Lys Ala Ala Tyr Pro Val
   930 935 940
   Ala Ser Leu Arg His Thr Lys Tyr Phe Thr Pro Val Arg Arg Ile Asp
   945 950 955 960
   Asn Ala Tyr Gly Asp Arg Asn Leu Val Cys Thr Cys Pro Pro Leu Glu
   965 970 975
   Asp Phe Ala Ile Asn Glu Asp
   980
<210> 11
   <211> 384
   <212> DNA
   <213> Corynebacterium jeikeium
<400> 11
   atgactgcac tgccaactga cttcctgtac tccgaagagc acgagtgggt taacacctcc     60
   gctgttgttg agggcgagac cgtgcgcgtg ggcattaccc acatcgccgc tgaggcgctg    120
   ggtgacatcg tgttcgtcga gctgccggag gttggctccg aggttgaggc cggcgaggct    180
   ttcggcgagg ttgagtccac caagtccgtt tccgacatct acgcaccggt ttctggcgag    240
   gttgtggctg tcaacgaggc gctggaagac aacgctggcc tgatcaacga agatccatac    300
   ggtgagggct ggctgtacga ggtcaaggtg accgaggccg gcgagctgat ggaggctgag    360
   gcttaccagg cggctaacga gtaa                                           384
<210> 12
   <211> 127
   <212> PRT
   <213> Corynebacterium jeikeium
<400> 12
   Met Thr Ala Leu Pro Thr Asp Phe Leu Tyr Ser Glu Glu His Glu Trp
   1               5                   10                  15
   Val Asn Thr Ser Ala Val Val Glu Gly Glu Thr Val Arg Val Gly Ile
               20                  25                  30
   Thr His Ile Ala Ala Glu Ala Leu Gly Asp Ile Val Phe Val Glu Leu
           35                  40                  45
   Pro Glu Val Gly Ser Glu Val Glu Ala Gly Glu Ala Phe Gly Glu Val
       50                  55                  60
   Glu Ser Thr Lys Ser Val Ser Asp Ile Tyr Ala Pro Val Ser Gly Glu
   65                  70                  75                  80
   Val Val Ala Val Asn Glu Ala Leu Glu Asp Asn Ala Gly Leu Ile Asn
                   85                  90                  95
   Glu Asp Pro Tyr Gly Glu Gly Trp Leu Tyr Glu Val Lys Val Thr Glu
               100                 105                 110
   Ala Gly Glu Leu Met Glu Ala Glu Ala Tyr Gln Ala Ala Asn Glu
           115                 120                 125
<210> 13
   <211> 1170
   <212> DNA
   <213> Corynebacterium jeikeium
<400> 13
   atgaccgaac ttaagaagac cgcgctgcac ctggtgcacg agaagttggg cgcgcgattt      60
   accgacttcg gcggctggga catgcctctg aagtacagca gtgagctgga cgagcaccac     120
   gctgtacgca atgccgtggg cgtattcgac ctctcccaca tgggtgaggt tcgcgtgacc     180
   ggcccgcagg cagcggagtt cctggaccac gcgctgattt cgaagctgtc ggcagtgaag     240
   gtcggcaagg cgaagtactc gatgatctgc accgaatccg gtggcatcat cgacgacctg     300
   atcacctacc gcctgggcga caacgagttc ctgatcgtgc cgaacgcggg caacgtggac     360
   aacgtggtct ccgcactgca gggccgcacc gagggctttg acgtggaggt taacaacgag     420
   tccgatgcga cctccatgat cgccgtacag gggcccaagg ccgcgcaggc gatgctggag     480
   atcgtggaga acgtcgtgga tgcacccgag gcatccggcg cgggcgagac cgttgccgag     540
   gctatcgagg ggctgggtta ctacgcggca ttcagcggtg ttgccgcagg tcagcccgtg     600
   ctggtggccc gcacaggcta taccggcgag gacggtttcg agctgatcgt ggctaacgat     660
   ggtgcggaga ccgtgtggac caaggctatg gaccaggctg cgcagctggg tggcctgccg     720
   tgtggcctgg cctgccgcga caccctgcgc ctggaggctg gcatgccgct gtacggcaac     780
   gagctatcgc tgaagctcac cccggtcgat gctgggctgg gcattcttgc ggcgacgaag     840
   tctaaggact ctttcgttgg tcgtgacgcc atcgtttccg ccaaggaaaa gggtacccag     900
   caggtactta tcgggctggc gggcgagggt cgccgcgctg cccgtggggg atacgaggtg     960
   tttgccggtg acggcgagaa ggccatcggt gccgtgacct ccggtgcact gtcgccgacg    1020
   ctgggccacc cggtggcatt ggcatacgtc gcgaagtccg cagtgtcctc cggcgcggcc    1080
   gctgagggtg cgaccgtgga ggtagacatc cgcggcaagc gctttgaata caaggttgtg    1140
   gcgctgccgt tctactcccg cgagaagtaa                                     1170
<210> 14
   <211> 389
   <212> PRT
   <213> Corynebacterium jeikeium
<400> 14
   Met Thr Glu Leu Lys Lys Thr Ala Leu His Leu Val His Glu Lys Leu
   1               5                   10                  15
   Gly Ala Arg Phe Thr Asp Phe Gly Gly Trp Asp Met Pro Leu Lys Tyr
               20                  25                  30
   Ser Ser Glu Leu Asp Glu His His Ala Val Arg Asn Ala Val Gly Val
           35                  40                  45
   Phe Asp Leu Ser His Met Gly Glu Val Arg Val Thr Gly Pro Gln Ala
       50                  55                  60
   Ala Glu Phe Leu Asp His Ala Leu Ile Ser Lys Leu Ser Ala Val Lys
   65 70 75 80
   Val Gly Lys Ala Lys Tyr Ser Met Ile Cys Thr Glu Ser Gly Gly Ile
   85 90 95
   Ile Asp Asp Leu Ile Thr Tyr Arg Leu Gly Asp Asn Glu Phe Leu Ile
   100 105 110
   Val Pro Asn Ala Gly Asn Val Asp Asn Val Val Ser Ala Leu Gln Gly
   115 120 125
   Arg Thr Glu Gly Phe Asp Val Glu Val Asn Asn Glu Ser Asp Ala Thr
   130 135 140
   Ser Met Ile Ala Val Gln Gly Pro Lys Ala Ala Gln Ala Met Leu Glu
   145 150 155 160
   Ile Val Glu Asn Val Val Asp Ala Pro Glu Ala Ser Gly Ala Gly Glu
   165 170 175
   Thr Val Ala Glu Ala Ile Glu Gly Leu Gly Tyr Tyr Ala Ala Phe Ser
   180 185 190
   Gly Val Ala Ala Gly Gln Pro Val Leu Val Ala Arg Thr Gly Tyr Thr
   195 200 205
   Gly Glu Asp Gly Phe Glu Leu Ile Val Ala Asn Asp Gly Ala Glu Thr
   210 215 220
   Val Trp Thr Lys Ala Met Asp Gln Ala Ala Gln Leu Gly Gly Leu Pro
   225 230 235 240
   Cys Gly Leu Ala Cys Arg Asp Thr Leu Arg Leu Glu Ala Gly Met Pro
   245 250 255
   Leu Tyr Gly Asn Glu Leu Ser Leu Lys Leu Thr Pro Val Asp Ala Gly
   260 265 270
   Leu Gly Ile Leu Ala Ala Thr Lys Ser Lys Asp Ser Phe Val Gly Arg
   275 280 285
   Asp Ala Ile Val Ser Ala Lys Glu Lys Gly Thr Gln Gln Val Leu Ile
   290 295 300
   Gly Leu Ala Gly Glu Gly Arg Arg Ala Ala Arg Gly Gly Tyr Glu Val
   305 310 315 320
   Phe Ala Gly Asp Gly Glu Lys Ala Ile Gly Ala Val Thr Ser Gly Ala
   325 330 335
   Leu Ser Pro Thr Leu Gly His Pro Val Ala Leu Ala Tyr Val Ala Lys
   340 345 350
   Ser Ala Val Ser Ser Gly Ala Ala Ala Glu Gly Ala Thr Val Glu Val
   355 360 365
   Asp Ile Arg Gly Lys Arg Phe Glu Tyr Lys Val Val Ala Leu Pro Phe
   370 375 380
   Tyr Ser Arg Glu Lys
   385
<210> 15
   <211> 1017
   <212> DNA
   <213> Escherichia coli
<400> 15
   atgtccacat tacgcctgct catctctgac tcttacgacc cgtggtttaa cctggcggtg      60
   gaagagtgta tttttcgcca aatgcccgcc acgcagcgcg ttctgtttct ctggcgcaat     120
   gccgacacgg tagtaattgg tcgcgcgcag aacccgtgga aagagtgtaa tacccggcgg     180
   atggaagaag ataacgtccg cctggcgcgg cgcagtagcg gtggcggcgc ggtgttccac     240
   gatctcggca atacctgctt tacctttatg gctggcaagc cggagtacga taaaactatc     300
   tccacgtcga ttgtgctcaa tgcgctgaac gcgctcggcg tcagcgccga agcgtccgga     360
   cgtaacgatc tggtggtgaa aaccgtcgaa ggcgaccgca aagtctcagg ctcggcctat     420
   cgcgaaacca aagatcgcgg cttccaccac ggcaccttgc tactcaatgc cgacctcagc     480
   cgcctggcaa actatctcaa tccggataaa aagaaactgg cggcgaaagg cattacgtcg     540
   gtacgttccc gcgtgaccaa cctcaccgag ctgttgccgg ggatcaccca tgagcaggtt     600
   tgcgaggcca taaccgaggc ctttttcgcc cattatggcg agcgcgtgga agcggaaatc     660
   atctccccga acaaaacgcc agacttgcca aacttcgccg aaacctttgc ccgccagagt     720
   agctgggaat ggaacttcgg tcaggctccg gcattctcgc atctgctgga tgaacgcttt     780
   acctggggcg gcgtggaact gcatttcgac gttgaaaaag gccatatcac ccgcgcccag     840
   gtgtttaccg acagcctcaa ccccgcgccg ctggaagccc tcgccggacg actgcaaggc     900
   tgcctgtacc gcgcagatat gctgcaacag gagtgcgaag cgctgttggt tgacttcccg     960
   gaacaggaaa aagagctacg ggagttatcg gcatggatgg cgggggctgt aaggtag       1017
<210> 16
   <211> 338
   <212> PRT
   <213> Escherichia coli
<400> 16
   Met Ser Thr Leu Arg Leu Leu Ile Ser Asp Ser Tyr Asp Pro Trp Phe
   1               5                   10                  15
   Asn Leu Ala Val Glu Glu Cys Ile Phe Arg Gln Met Pro Ala Thr Gln
               20                  25                  30
   Arg Val Leu Phe Leu Trp Arg Asn Ala Asp Thr Val Val Ile Gly Arg
           35                  40                  45
   Ala Gln Asn Pro Trp Lys Glu Cys Asn Thr Arg Arg Met Glu Glu Asp
       50                  55                  60
   Asn Val Arg Leu Ala Arg Arg Ser Ser Gly Gly Gly Ala Val Phe His
   65                  70                  75                  80
   Asp Leu Gly Asn Thr Cys Phe Thr Phe Met Ala Gly Lys Pro Glu Tyr
                   85                  90                  95
   Asp Lys Thr Ile Ser Thr Ser Ile Val Leu Asn Ala Leu Asn Ala Leu
               100                 105                 110
   Gly Val Ser Ala Glu Ala Ser Gly Arg Asn Asp Leu Val Val Lys Thr
           115                 120                 125
   Val Glu Gly Asp Arg Lys Val Ser Gly Ser Ala Tyr Arg Glu Thr Lys
       130                 135                 140
   Asp Arg Gly Phe His His Gly Thr Leu Leu Leu Asn Ala Asp Leu Ser
   145                 150                 155                 160
   Arg Leu Ala Asn Tyr Leu Asn Pro Asp Lys Lys Lys Leu Ala Ala Lys
                   165                 170                 175
   Gly Ile Thr Ser Val Arg Ser Arg Val Thr Asn Leu Thr Glu Leu Leu
               180                 185                 190
   Pro Gly Ile Thr His Glu Gln Val Cys Glu Ala Ile Thr Glu Ala Phe
           195                 200                 205
   Phe Ala His Tyr Gly Glu Arg Val Glu Ala Glu Ile Ile Ser Pro Asn
       210                 215                 220
   Lys Thr Pro Asp Leu Pro Asn Phe Ala Glu Thr Phe Ala Arg Gln Ser
   225                 230                 235                 240
   Ser Trp Glu Trp Asn Phe Gly Gln Ala Pro Ala Phe Ser His Leu Leu
                   245                 250                 255
   Asp Glu Arg Phe Thr Trp Gly Gly Val Glu Leu His Phe Asp Val Glu
   260 265 270
   Lys Gly His Ile Thr Arg Ala Gln Val Phe Thr Asp Ser Leu Asn Pro
   275 280 285
   Ala Pro Leu Glu Ala Leu Ala Gly Arg Leu Gln Gly Cys Leu Tyr Arg
   290 295 300
   Ala Asp Met Leu Gln Gln Glu Cys Glu Ala Leu Leu Val Asp Phe Pro
   305 310 315 320
   Glu Gln Glu Lys Glu Leu Arg Glu Leu Ser Ala Trp Met Ala Gly Ala
   325 330 335
   Val Arg
<210> 17
   <211> 1050
   <212> DNA
   <213> Corynebacterium jeikeium
<400> 17
   atgagtgtga ccgcagatgg acgccggatg ctacgcatcg aggcgaagaa tgccgaaacc 60
   cccatcgagt cgaagcctcg gtggatccgc acgaccgcga aggtcggccc ggagtatcgg 120
   gatattaaga atcgcgtgaa gggcgctggc ctgcacaccg tgtgccaaga ggctggctgc 180
   ccgaatatca acgagtgctg ggaggaccgc gaggcgacgt tcctcatcgg cggtgatacg 240
   tgttcccgcc gttgcgactt ctgccagatt aagtccggcc gcccgtcccc gctggatatg 300
   gacgagccac gccgcgtggc ggagaatgtc cgcgagatgg gtctgcgcta cgccaccatc 360
   accggcgtga cgcgcgatga cctggatgat gagggcgcgt ggctgtatgc cgaggttgtg 420
   aagaagattc acgagctgaa cccgaacacg ggtgtggaga atctgacgcc agatttttcc 480
   aaccgcccgg agctgctgaa ggtcgtcttc gattcccagc cggaggtgtt tgcccacaac 540
   ctggagacag ttccgcgcat ctttaagcgc atccgcccgg cctttaagta cgaccgttcc 600
   ctggaggtca ttcaggcagc tcacgattac ggcctggtga cgaagtccaa cctgatcctg 660
   ggcatgggtg agaagaagga agaggtccgc gcggctatca aggacctggc agacgccggc 720
   accgacattc tgacgattac ccagtacctg cgcccgtctt ccatgcacca cccgattgag 780
   cgttgggtga agccggagga gttcatggag cactccgacg cagcctacga gctgggcatc 840
   aaggctgtta tgtccggccc gttggtgcgt tcctcttacc gcgccggccg cctgtacgcg 900
   caggccaagc aggcgcgcgg cgaggctatt ccggagaacc tgaagcactt ggaggagact 960
   ctcgattcca ccacgtcgca ggaggcctct acactgctgg agcgctacgg cgcttcggag 1020
   gacacgccgg tcactgcgtc gcgccgctag 1050
<210> 18
   <211> 349
   <212> PRT
   <213> Corynebacterium jeikeium
<400> 18
   Met Ser Val Thr Ala Asp Gly Arg Arg Met Leu Arg Ile Glu Ala Lys
   1               5                   10                  15
   Asn Ala Glu Thr Pro Ile Glu Ser Lys Pro Arg Trp Ile Arg Thr Thr
               20                  25                  30
   Ala Lys Val Gly Pro Glu Tyr Arg Asp Ile Lys Asn Arg Val Lys Gly
           35                  40                  45
   Ala Gly Leu His Thr Val Cys Gln Glu Ala Gly Cys Pro Asn Ile Asn
       50                  55                  60
   Glu Cys Trp Glu Asp Arg Glu Ala Thr Phe Leu Ile Gly Gly Asp Thr
   65                  70                  75                  80
   Cys Ser Arg Arg Cys Asp Phe Cys Gln Ile Lys Ser Gly Arg Pro Ser
                   85                  90                  95
   Pro Leu Asp Met Asp Glu Pro Arg Arg Val Ala Glu Asn Val Arg Glu
               100                 105                 110
   Met Gly Leu Arg Tyr Ala Thr Ile Thr Gly Val Thr Arg Asp Asp Leu
           115                 120                 125
   Asp Asp Glu Gly Ala Trp Leu Tyr Ala Glu Val Val Lys Lys Ile His
       130                 135                 140
   Glu Leu Asn Pro Asn Thr Gly Val Glu Asn Leu Thr Pro Asp Phe Ser
   145                 150                 155                 160
   Asn Arg Pro Glu Leu Leu Lys Val Val Phe Asp Ser Gln Pro Glu Val
                   165                 170                 175
   Phe Ala His Asn Leu Glu Thr Val Pro Arg Ile Phe Lys Arg Ile Arg
               180                 185                 190
   Pro Ala Phe Lys Tyr Asp Arg Ser Leu Glu Val Ile Gln Ala Ala His
           195                 200                 205
   Asp Tyr Gly Leu Val Thr Lys Ser Asn Leu Ile Leu Gly Met Gly Glu
       210                 215                 220
   Lys Lys Glu Glu Val Arg Ala Ala Ile Lys Asp Leu Ala Asp Ala Gly
   225                 230                 235                 240
   Thr Asp Ile Leu Thr Ile Thr Gln Tyr Leu Arg Pro Ser Ser Met His
                   245                 250                 255
   His Pro Ile Glu Arg Trp Val Lys Pro Glu Glu Phe Met Glu His Ser
               260                 265                 270
   Asp Ala Ala Tyr Glu Leu Gly Ile Lys Ala Val Met Ser Gly Pro Leu
           275                 280                 285
   Val Arg Ser Ser Tyr Arg Ala Gly Arg Leu Tyr Ala Gln Ala Lys Gln
       290                 295                 300
   Ala Arg Gly Glu Ala Ile Pro Glu Asn Leu Lys His Leu Glu Glu Thr
   305                 310                 315                 320
   Leu Asp Ser Thr Thr Ser Gln Glu Ala Ser Thr Leu Leu Glu Arg Tyr
                   325                 330                 335
   Gly Ala Ser Glu Asp Thr Pro Val Thr Ala Ser Arg Arg
               340                 345
<210> 19
   <211> 759
   <212> DNA
   <213> Corynebacterium jeikeium
<400> 19
   atgggattcc agcaaggcag catccgcaaa gcaaacatgg gcacaaccgg cactaacgac      60
   ggggcaacca ccccaccagc caacaccagc accccagccg tagacatcga cgtccgcgac     120
   ctgggcaccg tggactacga ggacacttgg cacctccagg caaatctcgc cgcccagcgc     180
   gccgaagaaa aaatcccaga caccatcctc ctactccagc atccgccgac gtacaccgcc     240
   ggcaagcgca cccaggattc cgaccgccct accaacggcc tgccagtagt cgacgtcgac     300
   cgcggaggcc gcatcacctg gcatggaccc ggccagttgg tcgcatatcc catcatcaag     360
   ctggccgacc cggtggacgt ggtcgattat gtccgccgcc tggagcaggc gctaatccag     420
   acttgtgaag atcttggcct gcacggcacc ggccgcgtag aggggcgttc gggcgtgtgg     480
   ctgcctgcgg gcgtcattaa tggcgagctc aagcccgcac gtaagatagc cgcgatcggc     540
   atccgcgtga cgcgcggcgt gaccatgcac ggagtggccc tcaactgcga taacaccatg     600
   gagtattacg accacattgt gccttgtggg ctggcggatg cgggtgtcac gacgctcacc     660
   gaggagctgg ggcgcgatgt tagtgtttct gacgcctact catccctcgc ccacaacctc     720
   gttgatgctt tgaacggcga cttgccggtg cattcctag                            759
<210> 20
   <211> 252
   <212> PRT
   <213> Corynebacterium jeikeium
<400> 20
   Met Gly Phe Gln Gln Gly Ser Ile Arg Lys Ala Asn Met Gly Thr Thr
   1               5                   10                  15
   Gly Thr Asn Asp Gly Ala Thr Thr Pro Pro Ala Asn Thr Ser Thr Pro
               20                  25                  30
   Ala Val Asp Ile Asp Val Arg Asp Leu Gly Thr Val Asp Tyr Glu Asp
           35                  40                  45
   Thr Trp His Leu Gln Ala Asn Leu Ala Ala Gln Arg Ala Glu Glu Lys
       50                  55                  60
   Ile Pro Asp Thr Ile Leu Leu Leu Gln His Pro Pro Thr Tyr Thr Ala
   65                  70                  75                  80
   Gly Lys Arg Thr Gln Asp Ser Asp Arg Pro Thr Asn Gly Leu Pro Val
                   85                  90                  95
   Val Asp Val Asp Arg Gly Gly Arg Ile Thr Trp His Gly Pro Gly Gln
               100                 105                 110
   Leu Val Ala Tyr Pro Ile Ile Lys Leu Ala Asp Pro Val Asp Val Val
           115                 120                 125
   Asp Tyr Val Arg Arg Leu Glu Gln Ala Leu Ile Gln Thr Cys Glu Asp
       130                 135                 140
   Leu Gly Leu His Gly Thr Gly Arg Val Glu Gly Arg Ser Gly Val Trp
   145                 150                 155                 160
   Leu Pro Ala Gly Val Ile Asn Gly Glu Leu Lys Pro Ala Arg Lys Ile
                   165                 170                 175
   Ala Ala Ile Gly Ile Arg Val Thr Arg Gly Val Thr Met His Gly Val
               180                 185                 190
   Ala Leu Asn Cys Asp Asn Thr Met Glu Tyr Tyr Asp His Ile Val Pro
           195                 200                 205
   Cys Gly Leu Ala Asp Ala Gly Val Thr Thr Leu Thr Glu Glu Leu Gly
       210                 215                 220
   Arg Asp Val Ser Val Ser Asp Ala Tyr Ser Ser Leu Ala His Asn Leu
   225 230 235 240
   Val Asp Ala Leu Asn Gly Asp Leu Pro Val His Ser
   245 250
<210> 21
   <211> 7070
   <212> DNA
   <213> artificial
<220>
   <223> Plasmid pH273
<400> 21
   tcgagaggcc tgacgtcggg cccggtacca cgcgtcatat gactagttgg agaatcatga      60
   cctcagcatc tgccccaagc tttaaccccg gcaagggtcc cggctcagca gtcggaattg     120
   cccttttagg attcggaaca gtcggcactg aggtgatgcg tctgatgacc gagtacggtg     180
   atgaacttgc gcaccgcatt ggtggcccac tggaggttcg tggcattgct gtttctgata     240
   tctcaaagcc acgtgaaggc gttgcacctg agctgctcac tgaggacgct tttgcactca     300
   tcgagcgcga ggatgttgac atcgtcgttg aggttatcgg cggcattgag tacccacgtg     360
   aggtagttct cgcagctctg aaggccggca agtctgttgt taccgccaat aaggctcttg     420
   ttgcagctca ctctgctgag cttgctgatg cagcggaagc cgcaaacgtt gacctgtact     480
   tcgaggctgc tgttgcaggc gcaattccag tggttggccc actgcgtcgc tccctggctg     540
   gcgatcagat ccagtctgtg atgggcatcg ttaacggcac caccaacttc atcttggacg     600
   ccatggattc caccggcgct gactatgcag attctttggc tgaggcaact cgtttgggtt     660
   acgccgaagc tgatccaact gcagacgtcg aaggccatga cgccgcatcc aaggctgcaa     720
   ttttggcatc catcgctttc cacacccgtg ttaccgcgga tgatgtgtac tgcgaaggta     780
   tcagcaacat cagcgctgcc gacattgagg cagcacagca ggcaggccac accatcaagt     840
   tgttggccat ctgtgagaag ttcaccaaca aggaaggaaa gtcggctatt tctgctcgcg     900
   tgcacccgac tctattacct gtgtcccacc cactggcgtc ggtaaacaag tcctttaatg     960
   caatctttgt tgaagcagaa gcagctggtc gcctgatgtt ctacggaaac ggtgcaggtg    1020
   gcgcgccaac cgcgtctgct gtgcttggcg acgtcgttgg tgccgcacga aacaaggtgc    1080
   acggtggccg tgctccaggt gagtccacct acgctaacct gccgatcgct gatttcggtg    1140
   agaccaccac tcgttaccac ctcgacatgg atgtggaaga tcgcgtgggg gttttggctg    1200
   aattggctag cctgttctct gagcaaggaa tcttcctgcg tacaatccga caggaagagc    1260
   gcgatgatga tgcacgtctg atcgtggtca cccactctgc gctggaatct gatctttccc    1320
   gcaccgttga actgctgaag gctaagcctg ttgttaaggc aatcaacagt gtgatccgcc    1380
   tcgaaaggga ctaattttac tgacatggca attgaactga acgtcggtcg taaggttacc    1440
   gtcacggtac ctggatcttc tgcaaacctc ggacctggct ttgacacttt aggtttggca    1500
   ctgtcggtat acgacactgt cgaagtggaa attattccat ctggcttgga agtggaagtt 1560
   tttggcgaag gccaaggcga agtccctctt gatggctccc acctggtggt taaagctatt 1620
   cgtgctggcc tgaaggcagc tgacgctgaa gttcctggat tgcgagtggt gtgccacaac 1680
   aacattccgc agtctcgtgg tcttggctcc tctgctgcag cggcggttgc tggtgttgct 1740
   gcagctaatg gtttggcgga tttcccgctg actcaagagc agattgttca gttgtcctct 1800
   gcctttgaag gccacccaga taatgctgcg gcttctgtgc tgggtggagc agtggtgtcg 1860
   tggacaaatc tgtctatcga cggcaagagc cagccacagt atgctgctgt accacttgag 1920
   gtgcaggaca atattcgtgc gactgcgctg gttcctaatt tccacgcatc caccgaagct 1980
   gtgcgccgag tccttcccac tgaagtcact cacatcgatg cgcgatttaa cgtgtcccgc 2040
   gttgcagtga tgatcgttgc gttgcagcag cgtcctgatt tgctgtggga gggtactcgt 2100
   gaccgtctgc accagcctta tcgtgcagaa gtgttgccta ttacctctga gtgggtaaac 2160
   cgcctgcgca accgtggcta cgcggcatac ctttccggtg ccggcccaac cgccatggtg 2220
   ctgtccactg agccaattcc agacaaggtt ttggaagatg ctcgtgagtc tggcattaag 2280
   gtgcttgagc ttgaggttgc gggaccagtc aaggttgaag ttaaccaacc ttaggcccaa 2340
   caaggaaggc ccccttcgaa tcaagaaggg ggccttatta gtgcagcaat tattcgctga 2400
   acacgtgaac cttacaggtg cccggcgcgt tgagtggttt gagttccagc tggatgcggt 2460
   tgttttcacc gaggctttct tggatgaatc cggcgtggat ggcgcagacg aaggctgatg 2520
   ggcgtttgtc gttgaccaca aatgggcagc tgtgtagagc gagggagttt gcttcttcgg 2580
   tttcggtggg gtcaaagccc atttcgcgga ggcggttaat gagcggggag agggcttcgt 2640
   cgagttcttc ggcttcggcg tggttaatgc ccatgacgtg tgcccactgg gttccgatgg 2700
   aaagtgcttt ggcgcggagg tcggggttgt gcattgcgtc atcgtcgaca tcgccgagca 2760
   tgttggccat gagttcgatc agggtgatgt attctttggc gacagcgcgg ttgtcgggga 2820
   cgcgtgtttg gaagatgagg gaggggcggg atcctctaga cccgggattt aaatcgctag 2880
   cgggctgcta aaggaagcgg aacacgtaga aagccagtcc gcagaaacgg tgctgacccc 2940
   ggatgaatgt cagctactgg gctatctgga caagggaaaa cgcaagcgca aagagaaagc 3000
   aggtagcttg cagtgggctt acatggcgat agctagactg ggcggtttta tggacagcaa 3060
   gcgaaccgga attgccagct ggggcgccct ctggtaaggt tgggaagccc tgcaaagtaa 3120
   actggatggc tttcttgccg ccaaggatct gatggcgcag gggatcaaga tctgatcaag 3180
   agacaggatg aggatcgttt cgcatgattg aacaagatgg attgcacgca ggttctccgg 3240
   ccgcttgggt ggagaggcta ttcggctatg actgggcaca acagacaatc ggctgctctg 3300
   atgccgccgt gttccggctg tcagcgcagg ggcgcccggt tctttttgtc aagaccgacc 3360
   tgtccggtgc cctgaatgaa ctgcaggacg aggcagcgcg gctatcgtgg ctggccacga 3420
   cgggcgttcc ttgcgcagct gtgctcgacg ttgtcactga agcgggaagg gactggctgc 3480
   tattgggcga agtgccgggg caggatctcc tgtcatctca ccttgctcct gccgagaaag 3540
   tatccatcat ggctgatgca atgcggcggc tgcatacgct tgatccggct acctgcccat 3600
   tcgaccacca agcgaaacat cgcatcgagc gagcacgtac tcggatggaa gccggtcttg 3660
   tcgatcagga tgatctggac gaagagcatc aggggctcgc gccagccgaa ctgttcgcca 3720
   ggctcaaggc gcgcatgccc gacggcgagg atctcgtcgt gacccatggc gatgcctgct 3780
   tgccgaatat catggtggaa aatggccgct tttctggatt catcgactgt ggccggctgg 3840
   gtgtggcgga ccgctatcag gacatagcgt tggctacccg tgatattgct gaagagcttg 3900
   gcggcgaatg ggctgaccgc ttcctcgtgc tttacggtat cgccgctccc gattcgcagc 3960
   gcatcgcctt ctatcgcctt cttgacgagt tcttctgagc gggactctgg ggttcgaaat 4020
   gaccgaccaa gcgacgccca acctgccatc acgagatttc gattccaccg ccgccttcta 4080
   tgaaaggttg ggcttcggaa tcgttttccg ggacgccggc tggatgatcc tccagcgcgg 4140
   ggatctcatg ctggagttct tcgcccacgc tagcggcgcg ccggccggcc cggtgtgaaa 4200
   taccgcacag atgcgtaagg agaaaatacc gcatcaggcg ctcttccgct tcctcgctca 4260
   ctgactcgct gcgctcggtc gttcggctgc ggcgagcggt atcagctcac tcaaaggcgg 4320
   taatacggtt atccacagaa tcaggggata acgcaggaaa gaacatgtga gcaaaaggcc 4380
   agcaaaaggc caggaaccgt aaaaaggccg cgttgctggc gtttttccat aggctccgcc 4440
   cccctgacga gcatcacaaa aatcgacgct caagtcagag gtggcgaaac ccgacaggac 4500
   tataaagata ccaggcgttt ccccctggaa gctccctcgt gcgctctcct gttccgaccc 4560
   tgccgcttac cggatacctg tccgcctttc tcccttcggg aagcgtggcg ctttctcata 4620
   gctcacgctg taggtatctc agttcggtgt aggtcgttcg ctccaagctg ggctgtgtgc 4680
   acgaaccccc cgttcagccc gaccgctgcg ccttatccgg taactatcgt cttgagtcca 4740
   acccggtaag acacgactta tcgccactgg cagcagccac tggtaacagg attagcagag 4800
   cgaggtatgt aggcggtgct acagagttct tgaagtggtg gcctaactac ggctacacta 4860
   gaaggacagt atttggtatc tgcgctctgc tgaagccagt taccttcgga aaaagagttg 4920
   gtagctcttg atccggcaaa caaaccaccg ctggtagcgg tggttttttt gtttgcaagc 4980
   agcagattac gcgcagaaaa aaaggatctc aagaagatcc tttgatcttt tctacggggt 5040
   ctgacgctca gtggaacgaa aactcacgtt aagggatttt ggtcatgaga ttatcaaaaa 5100
   ggatcttcac ctagatcctt ttaaaggccg gccgcggccg ccatcggcat tttcttttgc 5160
   gtttttattt gttaactgtt aattgtcctt gttcaaggat gctgtctttg acaacagatg 5220
   ttttcttgcc tttgatgttc agcaggaagc tcggcgcaaa cgttgattgt ttgtctgcgt 5280
   agaatcctct gtttgtcata tagcttgtaa tcacgacatt gtttcctttc gcttgaggta 5340
   cagcgaagtg tgagtaagta aaggttacat cgttaggatc aagatccatt tttaacacaa 5400
   ggccagtttt gttcagcggc ttgtatgggc cagttaaaga attagaaaca taaccaagca 5460
   tgtaaatatc gttagacgta atgccgtcaa tcgtcatttt tgatccgcgg gagtcagtga 5520
   acaggtacca tttgccgttc attttaaaga cgttcgcgcg ttcaatttca tctgttactg 5580
   tgttagatgc aatcagcggt ttcatcactt ttttcagtgt gtaatcatcg tttagctcaa 5640
   tcataccgag agcgccgttt gctaactcag ccgtgcgttt tttatcgctt tgcagaagtt 5700
   tttgactttc ttgacggaag aatgatgtgc ttttgccata gtatgctttg ttaaataaag 5760
   attcttcgcc ttggtagcca tcttcagttc cagtgtttgc ttcaaatact aagtatttgt 5820
   ggcctttatc ttctacgtag tgaggatctc tcagcgtatg gttgtcgcct gagctgtagt 5880
   tgccttcatc gatgaactgc tgtacatttt gatacgtttt tccgtcaccg tcaaagattg 5940
   atttataatc ctctacaccg ttgatgttca aagagctgtc tgatgctgat acgttaactt 6000
   gtgcagttgt cagtgtttgt ttgccgtaat gtttaccgga gaaatcagtg tagaataaac 6060
   ggatttttcc gtcagatgta aatgtggctg aacctgacca ttcttgtgtt tggtctttta 6120
   ggatagaatc atttgcatcg aatttgtcgc tgtctttaaa gacgcggcca gcgtttttcc 6180
   agctgtcaat agaagtttcg ccgacttttt gatagaacat gtaaatcgat gtgtcatccg 6240
   catttttagg atctccggct aatgcaaaga cgatgtggta gccgtgatag tttgcgacag 6300
   tgccgtcagc gttttgtaat ggccagctgt cccaaacgtc caggcctttt gcagaagaga 6360
   tatttttaat tgtggacgaa tcaaattcag aaacttgata tttttcattt ttttgctgtt 6420
   cagggatttg cagcatatca tggcgtgtaa tatgggaaat gccgtatgtt tccttatatg 6480
   gcttttggtt cgtttctttc gcaaacgctt gagttgcgcc tcctgccagc agtgcggtag 6540
   taaaggttaa tactgttgct tgttttgcaa actttttgat gttcatcgtt catgtctcct 6600
   tttttatgta ctgtgttagc ggtctgcttc ttccagccct cctgtttgaa gatggcaagt 6660
   tagttacgca caataaaaaa agacctaaaa tatgtaaggg gtgacgccaa agtatacact 6720
   ttgcccttta cacattttag gtcttgcctg ctttatcagt aacaaacccg cgcgatttac 6780
   ttttcgacct cattctatta gactctcgtt tggattgcaa ctggtctatt ttcctctttt 6840
   gtttgataga aaatcataaa aggatttgca gactacgggc ctaaagaact aaaaaatcta 6900
   tctgtttctt ttcattctct gtatttttta tagtttctgt tgcatgggca taaagttgcc 6960
   tttttaatca caattcagaa aatatcataa tatctcattt cactaaataa tagtgaacgg 7020
   caggtatatg tgatgggtta aaaaggatcg gcggccgctc gatttaaatc 7070
<210> 22
   <211> 7070
   <212> DNA
   <213> artificial
<220>
   <223> Plasmid pH373
<400> 22
   tcgagaggcc tgacgtcggg cccggtacca cgcgtcatat gactagttgg agaatcatga     60
   cctcagcatc tgccccaagc tttaaccccg gcaagggtcc cggctcagca gtcggaattg    120
   cccttttagg attcggaaca gtcggcactg aggtgatgcg tctgatgacc gagtacggtg    180
   atgaacttgc gcaccgcatt ggtggcccac tggaggttcg tggcattgct gtttctgata    240
   tctcaaagcc acgtgaaggc gttgcacctg agctgctcac tgaggacgct tttgcactca    300
   tcgagcgcga ggatgttgac atcgtcgttg aggttatcgg cggcattgag tacccacgtg    360
   aggtagttct cgcagctctg aaggccggca agtctgttgt taccgccaat aaggctcttg    420
   ttgcagctca ctctgctgag cttgctgatg cagcggaagc cgcaaacgtt gacctgtact    480
   tcgaggctgc tgttgcaggc gcaattccag tggttggccc actgcgtcgc tccctggctg    540
   gcgatcagat ccagtctgtg atgggcatcg ttaacggcac caccaacttc atcttggacg    600
   ccatggattc caccggcgct gactatgcag attctttggc tgaggcaact cgtttgggtt    660
   acgccgaagc tgatccaact gcagacgtcg aaggccatga cgccgcatcc aaggctgcaa    720
   ttttggcatc catcgctttc cacacccgtg ttaccgcgga tgatgtgtac tgcgaaggta    780
   tcagcaacat cagcgctgcc gacattgagg cagcacagca ggcaggccac accatcaagt    840
   tgttggccat ctgtgagaag ttcaccaaca aggaaggaaa gtcggctatt tctgctcgcg    900
   tgcacccgac tctattacct gtgtcccacc cactggcgtc ggtaaacaag tcctttaatg    960
   caatctttgt tgaagcagaa gcagctggtc gcctgatgtt ctacggaaac ggtgcaggtg   1020
   gcgcgccaac cgcgtctgct gtgcttggcg acgtcgttgg tgccgcacga aacaaggtgc   1080
   acggtggccg tgctccaggt gagtccacct acgctaacct gccgatcgct gatttcggtg   1140
   agaccaccac tcgttaccac ctcgacatgg atgtggaaga tcgcgtgggg gttttggctg   1200
   aattggctag cctgttctct gagcaaggaa tcttcctgcg tacaatccga caggaagagc   1260
   gcgatgatga tgcacgtctg atcgtggtca cccactctgc gctggaatct gatctttccc   1320
   gcaccgttga actgctgaag gctaagcctg ttgttaaggc aatcaacagt gtgatccgcc   1380
   tcgaaaggga ctaattttac tgacatggca attgaactga acgtcggtcg taaggttacc   1440
   gtcacggtac ctggatcttc tgcaaacctc ggacctggct ttgacacttt aggtttggca   1500
   ctgtcggtat acgacactgt cgaagtggaa attattccat ctggcttgga agtggaagtt   1560
   tttggcgaag gccaaggcga agtccctctt gatggctccc acctggtggt taaagctatt   1620
   cgtgctggcc tgaaggcagc tgacgctgaa gttcctggat tgcgagtggt gtgccacaac   1680
   aacattccgc agtctcgtgg tcttggctcc tctgctgcag cggcggttgc tggtgttgct   1740
   gcagctaatg gtttggcgga tttcccgctg actcaagagc agattgttca gttgtcctct   1800
   gcctttgaag gccacccaga taatgctgcg gcttctgtgc tgggtggagc agtggtgtcg   1860
   tggacaaatc tgtctatcga cggcaagagc cagccacagt atgctgctgt accacttgag   1920
   gtgcaggaca atattcgtgc gactgcgctg gttcctaatt tccacgcatc caccgaagct 1980
   gtgcgccgag tccttcccac tgaagtcact cacatcgatg cgcgatttaa cgtgtcccgc 2040
   gttgcagtga tgatcgttgc gttgcagcag cgtcctgatt tgctgtggga gggtactcgt 2100
   gaccgtctgc accagcctta tcgtgcagaa gtgttgccta ttacctctga gtgggtaaac 2160
   cgcctgcgca accgtggcta cgcggcatac ctttccggtg ccggcccaac cgccatggtg 2220
   ctgtccactg agccaattcc agacaaggtt ttggaagatg ctcgtgagtc tggcattaag 2280
   gtgcttgagc ttgaggttgc gggaccagtc aaggttgaag ttaaccaacc ttaggcccaa 2340
   caaggaaggc ccccttcgaa tcaagaaggg ggccttatta gtgcagcaat tattcgctga 2400
   acacgtgaac cttacaggtg cccggcgcgt tgagtggttt gagttccagc tggatgcggt 2460
   tgttttcacc gaggctttct tggatgaatc cggcgtggat ggcgcagacg aaggctgatg 2520
   ggcgtttgtc gttgaccaca aatgggcagc tgtgtagagc gagggagttt gcttcttcgg 2580
   tttcggtggg gtcaaagccc atttcgcgga ggcggttaat gagcggggag agggcttcgt 2640
   cgagttcttc ggcttcggcg tggttaatgc ccatgacgtg tgcccactgg gttccgatgg 2700
   aaagtgcttt ggcgcggagg tcggggttgt gcattgcgtc atcgtcgaca tcgccgagca 2760
   tgttggccat gagttcgatc agggtgatgt attctttggc gacagcgcgg ttgtcgggga 2820
   cgcgtgtttg gaagatgagg gaggggcggg atcctctaga cccgggattt aaatcgctag 2880
   cgggctgcta aaggaagcgg aacacgtaga aagccagtcc gcagaaacgg tgctgacccc 2940
   ggatgaatgt cagctactgg gctatctgga caagggaaaa cgcaagcgca aagagaaagc 3000
   aggtagcttg cagtgggctt acatggcgat agctagactg ggcggtttta tggacagcaa 3060
   gcgaaccgga attgccagct ggggcgccct ctggtaaggt tgggaagccc tgcaaagtaa 3120
   actggatggc tttcttgccg ccaaggatct gatggcgcag gggatcaaga tctgatcaag 3180
   agacaggatg aggatcgttt cgcatgattg aacaagatgg attgcacgca ggttctccgg 3240
   ccgcttgggt ggagaggcta ttcggctatg actgggcaca acagacaatc ggctgctctg 3300
   atgccgccgt gttccggctg tcagcgcagg ggcgcccggt tctttttgtc aagaccgacc 3360
   tgtccggtgc cctgaatgaa ctgcaggacg aggcagcgcg gctatcgtgg ctggccacga 3420
   cgggcgttcc ttgcgcagct gtgctcgacg ttgtcactga agcgggaagg gactggctgc 3480
   tattgggcga agtgccgggg caggatctcc tgtcatctca ccttgctcct gccgagaaag 3540
   tatccatcat ggctgatgca atgcggcggc tgcatacgct tgatccggct acctgcccat 3600
   tcgaccacca agcgaaacat cgcatcgagc gagcacgtac tcggatggaa gccggtcttg 3660
   tcgatcagga tgatctggac gaagagcatc aggggctcgc gccagccgaa ctgttcgcca 3720
   ggctcaaggc gcgcatgccc gacggcgagg atctcgtcgt gacccatggc gatgcctgct 3780
   tgccgaatat catggtggaa aatggccgct tttctggatt catcgactgt ggccggctgg 3840
   gtgtggcgga ccgctatcag gacatagcgt tggctacccg tgatattgct gaagagcttg 3900
   gcggcgaatg ggctgaccgc ttcctcgtgc tttacggtat cgccgctccc gattcgcagc 3960
   gcatcgcctt ctatcgcctt cttgacgagt tcttctgagc gggactctgg ggttcgaaat 4020
   gaccgaccaa gcgacgccca acctgccatc acgagatttc gattccaccg ccgccttcta 4080
   tgaaaggttg ggcttcggaa tcgttttccg ggacgccggc tggatgatcc tccagcgcgg 4140
   ggatctcatg ctggagttct tcgcccacgc tagcggcgcg ccggccggcc cggtgtgaaa 4200
   taccgcacag atgcgtaagg agaaaatacc gcatcaggcg ctcttccgct tcctcgctca 4260
   ctgactcgct gcgctcggtc gttcggctgc ggcgagcggt atcagctcac tcaaaggcgg 4320
   taatacggtt atccacagaa tcaggggata acgcaggaaa gaacatgtga gcaaaaggcc 4380
   agcaaaaggc caggaaccgt aaaaaggccg cgttgctggc gtttttccat aggctccgcc 4440
   cccctgacga gcatcacaaa aatcgacgct caagtcagag gtggcgaaac ccgacaggac 4500
   tataaagata ccaggcgttt ccccctggaa gctccctcgt gcgctctcct gttccgaccc 4560
   tgccgcttac cggatacctg tccgcctttc tcccttcggg aagcgtggcg ctttctcata 4620
   gctcacgctg taggtatctc agttcggtgt aggtcgttcg ctccaagctg ggctgtgtgc 4680
   acgaaccccc cgttcagccc gaccgctgcg ccttatccgg taactatcgt cttgagtcca 4740
   acccggtaag acacgactta tcgccactgg cagcagccac tggtaacagg attagcagag 4800
   cgaggtatgt aggcggtgct acagagttct tgaagtggtg gcctaactac ggctacacta 4860
   gaaggacagt atttggtatc tgcgctctgc tgaagccagt taccttcgga aaaagagttg 4920
   gtagctcttg atccggcaaa caaaccaccg ctggtagcgg tggttttttt gtttgcaagc 4980
   agcagattac gcgcagaaaa aaaggatctc aagaagatcc tttgatcttt tctacggggt 5040
   ctgacgctca gtggaacgaa aactcacgtt aagggatttt ggtcatgaga ttatcaaaaa 5100
   ggatcttcac ctagatcctt ttaaaggccg gccgcggccg ccatcggcat tttcttttgc 5160
   gtttttattt gttaactgtt aattgtcctt gttcaaggat gctgtctttg acaacagatg 5220
   ttttcttgcc tttgatgttc agcaggaagc tcggcgcaaa cgttgattgt ttgtctgcgt 5280
   agaatcctct gtttgtcata tagcttgtaa tcacgacatt gtttcctttc gcttgaggta 5340
   cagcgaagtg tgagtaagta aaggttacat cgttaggatc aagatccatt tttaacacaa 5400
   ggccagtttt gttcagcggc ttgtatgggc cagttaaaga attagaaaca taaccaagca 5460
   tgtaaatatc gttagacgta atgccgtcaa tcgtcatttt tgatccgcgg gagtcagtga 5520
   acaggtacca tttgccgttc attttaaaga cgttcgcgcg ttcaatttca tctgttactg 5580
   tgttagatgc aatcagcggt ttcatcactt ttttcagtgt gtaatcatcg tttagctcaa 5640
   tcataccgag agcgccgttt gctaactcag ccgtgcgttt tttatcgctt tgcagaagtt 5700
   tttgactttc ttgacggaag aatgatgtgc ttttgccata gtatgctttg ttaaataaag 5760
   attcttcgcc ttggtagcca tcttcagttc cagtgtttgc ttcaaatact aagtatttgt 5820
   ggcctttatc ttctacgtag tgaggatctc tcagcgtatg gttgtcgcct gagctgtagt   5880
   tgccttcatc gatgaactgc tgtacatttt gatacgtttt tccgtcaccg tcaaagattg   5940
   atttataatc ctctacaccg ttgatgttca aagagctgtc tgatgctgat acgttaactt   6000
   gtgcagttgt cagtgtttgt ttgccgtaat gtttaccgga gaaatcagtg tagaataaac   6060
   ggatttttcc gtcagatgta aatgtggctg aacctgacca ttcttgtgtt tggtctttta   6120
   ggatagaatc atttgcatcg aatttgtcgc tgtctttaaa gacgcggcca gcgtttttcc   6180
   agctgtcaat agaagtttcg ccgacttttt gatagaacat gtaaatcgat gtgtcatccg   6240
   catttttagg atctccggct aatgcaaaga cgatgtggta gccgtgatag tttgcgacag   6300
   tgccgtcagc gttttgtaat ggccagctgt cccaaacgtc caggcctttt gcagaagaga   6360
   tatttttaat tgtggacgaa tcaaattcag aaacttgata tttttcattt ttttgctgtt   6420
   cagggatttg cagcatatca tggcgtgtaa tatgggaaat gccgtatgtt tccttatatg   6480
   gcttttggtt cgtttctttc gcaaacgctt gagttgcgcc tcctgccagc agtgcggtag   6540
   taaaggttaa tactgttgct tgttttgcaa actttttgat gttcatcgtt catgtctcct   6600
   tttttatgta ctgtgttagc ggtctgcttc ttccagccct cctgtttgaa gatggcaagt   6660
   tagttacgca caataaaaaa agacctaaaa tatgtaaggg gtgacgccaa agtatacact   6720
   ttgcccttta cacattttag gtcttgcctg ctttatcagt aacaaacccg cgcgatttac   6780
   ttttcgacct cattctatta gactctcgtt tggattgcaa ctggtctatt ttcctctttt   6840
   gtttgataga aaatcataaa aggatttgca gactacgggc ctaaagaact aaaaaatcta   6900
   tctgtttctt ttcattctct gtatttttta tagtttctgt tgcatgggca taaagttgcc   6960
   tttttaatca caattcagaa aatatcataa tatctcattt cactaaataa tagtgaacgg   7020
   caggtatatg tgatgggtta aaaaggatcg gcggccgctc gatttaaatc              7070
<210> 23
   <211> 8766
   <212> DNA
   <213> artificial
<220>
   <223> Plasmid pH304
<400> 23
<210> 24
   <211> 7070
   <212> DNA
   <213> artificial
<220>
   <223> Plasmid pH399
<400> 24
   tcgagaggcc tgacgtcggg cccggtacca cgcgtcatat gactagttgg agaatcatga      60
   cctcagcatc tgccccaagc tttaaccccg gcaagggtcc cggctcagca gtcggaattg     120
   cccttttagg attcggaaca gtcggcactg aggtgatgcg tctgatgacc gagtacggtg    180
   atgaacttgc gcaccgcatt ggtggcccac tggaggttcg tggcattgct gtttctgata     240
   tctcaaagcc acgtgaaggc gttgcacctg agctgctcac tgaggacgct tttgcactca     300
   tcgagcgcga ggatgttgac atcgtcgttg aggttatcgg cggcattgag tacccacgtg     360
   aggtagttct cgcagctctg aaggccggca agtctgttgt taccgccaat aaggctcttg     420
   ttgcagctca ctctgctgag cttgctgatg cagcggaagc cgcaaacgtt gacctgtact     480
   tcgaggctgc tgttgcaggc gcaattccag tggttggccc actgcgtcgc tccctggctg     540
   gcgatcagat ccagtctgtg atgggcatcg ttaacggcac caccaacttc atcttggacg     600
   ccatggattc caccggcgct gactatgcag attctttggc tgaggcaact cgtttgggtt     660
   acgccgaagc tgatccaact gcagacgtcg aaggccatga cgccgcatcc aaggctgcaa     720
   ttttggcatc catcgctttc cacacccgtg ttaccgcgga tgatgtgtac tgcgaaggta     780
   tcagcaacat cagcgctgcc gacattgagg cagcacagca ggcaggccac accatcaagt     840
   tgttggccat ctgtgagaag ttcaccaaca aggaaggaaa gtcggctatt tctgctcgcg     900
   tgcacccgac tctattacct gtgtcccacc cactggcgtc ggtaaacaag tcctttaatg     960
   caatctttgt tgaagcagaa gcagctggtc gcctgatgtt ctacggaaac ggtgcaggtg    1020
   gcgcgccaac cgcgtctgct gtgcttggcg acgtcgttgg tgccgcacga aacaaggtgc 1080
   acggtggccg tgctccaggt gagtccacct acgctaacct gccgatcgct gatttcggtg 1140
   agaccaccac tcgttaccac ctcgacatgg atgtggaaga tcgcgtgggg gttttggctg 1200
   aattggctag cctgttctct gagcaaggaa tcttcctgcg tacaatccga caggaagagc 1260
   gcgatgatga tgcacgtctg atcgtggtca cccactctgc gctggaatct gatctttccc 1320
   gcaccgttga actgctgaag gctaagcctg ttgttaaggc aatcaacagt gtgatccgcc 1380
   tcgaaaggga ctaattttac tgacatggca attgaactga acgtcggtcg taaggttacc 1440
   gtcacggtac ctggatcttc tgcaaacctc ggacctggct ttgacacttt aggtttggca 1500
   ctgtcggtat acgacactgt cgaagtggaa attattccat ctggcttgga agtggaagtt 1560
   tttggcgaag gccaaggcga agtccctctt gatggctccc acctggtggt taaagctatt 1620
   cgtgctggcc tgaaggcagc tgacgctgaa gttcctggat tgcgagtggt gtgccacaac 1680
   aacattccgc agtctcgtgg tcttggctcc gctgctgcag cggcggttgc tggtgttgct 1740
   gcagctaatg gtttggcgga tttcccgctg actcaagagc agattgttca gttgtcctct 1800
   gcctttgaag gccacccaga taatgctgcg gcttctgtgc tgggtggagc agtggtgtcg 1860
   tggacaaatc tgtctatcga cggcaagagc cagccacagt atgctgctgt accacttgag 1920
   gtgcaggaca atattcgtgc gactgcgctg gttcctaatt tccacgcatc caccgaagct 1980
   gtgcgccgag tccttcccac tgaagtcact cacatcgatg cgcgatttaa cgtgtcccgc 2040
   gttgcagtga tgatcgttgc gttgcagcag cgtcctgatt tgctgtggga gggtactcgt 2100
   gaccgtctgc accagcctta tcgtgcagaa gtgttgccta ttacctctga gtgggtaaac 2160
   cgcctgcgca accgtggcta cgcggcatac ctttccggtg ccggcccaac cgccatggtg 2220
   ctgtccactg agccaattcc agacaaggtt ttggaagatg ctcgtgagtc tggcattaag 2280
   gtgcttgagc ttgaggttgc gggaccagtc aaggttgaag ttaaccaacc ttaggcccaa 2340
   caaggaaggc ccccttcgaa tcaagaaggg ggccttatta gtgcagcaat tattcgctga 2400
   acacgtgaac cttacaggtg cccggcgcgt tgagtggttt gagttccagc tggatgcggt 2460
   tgttttcacc gaggctttct tggatgaatc cggcgtggat ggcgcagacg aaggctgatg 2520
   ggcgtttgtc gttgaccaca aatgggcagc tgtgtagagc gagggagttt gcttcttcgg 2580
   tttcggtggg gtcaaagccc atttcgcgga ggcggttaat gagcggggag agggcttcgt 2640
   cgagttcttc ggcttcggcg tggttaatgc ccatgacgtg tgcccactgg gttccgatgg 2700
   aaagtgcttt ggcgcggagg tcggggttgt gcattgcgtc atcgtcgaca tcgccgagca 2760
   tgttggccat gagttcgatc agggtgatgt attctttggc gacagcgcgg ttgtcgggga 2820
   cgcgtgtttg gaagatgagg gaggggcggg atcctctaga cccgggattt aaatcgctag 2880
   cgggctgcta aaggaagcgg aacacgtaga aagccagtcc gcagaaacgg tgctgacccc 2940
   ggatgaatgt cagctactgg gctatctgga caagggaaaa cgcaagcgca aagagaaagc 3000
   aggtagcttg cagtgggctt acatggcgat agctagactg ggcggtttta tggacagcaa 3060
   gcgaaccgga attgccagct ggggcgccct ctggtaaggt tgggaagccc tgcaaagtaa 3120
   actggatggc tttcttgccg ccaaggatct gatggcgcag gggatcaaga tctgatcaag 3180
   agacaggatg aggatcgttt cgcatgattg aacaagatgg attgcacgca ggttctccgg 3240
   ccgcttgggt ggagaggcta ttcggctatg actgggcaca acagacaatc ggctgctctg 3300
   atgccgccgt gttccggctg tcagcgcagg ggcgcccggt tctttttgtc aagaccgacc 3360
   tgtccggtgc cctgaatgaa ctgcaggacg aggcagcgcg gctatcgtgg ctggccacga 3420
   cgggcgttcc ttgcgcagct gtgctcgacg ttgtcactga agcgggaagg gactggctgc 3480
   tattgggcga agtgccgggg caggatctcc tgtcatctca ccttgctcct gccgagaaag 3540
   tatccatcat ggctgatgca atgcggcggc tgcatacgct tgatccggct acctgcccat 3600
   tcgaccacca agcgaaacat cgcatcgagc gagcacgtac tcggatggaa gccggtcttg 3660
   tcgatcagga tgatctggac gaagagcatc aggggctcgc gccagccgaa ctgttcgcca 3720
   ggctcaaggc gcgcatgccc gacggcgagg atctcgtcgt gacccatggc gatgcctgct 3780
   tgccgaatat catggtggaa aatggccgct tttctggatt catcgactgt ggccggctgg 3840
   gtgtggcgga ccgctatcag gacatagcgt tggctacccg tgatattgct gaagagcttg 3900
   gcggcgaatg ggctgaccgc ttcctcgtgc tttacggtat cgccgctccc gattcgcagc 3960
   gcatcgcctt ctatcgcctt cttgacgagt tcttctgagc gggactctgg ggttcgaaat 4020
   gaccgaccaa gcgacgccca acctgccatc acgagatttc gattccaccg ccgccttcta 4080
   tgaaaggttg ggcttcggaa tcgttttccg ggacgccggc tggatgatcc tccagcgcgg 4140
   ggatctcatg ctggagttct tcgcccacgc tagcggcgcg ccggccggcc cggtgtgaaa 4200
   taccgcacag atgcgtaagg agaaaatacc gcatcaggcg ctcttccgct tcctcgctca 4260
   ctgactcgct gcgctcggtc gttcggctgc ggcgagcggt atcagctcac tcaaaggcgg 4320
   taatacggtt atccacagaa tcaggggata acgcaggaaa gaacatgtga gcaaaaggcc 4380
   agcaaaaggc caggaaccgt aaaaaggccg cgttgctggc gtttttccat aggctccgcc 4440
   cccctgacga gcatcacaaa aatcgacgct caagtcagag gtggcgaaac ccgacaggac 4500
   tataaagata ccaggcgttt ccccctggaa gctccctcgt gcgctctcct gttccgaccc 4560
   tgccgcttac cggatacctg tccgcctttc tcccttcggg aagcgtggcg ctttctcata 4620
   gctcacgctg taggtatctc agttcggtgt aggtcgttcg ctccaagctg ggctgtgtgc 4680
   acgaaccccc cgttcagccc gaccgctgcg ccttatccgg taactatcgt cttgagtcca 4740
   acccggtaag acacgactta tcgccactgg cagcagccac tggtaacagg attagcagag 4800
   cgaggtatgt aggcggtgct acagagttct tgaagtggtg gcctaactac ggctacacta 4860
   gaaggacagt atttggtatc tgcgctctgc tgaagccagt taccttcgga aaaagagttg 4920
   gtagctcttg atccggcaaa caaaccaccg ctggtagcgg tggttttttt gtttgcaagc 4980
   agcagattac gcgcagaaaa aaaggatctc aagaagatcc tttgatcttt tctacggggt 5040
   ctgacgctca gtggaacgaa aactcacgtt aagggatttt ggtcatgaga ttatcaaaaa 5100
   ggatcttcac ctagatcctt ttaaaggccg gccgcggccg ccatcggcat tttcttttgc 5160
   gtttttattt gttaactgtt aattgtcctt gttcaaggat gctgtctttg acaacagatg 5220
   ttttcttgcc tttgatgttc agcaggaagc tcggcgcaaa cgttgattgt ttgtctgcgt 5280
   agaatcctct gtttgtcata tagcttgtaa tcacgacatt gtttcctttc gcttgaggta 5340
   cagcgaagtg tgagtaagta aaggttacat cgttaggatc aagatccatt tttaacacaa 5400
   ggccagtttt gttcagcggc ttgtatgggc cagttaaaga attagaaaca taaccaagca 5460
   tgtaaatatc gttagacgta atgccgtcaa tcgtcatttt tgatccgcgg gagtcagtga 5520
   acaggtacca tttgccgttc attttaaaga cgttcgcgcg ttcaatttca tctgttactg 5580
   tgttagatgc aatcagcggt ttcatcactt ttttcagtgt gtaatcatcg tttagctcaa 5640
   tcataccgag agcgccgttt gctaactcag ccgtgcgttt tttatcgctt tgcagaagtt 5700
   tttgactttc ttgacggaag aatgatgtgc ttttgccata gtatgctttg ttaaataaag 5760
   attcttcgcc ttggtagcca tcttcagttc cagtgtttgc ttcaaatact aagtatttgt 5820
   ggcctttatc ttctacgtag tgaggatctc tcagcgtatg gttgtcgcct gagctgtagt 5880
   tgccttcatc gatgaactgc tgtacatttt gatacgtttt tccgtcaccg tcaaagattg 5940
   atttataatc ctctacaccg ttgatgttca aagagctgtc tgatgctgat acgttaactt 6000
   gtgcagttgt cagtgtttgt ttgccgtaat gtttaccgga gaaatcagtg tagaataaac 6060
   ggatttttcc gtcagatgta aatgtggctg aacctgacca ttcttgtgtt tggtctttta 6120
   ggatagaatc atttgcatcg aatttgtcgc tgtctttaaa gacgcggcca gcgtttttcc 6180
   agctgtcaat agaagtttcg ccgacttttt gatagaacat gtaaatcgat gtgtcatccg 6240
   catttttagg atctccggct aatgcaaaga cgatgtggta gccgtgatag tttgcgacag 6300
   tgccgtcagc gttttgtaat ggccagctgt cccaaacgtc caggcctttt gcagaagaga 6360
   tatttttaat tgtggacgaa tcaaattcag aaacttgata tttttcattt ttttgctgtt 6420
   cagggatttg cagcatatca tggcgtgtaa tatgggaaat gccgtatgtt tccttatatg 6480
   gcttttggtt cgtttctttc gcaaacgctt gagttgcgcc tcctgccagc agtgcggtag 6540
   taaaggttaa tactgttgct tgttttgcaa actttttgat gttcatcgtt catgtctcct 6600
   tttttatgta ctgtgttagc ggtctgcttc ttccagccct cctgtttgaa gatggcaagt 6660
   tagttacgca caataaaaaa agacctaaaa tatgtaaggg gtgacgccaa agtatacact 6720
   ttgcccttta cacattttag gtcttgcctg ctttatcagt aacaaacccg cgcgatttac 6780
   ttttcgacct cattctatta gactctcgtt tggattgcaa ctggtctatt ttcctctttt 6840
   gtttgataga aaatcataaa aggatttgca gactacgggc ctaaagaact aaaaaatcta   6900
   tctgtttctt ttcattctct gtatttttta tagtttctgt tgcatgggca taaagttgcc   6960
   tttttaatca caattcagaa aatatcataa tatctcattt cactaaataa tagtgaacgg   7020
   caggtatatg tgatgggtta aaaaggatcg gcggccgctc gatttaaatc              7070
<210> 25
   <211> 6625
   <212> DNA
   <213> artificial
<220>
   <223> Plasmid pH484
<400> 25
   tcgagaggcc tgacgtcggg cccggtaccg ttgctcgctg atctttcggc ttaacaactt 60
   tgtattcaat cagtcgggca tagaaagaaa acgcaatgat ataggaacca actgccgcca 120
   aaaccagcca cacagagttg attgtttcgc cacgggagaa agcgattgct ccccaaccca 180
   ccgccgcgat aaccccaaag acaaggagac caacgcgggc ggtcggtgac attttagggg 240
   acttcttcac gcctactgga aggtcagtag cgttgctgta caccaaatca tcgtcattga 300
   tgttgtcagt ctgttttatg gtcacgatct ttactgtttt ctcttcgggt cgtttcaaag 360
   ccactatgcg tagaaacagc gggcagaaac agcgggcaga aactgtgtgc agaaatgcat 420
   gcagaaaaag gaaagttcgg ccagatgggt gtttctgtat gccgatgatc ggatctttga 480
   cagctgggta tgcgacaaat caccgagagt tgttaattct taacaatgga aaagtaacat 540
   tgagagatga tttataccat cctgcaccat ttagagtggg gctagtcata cccccataac 600
   cctagctgta cgcaatcgat ttcaaatcag ttggaaaaag tcaagaaaat tacccgagac 660
   atatgcggct taaagtttgg ctgccatgtg aatttttagc accctcaaca gttgagtgct 720
   ggcactctcg agggtagagt gccaaatagg ttgtttgaca cacagttgtt cacccgcgac 780
   gacggctgtg ctggaaaccc acaaccggca cacacaaaat ttttctcatg gccgttaccc 840
   tgcgaatgtc cacagggtag ctggtagttt gaaaatcaac gccgttgccc ttaggattca 900
   gtaactggca cattttgtaa tgcgctagat ctgtgtgctc agtcttccag gctgcttatc 960
   acagtgaaag caaaaccaat tcgtggctgc gaaagtcgta gccaccacga agtccaggag 1020
   gacatacaat gccaaagtac gacaattcca atgctgacca gtggggcttt gaaacccgct 1080
   ccattcacgc aggccagtca gtagacgcac agaccagcgc acgaaacctt ccgatctacc 1140
   aatccaccgc tttcgtgttc gactccgctg agcacgccaa gcagcgtttc gcacttgagg 1200
   atctaggccc tgtttactcc cgcctcacca acccaaccgt tgaggctttg gaaaaccgca 1260
   tcgcttccct cgaaggtggc gtccacgctg tagcgttctc ctccggacag gccgcaacca 1320
   ccaacgccat tttgaacctg gcaggagcgg gcgaccacat cgtcacctcc ccacgcctct 1380
   acggtggcac cgagactcta ttccttatca ctcttaaccg cctgggtatc gatgtttcct 1440
   tcgtggaaaa ccccgacgac cctgagtcct ggcaggcagc cgttcagcca aacaccaaag 1500
   cattcttcgg cgagactttc gccaacccac aggcagacgt cctggatatt cctgcggtgg 1560
   ctgaagttgc gcaccgcaac agcgttccac tgatcatcga caacaccatc gctaccgcag 1620
   cgctcgtgcg cccgctcgag ctcggcgcag acgttgtcgt cgcttccctc accaagttct 1680
   acaccggcaa cggctccgga ctgggcggcg tgcttatcga cggcggaaag ttcgattgga 1740
   ctgtcgaaaa ggatggaaag ccagtattcc cctacttcgt cactccagat gctgcttacc 1800
   acggattgaa gtacgcagac cttggtgcac cagccttcgg cctcaaggtt cgcgttggcc 1860
   ttctacgcga caccggctcc accctctccg cattcaacgc atgggctgca gtccagggca 1920
   tcgacaccct ttccctgcgc ctggagcgcc acaacgaaaa cgccatcaag gttgcagaat 1980
   tcctcaacaa ccacgagaag gtggaaaagg ttaacttcgc aggcctgaag gattcccctt 2040
   ggtacgcaac caaggaaaag cttggcctga agtacaccgg ctccgttctc accttcgaga 2100
   tcaagggcgg caaggatgag gcttgggcat ttatcgacgc cctgaagcta cactccaacc 2160
   ttgcaaacat cggcgatgtt cgctccctcg ttgttcaccc agcaaccacc acccattcac 2220
   agtccgacga agctggcctg gcacgcgcgg gcgttaccca gtccaccgtc cgcctgtccg 2280
   ttggcatcga gaccattgat gatatcatcg ctgacctcga aggcggcttt gctgcaatct 2340
   agcactagtt cggacctagg gatatcgtcg acatcgatgc tcttctgcgt taattaacaa 2400
   ttgggatcct ctagacccgg gatttaaatc gctagcgggc tgctaaagga agcggaacac 2460
   gtagaaagcc agtccgcaga aacggtgctg accccggatg aatgtcagct actgggctat 2520
   ctggacaagg gaaaacgcaa gcgcaaagag aaagcaggta gcttgcagtg ggcttacatg 2580
   gcgatagcta gactgggcgg ttttatggac agcaagcgaa ccggaattgc cagctggggc 2640
   gccctctggt aaggttggga agccctgcaa agtaaactgg atggctttct tgccgccaag 2700
   gatctgatgg cgcaggggat caagatctga tcaagagaca ggatgaggat cgtttcgcat 2760
   gattgaacaa gatggattgc acgcaggttc tccggccgct tgggtggaga ggctattcgg 2820
   ctatgactgg gcacaacaga caatcggctg ctctgatgcc gccgtgttcc ggctgtcagc 2880
   gcaggggcgc ccggttcttt ttgtcaagac cgacctgtcc ggtgccctga atgaactgca 2940
   ggacgaggca gcgcggctat cgtggctggc cacgacgggc gttccttgcg cagctgtgct 3000
   cgacgttgtc actgaagcgg gaagggactg gctgctattg ggcgaagtgc cggggcagga 3060
   tctcctgtca tctcaccttg ctcctgccga gaaagtatcc atcatggctg atgcaatgcg 3120
   gcggctgcat acgcttgatc cggctacctg cccattcgac caccaagcga aacatcgcat 3180
   cgagcgagca cgtactcgga tggaagccgg tcttgtcgat caggatgatc tggacgaaga 3240
   gcatcagggg ctcgcgccag ccgaactgtt cgccaggctc aaggcgcgca tgcccgacgg 3300
   cgaggatctc gtcgtgaccc atggcgatgc ctgcttgccg aatatcatgg tggaaaatgg 3360
   ccgcttttct ggattcatcg actgtggccg gctgggtgtg gcggaccgct atcaggacat 3420
   agcgttggct acccgtgata ttgctgaaga gcttggcggc gaatgggctg accgcttcct 3480
   cgtgctttac ggtatcgccg ctcccgattc gcagcgcatc gccttctatc gccttcttga 3540
   cgagttcttc tgagcgggac tctggggttc gaaatgaccg accaagcgac gcccaacctg 3600
   ccatcacgag atttcgattc caccgccgcc ttctatgaaa ggttgggctt cggaatcgtt 3660
   ttccgggacg ccggctggat gatcctccag cgcggggatc tcatgctgga gttcttcgcc 3720
   cacgctagcg gcgcgccggc cggcccggtg tgaaataccg cacagatgcg taaggagaaa 3780
   ataccgcatc aggcgctctt ccgcttcctc gctcactgac tcgctgcgct cggtcgttcg 3840
   gctgcggcga gcggtatcag ctcactcaaa ggcggtaata cggttatcca cagaatcagg 3900
   ggataacgca ggaaagaaca tgtgagcaaa aggccagcaa aaggccagga accgtaaaaa 3960
   ggccgcgttg ctggcgtttt tccataggct ccgcccccct gacgagcatc acaaaaatcg 4020
   acgctcaagt cagaggtggc gaaacccgac aggactataa agataccagg cgtttccccc 4080
   tggaagctcc ctcgtgcgct ctcctgttcc gaccctgccg cttaccggat acctgtccgc 4140
   ctttctccct tcgggaagcg tggcgctttc tcatagctca cgctgtaggt atctcagttc 4200
   ggtgtaggtc gttcgctcca agctgggctg tgtgcacgaa ccccccgttc agcccgaccg 4260
   ctgcgcctta tccggtaact atcgtcttga gtccaacccg gtaagacacg acttatcgcc 4320
   actggcagca gccactggta acaggattag cagagcgagg tatgtaggcg gtgctacaga 4380
   gttcttgaag tggtggccta actacggcta cactagaagg acagtatttg gtatctgcgc 4440
   tctgctgaag ccagttacct tcggaaaaag agttggtagc tcttgatccg gcaaacaaac 4500
   caccgctggt agcggtggtt tttttgtttg caagcagcag attacgcgca gaaaaaaagg 4560
   atctcaagaa gatcctttga tcttttctac ggggtctgac gctcagtgga acgaaaactc 4620
   acgttaaggg attttggtca tgagattatc aaaaaggatc ttcacctaga tccttttaaa 4680
   ggccggccgc ggccgccatc ggcattttct tttgcgtttt tatttgttaa ctgttaattg 4740
   tccttgttca aggatgctgt ctttgacaac agatgttttc ttgcctttga tgttcagcag 4800
   gaagctcggc gcaaacgttg attgtttgtc tgcgtagaat cctctgtttg tcatatagct 4860
   tgtaatcacg acattgtttc ctttcgcttg aggtacagcg aagtgtgagt aagtaaaggt 4920
   tacatcgtta ggatcaagat ccatttttaa cacaaggcca gttttgttca gcggcttgta 4980
   tgggccagtt aaagaattag aaacataacc aagcatgtaa atatcgttag acgtaatgcc 5040
   gtcaatcgtc atttttgatc cgcgggagtc agtgaacagg taccatttgc cgttcatttt 5100
   aaagacgttc gcgcgttcaa tttcatctgt tactgtgtta gatgcaatca gcggtttcat 5160
   cacttttttc agtgtgtaat catcgtttag ctcaatcata ccgagagcgc cgtttgctaa 5220
   ctcagccgtg cgttttttat cgctttgcag aagtttttga ctttcttgac ggaagaatga 5280
   tgtgcttttg ccatagtatg ctttgttaaa taaagattct tcgccttggt agccatcttc 5340
   agttccagtg tttgcttcaa atactaagta tttgtggcct ttatcttcta cgtagtgagg 5400
   atctctcagc gtatggttgt cgcctgagct gtagttgcct tcatcgatga actgctgtac 5460
   attttgatac gtttttccgt caccgtcaaa gattgattta taatcctcta caccgttgat 5520
   gttcaaagag ctgtctgatg ctgatacgtt aacttgtgca gttgtcagtg tttgtttgcc 5580
   gtaatgttta ccggagaaat cagtgtagaa taaacggatt tttccgtcag atgtaaatgt 5640
   ggctgaacct gaccattctt gtgtttggtc ttttaggata gaatcatttg catcgaattt 5700
   gtcgctgtct ttaaagacgc ggccagcgtt tttccagctg tcaatagaag tttcgccgac 5760
   tttttgatag aacatgtaaa tcgatgtgtc atccgcattt ttaggatctc cggctaatgc 5820
   aaagacgatg tggtagccgt gatagtttgc gacagtgccg tcagcgtttt gtaatggcca 5880
   gctgtcccaa acgtccaggc cttttgcaga agagatattt ttaattgtgg acgaatcaaa 5940
   ttcagaaact tgatattttt catttttttg ctgttcaggg atttgcagca tatcatggcg 6000
   tgtaatatgg gaaatgccgt atgtttcctt atatggcttt tggttcgttt ctttcgcaaa 6060
   cgcttgagtt gcgcctcctg ccagcagtgc ggtagtaaag gttaatactg ttgcttgttt 6120
   tgcaaacttt ttgatgttca tcgttcatgt ctcctttttt atgtactgtg ttagcggtct 6180
   gcttcttcca gccctcctgt ttgaagatgg caagttagtt acgcacaata aaaaaagacc 6240
   taaaatatgt aaggggtgac gccaaagtat acactttgcc ctttacacat tttaggtctt 6300
   gcctgcttta tcagtaacaa acccgcgcga tttacttttc gacctcattc tattagactc 6360
   tcgtttggat tgcaactggt ctattttcct cttttgtttg atagaaaatc ataaaaggat 6420
   ttgcagacta cgggcctaaa gaactaaaaa atctatctgt ttcttttcat tctctgtatt 6480
   ttttatagtt tctgttgcat gggcataaag ttgccttttt aatcacaatt cagaaaatat 6540
   cataatatct catttcacta aataatagtg aacggcaggt atatgtgatg ggttaaaaag 6600
   gatcggcggc cgctcgattt aaatc 6625
<210> 26
   <211> 363
   <212> DNA
   <213> artificial
<220>
   <223> Sequence of the promotor P497_P1284 = PgrESPEFTu>P497_P1284
<400> 26
   cggcttaaag tttggctgcc atgtgaattt ttagcaccct caacagttga gtgctggcac     60
   tctcgagggt agagtgccaa ataggttgtt tgacacacag ttgttcaccc gcgacgacgg    120
   ctgtgctgga aacccacaac cggcacacac aaaatttttc tcatggccgt taccctgcga    180
   atgtccacag ggtagctggt agtttgaaaa tcaacgccgt tgcccttagg attcagtaac    240
   tggcacattt tgtaatgcgc tagatctgtg tgctcagtct tccaggctgc ttatcacagt    300
   gaaagcaaaa ccaattcgtg gctgcgaaag tcgtagccac cacgaagtcc aggaggacat    360
   aca                                                                  363
<210> 27
   <211> 6350
   <212> DNA
   <213> artificial
<220>
   <223> Plasmid pH491
<400> 27
   tcgagctcgg cgcagacgtt gtcgtcgctt ccctcaccaa gttctacacc ggcaacggct      60
   ccggactggg cggcgtgctt atcgacggcg gaaagttcga ttggactgtc gaaaaggatg     120
   gaaagccagt attcccctac ttcgtcactc cagatgctgc ttaccacgga ttgaagtacg     180
   cagaccttgg tgcaccagcc ttcggcctca aggttcgcgt tggccttcta cgcgacaccg     240
   gctccaccct ctccgcattc aacgcatggg ctgcagtcca gggcatcgac accctttccc     300
   tgcgcctgga gcgccacaac gaaaacgcca tcaaggttgc agaattcctc aacaaccacg     360
   agaaggtgga aaaggttaac ttcgcaggcc tgaaggattc cccttggtac gcaaccaagg     420
   aaaagcttgg cctgaagtac accggctccg ttctcacctt cgagatcaag ggcggcaagg     480
   atgaggcttg ggcatttatc gacgccctga agctacactc caaccttgca aacatcggcg     540
   atgttcgctc cctcgttgtt cacccagcaa ccaccaccca ttcacagtcc gacgaagctg     600
   gcctggcacg cgcgggcgtt acccagtcca ccgtccgcct gtccgttggc atcgagacca     660
   ttgatgatat catcgctgac ctcgaaggcg gctttgctgc aatctagcac tagttcggac     720
   ctagggatat cgtcgagagc tgccaattat tccgggcttg tgacccgcta cccgataaat     780
   aggtcggctg aaaaatttcg ttgcaatatc aacaaaaagg cctatcattg ggaggtgtcg     840
   caccaagtac ttttgcgaag cgccatctga cggattttca aaagatgtat atgctcggtg     900
   cggaaaccta cgaaaggatt ttttacccat gcccaccctc gcgccttcag gtcaacttga     960
   aatccaagcg atcggtgatg tctccaccga agccggagca atcattacaa acgctgaaat    1020
   cgcctatcac cgctggggtg aataccgcgt agataaagaa ggacgcagca atgtcgttct    1080
   catcgaacac gccctcactg gagattccaa cgcagccgat tggtgggctg acttgctcgg    1140
   tcccggcaaa gccatcaaca ctgatattta ctgcgtgatc tgtaccaacg tcatcggtgg    1200
   ttgcaacggt tccaccggac ctggctccat gcatccagat ggaaatttct ggggtaatcg    1260
   cttccccgcc acgtccattc gtgatcaggt aaacgccgaa aaacaattcc tcgacgcact    1320
   cggcatcacc acggtcgccg cagtacttgg tggttccatg ggtggtgccc gcaccctaga    1380
   gtgggccgca atgtacccag aaactgttgg cgcagctgct gttcttgcag tttctgcacg    1440
   cgccagcgcc tggcaaatcg gcattcaatc cgcccaaatt aaggcgattg aaaacgacca    1500
   ccactggcac gaaggcaact actacgaatc cggctgcaac ccagccaccg gactcggcgc    1560
   cgcccgacgc atcgcccacc tcacctaccg tggcgaacta gaaatcgacg aacgcttcgg 1620
   caccaaagcc caaaagaacg aaaacccact cggtccctac cgcaagcccg accagcgctt 1680
   cgccgtggaa tcctacttgg actaccaagc agacaagcta gtacagcgtt tcgacgccgg 1740
   ctcctacgtc ttgctcaccg acgccctcaa ccgccacgac attggtcgcg accgcggagg 1800
   cctcaacaag gcactcgaat ccatcaaagt tccagtcctt gtcgcaggcg tagataccga 1860
   tattttgtac ccctaccacc agcaagaaca cctctccaga aacctgggaa atctactggc 1920
   aatggcaaaa atcgtatccc ctgtcggcca cgatgctttc ctcaccgaaa gccgccaaat 1980
   ggatcgcatc gtgaggaact tcttcagcct catctcccca gacgaagaca acccttcgac 2040
   ctacatcgag ttctacatct aacatatgac tagttcggac ctagggatat cgtcgacatc 2100
   gatgctcttc tgcgttaatt aacaattggg atcctctaga cccgggattt aaatcgctag 2160
   cgggctgcta aaggaagcgg aacacgtaga aagccagtcc gcagaaacgg tgctgacccc 2220
   ggatgaatgt cagctactgg gctatctgga caagggaaaa cgcaagcgca aagagaaagc 2280
   aggtagcttg cagtgggctt acatggcgat agctagactg ggcggtttta tggacagcaa 2340
   gcgaaccgga attgccagct ggggcgccct ctggtaaggt tgggaagccc tgcaaagtaa 2400
   actggatggc tttcttgccg ccaaggatct gatggcgcag gggatcaaga tctgatcaag 2460
   agacaggatg aggatcgttt cgcatgattg aacaagatgg attgcacgca ggttctccgg 2520
   ccgcttgggt ggagaggcta ttcggctatg actgggcaca acagacaatc ggctgctctg 2580
   atgccgccgt gttccggctg tcagcgcagg ggcgcccggt tctttttgtc aagaccgacc 2640
   tgtccggtgc cctgaatgaa ctgcaggacg aggcagcgcg gctatcgtgg ctggccacga 2700
   cgggcgttcc ttgcgcagct gtgctcgacg ttgtcactga agcgggaagg gactggctgc 2760
   tattgggcga agtgccgggg caggatctcc tgtcatctca ccttgctcct gccgagaaag 2820
   tatccatcat ggctgatgca atgcggcggc tgcatacgct tgatccggct acctgcccat 2880
   tcgaccacca agcgaaacat cgcatcgagc gagcacgtac tcggatggaa gccggtcttg 2940
   tcgatcagga tgatctggac gaagagcatc aggggctcgc gccagccgaa ctgttcgcca 3000
   ggctcaaggc gcgcatgccc gacggcgagg atctcgtcgt gacccatggc gatgcctgct 3060
   tgccgaatat catggtggaa aatggccgct tttctggatt catcgactgt ggccggctgg 3120
   gtgtggcgga ccgctatcag gacatagcgt tggctacccg tgatattgct gaagagcttg 3180
   gcggcgaatg ggctgaccgc ttcctcgtgc tttacggtat cgccgctccc gattcgcagc 3240
   gcatcgcctt ctatcgcctt cttgacgagt tcttctgagc gggactctgg ggttcgaaat 3300
   gaccgaccaa gcgacgccca acctgccatc acgagatttc gattccaccg ccgccttcta 3360
   tgaaaggttg ggcttcggaa tcgttttccg ggacgccggc tggatgatcc tccagcgcgg 3420
   ggatctcatg ctggagttct tcgcccacgc tagcggcgcg ccggccggcc cggtgtgaaa 3480
   taccgcacag atgcgtaagg agaaaatacc gcatcaggcg ctcttccgct tcctcgctca   3540
   ctgactcgct gcgctcggtc gttcggctgc ggcgagcggt atcagctcac tcaaaggcgg   3600
   taatacggtt atccacagaa tcaggggata acgcaggaaa gaacatgtga gcaaaaggcc   3660
   agcaaaaggc caggaaccgt aaaaaggccg cgttgctggc gtttttccat aggctccgcc   3720
   cccctgacga gcatcacaaa aatcgacgct caagtcagag gtggcgaaac ccgacaggac   3780
   tataaagata ccaggcgttt ccccctggaa gctccctcgt gcgctctcct gttccgaccc   3840
   tgccgcttac cggatacctg tccgcctttc tcccttcggg aagcgtggcg ctttctcata   3900
   gctcacgctg taggtatctc agttcggtgt aggtcgttcg ctccaagctg ggctgtgtgc   3960
   acgaaccccc cgttcagccc gaccgctgcg ccttatccgg taactatcgt cttgagtcca   4020
   acccggtaag acacgactta tcgccactgg cagcagccac tggtaacagg attagcagag   4080
   cgaggtatgt aggcggtgct acagagttct tgaagtggtg gcctaactac ggctacacta   4140
   gaaggacagt atttggtatc tgcgctctgc tgaagccagt taccttcgga aaaagagttg   4200
   gtagctcttg atccggcaaa caaaccaccg ctggtagcgg tggttttttt gtttgcaagc   4260
   agcagattac gcgcagaaaa aaaggatctc aagaagatcc tttgatcttt tctacggggt   4320
   ctgacgctca gtggaacgaa aactcacgtt aagggatttt ggtcatgaga ttatcaaaaa   4380
   ggatcttcac ctagatcctt ttaaaggccg gccgcggccg ccatcggcat tttcttttgc    4440
   gtttttattt gttaactgtt aattgtcctt gttcaaggat gctgtctttg acaacagatg   4500
   ttttcttgcc tttgatgttc agcaggaagc tcggcgcaaa cgttgattgt ttgtctgcgt   4560
   agaatcctct gtttgtcata tagcttgtaa tcacgacatt gtttcctttc gcttgaggta   4620
   cagcgaagtg tgagtaagta aaggttacat cgttaggatc aagatccatt tttaacacaa   4680
   ggccagtttt gttcagcggc ttgtatgggc cagttaaaga attagaaaca taaccaagca   4740
   tgtaaatatc gttagacgta atgccgtcaa tcgtcatttt tgatccgcgg gagtcagtga   4800
   acaggtacca tttgccgttc attttaaaga cgttcgcgcg ttcaatttca tctgttactg   4860
   tgttagatgc aatcagcggt ttcatcactt ttttcagtgt gtaatcatcg tttagctcaa   4920
   tcataccgag agcgccgttt gctaactcag ccgtgcgttt tttatcgctt tgcagaagtt   4980
   tttgactttc ttgacggaag aatgatgtgc ttttgccata gtatgctttg ttaaataaag   5040
   attcttcgcc ttggtagcca tcttcagttc cagtgtttgc ttcaaatact aagtatttgt   5100
   ggcctttatc ttctacgtag tgaggatctc tcagcgtatg gttgtcgcct gagctgtagt   5160
   tgccttcatc gatgaactgc tgtacatttt gatacgtttt tccgtcaccg tcaaagattg   5220
   atttataatc ctctacaccg ttgatgttca aagagctgtc tgatgctgat acgttaactt   5280
   gtgcagttgt cagtgtttgt ttgccgtaat gtttaccgga gaaatcagtg tagaataaac   5340
   ggatttttcc gtcagatgta aatgtggctg aacctgacca ttcttgtgtt tggtctttta   5400
   ggatagaatc atttgcatcg aatttgtcgc tgtctttaaa gacgcggcca gcgtttttcc   5460
   agctgtcaat agaagtttcg ccgacttttt gatagaacat gtaaatcgat gtgtcatccg 5520
   catttttagg atctccggct aatgcaaaga cgatgtggta gccgtgatag tttgcgacag 5580
   tgccgtcagc gttttgtaat ggccagctgt cccaaacgtc caggcctttt gcagaagaga 5640
   tatttttaat tgtggacgaa tcaaattcag aaacttgata tttttcattt ttttgctgtt 5700
   cagggatttg cagcatatca tggcgtgtaa tatgggaaat gccgtatgtt tccttatatg 5760
   gcttttggtt cgtttctttc gcaaacgctt gagttgcgcc tcctgccagc agtgcggtag 5820
   taaaggttaa tactgttgct tgttttgcaa actttttgat gttcatcgtt catgtctcct 5880
   tttttatgta ctgtgttagc ggtctgcttc ttccagccct cctgtttgaa gatggcaagt 5940
   tagttacgca caataaaaaa agacctaaaa tatgtaaggg gtgacgccaa agtatacact 6000
   ttgcccttta cacattttag gtcttgcctg ctttatcagt aacaaacccg cgcgatttac 6060
   ttttcgacct cattctatta gactctcgtt tggattgcaa ctggtctatt ttcctctttt 6120
   gtttgataga aaatcataaa aggatttgca gactacgggc ctaaagaact aaaaaatcta 6180
   tctgtttctt ttcattctct gtatttttta tagtttctgt tgcatgggca taaagttgcc 6240
   tttttaatca caattcagaa aatatcataa tatctcattt cactaaataa tagtgaacgg 6300
   caggtatatg tgatgggtta aaaaggatcg gcggccgctc gatttaaatc 6350
<210> 28
   <211> 5477
   <212> DNA
   <213> artificial
<220>
   <223> Plasmid pH429
<400> 28
   tcgagctctc caatctccac tgaggtactt aatccttccg gggaattcgg gcgcttaaat      60
   cgagaaatta ggccatcacc ttttaataac aatacaatga ataattggaa taggtcgaca     120
   cctttggagc ggagccggtt aaaattggca gcattcaccg aaagaaaagg agaaccacat     180
   gcttgcccta ggttggatta catggatcat tattggtggt ctagctggtt ggattgcctc     240
   caagattaaa ggcactgatg ctcagcaagg aattttgctg aacatagtcg tcggtattat     300
   cggtggtttg ttaggcggct ggctgcttgg aatcttcgga gtggatgttg ccggtggcgg     360
   cttgatcttc agcttcatca catgtctgat tggtgctgtc attttgctga cgatcgtgca     420
   gttcttcact cggaagaagt aatctgcttt aaatccgtag ggcctgttga tatttcgata     480
   tcaacaggcc ttttggtcat tttggggtgg aaaaagcgct agacttgcct gtggattaaa     540
   actatacgaa ccggtttgtc tatattggtg ttagacagtt cgtcgtatct tgaaacagac     600
   caacccgaaa ggacgtggcc gaacgtggct gctagctaat ccttgatggt ggacttgctg     660
   gatctcgatt ggtccacaac atcagtcctc ttgagacggc tcgcgatttg gctcggcagt     720
   tgttgtcggc tccacctgcg gactactcaa tttagtttct tcattttccg aaggggtatc 780
   ttcgttgggg gaggcgtcga taagcccctt ctttttagct ttaacctcag cgcgacgctg 840
   ctttaagcgc tgcatggcgg cgcggttcat ttcacgttgc gtttcgcgcc tcttgttcgc 900
   gatttctttg cgggcctgtt ttgcttcgtt gatttcggca gtacgggttt tggtgagttc 960
   cacgtttgtt gcgtgaagcg ttgaggcgtt ccatggggtg agaatcatca gggcgcggtt 1020
   tttgcgtcgt gtccacagga agatgcgctt ttctttttgt tttgcgcggt agatgtcgcg 1080
   ctgctctagg tggtgcactt tgaaatcgtc ggtaagtggg tatttgcgtt ccaaaatgac 1140
   catcatgatg attgtttgga ggagcgtcca caggttgttg ctgacgcgtc atatgactag 1200
   ttcggaccta gggatatcgt cgacatcgat gctcttctgc gttaattaac aattgggatc 1260
   ctctagaccc gggatttaaa tcgctagcgg gctgctaaag gaagcggaac acgtagaaag 1320
   ccagtccgca gaaacggtgc tgaccccgga tgaatgtcag ctactgggct atctggacaa 1380
   gggaaaacgc aagcgcaaag agaaagcagg tagcttgcag tgggcttaca tggcgatagc 1440
   tagactgggc ggttttatgg acagcaagcg aaccggaatt gccagctggg gcgccctctg 1500
   gtaaggttgg gaagccctgc aaagtaaact ggatggcttt cttgccgcca aggatctgat 1560
   ggcgcagggg atcaagatct gatcaagaga caggatgagg atcgtttcgc atgattgaac 1620
   aagatggatt gcacgcaggt tctccggccg cttgggtgga gaggctattc ggctatgact 1680
   gggcacaaca gacaatcggc tgctctgatg ccgccgtgtt ccggctgtca gcgcaggggc 1740
   gcccggttct ttttgtcaag accgacctgt ccggtgccct gaatgaactg caggacgagg 1800
   cagcgcggct atcgtggctg gccacgacgg gcgttccttg cgcagctgtg ctcgacgttg 1860
   tcactgaagc gggaagggac tggctgctat tgggcgaagt gccggggcag gatctcctgt 1920
   catctcacct tgctcctgcc gagaaagtat ccatcatggc tgatgcaatg cggcggctgc 1980
   atacgcttga tccggctacc tgcccattcg accaccaagc gaaacatcgc atcgagcgag 2040
   cacgtactcg gatggaagcc ggtcttgtcg atcaggatga tctggacgaa gagcatcagg 2100
   ggctcgcgcc agccgaactg ttcgccaggc tcaaggcgcg catgcccgac ggcgaggatc 2160
   tcgtcgtgac ccatggcgat gcctgcttgc cgaatatcat ggtggaaaat ggccgctttt 2220
   ctggattcat cgactgtggc cggctgggtg tggcggaccg ctatcaggac atagcgttgg 2280
   ctacccgtga tattgctgaa gagcttggcg gcgaatgggc tgaccgcttc ctcgtgcttt 2340
   acggtatcgc cgctcccgat tcgcagcgca tcgccttcta tcgccttctt gacgagttct 2400
   tctgagcggg actctggggt tcgaaatgac cgaccaagcg acgcccaacc tgccatcacg 2460
   agatttcgat tccaccgccg ccttctatga aaggttgggc ttcggaatcg ttttccggga 2520
   cgccggctgg atgatcctcc agcgcgggga tctcatgctg gagttcttcg cccacgctag 2580
   cggcgcgccg gccggcccgg tgtgaaatac cgcacagatg cgtaaggaga aaataccgca 2640
   tcaggcgctc ttccgcttcc tcgctcactg actcgctgcg ctcggtcgtt cggctgcggc 2700
   gagcggtatc agctcactca aaggcggtaa tacggttatc cacagaatca ggggataacg 2760
   caggaaagaa catgtgagca aaaggccagc aaaaggccag gaaccgtaaa aaggccgcgt 2820
   tgctggcgtt tttccatagg ctccgccccc ctgacgagca tcacaaaaat cgacgctcaa 2880
   gtcagaggtg gcgaaacccg acaggactat aaagatacca ggcgtttccc cctggaagct 2940
   ccctcgtgcg ctctcctgtt ccgaccctgc cgcttaccgg atacctgtcc gcctttctcc 3000
   cttcgggaag cgtggcgctt tctcatagct cacgctgtag gtatctcagt tcggtgtagg 3060
   tcgttcgctc caagctgggc tgtgtgcacg aaccccccgt tcagcccgac cgctgcgcct 3120
   tatccggtaa ctatcgtctt gagtccaacc cggtaagaca cgacttatcg ccactggcag 3180
   cagccactgg taacaggatt agcagagcga ggtatgtagg cggtgctaca gagttcttga 3240
   agtggtggcc taactacggc tacactagaa ggacagtatt tggtatctgc gctctgctga 3300
   agccagttac cttcggaaaa agagttggta gctcttgatc cggcaaacaa accaccgctg 3360
   gtagcggtgg tttttttgtt tgcaagcagc agattacgcg cagaaaaaaa ggatctcaag 3420
   aagatccttt gatcttttct acggggtctg acgctcagtg gaacgaaaac tcacgttaag 3480
   ggattttggt catgagatta tcaaaaagga tcttcaccta gatcctttta aaggccggcc 3540
   gcggccgcca tcggcatttt cttttgcgtt tttatttgtt aactgttaat tgtccttgtt 3600
   caaggatgct gtctttgaca acagatgttt tcttgccttt gatgttcagc aggaagctcg 3660
   gcgcaaacgt tgattgtttg tctgcgtaga atcctctgtt tgtcatatag cttgtaatca 3720
   cgacattgtt tcctttcgct tgaggtacag cgaagtgtga gtaagtaaag gttacatcgt 3780
   taggatcaag atccattttt aacacaaggc cagttttgtt cagcggcttg tatgggccag 3840
   ttaaagaatt agaaacataa ccaagcatgt aaatatcgtt agacgtaatg ccgtcaatcg 3900
   tcatttttga tccgcgggag tcagtgaaca ggtaccattt gccgttcatt ttaaagacgt 3960
   tcgcgcgttc aatttcatct gttactgtgt tagatgcaat cagcggtttc atcacttttt 4020
   tcagtgtgta atcatcgttt agctcaatca taccgagagc gccgtttgct aactcagccg 4080
   tgcgtttttt atcgctttgc agaagttttt gactttcttg acggaagaat gatgtgcttt 4140
   tgccatagta tgctttgtta aataaagatt cttcgccttg gtagccatct tcagttccag 4200
   tgtttgcttc aaatactaag tatttgtggc ctttatcttc tacgtagtga ggatctctca 4260
   gcgtatggtt gtcgcctgag ctgtagttgc cttcatcgat gaactgctgt acattttgat 4320
   acgtttttcc gtcaccgtca aagattgatt tataatcctc tacaccgttg atgttcaaag 4380
   agctgtctga tgctgatacg ttaacttgtg cagttgtcag tgtttgtttg ccgtaatgtt 4440
   taccggagaa atcagtgtag aataaacgga tttttccgtc agatgtaaat gtggctgaac 4500
   ctgaccattc ttgtgtttgg tcttttagga tagaatcatt tgcatcgaat ttgtcgctgt 4560
   ctttaaagac gcggccagcg tttttccagc tgtcaataga agtttcgccg actttttgat 4620
   agaacatgta aatcgatgtg tcatccgcat ttttaggatc tccggctaat gcaaagacga 4680
   tgtggtagcc gtgatagttt gcgacagtgc cgtcagcgtt ttgtaatggc cagctgtccc 4740
   aaacgtccag gccttttgca gaagagatat ttttaattgt ggacgaatca aattcagaaa 4800
   cttgatattt ttcatttttt tgctgttcag ggatttgcag catatcatgg cgtgtaatat 4860
   gggaaatgcc gtatgtttcc ttatatggct tttggttcgt ttctttcgca aacgcttgag 4920
   ttgcgcctcc tgccagcagt gcggtagtaa aggttaatac tgttgcttgt tttgcaaact 4980
   ttttgatgtt catcgttcat gtctcctttt ttatgtactg tgttagcggt ctgcttcttc 5040
   cagccctcct gtttgaagat ggcaagttag ttacgcacaa taaaaaaaga cctaaaatat 5100
   gtaaggggtg acgccaaagt atacactttg ccctttacac attttaggtc ttgcctgctt 5160
   tatcagtaac aaacccgcgc gatttacttt tcgacctcat tctattagac tctcgtttgg 5220
   attgcaactg gtctattttc ctcttttgtt tgatagaaaa tcataaaagg atttgcagac 5280
   tacgggccta aagaactaaa aaatctatct gtttcttttc attctctgta ttttttatag 5340
   tttctgttgc atgggcataa agttgccttt ttaatcacaa ttcagaaaat atcataatat 5400
   ctcatttcac taaataatag tgaacggcag gtatatgtga tgggttaaaa aggatcggcg 5460
   gccgctcgat ttaaatc 5477
<210> 29
   <211> 5697
   <212> DNA
   <213> artificial
<220>
   <223> Plasmid pH449
<400> 29
   tcgaggcgtc ttccggtgtc atggttgaac cgaattccag cacaatattt tccggtttaa    60
   agcaatcgat cacatagtcg attttgtcca accactgaaa acctgcaagg accacccaat   120
   cccctgcagc atgttcagca accattggca gcggcggata gcgaacttcc cccttttctc   180
   ccgttgccat tttcgcgtca ctgatcaggt gactgagctt tttgtagcct tccggatttt   240
   tacacaagac tgtcaacacg ccttcttgca gactcagctc cgcaccataa acggtatgca   300
   ttccagcttc cgcggcagct tccgcaaatc tcactgcacc ataaaaacca tccctatcca   360
   tgactgatag agcaacaagt cctaactttt tggcctgcac aaccacatca gacggatccg   420
   atgcgccagt gagaaagtta taactgctgg tggcatgcag ctcggcaaaa ggaaccgacg   480
   cttccccctg catggcagat gaaggcgcct gcgcatccgg ctcatgcagc accggacgca   540
   gagattcgac ctttttacct gagaggattc tttccaattt ggaccacgat aatggcctgc   600
   cgttaaagct tcccccgcca ttccattcca taatgatagg atacattttt agaacaaatt   660
   ttccaataag ttttccacgc cagccggaga aggaaataga ccaagctgta cagatcgacg   720
   cgtcctggct gagtacaacg tcggctccgg cgcagacctc accccagttg gctccagcga   780
   aatcgtgcca ctggcactat tctggaagga ccacgactcc atcgacggca ttgacggcga 840
   gtccgttgcc atccctaacg atccttccaa ccagggccgc gccatcaacg ttctcgttca 900
   ggcaggtctg gtcaccctga agaccccagg tctggtcacc ccagctccag tcgatatcga 960
   cgaggcagct tccaaggttt ccgtcatccc agtcgacgca gctcaggcac caaccgctta 1020
   ccaggagggt cgcccagcga tcatcaacaa ctccttcctt gaccgcgcag gcatcgatcc 1080
   aaacctcgcg gtcttcgaag atgatcctga gtctgaagaa gcagagccat acatcaacgt 1140
   cttcgtcacc aaggctgagg acaaggacga tgccaacatc gcccgcctcg ttgagctgtg 1200
   gcacgaccca gaggttctgg ctgcagtaga ccgcgactct gagggcacct ccgtcccagt 1260
   tgatcgtcca ggagctgacc ttcaggaaat ccttgatcgc cttgaggctg atcaggaaaa 1320
   cgcataatct cttttgagtt ctttgcatac ccatgtgcag atttctttgc acaatcacag 1380
   cctgaaaatc agactgtgaa cttcaaacgc atatgactag ttcggaccta gggatatcgt 1440
   cgacatcgat gctcttctgc gttaattaac aattgggatc ctctagaccc gggatttaaa 1500
   tcgctagcgg gctgctaaag gaagcggaac acgtagaaag ccagtccgca gaaacggtgc 1560
   tgaccccgga tgaatgtcag ctactgggct atctggacaa gggaaaacgc aagcgcaaag 1620
   agaaagcagg tagcttgcag tgggcttaca tggcgatagc tagactgggc ggttttatgg 1680
   acagcaagcg aaccggaatt gccagctggg gcgccctctg gtaaggttgg gaagccctgc 1740
   aaagtaaact ggatggcttt cttgccgcca aggatctgat ggcgcagggg atcaagatct 1800
   gatcaagaga caggatgagg atcgtttcgc atgattgaac aagatggatt gcacgcaggt 1860
   tctccggccg cttgggtgga gaggctattc ggctatgact gggcacaaca gacaatcggc 1920
   tgctctgatg ccgccgtgtt ccggctgtca gcgcaggggc gcccggttct ttttgtcaag 1980
   accgacctgt ccggtgccct gaatgaactg caggacgagg cagcgcggct atcgtggctg 2040
   gccacgacgg gcgttccttg cgcagctgtg ctcgacgttg tcactgaagc gggaagggac 2100
   tggctgctat tgggcgaagt gccggggcag gatctcctgt catctcacct tgctcctgcc 2160
   gagaaagtat ccatcatggc tgatgcaatg cggcggctgc atacgcttga tccggctacc 2220
   tgcccattcg accaccaagc gaaacatcgc atcgagcgag cacgtactcg gatggaagcc 2280
   ggtcttgtcg atcaggatga tctggacgaa gagcatcagg ggctcgcgcc agccgaactg 2340
   ttcgccaggc tcaaggcgcg catgcccgac ggcgaggatc tcgtcgtgac ccatggcgat 2400
   gcctgcttgc cgaatatcat ggtggaaaat ggccgctttt ctggattcat cgactgtggc 2460
   cggctgggtg tggcggaccg ctatcaggac atagcgttgg ctacccgtga tattgctgaa 2520
   gagcttggcg gcgaatgggc tgaccgcttc ctcgtgcttt acggtatcgc cgctcccgat 2580
   tcgcagcgca tcgccttcta tcgccttctt gacgagttct tctgagcggg actctggggt 2640
   tcgaaatgac cgaccaagcg acgcccaacc tgccatcacg agatttcgat tccaccgccg 2700
   ccttctatga aaggttgggc ttcggaatcg ttttccggga cgccggctgg atgatcctcc 2760
   agcgcgggga tctcatgctg gagttcttcg cccacgctag cggcgcgccg gccggcccgg 2820
   tgtgaaatac cgcacagatg cgtaaggaga aaataccgca tcaggcgctc ttccgcttcc 2880
   tcgctcactg actcgctgcg ctcggtcgtt cggctgcggc gagcggtatc agctcactca 2940
   aaggcggtaa tacggttatc cacagaatca ggggataacg caggaaagaa catgtgagca 3000
   aaaggccagc aaaaggccag gaaccgtaaa aaggccgcgt tgctggcgtt tttccatagg 3060
   ctccgccccc ctgacgagca tcacaaaaat cgacgctcaa gtcagaggtg gcgaaacccg 3120
   acaggactat aaagatacca ggcgtttccc cctggaagct ccctcgtgcg ctctcctgtt 3180
   ccgaccctgc cgcttaccgg atacctgtcc gcctttctcc cttcgggaag cgtggcgctt 3240
   tctcatagct cacgctgtag gtatctcagt tcggtgtagg tcgttcgctc caagctgggc 3300
   tgtgtgcacg aaccccccgt tcagcccgac cgctgcgcct tatccggtaa ctatcgtctt 3360
   gagtccaacc cggtaagaca cgacttatcg ccactggcag cagccactgg taacaggatt 3420
   agcagagcga ggtatgtagg cggtgctaca gagttcttga agtggtggcc taactacggc 3480
   tacactagaa ggacagtatt tggtatctgc gctctgctga agccagttac cttcggaaaa 3540
   agagttggta gctcttgatc cggcaaacaa accaccgctg gtagcggtgg tttttttgtt 3600
   tgcaagcagc agattacgcg cagaaaaaaa ggatctcaag aagatccttt gatcttttct 3660
   acggggtctg acgctcagtg gaacgaaaac tcacgttaag ggattttggt catgagatta 3720
   tcaaaaagga tcttcaccta gatcctttta aaggccggcc gcggccgcca tcggcatttt 3780
   cttttgcgtt tttatttgtt aactgttaat tgtccttgtt caaggatgct gtctttgaca 3840
   acagatgttt tcttgccttt gatgttcagc aggaagctcg gcgcaaacgt tgattgtttg 3900
   tctgcgtaga atcctctgtt tgtcatatag cttgtaatca cgacattgtt tcctttcgct 3960
   tgaggtacag cgaagtgtga gtaagtaaag gttacatcgt taggatcaag atccattttt 4020
   aacacaaggc cagttttgtt cagcggcttg tatgggccag ttaaagaatt agaaacataa 4080
   ccaagcatgt aaatatcgtt agacgtaatg ccgtcaatcg tcatttttga tccgcgggag 4140
   tcagtgaaca ggtaccattt gccgttcatt ttaaagacgt tcgcgcgttc aatttcatct 4200
   gttactgtgt tagatgcaat cagcggtttc atcacttttt tcagtgtgta atcatcgttt 4260
   agctcaatca taccgagagc gccgtttgct aactcagccg tgcgtttttt atcgctttgc 4320
   agaagttttt gactttcttg acggaagaat gatgtgcttt tgccatagta tgctttgtta 4380
   aataaagatt cttcgccttg gtagccatct tcagttccag tgtttgcttc aaatactaag 4440
   tatttgtggc ctttatcttc tacgtagtga ggatctctca gcgtatggtt gtcgcctgag 4500
   ctgtagttgc cttcatcgat gaactgctgt acattttgat acgtttttcc gtcaccgtca 4560
   aagattgatt tataatcctc tacaccgttg atgttcaaag agctgtctga tgctgatacg 4620
   ttaacttgtg cagttgtcag tgtttgtttg ccgtaatgtt taccggagaa atcagtgtag 4680
   aataaacgga tttttccgtc agatgtaaat gtggctgaac ctgaccattc ttgtgtttgg 4740
   tcttttagga tagaatcatt tgcatcgaat ttgtcgctgt ctttaaagac gcggccagcg 4800
   tttttccagc tgtcaataga agtttcgccg actttttgat agaacatgta aatcgatgtg 4860
   tcatccgcat ttttaggatc tccggctaat gcaaagacga tgtggtagcc gtgatagttt 4920
   gcgacagtgc cgtcagcgtt ttgtaatggc cagctgtccc aaacgtccag gccttttgca 4980
   gaagagatat ttttaattgt ggacgaatca aattcagaaa cttgatattt ttcatttttt 5040
   tgctgttcag ggatttgcag catatcatgg cgtgtaatat gggaaatgcc gtatgtttcc 5100
   ttatatggct tttggttcgt ttctttcgca aacgcttgag ttgcgcctcc tgccagcagt 5160
   gcggtagtaa aggttaatac tgttgcttgt tttgcaaact ttttgatgtt catcgttcat 5220
   gtctcctttt ttatgtactg tgttagcggt ctgcttcttc cagccctcct gtttgaagat 5280
   ggcaagttag ttacgcacaa taaaaaaaga cctaaaatat gtaaggggtg acgccaaagt 5340
   atacactttg ccctttacac attttaggtc ttgcctgctt tatcagtaac aaacccgcgc 5400
   gatttacttt tcgacctcat tctattagac tctcgtttgg attgcaactg gtctattttc 5460
   ctcttttgtt tgatagaaaa tcataaaagg atttgcagac tacgggccta aagaactaaa 5520
   aaatctatct gtttcttttc attctctgta ttttttatag tttctgttgc atgggcataa 5580
   agttgccttt ttaatcacaa ttcagaaaat atcataatat ctcatttcac taaataatag 5640
   tgaacggcag gtatatgtga tgggttaaaa aggatcggcg gccgctcgat ttaaatc 5697
<210> 30
   <211> 7318
   <212> DNA
   <213> artificial
<220>
   <223> Plasmid pOM427
<400> 30
   ggccgctcga tttaaatctc gagctctgga gtgcgacagg tttgatgata aaaaattagc      60
   gcaagaagac aaaaatcacc ttgcgctaat gctctgttac aggtcactaa taccatctaa     120
   gtagttgatt catagtgact gcatatgtaa gtatttcctt agataacaat tgattgaatg     180
   tatgcaaata aatgcataca ccataggtgt ggtttaattt gatgcccttt ttcagggctg     240
   gaatgtgtaa gagcggggtt atttatgctg ttgttttttt gttactcggg aagggcttta     300
   cctcttccgc ataaacgctt ccatcagcgt ttatagttaa aaaaatcttt cggggggatg     360
   gggagtaagc ttgtgttatc cgctcgggcc caatccgcaa gctccaccga ctcgttggcg     420
   tgcgactcta gataaatatc aagcagctgg ccgccaataa cctcagtacg catgccacgc     480
   caagcatccc tcgtgcgggc caatgcctct gcactcaaac cggaatcctg cagcatgtct     540
   tctgcccaca ccaatgccat atcgccagcc aaaatcgaga ctgaaacgcc aaagtgctcg     600
   ggatcgcctt cgaaattatt ggcgcggtga tcagcttcca cagcccggtg aactgtgggg 660
   gctccgcgcc gggtatcaga agaatcgata atatcgtcat gaatcaaggc acaagcctgg 720
   atgaattcga gactcgctgc ggcgtcaagg acggactcaa gtttttcaga agaattctta 780
   tggccttgcg ccgccaggaa accagcccac gcataaagag gacggattcg ctttcctcca 840
   ttgagcacga aactgcgaag atgggccaca gcatctgtga caggagcgcc gatatcagca 900
   attgttagct cttgagcatc gaggaactgc gtcaaacgat ctcgcacgac ctccggaaat 960
   ttgtcgaggt caaggtcatg ggcatcgaaa ctgctcaagg agacgtcctt caatcgaata 1020
   gggggatgcg ggctgaattt tggtggaggt gaataaatgc cagaggcagt cccaacaaaa 1080
   cactctcatc acactaagat acccgtcgac tcatacgtta aatctatcac cgcaagggat 1140
   aaatatctaa caccgtgcgt gttgactatt ttacctctgg cggtgataat ggttgcatgt 1200
   actaaggagg attaattaat gtccctaacg aacatcccag cctcatctca atgggcaatt 1260
   agcgacgttt tgaagcgtcc ttcacccggc cgagtacctt tttctgtcga gtttatgcca 1320
   ccccgcgacg atgcagctga agagcgtctt taccgcgcag cagaggtctt ccatgacctc 1380
   ggtgcatcgt ttgtctccgt gacttatggt gctggcggat caacccgtga gagaacctca 1440
   cgtattgctc gacgattagc gaaacaaccg ttgaccactc tggtgcacct gaccctggtt 1500
   aaccacactc gcgaagagat gaaggcaatt cttcgggaat acctagagct gggattaaca 1560
   aacctgttgg cgcttcgagg agatccgcct ggagacccat taggcgattg ggtgagcacc 1620
   gatggaggac tgaactatgc ctctgagctc atcgatctta ttaagtccac tcctgagttc 1680
   cgggaattcg acctcggtat cgcctccttc cccgaagggc atttccgggc gaaaactcta 1740
   gaagaagaca ccaaatacac tctggcgaag ctgcgtggag gggcagagta ctccatcacg 1800
   cagatgttct ttgatgtgga agactacctg cgacttcgtg atcgccggat cctgttttgg 1860
   cggatgagag aagattttca gcctgataca gattaaatca gaacgcagaa gcggtctgat 1920
   aaaacagaat ttgcctggcg gcagtagcgc ggtggtccca cctgacccca tgccgaactc 1980
   agaagtgaaa cgccgtagcg ccgatggtag tgtggggtct ccccatgcga gagtagggaa 2040
   ctgccaggca tcaaataaaa cgaaaggctc agtcgaaaga ctgggccttt cgttttatct 2100
   gttgtttgtc ggtgaacgct ctcctgagta ggacaaatcc gccgggagcg gatttgaacg 2160
   ttgcgaagca acggcccgga gggtggcggg caggacgccc gccataaact gccaggcatc 2220
   aaattaagca gaaggccatc ctgacggatg gcctttttgc gtttctacaa actcttggta 2280
   cgggatttaa atgatccgct agcgggctgc taaaggaagc ggaacacgta gaaagccagt 2340
   ccgcagaaac ggtgctgacc ccggatgaat gtcagctact gggctatctg gacaagggaa 2400
   aacgcaagcg caaagagaaa gcaggtagct tgcagtgggc ttacatggcg atagctagac 2460
   tgggcggttt tatggacagc aagcgaaccg gaattgccag ctggggcgcc ctctggtaag 2520
   gttgggaagc cctgcaaagt aaactggatg gctttcttgc cgccaaggat ctgatggcgc 2580
   aggggatcaa gatctgatca agagacagga tgaggatcgt ttcgcatgat tgaacaagat 2640
   ggattgcacg caggttctcc ggccgcttgg gtggagaggc tattcggcta tgactgggca 2700
   caacagacaa tcggctgctc tgatgccgcc gtgttccggc tgtcagcgca ggggcgcccg 2760
   gttctttttg tcaagaccga cctgtccggt gccctgaatg aactgcagga cgaggcagcg 2820
   cggctatcgt ggctggccac gacgggcgtt ccttgcgcag ctgtgctcga cgttgtcact 2880
   gaagcgggaa gggactggct gctattgggc gaagtgccgg ggcaggatct cctgtcatct 2940
   caccttgctc ctgccgagaa agtatccatc atggctgatg caatgcggcg gctgcatacg 3000
   cttgatccgg ctacctgccc attcgaccac caagcgaaac atcgcatcga gcgagcacgt 3060
   actcggatgg aagccggtct tgtcgatcag gatgatctgg acgaagagca tcaggggctc 3120
   gcgccagccg aactgttcgc caggctcaag gcgcgcatgc ccgacggcga ggatctcgtc 3180
   gtgacccatg gcgatgcctg cttgccgaat atcatggtgg aaaatggccg cttttctgga 3240
   ttcatcgact gtggccggct gggtgtggcg gaccgctatc aggacatagc gttggctacc 3300
   cgtgatattg ctgaagagct tggcggcgaa tgggctgacc gcttcctcgt gctttacggt 3360
   atcgccgctc ccgattcgca gcgcatcgcc ttctatcgcc ttcttgacga gttcttctga 3420
   gcgggactct ggggttcgaa atgaccgacc aagcgacgcc caacctgcca tcacgagatt 3480
   tcgattccac cgccgccttc tatgaaaggt tgggcttcgg aatcgttttc cgggacgccg 3540
   gctggatgat cctccagcgc ggggatctca tgctggagtt cttcgcccac gctagcggcg 3600
   cgccacgggt gcgcatgatc gtgctcctgt cgttgaggac ccggctaggc tggcggggtt 3660
   gccttactgg ttagcagaat gaatcaccga tacgcgagcg aacgtgaagc gactgctgct 3720
   gcaaaacgtc tgcgacctga gcaacaacat gaatggtctt cggtttccgt gtttcgtaaa 3780
   gtctggaaac gcggaagtca gcgccctgca ccattatgtt ccggatctgc atcgcaggat 3840
   gctgctggct accctgtgga acacctacat ctgtattaac gaagcgctgg cattgaccct 3900
   gagtgatttt tctctggtcc cgccgcatcc ataccgccag ttgtttaccc tcacaacgtt 3960
   ccagtaaccg ggcatgttca tcatcagtaa cccgtatcgt gagcatcctc tctcgtttca 4020
   tcggtatcat tacccccatg aacagaaatc ccccttacac ggaggcatca gtgaccaaac 4080
   aggaaaaaac cgcccttaac atggcccgct ttatcagaag ccagacatta acgcttctgg 4140
   agaaactcaa cgagctggac gcggatgaac aggcagacat ctgtgaatcg cttcacgacc 4200
   acgctgatga gctttaccgc agctgcctcg cgcgtttcgg tgatgacggt gaaaacctct 4260
   gacacatgca gctcccggag acggtcacag cttgtctgta agcggatgcc gggagcagac 4320
   aagcccgtca gggcgcgtca gcgggtgttg gcgggtgtcg gggcgcagcc atgacccagt 4380
   cacgtagcga tagcggagtg tatactggct taactatgcg gcatcagagc agattgtact 4440
   gagagtgcac catatgcggt gtgaaatacc gcacagatgc gtaaggagaa aataccgcat 4500
   caggcgctct tccgcttcct cgctcactga ctcgctgcgc tcggtcgttc ggctgcggcg 4560
   agcggtatca gctcactcaa aggcggtaat acggttatcc acagaatcag gggataacgc 4620
   aggaaagaac atgtgagcaa aaggccagca aaaggccagg aaccgtaaaa aggccgcgtt 4680
   gctggcgttt ttccataggc tccgcccccc tgacgagcat cacaaaaatc gacgctcaag 4740
   tcagaggtgg cgaaacccga caggactata aagataccag gcgtttcccc ctggaagctc 4800
   cctcgtgcgc tctcctgttc cgaccctgcc gcttaccgga tacctgtccg cctttctccc 4860
   ttcgggaagc gtggcgcttt ctcatagctc acgctgtagg tatctcagtt cggtgtaggt 4920
   cgttcgctcc aagctgggct gtgtgcacga accccccgtt cagcccgacc gctgcgcctt 4980
   atccggtaac tatcgtcttg agtccaaccc ggtaagacac gacttatcgc cactggcagc 5040
   agccactggt aacaggatta gcagagcgag gtatgtaggc ggtgctacag agttcttgaa 5100
   gtggtggcct aactacggct acactagaag gacagtattt ggtatctgcg ctctgctgaa 5160
   gccagttacc ttcggaaaaa gagttggtag ctcttgatcc ggcaaacaaa ccaccgctgg 5220
   tagcggtggt ttttttgttt gcaagcagca gattacgcgc agaaaaaaag gatctcaaga 5280
   agatcctttg atcttttcta cggggtctga cgctcagtgg aacgaaaact cacgttaagg 5340
   gattttggtc atgagattat caaaaaggat cttcacctag atccttttaa aggccggccg 5400
   cggccgccat cggcattttc ttttgcgttt ttatttgtta actgttaatt gtccttgttc 5460
   aaggatgctg tctttgacaa cagatgtttt cttgcctttg atgttcagca ggaagctcgg 5520
   cgcaaacgtt gattgtttgt ctgcgtagaa tcctctgttt gtcatatagc ttgtaatcac 5580
   gacattgttt cctttcgctt gaggtacagc gaagtgtgag taagtaaagg ttacatcgtt 5640
   aggatcaaga tccattttta acacaaggcc agttttgttc agcggcttgt atgggccagt 5700
   taaagaatta gaaacataac caagcatgta aatatcgtta gacgtaatgc cgtcaatcgt 5760
   catttttgat ccgcgggagt cagtgaacag gtaccatttg ccgttcattt taaagacgtt 5820
   cgcgcgttca atttcatctg ttactgtgtt agatgcaatc agcggtttca tcactttttt 5880
   cagtgtgtaa tcatcgttta gctcaatcat accgagagcg ccgtttgcta actcagccgt 5940
   gcgtttttta tcgctttgca gaagtttttg actttcttga cggaagaatg atgtgctttt 6000
   gccatagtat gctttgttaa ataaagattc ttcgccttgg tagccatctt cagttccagt 6060
   gtttgcttca aatactaagt atttgtggcc tttatcttct acgtagtgag gatctctcag 6120
   cgtatggttg tcgcctgagc tgtagttgcc ttcatcgatg aactgctgta cattttgata 6180
   cgtttttccg tcaccgtcaa agattgattt ataatcctct acaccgttga tgttcaaaga 6240
   gctgtctgat gctgatacgt taacttgtgc agttgtcagt gtttgtttgc cgtaatgttt 6300
   accggagaaa tcagtgtaga ataaacggat ttttccgtca gatgtaaatg tggctgaacc 6360
   tgaccattct tgtgtttggt cttttaggat agaatcattt gcatcgaatt tgtcgctgtc 6420
   tttaaagacg cggccagcgt ttttccagct gtcaatagaa gtttcgccga ctttttgata 6480
   gaacatgtaa atcgatgtgt catccgcatt tttaggatct ccggctaatg caaagacgat 6540
   gtggtagccg tgatagtttg cgacagtgcc gtcagcgttt tgtaatggcc agctgtccca 6600
   aacgtccagg ccttttgcag aagagatatt tttaattgtg gacgaatcaa attcagaaac 6660
   ttgatatttt tcattttttt gctgttcagg gatttgcagc atatcatggc gtgtaatatg 6720
   ggaaatgccg tatgtttcct tatatggctt ttggttcgtt tctttcgcaa acgcttgagt 6780
   tgcgcctcct gccagcagtg cggtagtaaa ggttaatact gttgcttgtt ttgcaaactt 6840
   tttgatgttc atcgttcatg tctccttttt tatgtactgt gttagcggtc tgcttcttcc 6900
   agccctcctg tttgaagatg gcaagttagt tacgcacaat aaaaaaagac ctaaaatatg 6960
   taaggggtga cgccaaagta tacactttgc cctttacaca ttttaggtct tgcctgcttt 7020
   atcagtaaca aacccgcgcg atttactttt cgacctcatt ctattagact ctcgtttgga 7080
   ttgcaactgg tctattttcc tcttttgttt gatagaaaat cataaaagga tttgcagact 7140
   acgggcctaa agaactaaaa aatctatctg tttcttttca ttctctgtat tttttatagt 7200
   ttctgttgca tgggcataaa gttgcctttt taatcacaat tcagaaaata tcataatatc 7260
   tcatttcact aaataatagt gaacggcagg tatatgtgat gggttaaaaa ggatcggc 7318
<210> 31
   <211> 5715
   <212> DNA
   <213> artificial
<220>
   <223> Plasmid pCLIK5APsodTKT
<400> 31
   cgcgtcggca aattagtcga atgaagttaa ttaaaagttc ccgaatcaat ctttttaatg 60
   ttttcaaacc atttgaaggt gtgctgaccc aggtggacgc caacctttaa aaagcttcag 120
   acttttattt ccacttcata aaaactgcct gtgacgattc cgttaaagat tgtgccaaat 180
   cactgcgcaa aactcgcgcg gaaccagacc ttgccatgct atcgcctatt cacactattt 240
   gagtaatcgg aaatagatgg gtgtagacgc ttgattggcg gacggttcac agcggacgat 300
   ttcaggccct cgtagctcga gagtttgaag gggtccgatt cgttccgttc gtgacgcttt 360
   gtgaggtttt ttgacgttgc accgtattgc ttgccgaaca tttttctttt cctttcggtt 420
   tttcgagaat tttcacctac aaaagcccac gtcacagctc ccagacttaa gattgatcac 480
   acctttgaca catttgaacc acagttggtt ataaaatggg ttcaacatca ctatggttag 540
   aggtgttgac gggtcagatt aagcaaagac tactttcggg gtagatcacc tttgccaaat 600
   ttgaaccaat taacctaagt cgtagatctg atcatcggat ctaacgaaaa cgaaccaaaa 660
   ctttggtccc ggtttaaccc aggaaggata gctgccaatt attccgggct tgtgacccgc 720
   tacccgataa ataggtcggc tgaaaaattt cgttgcaata tcaacaaaaa ggcctatcat 780
   tgggaggtgt cgcaccaagt acttttgcga agcgccatct gacggatttt caaaagatgt     840
   atatgctcgg tgcggaaacc tacgaaagga ttttttaccc ttgaccacct tgacgctgtc      900
   acctgaactt caggcgctca ctgtacgcaa ttacccctct gattggtccg atgtggacac     960
   caaggctgta gacactgttc gtgtcctcgc tgcagacgct gtagaaaact gtggctccgg    1020
   ccacccaggc accgcaatga gcctggctcc ccttgcatac accttgtacc agcgggttat    1080
   gaacgtagat ccacaggaca ccaactgggc aggccgtgac cgcttcgttc tttcttgtgg    1140
   ccactcctct ttgacccagt acatccagct ttacttgggt ggattcggcc ttgagatgga    1200
   tgacctgaag gctctgcgca cctgggattc cttgacccca ggacaccctg agtaccgcca    1260
   caccaagggc gttgagatca ccactggccc tcttggccag ggtcttgcat ctgcagttgg    1320
   tatggccatg gctgctcgtc gtgagcgtgg cctattcgac ccaaccgctg ctgagggcga    1380
   atccccattc gaccaccaca tctacgtcat tgcttctgat gggtcgacat cgatgctctt    1440
   ctgcgttaat taacaattgg gatcctctag acccgggatt taaatgatcc gctagcgggc    1500
   tgctaaagga agcggaacac gtagaaagcc agtccgcaga aacggtgctg accccggatg    1560
   aatgtcagct actgggctat ctggacaagg gaaaacgcaa gcgcaaagag aaagcaggta    1620
   gcttgcagtg ggcttacatg gcgatagcta gactgggcgg ttttatggac agcaagcgaa    1680
   ccggaattgc cagctggggc gccctctggt aaggttggga agccctgcaa agtaaactgg    1740
   atggctttct tgccgccaag gatctgatgg cgcaggggat caagatctga tcaagagaca    1800
   ggatgaggat cgtttcgcat gattgaacaa gatggattgc acgcaggttc tccggccgct    1860
   tgggtggaga ggctattcgg ctatgactgg gcacaacaga caatcggctg ctctgatgcc    1920
   gccgtgttcc ggctgtcagc gcaggggcgc ccggttcttt ttgtcaagac cgacctgtcc    1980
   ggtgccctga atgaactgca ggacgaggca gcgcggctat cgtggctggc cacgacgggc    2040
   gttccttgcg cagctgtgct cgacgttgtc actgaagcgg gaagggactg gctgctattg    2100
   ggcgaagtgc cggggcagga tctcctgtca tctcaccttg ctcctgccga gaaagtatcc    2160
   atcatggctg atgcaatgcg gcggctgcat acgcttgatc cggctacctg cccattcgac    2220
   caccaagcga aacatcgcat cgagcgagca cgtactcgga tggaagccgg tcttgtcgat    2280
   caggatgatc tggacgaaga gcatcagggg ctcgcgccag ccgaactgtt cgccaggctc    2340
   aaggcgcgca tgcccgacgg cgaggatctc gtcgtgaccc atggcgatgc ctgcttgccg    2400
   aatatcatgg tggaaaatgg ccgcttttct ggattcatcg actgtggccg gctgggtgtg    2460
   gcggaccgct atcaggacat agcgttggct acccgtgata ttgctgaaga gcttggcggc    2520
   gaatgggctg accgcttcct cgtgctttac ggtatcgccg ctcccgattc gcagcgcatc    2580
   gccttctatc gccttcttga cgagttcttc tgagcgggac tctggggttc gaaatgaccg    2640
   accaagcgac gcccaacctg ccatcacgag atttcgattc caccgccgcc ttctatgaaa    2700
   ggttgggctt cggaatcgtt ttccgggacg ccggctggat gatcctccag cgcggggatc    2760
   tcatgctgga gttcttcgcc cacgctagcg gcgcgccggc cggcccggtg tgaaataccg 2820
   cacagatgcg taaggagaaa ataccgcatc aggcgctctt ccgcttcctc gctcactgac 2880
   tcgctgcgct cggtcgttcg gctgcggcga gcggtatcag ctcactcaaa ggcggtaata 2940
   cggttatcca cagaatcagg ggataacgca ggaaagaaca tgtgagcaaa aggccagcaa 3000
   aaggccagga accgtaaaaa ggccgcgttg ctggcgtttt tccataggct ccgcccccct 3060
   gacgagcatc acaaaaatcg acgctcaagt cagaggtggc gaaacccgac aggactataa 3120
   agataccagg cgtttccccc tggaagctcc ctcgtgcgct ctcctgttcc gaccctgccg 3180
   cttaccggat acctgtccgc ctttctccct tcgggaagcg tggcgctttc tcatagctca 3240
   cgctgtaggt atctcagttc ggtgtaggtc gttcgctcca agctgggctg tgtgcacgaa 3300
   ccccccgttc agcccgaccg ctgcgcctta tccggtaact atcgtcttga gtccaacccg 3360
   gtaagacacg acttatcgcc actggcagca gccactggta acaggattag cagagcgagg 3420
   tatgtaggcg gtgctacaga gttcttgaag tggtggccta actacggcta cactagaagg 3480
   acagtatttg gtatctgcgc tctgctgaag ccagttacct tcggaaaaag agttggtagc 3540
   tcttgatccg gcaaacaaac caccgctggt agcggtggtt tttttgtttg caagcagcag 3600
   attacgcgca gaaaaaaagg atctcaagaa gatcctttga tcttttctac ggggtctgac 3660
   gctcagtgga acgaaaactc acgttaaggg attttggtca tgagattatc aaaaaggatc 3720
   ttcacctaga tccttttaaa ggccggccgc ggccgccatc ggcattttct tttgcgtttt 3780
   tatttgttaa ctgttaattg tccttgttca aggatgctgt ctttgacaac agatgttttc 3840
   ttgcctttga tgttcagcag gaagctcggc gcaaacgttg attgtttgtc tgcgtagaat 3900
   cctctgtttg tcatatagct tgtaatcacg acattgtttc ctttcgcttg aggtacagcg 3960
   aagtgtgagt aagtaaaggt tacatcgtta ggatcaagat ccatttttaa cacaaggcca 4020
   gttttgttca gcggcttgta tgggccagtt aaagaattag aaacataacc aagcatgtaa 4080
   atatcgttag acgtaatgcc gtcaatcgtc atttttgatc cgcgggagtc agtgaacagg 4140
   taccatttgc cgttcatttt aaagacgttc gcgcgttcaa tttcatctgt tactgtgtta 4200
   gatgcaatca gcggtttcat cacttttttc agtgtgtaat catcgtttag ctcaatcata 4260
   ccgagagcgc cgtttgctaa ctcagccgtg cgttttttat cgctttgcag aagtttttga 4320
   ctttcttgac ggaagaatga tgtgcttttg ccatagtatg ctttgttaaa taaagattct 4380
   tcgccttggt agccatcttc agttccagtg tttgcttcaa atactaagta tttgtggcct 4440
   ttatcttcta cgtagtgagg atctctcagc gtatggttgt cgcctgagct gtagttgcct 4500
   tcatcgatga actgctgtac attttgatac gtttttccgt caccgtcaaa gattgattta 4560
   taatcctcta caccgttgat gttcaaagag ctgtctgatg ctgatacgtt aacttgtgca 4620
   gttgtcagtg tttgtttgcc gtaatgttta ccggagaaat cagtgtagaa taaacggatt 4680
   tttccgtcag atgtaaatgt ggctgaacct gaccattctt gtgtttggtc ttttaggata 4740
   gaatcatttg catcgaattt gtcgctgtct ttaaagacgc ggccagcgtt tttccagctg 4800
   tcaatagaag tttcgccgac tttttgatag aacatgtaaa tcgatgtgtc atccgcattt 4860
   ttaggatctc cggctaatgc aaagacgatg tggtagccgt gatagtttgc gacagtgccg 4920
   tcagcgtttt gtaatggcca gctgtcccaa acgtccaggc cttttgcaga agagatattt 4980
   ttaattgtgg acgaatcaaa ttcagaaact tgatattttt catttttttg ctgttcaggg 5040
   atttgcagca tatcatggcg tgtaatatgg gaaatgccgt atgtttcctt atatggcttt 5100
   tggttcgttt ctttcgcaaa cgcttgagtt gcgcctcctg ccagcagtgc ggtagtaaag 5160
   gttaatactg ttgcttgttt tgcaaacttt ttgatgttca tcgttcatgt ctcctttttt 5220
   atgtactgtg ttagcggtct gcttcttcca gccctcctgt ttgaagatgg caagttagtt 5280
   acgcacaata aaaaaagacc taaaatatgt aaggggtgac gccaaagtat acactttgcc 5340
   ctttacacat tttaggtctt gcctgcttta tcagtaacaa acccgcgcga tttacttttc 5400
   gacctcattc tattagactc tcgtttggat tgcaactggt ctattttcct cttttgtttg 5460
   atagaaaatc ataaaaggat ttgcagacta cgggcctaaa gaactaaaaa atctatctgt 5520
   ttcttttcat tctctgtatt ttttatagtt tctgttgcat gggcataaag ttgccttttt 5580
   aatcacaatt cagaaaatat cataatatct catttcacta aataatagtg aacggcaggt 5640
   atatgtgatg ggttaaaaag gatcggcggc cgctcgattt aaatctcgag aggcctgacg 5700
   tcgggcccgg tacca 5715
<210> 32
   <211> 5083
   <212> DNA
   <213> artificial
<220>
   <223> Plasmid pH626 int SacB delta sdaA
<400> 32 .
   ctagacccgg gatttaaatc gctagcgggc tgctaaagga agcggaacac gtagaaagcc     60
   agtccgcaga aacggtgctg accccggatg aatgtcagct actgggctat ctggacaagg    120
   gaaaacgcaa gcgcaaagag aaagcaggta gcttgcagtg ggcttacatg gcgatagcta    180
   gactgggcgg ttttatggac agcaagcgaa ccggaattgc cagctggggc gccctctggt    240
   aaggttggga agccctgcaa agtaaactgg atggctttct tgccgccaag gatctgatgg    300
   cgcaggggat caagatctga tcaagagaca ggatgaggat cgtttcgcat gattgaacaa    360
   gatggattgc acgcaggttc tccggccgct tgggtggaga ggctattcgg ctatgactgg    420
   gcacaacaga caatcggctg ctctgatgcc gccgtgttcc ggctgtcagc gcaggggcgc    480
   ccggttcttt ttgtcaagac cgacctgtcc ggtgccctga atgaactgca ggacgaggca    540
   gcgcggctat cgtggctggc cacgacgggc gttccttgcg cagctgtgct cgacgttgtc    600
   actgaagcgg gaagggactg gctgctattg ggcgaagtgc cggggcagga tctcctgtca    660
   tctcaccttg ctcctgccga gaaagtatcc atcatggctg atgcaatgcg gcggctgcat    720
   acgcttgatc cggctacctg cccattcgac caccaagcga aacatcgcat cgagcgagca    780
   cgtactcgga tggaagccgg tcttgtcgat caggatgatc tggacgaaga gcatcagggg    840
   ctcgcgccag ccgaactgtt cgccaggctc aaggcgcgca tgcccgacgg cgaggatctc    900
   gtcgtgaccc atggcgatgc ctgcttgccg aatatcatgg tggaaaatgg ccgcttttct    960
   ggattcatcg actgtggccg gctgggtgtg gcggaccgct atcaggacat agcgttggct   1020
   acccgtgata ttgctgaaga gcttggcggc gaatgggctg accgcttcct cgtgctttac   1080
   ggtatcgccg ctcccgattc gcagcgcatc gccttctatc gccttcttga cgagttcttc   1140
   tgagcgggac tctggggttc gaaatgaccg accaagcgac gcccaacctg ccatcacgag   1200
   atttcgattc caccgccgcc ttctatgaaa ggttgggctt cggaatcgtt ttccgggacg   1260
   ccggctggat gatcctccag cgcggggatc tcatgctgga gttcttcgcc cacgctagcg   1320
   gcgcgccggc cggcccggtg tgaaataccg cacagatgcg taaggagaaa ataccgcatc   1380
   aggcgctctt ccgcttcctc gctcactgac tcgctgcgct cggtcgttcg gctgcggcga   1440
   gcggtatcag ctcactcaaa ggcggtaata cggttatcca cagaatcagg ggataacgca   1500
   ggaaagaaca tgtgagcaaa aggccagcaa aaggccagga accgtaaaaa ggccgcgttg   1560
   ctggcgtttt tccataggct ccgcccccct gacgagcatc acaaaaatcg acgctcaagt   1620
   cagaggtggc gaaacccgac aggactataa agataccagg cgtttccccc tggaagctcc   1680
   ctcgtgcgct ctcctgttcc gaccctgccg cttaccggat acctgtccgc ctttctccct   1740
   tcgggaagcg tggcgctttc tcatagctca cgctgtaggt atctcagttc ggtgtaggtc   1800
   gttcgctcca agctgggctg tgtgcacgaa ccccccgttc agcccgaccg ctgcgcctta   1860
   tccggtaact atcgtcttga gtccaacccg gtaagacacg acttatcgcc actggcagca   1920
   gccactggta acaggattag cagagcgagg tatgtaggcg gtgctacaga gttcttgaag   1980
   tggtggccta actacggcta cactagaagg acagtatttg gtatctgcgc tctgctgaag   2040
   ccagttacct tcggaaaaag agttggtagc tcttgatccg gcaaacaaac caccgctggt   2100
   agcggtggtt tttttgtttg caagcagcag attacgcgca gaaaaaaagg atctcaagaa   2160
   gatcctttga tcttttctac ggggtctgac gctcagtgga acgaaaactc acgttaaggg   2220
   attttggtca tgagattatc aaaaaggatc ttcacctaga tccttttaaa ggccggccgc   2280
   ggccgccatc ggcattttct tttgcgtttt tatttgttaa ctgttaattg tccttgttca   2340
   aggatgctgt ctttgacaac agatgttttc ttgcctttga tgttcagcag gaagctcggc   2400
   gcaaacgttg attgtttgtc tgcgtagaat cctctgtttg tcatatagct tgtaatcacg   2460
   acattgtttc ctttcgcttg aggtacagcg aagtgtgagt aagtaaaggt tacatcgtta   2520
   ggatcaagat ccatttttaa cacaaggcca gttttgttca gcggcttgta tgggccagtt 2580
   aaagaattag aaacataacc aagcatgtaa atatcgttag acgtaatgcc gtcaatcgtc 2640
   atttttgatc cgcgggagtc agtgaacagg taccatttgc cgttcatttt aaagacgttc 2700
   gcgcgttcaa tttcatctgt tactgtgtta gatgcaatca gcggtttcat cacttttttc 2760
   agtgtgtaat catcgtttag ctcaatcata ccgagagcgc cgtttgctaa ctcagccgtg 2820
   cgttttttat cgctttgcag aagtttttga ctttcttgac ggaagaatga tgtgcttttg 2880
   ccatagtatg ctttgttaaa taaagattct tcgccttggt agccatcttc agttccagtg 2940
   tttgcttcaa atactaagta tttgtggcct ttatcttcta cgtagtgagg atctctcagc 3000
   gtatggttgt cgcctgagct gtagttgcct tcatcgatga actgctgtac attttgatac 3060
   gtttttccgt caccgtcaaa gattgattta taatcctcta caccgttgat gttcaaagag 3120
   ctgtctgatg ctgatacgtt aacttgtgca gttgtcagtg tttgtttgcc gtaatgttta 3180
   ccggagaaat cagtgtagaa taaacggatt tttccgtcag atgtaaatgt ggctgaacct 3240
   gaccattctt gtgtttggtc ttttaggata gaatcatttg catcgaattt gtcgctgtct 3300
   ttaaagacgc ggccagcgtt tttccagctg tcaatagaag tttcgccgac tttttgatag 3360
   aacatgtaaa tcgatgtgtc atccgcattt ttaggatctc cggctaatgc aaagacgatg 3420
   tggtagccgt gatagtttgc gacagtgccg tcagcgtttt gtaatggcca gctgtcccaa 3480
   acgtccaggc cttttgcaga agagatattt ttaattgtgg acgaatcaaa ttcagaaact 3540
   tgatattttt catttttttg ctgttcaggg atttgcagca tatcatggcg tgtaatatgg 3600
   gaaatgccgt atgtttcctt atatggcttt tggttcgttt ctttcgcaaa cgcttgagtt 3660
   gcgcctcctg ccagcagtgc ggtagtaaag gttaatactg ttgcttgttt tgcaaacttt 3720
   ttgatgttca tcgttcatgt ctcctttttt atgtactgtg ttagcggtct gcttcttcca 3780
   gccctcctgt ttgaagatgg caagttagtt acgcacaata aaaaaagacc taaaatatgt 3840
   aaggggtgac gccaaagtat acactttgcc ctttacacat tttaggtctt gcctgcttta 3900
   tcagtaacaa acccgcgcga tttacttttc gacctcattc tattagactc tcgtttggat 3960
   tgcaactggt ctattttcct cttttgtttg atagaaaatc ataaaaggat ttgcagacta 4020
   cgggcctaaa gaactaaaaa atctatctgt ttcttttcat tctctgtatt ttttatagtt 4080
   tctgttgcat gggcataaag ttgccttttt aatcacaatt cagaaaatat cataatatct 4140
   catttcacta aataatagtg aacggcaggt atatgtgatg ggttaaaaag gatcggcggc 4200
   cgctcgattt aaatctcgag aggcctgacg tcgggcccgg taccacgcgt gccgatcttc 4260
   tcaaaggaca cgacggaaac ggctaaattc gcggatctcc gtttaaggca ttgaagcatt 4320
   tggaggcccc aagacatgac ccagaccctg taaagcgctt aaacggcgtt ttagagggtc 4380
   atagttttgg gacaagtggg acaagtgtga atcctgaaag cttccagggc aaggatccac 4440
   cacaaaccgg ccatcgccct ttggaatcgg tccgaaaatt gcaggtacag agccttttac 4500
   cgagaaaatc caccacagat tgctgaaatt tcgtgatctg tggtggattc gtgcaacttc 4560
   agactcttac ggaggcgatg gaccaaaaac aactacaatc aagcagatca ccttgtacac 4620
   caccatagaa aaggcccacc ctcagcccgg tacggcttta acacggcttg gattttgtct 4680
   tgcttggcga ggtaggactg gcactgggcg tcgataagct cagctgacca cccggtgacc 4740
   tgcgccattg cttcggccgt cgcacgcacg gcagccggtt gcacataacc cagcgtgccg 4800
   agcacgatgc gacgatccag cacgtccgcc aggtcgacgg ccgcctcctc ggcgacagca 4860
   aaaacggcct gcgccgcgat atcaaggttg tctgggtcaa gtcggcgccc caggtcgggt 4920
   tgctttgcga cgagatccag cactttttca tgctcagttc catacagtct ggccagatgc 4980
   acgcggattt catcatccac atccagctcg gggtggctgc gaagcgctga ctcaaaggaa 5040
   tcagccacgg actcatacgc gccaaaagaa gtactcaacg gct 5083
<210> 33
   <211> 6995
   <212> DNA
   <213> artificial
<220>
   <223> Plasmid pOM253
<400> 33
   aaatcgctag cgggctgcta aaggaagcgg aacacgtaga aagccagtcc gcagaaacgg 60
   tgctgacccc ggatgaatgt cagctactgg gctatctgga caagggaaaa cgcaagcgca 120
   aagagaaagc aggtagcttg cagtgggctt acatggcgat agctagactg ggcggtttta 180
   tggacagcaa gcgaaccgga attgccagct ggggcgccct ctggtaaggt tgggaagccc 240
   tgcaaagtaa actggatggc tttcttgccg ccaaggatct gatggcgcag gggatcaaga 300
   tctgatcaag agacaggatg aggatcgttt cgcatgattg aacaagatgg attgcacgca 360
   ggttctccgg ccgcttgggt ggagaggcta ttcggctatg actgggcaca acagacaatc 420
   ggctgctctg atgccgccgt gttccggctg tcagcgcagg ggcgcccggt tctttttgtc 480
   aagaccgacc tgtccggtgc cctgaatgaa ctgcaggacg aggcagcgcg gctatcgtgg 540
   ctggccacga cgggcgttcc ttgcgcagct gtgctcgacg ttgtcactga agcgggaagg 600
   gactggctgc tattgggcga agtgccgggg caggatctcc tgtcatctca ccttgctcct 660
   gccgagaaag tatccatcat ggctgatgca atgcggcggc tgcatacgct tgatccggct 720
   acctgcccat tcgaccacca agcgaaacat cgcatcgagc gagcacgtac tcggatggaa 780
   gccggtcttg tcgatcagga tgatctggac gaagagcatc aggggctcgc gccagccgaa 840
   ctgttcgcca ggctcaaggc gcgcatgccc gacggcgagg atctcgtcgt gacccatggc 900
   gatgcctgct tgccgaatat catggtggaa aatggccgct tttctggatt catcgactgt 960
   ggccggctgg gtgtggcgga ccgctatcag gacatagcgt tggctacccg tgatattgct 1020
   gaagagcttg gcggcgaatg ggctgaccgc ttcctcgtgc tttacggtat cgccgctccc 1080
   gattcgcagc gcatcgcctt ctatcgcctt cttgacgagt tcttctgagc gggactctgg 1140
   ggttcgaaat gaccgaccaa gcgacgccca acctgccatc acgagatttc gattccaccg 1200
   ccgccttcta tgaaaggttg ggcttcggaa tcgttttccg ggacgccggc tggatgatcc 1260
   tccagcgcgg ggatctcatg ctggagttct tcgcccacgc tagcggcgcg ccggccggcc 1320
   cggtgtgaaa taccgcacag atgcgtaagg agaaaatacc gcatcaggcg ctcttccgct 1380
   tcctcgctca ctgactcgct gcgctcggtc gttcggctgc ggcgagcggt atcagctcac 1440
   tcaaaggcgg taatacggtt atccacagaa tcaggggata acgcaggaaa gaacatgtga 1500
   gcaaaaggcc agcaaaaggc caggaaccgt aaaaaggccg cgttgctggc gtttttccat 1560
   aggctccgcc cccctgacga gcatcacaaa aatcgacgct caagtcagag gtggcgaaac 1620
   ccgacaggac tataaagata ccaggcgttt ccccctggaa gctccctcgt gcgctctcct 1680
   gttccgaccc tgccgcttac cggatacctg tccgcctttc tcccttcggg aagcgtggcg 1740
   ctttctcata gctcacgctg taggtatctc agttcggtgt aggtcgttcg ctccaagctg 1800
   ggctgtgtgc acgaaccccc cgttcagccc gaccgctgcg ccttatccgg taactatcgt 1860
   cttgagtcca acccggtaag acacgactta tcgccactgg cagcagccac tggtaacagg 1920
   attagcagag cgaggtatgt aggcggtgct acagagttct tgaagtggtg gcctaactac 1980
   ggctacacta gaaggacagt atttggtatc tgcgctctgc tgaagccagt taccttcgga 2040
   aaaagagttg gtagctcttg atccggcaaa caaaccaccg ctggtagcgg tggttttttt 2100
   gtttgcaagc agcagattac gcgcagaaaa aaaggatctc aagaagatcc tttgatcttt 2160
   tctacggggt ctgacgctca gtggaacgaa aactcacgtt aagggatttt ggtcatgaga 2220
   ttatcaaaaa ggatcttcac ctagatcctt ttaaaggccg gccgcggccg ccatcggcat 2280
   tttcttttgc gtttttattt gttaactgtt aattgtcctt gttcaaggat gctgtctttg 2340
   acaacagatg ttttcttgcc tttgatgttc agcaggaagc tcggcgcaaa cgttgattgt 2400
   ttgtctgcgt agaatcctct gtttgtcata tagcttgtaa tcacgacatt gtttcctttc 2460
   gcttgaggta cagcgaagtg tgagtaagta aaggttacat cgttaggatc aagatccatt 2520
   tttaacacaa ggccagtttt gttcagcggc ttgtatgggc cagttaaaga attagaaaca 2580
   taaccaagca tgtaaatatc gttagacgta atgccgtcaa tcgtcatttt tgatccgcgg 2640
   gagtcagtga acaggtacca tttgccgttc attttaaaga cgttcgcgcg ttcaatttca 2700
   tctgttactg tgttagatgc aatcagcggt ttcatcactt ttttcagtgt gtaatcatcg 2760
   tttagctcaa tcataccgag agcgccgttt gctaactcag ccgtgcgttt tttatcgctt 2820
   tgcagaagtt tttgactttc ttgacggaag aatgatgtgc ttttgccata gtatgctttg 2880
   ttaaataaag attcttcgcc ttggtagcca tcttcagttc cagtgtttgc ttcaaatact 2940
   aagtatttgt ggcctttatc ttctacgtag tgaggatctc tcagcgtatg gttgtcgcct 3000
   gagctgtagt tgccttcatc gatgaactgc tgtacatttt gatacgtttt tccgtcaccg 3060
   tcaaagattg atttataatc ctctacaccg ttgatgttca aagagctgtc tgatgctgat 3120
   acgttaactt gtgcagttgt cagtgtttgt ttgccgtaat gtttaccgga gaaatcagtg 3180
   tagaataaac ggatttttcc gtcagatgta aatgtggctg aacctgacca ttcttgtgtt 3240
   tggtctttta ggatagaatc atttgcatcg aatttgtcgc tgtctttaaa gacgcggcca 3300
   gcgtttttcc agctgtcaat agaagtttcg ccgacttttt gatagaacat gtaaatcgat 3360
   gtgtcatccg catttttagg atctccggct aatgcaaaga cgatgtggta gccgtgatag 3420
   tttgcgacag tgccgtcagc gttttgtaat ggccagctgt cccaaacgtc caggcctttt 3480
   gcagaagaga tatttttaat tgtggacgaa tcaaattcag aaacttgata tttttcattt 3540
   ttttgctgtt cagggatttg cagcatatca tggcgtgtaa tatgggaaat gccgtatgtt 3600
   tccttatatg gcttttggtt cgtttctttc gcaaacgctt gagttgcgcc tcctgccagc 3660
   agtgcggtag taaaggttaa tactgttgct tgttttgcaa actttttgat gttcatcgtt 3720
   catgtctcct tttttatgta ctgtgttagc ggtctgcttc ttccagccct cctgtttgaa 3780
   gatggcaagt tagttacgca caataaaaaa agacctaaaa tatgtaaggg gtgacgccaa 3840
   agtatacact ttgcccttta cacattttag gtcttgcctg ctttatcagt aacaaacccg 3900
   cgcgatttac ttttcgacct cattctatta gactctcgtt tggattgcaa ctggtctatt 3960
   ttcctctttt gtttgataga aaatcataaa aggatttgca gactacgggc ctaaagaact   4020
   aaaaaatcta tctgtttctt ttcattctct gtatttttta tagtttctgt tgcatgggca   4080
   taaagttgcc tttttaatca caattcagaa aatatcataa tatctcattt cactaaataa 4140
   tagtgaacgg caggtatatg tgatgggtta aaaaggatcg gcggccgctc gatttgggtc 4200
   tagaggaggt gaaacaatgt cccagaatgg ccgtccagta gtcctcatcg ccgataagct 4260
   tgcgcagtcc actgttgacg cgcttggaga tgcagtagaa gtccgttggg ttgacggacc 4320
   taaccgccca gaactgcttg atgcagttaa ggaagcggac gcactgctcg tgcgttctgc 4380
   taccactgtc gatgctgaag tcatcgccgc tgcccctaac ttgaagatcg tcggtcgtgc 4440
   cggcgtgggc ttggacaacg ttgacatccc tgctgccact gaagctggcg tcatggttgc 4500
   taacgcaccg acctctaata ttcactccgc ttgtgagcac gcaatttctt tgctgctgtc 4560
   tactgctcgc cagatccctg ctgctgatgc gacgctgcgt gagggcgagt ggaagcggtc 4620
   ttctttcaac ggtgtggaaa ttttcggaaa aactgtcggt atcgtcggtt ttggccacat 4680
   tggtcagttg tttgctcagc gtcttgctgc gtttgagacc accattgttg cttacgatcc 4740
   ttacgctaac cctgctcgtg cggctcagct gaacgttgag ttggttgagt tggatgagct 4800
   gatgagccgt tctgactttg tcaccattca ccttcctaag accaaggaaa ctgctggcat 4860
   gtttgatgcg cagctccttg ctaagtccaa gaagggccag atcatcatca acgctgctcg 4920
   tggtggcctt gttgatgagc aggctttggc tgatgcgatt gagtccggtc acatctgcgg 4980
   ccgcacagcg atcccagagg aaatatcctc tggggtcgct gtgtcgacct taaagtttgg 5040
   ctgccatgtg aatttttagc accctcaaca gttgagtgct ggcactctcg ggggtagagt 5100
   gccaaatagg ttgtttgaca cacagttgtt cacccgcgac gacggctgtg ctggaaaccc 5160
   acaaccggca cacacaaaat ttttctagag gagggattca tcatgaatac atacgaacaa 5220
   attaataaag tgaaaaaaat acttcggaaa catttaaaaa ataaccttat tggtacttac 5280
   atgtttggat caggagttga gagtggacta aaaccaaata gtgatcttga ctttttagtc 5340
   gtcgtatctg aaccattgac agatcaaagt aaagaaatac ttatacaaaa aattagacct 5400
   atttcaaaaa aaataggaga taaaagcaac ttacgatata ttgaattaac aattattatt 5460
   cagcaagaaa tggtaccgtg gaatcatcct cccaaacaag aatttattta tggagaatgg 5520
   ttacaagagc tttatgaaca aggatacatt cctcagaagg aattaaattc agatttaacc 5580
   ataatgcttt accaagcaaa acgaaaaaat aaaagaatat acggaaatta tgacttagag 5640
   gaattactac ctgatattcc attttctgat gtgagaagag ccattatgga ttcgtcagag 5700
   gaattaatag ataattatca ggatgatgaa accaactcta tattaacttt atgccgtatg 5760
   attttaacta tggacacggg taaaatcata ccaaaagata ttgcgggaaa tgcagtggct 5820
   gaatcttctc cattagaaca tagggagaga attttgttag cagttcgtag ttatcttgga 5880
   gagaatattg aatggactaa tgaaaatgta aatttaacta taaactattt aaataacaga 5940
   ttaaaaaaat tataaaaaaa ttgaaaaaat ggtggaaaca cttttttcaa tttttttgtt 6000
   ttattattta atatttggga aatattcatt ctaattggta atcagatttt agaaaacaat 6060
   aaacccttgc atagggggat cgatatccgt ttaggctggg cggatccgcc ctcccgcacg 6120
   ctttgcggga gggcggtacc aggggtgctt ctactgaaga ggctcaggat cgtgcgggta 6180
   ctgacgttgc tgattctgtg ctcaaggcgc tggctggcga gttcgtggcg gatgctgtga 6240
   acgtttccgg tggtcgcgtg ggcgaagagg ttgctgtgtg gatggatctg gctcgcaagc 6300
   ttggtcttct tgctggcaag cttgtcgacg ccgccccagt ctccattgag gttgaggctc 6360
   gaggcgagct ttcttccgag caggtcgatg cacttggttt gtccgctgtt cgtggtttgt 6420
   tctccggaat tatcgaagag tccgttactt tcgtcaacgc tcctcgcatt gctgaagagc 6480
   gtggcctgga catctccgtg aagaccaact ctgagtctgt tactcaccgt tccgtcctgc 6540
   aggtcaaggt cattactggc agcggcgcga gcgcaactgt tgttggtgcc ctgactggtc 6600
   ttgagcgcgt tgagaagatc acccgcatca atggccgtgg cctggatctg cgcgcagagg 6660
   gtctgaacct cttcctgcag tacactgacg ctcctggtgc actgggtacc gttggtacca 6720
   agctgggtgc tgctggcatc aacatcgagg ctgctgcgtt gactcaggct gagaagggtg 6780
   acggcgctgt cctgatcctg cgtgttgagt ccgctgtctc tgaagagctg gaagctgaaa 6840
   tcaacgctga gttgggtgct acttccttcc aggttgatct tgactaatta gagatccatt 6900
   tgcttgaacc gccttcccat ctttgaattc attcaaggtg gtaaggcggt tttcgctctt 6960
   ttaatacagt tttaatggta gatttgggat ccctc 6995
<210> 34
   <211> 11491
   <212> DNA
   <213> artificial
<220>
   <223> Plasmid pOM615
<400> 34
   ttgatcagcg gccgcacagc gatcccagag gaaatatcct ctggggtcgc tgtgtcgacc 60
   ttaaagtttg gctgccatgt gaatttttag caccctcaac agttgagtgc tggcactctc 120
   gggggtagag tgccaaatag gttgtttgac acacagttgt tcacccgcga cgacggctgt 180
   gctggaaacc cacaaccggc acacacaaaa tttttctaga aggaggtgat agtatgtctt 240
   ctgcagctac ccgccgtaat tcagccccct tcgttcagcg ccacatcggc ccaaaccagg 300
   ccgataccca ggagatcctc gattacctgg gctatgaatc ttccgccgcg ctggccgacg 360
   atgccctccc gaagtcgatc cgccaggcag gcccgatcgg cctgccggag gcactggatg 420
   agacggacac cctggccgcc ctgcgtgctt acgctgacaa gaacgtgcag aagcagcagc 480
   tgatcggcaa cggttacttc gacacgatca ctccggccgt gattcgccgc aacgtggtgg 540
   agaacccggg ctggtacacc gcctacaccc cctaccagcc ggaaatctcc caggggcgcc 600
   tcgaggccct gctgaacttc cagacgatgg tgcaggacct gaccggcctg ccagtggccg 660
   gtgcttcgct gttagacgaa gccaccgcag tggccgaggc cgtgcagctg atggctcgcg 720
   gcaatgcgaa ggctgccaag aagggcggcg tggtgctgct ggattcctcc ctgcaccagc 780
   agtccatcac cgtaaccctg gcgcgcgctg aggctgcggg tatcccggtg gaggtcgtgg 840
   acctggacgg cgaagatgct accgccgcgt ttgagggccg cgagaacctc gtcggcgtgg 900
   tgctttccaa ccccggctcc accggccgcg tccgcgacct gtccggtctg atctctgcgg 960
   cgaaggagac cggcgctctg gtgacggtcg cttgtgacct gctggctcag gttctggtga 1020
   cctccccggg ctcccaaggt gccgacattg ctgtcggctc cgcccagcgc ttcggcgtgc 1080
   cgctattctt cggtggcccg cacgcgggct tcatctcctg caccgaggct ctgcagcgta 1140
   agctgccggg ccgcatcgtg ggcgtgtccg tggatgccga gggcaccccg gcctaccgct 1200
   tggctctgca gacccgtgag cagcacattc gccgcgacaa ggccaccagt aacatctgta 1260
   ccgctcaggc tctgctggcc gtcgttgccg gtttctacgc ggtctggcac ggcccagccg 1320
   gcctgcgcgc catcgccgag ggggtgcacg cccgcgcgac cgccctggcc gttgccctgt 1380
   ccgaggctgg cctgacgctg gcgcacgata ccttcttcga caccgtcacc gttgacgtgt 1440
   ctggttcttc tcttggtgac gcccccacgg ctctgcgcgc cgcagccgag gcaggctaca 1500
   acctgcgcca ggttaacgat tccttcgtcg gcatctctgt cggcgagtcc accacggacg 1560
   aggacattgc caagctgatc gaggtgctgg gctcccgcac cggcgaggtc aactccgcga 1620
   gcttcgacgt caccgccggc ccgctgggcg aggccggcgt gctgcgcgcg gaggacgagg 1680
   agattctgac ccacccgatc ttcaccgcca tcacttccga gacccagatg atgcgctaca 1740
   tgcgcaagct ggccgaccgc gacctggcgc tggatcgtac gatgatcccg ctgggctcct 1800
   gcaccatgaa gctgaacgcg gccgtctcca tggagccgat cacctggcct ggcttcgcag 1860
   gcatccaccc gcacgtcccg gccgagcagg cgcagggctg gctggagctc atcgaggacc 1920
   tggaggagcg cctggcgaag atcaccggct acgccaaggt ttccgtccag ccgaacgcgg 1980
   gctcccaggg cgagttcgcc ggcctgctgg cgatccaccg ctaccaccag tcccgcggcg 2040
   acgatcagcg tgacatcgtt ctgatcccgg cctccgcgca cggcaccaac gctgcctctg 2100
   cagcgctggc gggcctgaag gtcgtggctg tgaagaatgc cgaagacggc tccatcgacg 2160
   tgccggacct ggaggccaag ctggagaagt acggcgagca gaccgccgcc atcatgctga 2220
   cctacccctc cacccacggc gtgttcgagg agcaggtgcg cgacgtctgc cagaaggttc 2280
   acgacgctgg cggccaggtg tacgtcgacg gcgctaacct gaacgccctg gtcggcctgg 2340
   cccagccggg cgagttcggc ggcgacgtat cccacctgaa cctgcacaag accttcacca 2400
   tcccgcacgg tggtggcggc ccgggcgttg gcccggtgtg cgtggcagag cacctgatcc 2460
   cgttcctgcc caccgacccg aacgccgacg ttatcgaggg cgatgctgcc ctgcagtccg 2520
   gccagccggt ctccggcgcg cagtacggct ccgctggcgt gctgccgatc acctggtcct 2580
   acatcgcaca gatgggcgac gaaggcctga ctgaggcctc ccgcatggcc ctggtgaacg 2640
   ccaactacgt ttcccgcaag ctggaggact actacccgac gctgtacaag ggcgacaccg 2700
   gcctggtcgc ccacgagtgc atcctggacc tgcgtgaact cacgaaggcc tccggcatca 2760
   ccgccgagga cgtatccaag cgcctgatgg acttcggctt ccacgccccg accttggcct 2820
   tcccggtcgc cggcaccctg atgatggagc ccaccgagtc ggaggacaag gaagagctgg 2880
   accgcttcat cgaagcgatg atcaccatcc acggggagat ccaggaagtt atcgacggca 2940
   aggtcaccgc cgaacagtcc gttctgcgcc acgcgccgtt taccgcctat tctgttgtgc 3000
   gggacgactt cgaggaagct gtatccggtg gtcacttcag ccgtgccaag gccgcatacc 3060
   cggtggctag cctgcgacac accaagtact tcacccccgt gcgtcgtatc gacaacgcct 3120
   atggcgaccg caatctcgtg tgcacctgcc cgcccctgga agacttcgca attaacgagg 3180
   actagggtcc gctggagcta agggcgtcac aaagagaaga aggagaacaa gacatgaccg 3240
   aacttaagaa gaccgcgctg cacctggtgc acgagaagtt gggcgcgcga tttaccgact 3300
   tcggcggctg ggacatgcct ctgaagtaca gcagtgagct ggacgagcac cacgctgtac 3360
   gcaatgccgt gggcgtattc gacctctccc acatgggtga ggttcgcgtg accggcccgc 3420
   aggcagcgga gttcctggac cacgcgctga tttcgaagct gtcggcagtg aaggtcggca 3480
   aggcgaagta ctcgatgatc tgcaccgaat ccggtggcat catcgacgac ctgatcacct 3540
   accgcctggg cgacaacgag ttcctgatcg tgccgaacgc gggcaacgtg gacaacgtgg 3600
   tctccgcact gcagggccgc accgagggct ttgacgtgga ggttaacaac gagtccgatg 3660
   cgacctccat gatcgccgta caggggccca aggccgcgca ggcgatgctg gagatcgtgg 3720
   agaacgtcgt ggatgcaccc gaggcatccg gcgcgggcga gaccgttgcc gaggctatcg 3780
   aggggctggg ttactacgcg gcattcagcg gtgttgccgc aggtcagccc gtgctggtgg 3840
   cccgcacagg ctataccggc gaggacggtt tcgagctgat cgtggctaac gatggtgcgg 3900
   agaccgtgtg gaccaaggct atggaccagg ctgcgcagct gggtggcctg ccgtgtggcc 3960
   tggcctgccg cgacaccctg cgcctggagg ctggcatgcc gctgtacggc aacgagctat 4020
   cgctgaagct caccccggtc gatgctgggc tgggcattct tgcggcgacg aagtctaagg 4080
   actctttcgt tggtcgtgac gccatcgttt ccgccaagga aaagggtacc cagcaggtac 4140
   ttatcgggct ggcgggcgag ggtcgccgcg ctgcccgtgg gggatacgag gtgtttgccg 4200
   gtgacggcga gaaggccatc ggtgccgtga cctccggtgc actgtcgccg acgctgggcc 4260
   acccggtggc attggcatac gtcgcgaagt ccgcagtgtc ctccggcgcg gccgctgagg 4320
   gtgcgaccgt ggaggtagac atccgcggca agcgctttga atacaaggtt gtggcgctgc 4380
   cgttctactc ccgcgagaag taacgcaaga agtaacgaaa agggttgtct gcccgctggt 4440
   gtaaggtttg ggcagacaac cacagaagta accacacaag gtttcacaag agaggcttaa 4500
   aacaatgact gcactgccaa ctgacttcct gtactccgaa gagcacgagt gggttaacac 4560
   ctccgctgtt gttgagggcg agaccgtgcg cgtgggcatt acccacatcg ccgctgaggc 4620
   gctgggtgac atcgtgttcg tcgagctgcc ggaggttggc tccgaggttg aggccggcga 4680
   ggctttcggc gaggttgagt ccaccaagtc cgtttccgac atctacgcac cggtttctgg 4740
   cgaggttgtg gctgtcaacg aggcgctgga agacaacgct ggcctgatca acgaagatcc 4800
   atacggtgag ggctggctgt acgaggtcaa ggtgaccgag gccggcgagc tgatggaggc 4860
   tgaggcttac caggcggcta acgagtaaaa caagacccta tggtcggttc catgttcgca 4920
   ttaatcctcg gcttggccct gttgggcctt gccgtgtggt tactcctgcg agcaaaagat 4980
   agcaccggat ccgccctccc gcacgctttg cgggagggct tttcttttcc cggtatttaa 5040
   atcgctagcg ggctgctaaa ggaagcggaa cacgtagaaa gccagtccgc agaaacggtg 5100
   ctgaccccgg atgaatgtca gctactgggc tatctggaca agggaaaacg caagcgcaaa 5160
   gagaaagcag gtagcttgca gtgggcttac atggcgatag ctagactggg cggttttatg 5220
   gacagcaagc gaaccggaat tgccagctgg ggcgccctct ggtaaggttg ggaagccctg 5280
   caaagtaaac tggatggctt tcttgccgcc aaggatctga tggcgcaggg gatcaagatc 5340
   tgatcaagag acaggatgag gatcgtttcg catgattgaa caagatggat tgcacgcagg 5400
   ttctccggcc gcttgggtgg agaggctatt cggctatgac tgggcacaac agacaatcgg 5460
   ctgctctgat gccgccgtgt tccggctgtc agcgcagggg cgcccggttc tttttgtcaa 5520
   gaccgacctg tccggtgccc tgaatgaact gcaggacgag gcagcgcggc tatcgtggct 5580
   ggccacgacg ggcgttcctt gcgcagctgt gctcgacgtt gtcactgaag cgggaaggga 5640
   ctggctgcta ttgggcgaag tgccggggca ggatctcctg tcatctcacc ttgctcctgc 5700
   cgagaaagta tccatcatgg ctgatgcaat gcggcggctg catacgcttg atccggctac 5760
   ctgcccattc gaccaccaag cgaaacatcg catcgagcga gcacgtactc ggatggaagc 5820
   cggtcttgtc gatcaggatg atctggacga agagcatcag gggctcgcgc cagccgaact 5880
   gttcgccagg ctcaaggcgc gcatgcccga cggcgaggat ctcgtcgtga cccatggcga 5940
   tgcctgcttg ccgaatatca tggtggaaaa tggccgcttt tctggattca tcgactgtgg 6000
   ccggctgggt gtggcggacc gctatcagga catagcgttg gctacccgtg atattgctga 6060
   agagcttggc ggcgaatggg ctgaccgctt cctcgtgctt tacggtatcg ccgctcccga 6120
   ttcgcagcgc atcgccttct atcgccttct tgacgagttc ttctgagcgg gactctgggg 6180
   ttcgaaatga ccgaccaagc gacgcccaac ctgccatcac gagatttcga ttccaccgcc 6240
   gccttctatg aaaggttggg cttcggaatc gttttccggg acgccggctg gatgatcctc 6300
   cagcgcgggg atctcatgct ggagttcttc gcccacgcta gtttaaactg cggatcagtg 6360
   agggtttgta actgcgggtc aaggatctgg atttcgatca cggcacgatc atcgtgcggg 6420
   agggcaaggg ctccaaggat cgggccttga tgttacccga gagcttggca cccagcctgc 6480
   gcgagcaggg gaattgatcc ggtggatgac cttttgaatg acctttaata gattatatta 6540
   ctaattaatt ggggacccta gaggtcccct tttttatttt aaaaattttt tcacaaaacg 6600
   gtttacaagc ataacgggtt ttgctgcccg caaacgggct gttctggtgt tgctagtttg 6660
   ttatcagaat cgcagatccg gcttcaggtt tgccggctga aagcgctatt tcttccagaa 6720
   ttgccatgat ttttccccca cgggaggcgt cactggctcc cgtgttgtcg gcagctttga 6780
   ttcgataagc agcatcgcct gtttcaggct gtctatgtgt gactgttgag ctgtaacaag 6840
   ttgtctcagg tgttcaattt catgttctag ttgctttgtt ttactggttt cacctgttct 6900
   attaggtgtt acatgctgtt catctgttac attgtcgatc tgttcatggt gaacagcttt 6960
   aaatgcacca aaaactcgta aaagctctga tgtatctatc ttttttacac cgttttcatc 7020
   tgtgcatatg gacagttttc cctttgatat ctaacggtga acagttgttc tacttttgtt 7080
   tgttagtctt gatgcttcac tgatagatac aagagccata agaacctcag atccttccgt 7140
   atttagccag tatgttctct agtgtggttc gttgtttttg cgtgagccat gagaacgaac 7200
   cattgagatc atgcttactt tgcatgtcac tcaaaaattt tgcctcaaaa ctggtgagct 7260
   gaatttttgc agttaaagca tcgtgtagtg tttttcttag tccgttacgt aggtaggaat 7320
   ctgatgtaat ggttgttggt attttgtcac cattcatttt tatctggttg ttctcaagtt 7380
   cggttacgag atccatttgt ctatctagtt caacttggaa aatcaacgta tcagtcgggc 7440
   ggcctcgctt atcaaccacc aatttcatat tgctgtaagt gtttaaatct ttacttattg 7500
   gtttcaaaac ccattggtta agccttttaa actcatggta gttattttca agcattaaca 7560
   tgaacttaaa ttcatcaagg ctaatctcta tatttgcctt gtgagttttc ttttgtgtta 7620
   gttcttttaa taaccactca taaatcctca tagagtattt gttttcaaaa gacttaacat 7680
   gttccagatt atattttatg aattttttta actggaaaag ataaggcaat atctcttcac 7740
   taaaaactaa ttctaatttt tcgcttgaga acttggcata gtttgtccac tggaaaatct 7800
   caaagccttt aaccaaagga ttcctgattt ccacagttct cgtcatcagc tctctggttg 7860
   ctttagctaa tacaccataa gcattttccc tactgatgtt catcatctga gcgtattggt 7920
   tataagtgaa cgataccgtc cgttctttcc ttgtagggtt ttcaatcgtg gggttgagta 7980
   gtgccacaca gcataaaatt agcttggttt catgctccgt taagtcatag cgactaatcg 8040
   ctagttcatt tgctttgaaa acaactaatt cagacataca tctcaattgg tctaggtgat 8100
   tttaatcact ataccaattg agatgggcta gtcaatgata attactagtc cttttccttt 8160
   gagttgtggg tatctgtaaa ttctgctaga cctttgctgg aaaacttgta aattctgcta 8220
   gaccctctgt aaattccgct agacctttgt gtgttttttt tgtttatatt caagtggtta 8280
   taatttatag aataaagaaa gaataaaaaa agataaaaag aatagatccc agccctgtgt 8340
   ataactcact actttagtca gttccgcagt attacaaaag gatgtcgcaa acgctgtttg 8400
   ctcctctaca aaacagacct taaaacccta aaggcttaag tagcaccctc gcaagctcgg 8460
   gcaaatcgct gaatattcct tttgtctccg accatcaggc acctgagtcg ctgtcttttt 8520
   cgtgacattc agttcgctgc gctcacggct ctggcagtga atgggggtaa atggcactac 8580
   aggcgccttt tatggattca tgcaaggaaa ctacccataa tacaagaaaa gcccgtcacg 8640
   ggcttctcag ggcgttttat ggcgggtctg ctatgtggtg ctatctgact ttttgctgtt 8700
   cagcagttcc tgccctctga ttttccagtc tgaccacttc ggattatccc gtgacaggtc 8760
   attcagactg gctaatgcac ccagtaaggc agcggtatca tcaacaggct tagtttaaac 8820
   cgcaaagtcc cgcttcgtga aaattttcgt gccgcgtgat tttccgccaa aaactttaac 8880
   gaacgttcgt tataatggtg tcatgacctt cacgacgaag tactaaaatt ggcccgaatc 8940
   atcagctatg gatctctctg atgtcgcgct ggagtccgac gcgctcgatg ctgccgtcga 9000
   tttaaaaacg gtgatcggat ttttccgagc tctcgatacg acggacgcgc cagcatcacg 9060
   agactgggcc agtgccgcga gcgacctaga aactctcgtg gcggatcttg aggagctggc 9120
   tgacgagctg cgtgctcggc cagcgccagg aggacgcaca gtagtggagg atgcaatcag 9180
   ttgcgcctac tgcggtggcc tgattcctcc ccggcctgac ccgcgaggac ggcgcgcaaa 9240
   atattgctca gatgcgtgtc gtgccgcagc cagccgcgag cgcgccaaca aacgccacgc 9300
   cgaggagctg gaggcggcta ggtcgcaaat ggcgctggaa gtgcgtcccc cgagcgaaat 9360
   tttggccatg gtcgtcacag agctggaagc ggcagcgaga attatcgcga tcgtggcggt 9420
   gcccgcaggc atgacaaaca tcgtaaatgc cgcgtttcgt gtgccgtggc cgcccaggac 9480
   gtgtcagcgc cgccaccacc tgcaccgaat cggcagcagc gtcgcgcgtc gaaaaagcgc 9540
   acaggcggca agaagcgata agctgcacga atacctgaaa aatgttgaac gccccgtgag 9600
   cggtaactca cagggcgtcg gctaaccccc agtccaaacc tgggagaaag cgctcaaaaa 9660
   tgactctagc ggattcacga gacattgaca caccggcctg gaaattttcc gctgatctgt 9720
   tcgacaccca tcccgagctc gcgctgcgat cacgtggctg gacgagcgaa gaccgccgcg 9780
   aattcctcgc tcacctgggc agagaaaatt tccagggcag caagacccgc gacttcgcca 9840
   gcgcttggat caaagacccg gacacggaga aacacagccg aagttatacc gagttggttc 9900
   aaaatcgctt gcccggtgcc agtatgttgc tctgacgcac gcgcagcacg cagccgtgct 9960
   tgtcctggac attgatgtgc cgagccacca ggccggcggg aaaatcgagc acgtaaaccc 10020
   cgaggtctac gcgattttgg agcgctgggc acgcctggaa aaagcgccag cttggatcgg 10080
   cgtgaatcca ctgagcggga aatgccagct catctggctc attgatccgg tgtatgccgc 10140
   agcaggcatg agcagcccga atatgcgcct gctggctgca acgaccgagg aaatgacccg 10200
   cgttttcggc gctgaccagg ctttttcaca taggctgagc cgtggccact gcactctccg 10260
   acgatcccag ccgtaccgct ggcatgccca gcacaatcgc gtggatcgcc tagctgatct 10320
   tatggaggtt gctcgcatga tctcaggcac agaaaaacct aaaaaacgct atgagcagga 10380
   gttttctagc ggacgggcac gtatcgaagc ggcaagaaaa gccactgcgg aagcaaaagc 10440
   acttgccacg cttgaagcaa gcctgccgag cgccgctgaa gcgtctggag agctgatcga 10500
   cggcgtccgt gtcctctgga ctgctccagg gcgtgccgcc cgtgatgaga cggcttttcg 10560
   ccacgctttg actgtgggat accagttaaa agcggctggt gagcgcctaa aagacaccaa 10620
   gggtcatcga gcctacgagc gtgcctacac cgtcgctcag gcggtcggag gaggccgtga 10680
   gcctgatctg ccgccggact gtgaccgcca gacggattgg ccgcgacgtg tgcgcggcta 10740
   cgtcgctaaa ggccagccag tcgtccctgc tcgtcagaca gagacgcaga gccagccgag 10800
   gcgaaaagct ctggccacta tgggaagacg tggcggtaaa aaggccgcag aacgctggaa 10860
   agacccaaac agtgagtacg cccgagcaca gcgagaaaaa ctagctaagt ccagtcaacg 10920
   acaagctagg aaagctaaag gaaatcgctt gaccattgca ggttggttta tgactgttga 10980
   gggagagact ggctcgtggc cgacaatcaa tgaagctatg tctgaattta gcgtgtcacg 11040
   tcagaccgtg aatagagcac ttaaggtctg cgggcattga acttccacga ggacgccgaa 11100
   agcttcccag taaatgtgcc atctcgtagg cagaaaacgg ttcccccgta gggtctctct 11160
   cttggcctcc tttctaggtc gggctgattg ctcttgaagc tctctagggg ggctcacacc 11220
   ataggcagat aacgttcccc accggctcgc ctcgtaagcg cacaaggact gctcccaaag 11280
   atcttcaaag ccactgccgc gactgccttc gcgaagcctt gccccgcgga aatttcctcc  11340
   accgagttcg tgcacacccc tatgccaagc ttctttcacc ctaaattcga gagattggat  11400
   tcttaccgtg gaaattcttc gcaaaaatcg tcccctgatc gcccttgcga cgttggcgtc  11460
   ggtgccgctg gttgcgcttg gcttgaccga c                                 11491
<210> 35
   <211> 11639
   <212> DNA
   <213> artificial
<220>
   <223> Plasmid pOM616
<400> 35
   ttgatcagcg gccgcttcgc gaagcttgtc gaccgaaaca gcagttataa ggcatgaagc     60
   tgtccggttt ttgcaaaagt ggctgtgact gtaaaaagaa atcgaaaaag accgttttgt    120
   gtgaaaacgg tctttttgtt tccttttaac caactgccat aactcgaggc tattgacgac    180
   agctatggtt cactgtccac caaccaaaac tgtgctcagt accgccaata tttctccctt    240
   gaggggtaca aagaggtgtc cctagaagag atccacgctg tgtaaaaatt ttacaaaaag    300
   gtattgactt tccctacagg gtgtgtaata atttaattac aggcgggggc aaccccgcct    360
   gttctagaag gaggtgatag tatgtcttct gcagctaccc gccgtaattc agcccccttc    420
   gttcagcgcc acatcggccc aaaccaggcc gatacccagg agatcctcga ttacctgggc    480
   tatgaatctt ccgccgcgct ggccgacgat gccctcccga agtcgatccg ccaggcaggc    540
   ccgatcggcc tgccggaggc actggatgag acggacaccc tggccgccct gcgtgcttac    600
   gctgacaaga acgtgcagaa gcagcagctg atcggcaacg gttacttcga cacgatcact    660
   ccggccgtga ttcgccgcaa cgtggtggag aacccgggct ggtacaccgc ctacaccccc    720
   taccagccgg aaatctccca ggggcgcctc gaggccctgc tgaacttcca gacgatggtg    780
   caggacctga ccggcctgcc agtggccggt gcttcgctgt tagacgaagc caccgcagtg    840
   gccgaggccg tgcagctgat ggctcgcggc aatgcgaagg ctgccaagaa gggcggcgtg    900
   gtgctgctgg attcctccct gcaccagcag tccatcaccg taaccctggc gcgcgctgag    960
   gctgcgggta tcccggtgga ggtcgtggac ctggacggcg aagatgctac cgccgcgttt   1020
   gagggccgcg agaacctcgt cggcgtggtg ctttccaacc ccggctccac cggccgcgtc   1080
   cgcgacctgt ccggtctgat ctctgcggcg aaggagaccg gcgctctggt gacggtcgct   1140
   tgtgacctgc tggctcaggt tctggtgacc tccccgggct cccaaggtgc cgacattgct   1200
   gtcggctccg cccagcgctt cggcgtgccg ctattcttcg gtggcccgca cgcgggcttc   1260
   atctcctgca ccgaggctct gcagcgtaag ctgccgggcc gcatcgtggg cgtgtccgtg   1320
   gatgccgagg gcaccccggc ctaccgcttg gctctgcaga cccgtgagca gcacattcgc   1380
   cgcgacaagg ccaccagtaa catctgtacc gctcaggctc tgctggccgt cgttgccggt 1440
   ttctacgcgg tctggcacgg cccagccggc ctgcgcgcca tcgccgaggg ggtgcacgcc 1500
   cgcgcgaccg ccctggccgt tgccctgtcc gaggctggcc tgacgctggc gcacgatacc 1560
   ttcttcgaca ccgtcaccgt tgacgtgtct ggttcttctc ttggtgacgc ccccacggct 1620
   ctgcgcgccg cagccgaggc aggctacaac ctgcgccagg ttaacgattc cttcgtcggc 1680
   atctctgtcg gcgagtccac cacggacgag gacattgcca agctgatcga ggtgctgggc 1740
   tcccgcaccg gcgaggtcaa ctccgcgagc ttcgacgtca ccgccggccc gctgggcgag 1800
   gccggcgtgc tgcgcgcgga ggacgaggag attctgaccc acccgatctt caccgccatc 1860
   acttccgaga cccagatgat gcgctacatg cgcaagctgg ccgaccgcga cctggcgctg 1920
   gatcgtacga tgatcccgct gggctcctgc accatgaagc tgaacgcggc cgtctccatg 1980
   gagccgatca cctggcctgg cttcgcaggc atccacccgc acgtcccggc cgagcaggcg 2040
   cagggctggc tggagctcat cgaggacctg gaggagcgcc tggcgaagat caccggctac 2100
   gccaaggttt ccgtccagcc gaacgcgggc tcccagggcg agttcgccgg cctgctggcg 2160
   atccaccgct accaccagtc ccgcggcgac gatcagcgtg acatcgttct gatcccggcc 2220
   tccgcgcacg gcaccaacgc tgcctctgca gcgctggcgg gcctgaaggt cgtggctgtg 2280
   aagaatgccg aagacggctc catcgacgtg ccggacctgg aggccaagct ggagaagtac 2340
   ggcgagcaga ccgccgccat catgctgacc tacccctcca cccacggcgt gttcgaggag 2400
   caggtgcgcg acgtctgcca gaaggttcac gacgctggcg gccaggtgta cgtcgacggc 2460
   gctaacctga acgccctggt cggcctggcc cagccgggcg agttcggcgg cgacgtatcc 2520
   cacctgaacc tgcacaagac cttcaccatc ccgcacggtg gtggcggccc gggcgttggc 2580
   ccggtgtgcg tggcagagca cctgatcccg ttcctgccca ccgacccgaa cgccgacgtt 2640
   atcgagggcg atgctgccct gcagtccggc cagccggtct ccggcgcgca gtacggctcc 2700
   gctggcgtgc tgccgatcac ctggtcctac atcgcacaga tgggcgacga aggcctgact 2760
   gaggcctccc gcatggccct ggtgaacgcc aactacgttt cccgcaagct ggaggactac 2820
   tacccgacgc tgtacaaggg cgacaccggc ctggtcgccc acgagtgcat cctggacctg 2880
   cgtgaactca cgaaggcctc cggcatcacc gccgaggacg tatccaagcg cctgatggac 2940
   ttcggcttcc acgccccgac cttggccttc ccggtcgccg gcaccctgat gatggagccc 3000
   accgagtcgg aggacaagga agagctggac cgcttcatcg aagcgatgat caccatccac 3060
   ggggagatcc aggaagttat cgacggcaag gtcaccgccg aacagtccgt tctgcgccac 3120
   gcgccgttta ccgcctattc tgttgtgcgg gacgacttcg aggaagctgt atccggtggt 3180
   cacttcagcc gtgccaaggc cgcatacccg gtggctagcc tgcgacacac caagtacttc 3240
   acccccgtgc gtcgtatcga caacgcctat ggcgaccgca atctcgtgtg cacctgcccg 3300
   cccctggaag acttcgcaat taacgaggac tagggtccgc tggagctaag ggcgtcacaa 3360
   agagaagaag gagaacaaga catgaccgaa cttaagaaga ccgcgctgca cctggtgcac 3420
   gagaagttgg gcgcgcgatt taccgacttc ggcggctggg acatgcctct gaagtacagc 3480
   agtgagctgg acgagcacca cgctgtacgc aatgccgtgg gcgtattcga cctctcccac 3540
   atgggtgagg ttcgcgtgac cggcccgcag gcagcggagt tcctggacca cgcgctgatt 3600
   tcgaagctgt cggcagtgaa ggtcggcaag gcgaagtact cgatgatctg caccgaatcc 3660
   ggtggcatca tcgacgacct gatcacctac cgcctgggcg acaacgagtt cctgatcgtg 3720
   ccgaacgcgg gcaacgtgga caacgtggtc tccgcactgc agggccgcac cgagggcttt 3780
   gacgtggagg ttaacaacga gtccgatgcg acctccatga tcgccgtaca ggggcccaag 3840
   gccgcgcagg cgatgctgga gatcgtggag aacgtcgtgg atgcacccga ggcatccggc 3900
   gcgggcgaga ccgttgccga ggctatcgag gggctgggtt actacgcggc attcagcggt 3960
   gttgccgcag gtcagcccgt gctggtggcc cgcacaggct ataccggcga ggacggtttc 4020
   gagctgatcg tggctaacga tggtgcggag accgtgtgga ccaaggctat ggaccaggct 4080
   gcgcagctgg gtggcctgcc gtgtggcctg gcctgccgcg acaccctgcg cctggaggct 4140
   ggcatgccgc tgtacggcaa cgagctatcg ctgaagctca ccccggtcga tgctgggctg 4200
   ggcattcttg cggcgacgaa gtctaaggac tctttcgttg gtcgtgacgc catcgtttcc 4260
   gccaaggaaa agggtaccca gcaggtactt atcgggctgg cgggcgaggg tcgccgcgct 4320
   gcccgtgggg gatacgaggt gtttgccggt gacggcgaga aggccatcgg tgccgtgacc 4380
   tccggtgcac tgtcgccgac gctgggccac ccggtggcat tggcatacgt cgcgaagtcc 4440
   gcagtgtcct ccggcgcggc cgctgagggt gcgaccgtgg aggtagacat ccgcggcaag 4500
   cgctttgaat acaaggttgt ggcgctgccg ttctactccc gcgagaagta acgcaagaag 4560
   taacgaaaag ggttgtctgc ccgctggtgt aaggtttggg cagacaacca cagaagtaac 4620
   cacacaaggt ttcacaagag aggcttaaaa caatgactgc actgccaact gacttcctgt 4680
   actccgaaga gcacgagtgg gttaacacct ccgctgttgt tgagggcgag accgtgcgcg 4740
   tgggcattac ccacatcgcc gctgaggcgc tgggtgacat cgtgttcgtc gagctgccgg 4800
   aggttggctc cgaggttgag gccggcgagg ctttcggcga ggttgagtcc accaagtccg 4860
   tttccgacat ctacgcaccg gtttctggcg aggttgtggc tgtcaacgag gcgctggaag 4920
   acaacgctgg cctgatcaac gaagatccat acggtgaggg ctggctgtac gaggtcaagg 4980
   tgaccgaggc cggcgagctg atggaggctg aggcttacca ggcggctaac gagtaaaaca 5040
   agaccctatg gtcggttcca tgttcgcatt aatcctcggc ttggccctgt tgggccttgc 5100
   cgtgtggtta ctcctgcgag caaaagatag caccggatcc gccctcccgc acgctttgcg 5160
   ggagggcttt tcttttcccg gtatttaaat cgctagcggg ctgctaaagg aagcggaaca 5220
   cgtagaaagc cagtccgcag aaacggtgct gaccccggat gaatgtcagc tactgggcta 5280
   tctggacaag ggaaaacgca agcgcaaaga gaaagcaggt agcttgcagt gggcttacat 5340
   ggcgatagct agactgggcg gttttatgga cagcaagcga accggaattg ccagctgggg 5400
   cgccctctgg taaggttggg aagccctgca aagtaaactg gatggctttc ttgccgccaa 5460
   ggatctgatg gcgcagggga tcaagatctg atcaagagac aggatgagga tcgtttcgca 5520
   tgattgaaca agatggattg cacgcaggtt ctccggccgc ttgggtggag aggctattcg 5580
   gctatgactg ggcacaacag acaatcggct gctctgatgc cgccgtgttc cggctgtcag 5640
   cgcaggggcg cccggttctt tttgtcaaga ccgacctgtc cggtgccctg aatgaactgc 5700
   aggacgaggc agcgcggcta tcgtggctgg ccacgacggg cgttccttgc gcagctgtgc 5760
   tcgacgttgt cactgaagcg ggaagggact ggctgctatt gggcgaagtg ccggggcagg 5820
   atctcctgtc atctcacctt gctcctgccg agaaagtatc catcatggct gatgcaatgc 5880
   ggcggctgca tacgcttgat ccggctacct gcccattcga ccaccaagcg aaacatcgca 5940
   tcgagcgagc acgtactcgg atggaagccg gtcttgtcga tcaggatgat ctggacgaag 6000
   agcatcaggg gctcgcgcca gccgaactgt tcgccaggct caaggcgcgc atgcccgacg 6060
   gcgaggatct cgtcgtgacc catggcgatg cctgcttgcc gaatatcatg gtggaaaatg 6120
   gccgcttttc tggattcatc gactgtggcc ggctgggtgt ggcggaccgc tatcaggaca 6180
   tagcgttggc tacccgtgat attgctgaag agcttggcgg cgaatgggct gaccgcttcc 6240
   tcgtgcttta cggtatcgcc gctcccgatt cgcagcgcat cgccttctat cgccttcttg 6300
   acgagttctt ctgagcggga ctctggggtt cgaaatgacc gaccaagcga cgcccaacct 6360
   gccatcacga gatttcgatt ccaccgccgc cttctatgaa aggttgggct tcggaatcgt 6420
   tttccgggac gccggctgga tgatcctcca gcgcggggat ctcatgctgg agttcttcgc 6480
   ccacgctagt ttaaactgcg gatcagtgag ggtttgtaac tgcgggtcaa ggatctggat 6540
   ttcgatcacg gcacgatcat cgtgcgggag ggcaagggct ccaaggatcg ggccttgatg 6600
   ttacccgaga gcttggcacc cagcctgcgc gagcagggga attgatccgg tggatgacct 6660
   tttgaatgac ctttaataga ttatattact aattaattgg ggaccctaga ggtccccttt 6720
   tttattttaa aaattttttc acaaaacggt ttacaagcat aacgggtttt gctgcccgca 6780
   aacgggctgt tctggtgttg ctagtttgtt atcagaatcg cagatccggc ttcaggtttg 6840
   ccggctgaaa gcgctatttc ttccagaatt gccatgattt tttccccacg ggaggcgtca 6900
   ctggctcccg tgttgtcggc agctttgatt cgataagcag catcgcctgt ttcaggctgt 6960
   ctatgtgtga ctgttgagct gtaacaagtt gtctcaggtg ttcaatttca tgttctagtt 7020
   gctttgtttt actggtttca cctgttctat taggtgttac atgctgttca tctgttacat 7080
   tgtcgatctg ttcatggtga acagctttaa atgcaccaaa aactcgtaaa agctctgatg 7140
   tatctatctt ttttacaccg ttttcatctg tgcatatgga cagttttccc tttgatatct 7200
   aacggtgaac agttgttcta cttttgtttg ttagtcttga tgcttcactg atagatacaa 7260
   gagccataag aacctcagat ccttccgtat ttagccagta tgttctctag tgtggttcgt 7320
   tgtttttgcg tgagccatga gaacgaacca ttgagatcat gcttactttg catgtcactc 7380
   aaaaattttg cctcaaaact ggtgagctga atttttgcag ttaaagcatc gtgtagtgtt 7440
   tttcttagtc cgttacgtag gtaggaatct gatgtaatgg ttgttggtat tttgtcacca 7500
   ttcattttta tctggttgtt ctcaagttcg gttacgagat ccatttgtct atctagttca 7560
   acttggaaaa tcaacgtatc agtcgggcgg cctcgcttat caaccaccaa tttcatattg 7620
   ctgtaagtgt ttaaatcttt acttattggt ttcaaaaccc attggttaag ccttttaaac 7680
   tcatggtagt tattttcaag cattaacatg aacttaaatt catcaaggct aatctctata 7740
   tttgccttgt gagttttctt ttgtgttagt tcttttaata accactcata aatcctcata 7800
   gagtatttgt tttcaaaaga cttaacatgt tccagattat attttatgaa tttttttaac 7860
   tggaaaagat aaggcaatat ctcttcacta aaaactaatt ctaatttttc gcttgagaac 7920
   ttggcatagt ttgtccactg gaaaatctca aagcctttaa ccaaaggatt cctgatttcc 7980
   acagttctcg tcatcagctc tctggttgct ttagctaata caccataagc attttcccta 8040
   ctgatgttca tcatctgagc gtattggtta taagtgaacg ataccgtccg ttctttcctt 8100
   gtagggtttt caatcgtggg gttgagtagt gccacacagc ataaaattag cttggtttca 8160
   tgctccgtta agtcatagcg actaatcgct agttcatttg ctttgaaaac aactaattca 8220
   gacatacatc tcaattggtc taggtgattt taatcactat accaattgag atgggctagt 8280
   caatgataat tactagtcct tttcctttga gttgtgggta tctgtaaatt ctgctagacc 8340
   tttgctggaa aacttgtaaa ttctgctaga ccctctgtaa attccgctag acctttgtgt 8400
   gttttttttg tttatattca agtggttata atttatagaa taaagaaaga ataaaaaaag 8460
   ataaaaagaa tagatcccag ccctgtgtat aactcactac tttagtcagt tccgcagtat 8520
   tacaaaagga tgtcgcaaac gctgtttgct cctctacaaa acagacctta aaaccctaaa 8580
   ggcttaagta gcaccctcgc aagctcgggc aaatcgctga atattccttt tgtctccgac 8640
   catcaggcac ctgagtcgct gtctttttcg tgacattcag ttcgctgcgc tcacggctct 8700
   ggcagtgaat gggggtaaat ggcactacag gcgcctttta tggattcatg caaggaaact 8760
   acccataata caagaaaagc ccgtcacggg cttctcaggg cgttttatgg cgggtctgct 8820
   atgtggtgct atctgacttt ttgctgttca gcagttcctg ccctctgatt ttccagtctg 8880
   accacttcgg attatcccgt gacaggtcat tcagactggc taatgcaccc agtaaggcag 8940
   cggtatcatc aacaggctta gtttaaaccg caaagtcccg cttcgtgaaa attttcgtgc 9000
   cgcgtgattt tccgccaaaa actttaacga acgttcgtta taatggtgtc atgaccttca 9060
   cgacgaagta ctaaaattgg cccgaatcat cagctatgga tctctctgat gtcgcgctgg 9120
   agtccgacgc gctcgatgct gccgtcgatt taaaaacggt gatcggattt ttccgagctc 9180
   tcgatacgac ggacgcgcca gcatcacgag actgggccag tgccgcgagc gacctagaaa 9240
   ctctcgtggc ggatcttgag gagctggctg acgagctgcg tgctcggcca gcgccaggag 9300
   gacgcacagt agtggaggat gcaatcagtt gcgcctactg cggtggcctg attcctcccc 9360
   ggcctgaccc gcgaggacgg cgcgcaaaat attgctcaga tgcgtgtcgt gccgcagcca 9420
   gccgcgagcg cgccaacaaa cgccacgccg aggagctgga ggcggctagg tcgcaaatgg 9480
   cgctggaagt gcgtcccccg agcgaaattt tggccatggt cgtcacagag ctggaagcgg 9540
   cagcgagaat tatcgcgatc gtggcggtgc ccgcaggcat gacaaacatc gtaaatgccg 9600
   cgtttcgtgt gccgtggccg cccaggacgt gtcagcgccg ccaccacctg caccgaatcg 9660
   gcagcagcgt cgcgcgtcga aaaagcgcac aggcggcaag aagcgataag ctgcacgaat 9720
   acctgaaaaa tgttgaacgc cccgtgagcg gtaactcaca gggcgtcggc taacccccag 9780
   tccaaacctg ggagaaagcg ctcaaaaatg actctagcgg attcacgaga cattgacaca 9840
   ccggcctgga aattttccgc tgatctgttc gacacccatc ccgagctcgc gctgcgatca 9900
   cgtggctgga cgagcgaaga ccgccgcgaa ttcctcgctc acctgggcag agaaaatttc 9960
   cagggcagca agacccgcga cttcgccagc gcttggatca aagacccgga cacggagaaa 10020
   cacagccgaa gttataccga gttggttcaa aatcgcttgc ccggtgccag tatgttgctc 10080
   tgacgcacgc gcagcacgca gccgtgcttg tcctggacat tgatgtgccg agccaccagg 10140
   ccggcgggaa aatcgagcac gtaaaccccg aggtctacgc gattttggag cgctgggcac 10200
   gcctggaaaa agcgccagct tggatcggcg tgaatccact gagcgggaaa tgccagctca 10260
   tctggctcat tgatccggtg tatgccgcag caggcatgag cagcccgaat atgcgcctgc 10320
   tggctgcaac gaccgaggaa atgacccgcg ttttcggcgc tgaccaggct ttttcacata 10380
   ggctgagccg tggccactgc actctccgac gatcccagcc gtaccgctgg catgcccagc 10440
   acaatcgcgt ggatcgccta gctgatctta tggaggttgc tcgcatgatc tcaggcacag 10500
   aaaaacctaa aaaacgctat gagcaggagt tttctagcgg acgggcacgt atcgaagcgg 10560
   caagaaaagc cactgcggaa gcaaaagcac ttgccacgct tgaagcaagc ctgccgagcg 10620
   ccgctgaagc gtctggagag ctgatcgacg gcgtccgtgt cctctggact gctccagggc 10680
   gtgccgcccg tgatgagacg gcttttcgcc acgctttgac tgtgggatac cagttaaaag 10740
   cggctggtga gcgcctaaaa gacaccaagg gtcatcgagc ctacgagcgt gcctacaccg 10800
   tcgctcaggc ggtcggagga ggccgtgagc ctgatctgcc gccggactgt gaccgccaga 10860
   cggattggcc gcgacgtgtg cgcggctacg tcgctaaagg ccagccagtc gtccctgctc 10920
   gtcagacaga gacgcagagc cagccgaggc gaaaagctct ggccactatg ggaagacgtg 10980
   gcggtaaaaa ggccgcagaa cgctggaaag acccaaacag tgagtacgcc cgagcacagc 11040
   gagaaaaact agctaagtcc agtcaacgac aagctaggaa agctaaagga aatcgcttga 11100
   ccattgcagg ttggtttatg actgttgagg gagagactgg ctcgtggccg acaatcaatg 11160
   aagctatgtc tgaatttagc gtgtcacgtc agaccgtgaa tagagcactt aaggtctgcg 11220
   ggcattgaac ttccacgagg acgccgaaag cttcccagta aatgtgccat ctcgtaggca 11280
   gaaaacggtt cccccgtagg gtctctctct tggcctcctt tctaggtcgg gctgattgct 11340
   cttgaagctc tctagggggg ctcacaccat aggcagataa cgttccccac cggctcgcct 11400
   cgtaagcgca caaggactgc tcccaaagat cttcaaagcc actgccgcga ctgccttcgc 11460
   gaagccttgc cccgcggaaa tttcctccac cgagttcgtg cacaccccta tgccaagctt 11520
   ctttcaccct aaattcgaga gattggattc ttaccgtgga aattcttcgc aaaaatcgtc 11580
   ccctgatcgc ccttgcgacg ttggcgtcgg tgccgctggt tgcgcttggc ttgaccgac 11639
<210> 36
   <211> 13915
   <212> DNA
   <213> artificial
<220>
   <223> Plasmid pOM620AF
<400> 36
   ttgatcagcg gccgcacagc gatcccagag gaaatatcct ctggggtcgc tgtgtcgacc     60
   ttaaagtttg gctgccatgt gaatttttag caccctcaac agttgagtgc tggcactctc    120
   gggggtagag tgccaaatag gttgtttgac acacagttgt tcacccgcga cgacggctgt    180
   gctggaaacc cacaaccggc acacacaaaa tttttctaga aggaggtgat agtatgtctt    240
   ctgcagctac ccgccgtaat tcagccccct tcgttcagcg ccacatcggc ccaaaccagg    300
   ccgataccca ggagatcctc gattacctgg gctatgaatc ttccgccgcg ctggccgacg    360
   atgccctccc gaagtcgatc cgccaggcag gcccgatcgg cctgccggag gcactggatg    420
   agacggacac cctggccgcc ctgcgtgctt acgctgacaa gaacgtgcag aagcagcagc    480
   tgatcggcaa cggttacttc gacacgatca ctccggccgt gattcgccgc aacgtggtgg    540
   agaacccggg ctggtacacc gcctacaccc cctaccagcc ggaaatctcc caggggcgcc    600
   tcgaggccct gctgaacttc cagacgatgg tgcaggacct gaccggcctg ccagtggccg    660
   gtgcttcgct gttagacgaa gccaccgcag tggccgaggc cgtgcagctg atggctcgcg    720
   gcaatgcgaa ggctgccaag aagggcggcg tggtgctgct ggattcctcc ctgcaccagc    780
   agtccatcac cgtaaccctg gcgcgcgctg aggctgcggg tatcccggtg gaggtcgtgg    840
   acctggacgg cgaagatgct accgccgcgt ttgagggccg cgagaacctc gtcggcgtgg    900
   tgctttccaa ccccggctcc accggccgcg tccgcgacct gtccggtctg atctctgcgg    960
   cgaaggagac cggcgctctg gtgacggtcg cttgtgacct gctggctcag gttctggtga   1020
   cctccccggg ctcccaaggt gccgacattg ctgtcggctc cgcccagcgc ttcggcgtgc   1080
   cgctattctt cggtggcccg cacgcgggct tcatctcctg caccgaggct ctgcagcgta   1140
   agctgccggg ccgcatcgtg ggcgtgtccg tggatgccga gggcaccccg gcctaccgct 1200
   tggctctgca gacccgtgag cagcacattc gccgcgacaa ggccaccagt aacatctgta 1260
   ccgctcaggc tctgctggcc gtcgttgccg gtttctacgc ggtctggcac ggcccagccg 1320
   gcctgcgcgc catcgccgag gggtgcacgc cccgcgcgac cgccctggcc gttgccctgt 1380
   ccgaggctgg cctgacgctg gcgcacgata ccttcttcga caccgtcacc gttgacgtgt 1440
   ctggttcttc tcttggtgac gcccccacgg ctctgcgcgc cgcagccgag gcaggctaca 1500
   acctgcgcca ggttaacgat tccttcgtcg gcatctctgt cggcgagtcc accacggacg 1560
   aggacattgc caagctgatc gaggtgctgg gctcccgcac cggcgaggtc aactccgcga 1620
   gcttcgacgt caccgccggc ccgctgggcg aggccggcgt gctgcgcgcg gaggacgagg 1680
   agattctgac ccacccgatc ttcaccgcca tcacttccga gacccagatg atgcgctaca 1740
   tgcgcaagct ggccgaccgc gacctggcgc tggatcgtac gatgatcccg ctgggctcct 1800
   gcaccatgaa gctgaacgcg gccgtctcca tggagccgat cacctggcct ggcttcgcag 1860
   gcatccaccc gcacgtcccg gccgagcagg cgcagggctg gctggagctc atcgaggacc 1920
   tggaggagcg cctggcgaag atcaccggct acgccaaggt ttccgtccag ccgaacgcgg 1980
   gctcccaggg cgagttcgcc ggcctgctgg cgatccaccg ctaccaccag tcccgcggcg 2040
   acgatcagcg tgacatcgtt ctgatcccgg cctccgcgca cggcaccaac gctgcctctg 2100
   cagcgctggc gggcctgaag gtcgtggctg tgaagaatgc cgaagacggc tccatcgacg 2160
   tgccggacct ggaggccaag ctggagaagt acggcgagca gaccgccgcc atcatgctga 2220
   cctacccctc cacccacggc gtgttcgagg agcaggtgcg cgacgtctgc cagaaggttc 2280
   acgacgctgg cggccaggtg tacgtcgacg gcgctaacct gaacgccctg gtcggcctgg 2340
   cccagccggg cgagttcggc ggcgacgtat cccacctgaa cctgcacaag accttcacca 2400
   tcccgcacgg tggtggcggc ccgggcgttg gcccggtgtg cgtggcagag cacctgatcc 2460
   cgttcctgcc caccgacccg aacgccgacg ttatcgaggg cgatgctgcc ctgcagtccg 2520
   gccagccggt ctccggcgcg cagtacggct ccgctggcgt gctgccgatc acctggtcct 2580
   acatcgcaca gatgggcgac gaaggcctga ctgaggcctc ccgcatggcc ctggtgaacg 2640
   ccaactacgt ttcccgcaag ctggaggact actacccgac gctgtacaag ggcgacaccg 2700
   gcctggtcgc ccacgagtgc atcctggacc tgcgtgaact cacgaaggcc tccggcatca 2760
   ccgccgagga cgtatccaag cgcctgatgg acttcggctt ccacgccccg accttggcct 2820
   tcccggtcgc cggcaccctg atgatggagc ccaccgagtc ggaggacaag gaagagctgg 2880
   accgcttcat cgaagcgatg atcaccatcc acggggagat ccaggaagtt atcgacggca 2940
   aggtcaccgc cgaacagtcc gttctgcgcc acgcgccgtt taccgcctat tctgttgtgc 3000
   gggacgactt cgaggaagct gtatccggtg gtcacttcag ccgtgccaag gccgcatacc 3060
   cggtggctag cctgcgacac accaagtact tcacccccgt gcgtcgtatc gacaacgcct 3120
   atggcgaccg caatctcgtg tgcacctgcc cgcccctgga agacttcgca attaacgagg 3180
   actagggtcc gctggagcta agggcgtcac aaagagaaga aggagaacaa gacatgaccg 3240
   aacttaagaa gaccgcgctg cacctggtgc acgagaagtt gggcgcgcga tttaccgact 3300
   tcggcggctg ggacatgcct ctgaagtaca gcagtgagct ggacgagcac cacgctgtac 3360
   gcaatgccgt gggcgtattc gacctctccc acatgggtga ggttcgcgtg accggcccgc 3420
   aggcagcgga gttcctggac cacgcgctga tttcgaagct gtcggcagtg aaggtcggca 3480
   aggcgaagta ctcgatgatc tgcaccgaat ccggtggcat catcgacgac ctgatcacct 3540
   accgcctggg cgacaacgag ttcctgatcg tgccgaacgc gggcaacgtg gacaacgtgg 3600
   tctccgcact gcagggccgc accgagggct ttgacgtgga ggttaacaac gagtccgatg 3660
   cgacctccat gatcgccgta caggggccca aggccgcgca ggcgatgctg gagatcgtgg 3720
   agaacgtcgt ggatgcaccc gaggcatccg gcgcgggcga gaccgttgcc gaggctatcg 3780
   aggggctggg ttactacgcg gcattcagcg gtgttgccgc aggtcagccc gtgctggtgg 3840
   cccgcacagg ctataccggc gaggacggtt tcgagctgat cgtggctaac gatggtgcgg 3900
   agaccgtgtg gaccaaggct atggaccagg ctgcgcagct gggtggcctg ccgtgtggcc 3960
   tggcctgccg cgacaccctg cgcctggagg ctggcatgcc gctgtacggc aacgagctat 4020
   cgctgaagct caccccggtc gatgctgggc tgggcattct tgcggcgacg aagtctaagg 4080
   actctttcgt tggtcgtgac gccatcgttt ccgccaagga aaagggtacc cagcaggtac 4140
   ttatcgggct ggcgggcgag ggtcgccgcg ctgcccgtgg gggatacgag gtgtttgccg 4200
   gtgacggcga gaaggccatc ggtgccgtga cctccggtgc actgtcgccg acgctgggcc 4260
   acccggtggc attggcatac gtcgcgaagt ccgcagtgtc ctccggcgcg gccgctgagg 4320
   gtgcgaccgt ggaggtagac atccgcggca agcgctttga atacaaggtt gtggcgctgc 4380
   cgttctactc ccgcgagaag taacgcaaga agtaacgaaa agggttgtct gcccgctggt 4440
   gtaaggtttg ggcagacaac cacagaagta accacacaag gtttcacaag agaggcttaa 4500
   aacaatgact gcactgccaa ctgacttcct gtactccgaa gagcacgagt gggttaacac 4560
   ctccgctgtt gttgagggcg agaccgtgcg cgtgggcatt acccacatcg ccgctgaggc 4620
   gctgggtgac atcgtgttcg tcgagctgcc ggaggttggc tccgaggttg aggccggcga 4680
   ggctttcggc gaggttgagt ccaccaagtc cgtttccgac atctacgcac cggtttctgg 4740
   cgaggttgtg gctgtcaacg aggcgctgga agacaacgct ggcctgatca acgaagatcc 4800
   atacggtgag ggctggctgt acgaggtcaa ggtgaccgag gccggcgagc tgatggaggc 4860
   tgaggcttac caggcggcta acgagtaaaa caagacccta tggtcggttc catgttcgca 4920
   ttaatcctcg gcttggccct gttgggcctt gccgtgtggt tactcctgcg agcaaaagat 4980
   agcaccggat ccgccctccc gcacgctttg cgggagggct tttcttttcc cggtatttca 5040
   gtagctcggg ggcgtctcat tcagcaaacc tagctccata tccgcctctg ggctctccca 5100
   ccccgcacca aaatctctcg cgagaggcct tcagggccct taaatgtcgg aaaagttgca 5160
   atttgttaat gctactggtt ttcaaatatg caaatgcgca ggtcaagggg cttttgggaa 5220
   agtggaaacc aatcccgatt tgaagaattg caacttttcc gacattaagc ggccttcagg 5280
   aagtgccaaa caccggaggc accagcatca acgtcaccac ctggcatatc tagcaccatc 5340
   agaagcttca gcctcactac aggcggaacc tgacgccagc gggacctcac ccagcgggac 5400
   agaacccagc ggaacttggc gccagcggaa ccgcagccac cagctcccac cagcacccag 5460
   cagcgccaag gcaccactgg agtatcatcg ccaccatggg attccagcaa ggcagcatcc 5520
   gcaaagcaaa catgggcaca accggcacta acgacggggc aaccacccca ccagccaaca 5580
   ccagcacccc agccgtagac atcgacgtcc gcgacctggg caccgtggac tacgaggaca 5640
   cttggcacct ccaggcaaat ctcgccgccc agcgcgccga agaaaaaatc ccagacacca 5700
   tcctcctact ccagcatccg ccgacgtaca ccgccggcaa gcgcacccag gattccgacc 5760
   gccctaccaa cggcctgcca gtagtcgacg tcgaccgcgg aggccgcatc acctggcatg 5820
   gacccggcca gttggtcgca tatcccatca tcaagctggc cgacccggtg gacgtggtcg 5880
   attatgtccg ccgcctggag caggcgctaa tccagacttg tgaagatctt ggcctgcacg 5940
   gcaccggccg cgtagagggg cgttcgggcg tgtggctgcc tgcgggcgtc attaatggcg 6000
   agctcaagcc cgcacgtaag atagccgcga tcggcatccg cgtgacgcgc ggcgtgacca 6060
   tgcacggagt ggccctcaac tgcgataaca ccatggagta ttacgaccac attgtgcctt 6120
   gtgggctggc ggatgcgggt gtcacgacgc tcaccgagga gctggggcgc gatgttagtg 6180
   tttctgacgc ctactcatcc ctcgcccaca acctcgttga tgctttgaac ggcgacttgc 6240
   cggtgcattc ctaggggaaa acccgaggtg gattcctata cggcacccag cgtaggctag 6300
   tcggcatgag tgtgaccgca gatggacgcc ggatgctacg catcgaggcg aagaatgccg 6360
   aaacccccat cgagtcgaag cctcggtgga tccgcacgac cgcgaaggtc ggcccggagt 6420
   atcgggatat taagaatcgc gtgaagggcg ctggcctgca caccgtgtgc caagaggctg 6480
   gctgcccgaa tatcaacgag tgctgggagg accgcgaggc gacgttcctc atcggcggtg 6540
   atacgtgttc ccgccgttgc gacttctgcc agattaagtc cggccgcccg tccccgctgg 6600
   atatggacga gccacgccgc gtggcggaga atgtccgcga gatgggtctg cgctacgcca 6660
   ccatcaccgg cgtgacgcgc gatgacctgg atgatgaggg cgcgtggctg tatgccgagg 6720
   ttgtgaagaa gattcacgag ctgaacccga acacgggtgt ggagaatctg acgccagatt 6780
   tttccaaccg cccggagctg ctgaaggtcg tcttcgattc ccagccggag gtgtttgccc 6840
   acaacctgga gacagttccg cgcatcttta agcgcatccg cccggccttt aagtacgacc 6900
   gttccctgga ggtcattcag gcagctcacg attacggcct ggtgacgaag tccaacctga 6960
   tcctgggcat gggtgagaag aaggaagagg tccgcgcggc tatcaaggac ctggcagacg 7020
   ccggcaccga cattctgacg attacccagt acctgcgccc gtcttccatg caccacccga 7080
   ttgagcgttg ggtgaagccg gaggagttca tggagcactc cgacgcagcc tacgagctgg 7140
   gcatcaaggc tgttatgtcc ggcccgttgg tgcgttcctc ttaccgcgcc ggccgcctgt 7200
   acgcgcaggc caagcaggcg cgcggcgagg ctattccgga gaacctgaag cacttggagg 7260
   agactctcga ttccaccacg tcgcaggagg cctctacact gctggagcgc tacggcgctt 7320
   cggaggacac gccggtcact gcgtcgcgcc gctagctcgg cggggcactt tcgccccgcc 7380
   tttattcatg ccttattgtt gaatccatgg cgaaggataa aaaggacgta agggtcaagg 7440
   aagagaagaa ggcagctaag gcaaatcgct agcgggctgc taaaggaagc ggaacacgta 7500
   gaaagccagt ccgcagaaac ggtgctgacc ccggatgaat gtcagctact gggctatctg 7560
   gacaagggaa aacgcaagcg caaagagaaa gcaggtagct tgcagtgggc ttacatggcg 7620
   atagctagac tgggcggttt tatggacagc aagcgaaccg gaattgccag ctggggcgcc 7680
   ctctggtaag gttgggaagc cctgcaaagt aaactggatg gctttcttgc cgccaaggat 7740
   ctgatggcgc aggggatcaa gatctgatca agagacagga tgaggatcgt ttcgcatgat 7800
   tgaacaagat ggattgcacg caggttctcc ggccgcttgg gtggagaggc tattcggcta 7860
   tgactgggca caacagacaa tcggctgctc tgatgccgcc gtgttccggc tgtcagcgca 7920
   ggggcgcccg gttctttttg tcaagaccga cctgtccggt gccctgaatg aactgcagga 7980
   cgaggcagcg cggctatcgt ggctggccac gacgggcgtt ccttgcgcag ctgtgctcga 8040
   cgttgtcact gaagcgggaa gggactggct gctattgggc gaagtgccgg ggcaggatct 8100
   cctgtcatct caccttgctc ctgccgagaa agtatccatc atggctgatg caatgcggcg 8160
   gctgcatacg cttgatccgg ctacctgccc attcgaccac caagcgaaac atcgcatcga 8220
   gcgagcacgt actcggatgg aagccggtct tgtcgatcag gatgatctgg acgaagagca 8280
   tcaggggctc gcgccagccg aactgttcgc caggctcaag gcgcgcatgc ccgacggcga 8340
   ggatctcgtc gtgacccatg gcgatgcctg cttgccgaat atcatggtgg aaaatggccg 8400
   cttttctgga ttcatcgact gtggccggct gggtgtggcg gaccgctatc aggacatagc 8460
   gttggctacc cgtgatattg ctgaagagct tggcggcgaa tgggctgacc gcttcctcgt 8520
   gctttacggt atcgccgctc ccgattcgca gcgcatcgcc ttctatcgcc ttcttgacga 8580
   gttcttctga gcgggactct ggggttcgaa atgaccgacc aagcgacgcc caacctgcca 8640
   tcacgagatt tcgattccac cgccgccttc tatgaaaggt tgggcttcgg aatcgttttc 8700
   cgggacgccg gctggatgat cctccagcgc ggggatctca tgctggagtt cttcgcccac 8760
   gctagtttaa actgcggatc agtgagggtt tgtaactgcg ggtcaaggat ctggatttcg 8820
   atcacggcac gatcatcgtg cgggagggca agggctccaa ggatcgggcc ttgatgttac 8880
   ccgagagctt ggcacccagc ctgcgcgagc aggggaattg atccggtgga tgaccttttg 8940
   aatgaccttt aatagattat attactaatt aattggggac cctagaggtc ccctttttta 9000
   ttttaaaaat tttttcacaa aacggtttac aagcataacg ggttttgctg cccgcaaacg 9060
   ggctgttctg gtgttgctag tttgttatca gaatcgcaga tccggcttca ggtttgccgg 9120
   ctgaaagcgc tatttcttcc agaattgcca tgattttttc cccacgggag gcgtcactgg 9180
   ctcccgtgtt gtcggcagct ttgattcgat aagcagcatc gcctgtttca ggctgtctat 9240
   gtgtgactgt tgagctgtaa caagttgtct caggtgttca atttcatgtt ctagttgctt 9300
   tgttttactg gtttcacctg ttctattagg tgttacatgc tgttcatctg ttacattgtc 9360
   gatctgttca tggtgaacag ctttaaatgc accaaaaact cgtaaaagct ctgatgtatc 9420
   tatctttttt acaccgtttt catctgtgca tatggacagt tttccctttg atatctaacg 9480
   gtgaacagtt gttctacttt tgtttgttag tcttgatgct tcactgatag atacaagagc 9540
   cataagaacc tcagatcctt ccgtatttag ccagtatgtt ctctagtgtg gttcgttgtt 9600
   tttgcgtgag ccatgagaac gaaccattga gatcatgctt actttgcatg tcactcaaaa 9660
   attttgcctc aaaactggtg agctgaattt ttgcagttaa agcatcgtgt agtgtttttc 9720
   ttagtccgtt acgtaggtag gaatctgatg taatggttgt tggtattttg tcaccattca 9780
   tttttatctg gttgttctca agttcggtta cgagatccat ttgtctatct agttcaactt 9840
   ggaaaatcaa cgtatcagtc gggcggcctc gcttatcaac caccaatttc atattgctgt 9900
   aagtgtttaa atctttactt attggtttca aaacccattg gttaagcctt ttaaactcat 9960
   ggtagttatt ttcaagcatt aacatgaact taaattcatc aaggctaatc tctatatttg 10020
   ccttgtgagt tttcttttgt gttagttctt ttaataacca ctcataaatc ctcatagagt 10080
   atttgttttc aaaagactta acatgttcca gattatattt tatgaatttt tttaactgga 10140
   aaagataagg caatatctct tcactaaaaa ctaattctaa tttttcgctt gagaacttgg 10200
   catagtttgt ccactggaaa atctcaaagc ctttaaccaa aggattcctg atttccacag 10260
   ttctcgtcat cagctctctg gttgctttag ctaatacacc ataagcattt tccctactga 10320
   tgttcatcat ctgagcgtat tggttataag tgaacgatac cgtccgttct ttccttgtag 10380
   ggttttcaat cgtggggttg agtagtgcca cacagcataa aattagcttg gtttcatgct 10440
   ccgttaagtc atagcgacta atcgctagtt catttgcttt gaaaacaact aattcagaca 10500
   tacatctcaa ttggtctagg tgattttaat cactatacca attgagatgg gctagtcaat 10560
   gataattact agtccttttc ctttgagttg tgggtatctg taaattctgc tagacctttg 10620
   ctggaaaact tgtaaattct gctagaccct ctgtaaattc cgctagacct ttgtgtgttt 10680
   tttttgttta tattcaagtg gttataattt atagaataaa gaaagaataa aaaaagataa 10740
   aaagaataga tcccagccct gtgtataact cactacttta gtcagttccg cagtattaca 10800
   aaaggatgtc gcaaacgctg tttgctcctc tacaaaacag accttaaaac cctaaaggct 10860
   taagtagcac cctcgcaagc tcgggcaaat cgctgaatat tccttttgtc tccgaccatc 10920
   aggcacctga gtcgctgtct ttttcgtgac attcagttcg ctgcgctcac ggctctggca 10980
   gtgaatgggg gtaaatggca ctacaggcgc cttttatgga ttcatgcaag gaaactaccc 11040
   ataatacaag aaaagcccgt cacgggcttc tcagggcgtt ttatggcggg tctgctatgt 11100
   ggtgctatct gactttttgc tgttcagcag ttcctgccct ctgattttcc agtctgacca 11160
   cttcggatta tcccgtgaca ggtcattcag actggctaat gcacccagta aggcagcggt 11220
   atcatcaaca ggcttagttt aaaccgcaaa gtcccgcttc gtgaaaattt tcgtgccgcg 11280
   tgattttccg ccaaaaactt taacgaacgt tcgttataat ggtgtcatga ccttcacgac 11340
   gaagtactaa aattggcccg aatcatcagc tatggatctc tctgatgtcg cgctggagtc 11400
   cgacgcgctc gatgctgccg tcgatttaaa aacggtgatc ggatttttcc gagctctcga 11460
   tacgacggac gcgccagcat cacgagactg ggccagtgcc gcgagcgacc tagaaactct 11520
   cgtggcggat cttgaggagc tggctgacga gctgcgtgct cggccagcgc caggaggacg 11580
   cacagtagtg gaggatgcaa tcagttgcgc ctactgcggt ggcctgattc ctccccggcc 11640
   tgacccgcga ggacggcgcg caaaatattg ctcagatgcg tgtcgtgccg cagccagccg 11700
   cgagcgcgcc aacaaacgcc acgccgagga gctggaggcg gctaggtcgc aaatggcgct 11760
   ggaagtgcgt cccccgagcg aaattttggc catggtcgtc acagagctgg aagcggcagc 11820
   gagaattatc gcgatcgtgg cggtgcccgc aggcatgaca aacatcgtaa atgccgcgtt 11880
   tcgtgtgccg tggccgccca ggacgtgtca gcgccgccac cacctgcacc gaatcggcag 11940
   cagcgtcgcg cgtcgaaaaa gcgcacaggc ggcaagaagc gataagctgc acgaatacct 12000
   gaaaaatgtt gaacgccccg tgagcggtaa ctcacagggc gtcggctaac ccccagtcca 12060
   aacctgggag aaagcgctca aaaatgactc tagcggattc acgagacatt gacacaccgg 12120
   cctggaaatt ttccgctgat ctgttcgaca cccatcccga gctcgcgctg cgatcacgtg 12180
   gctggacgag cgaagaccgc cgcgaattcc tcgctcacct gggcagagaa aatttccagg 12240
   gcagcaagac ccgcgacttc gccagcgctt ggatcaaaga cccggacacg gagaaacaca 12300
   gccgaagtta taccgagttg gttcaaaatc gcttgcccgg tgccagtatg ttgctctgac 12360
   gcacgcgcag cacgcagccg tgcttgtcct ggacattgat gtgccgagcc accaggccgg 12420
   cgggaaaatc gagcacgtaa accccgaggt ctacgcgatt ttggagcgct gggcacgcct 12480
   ggaaaaagcg ccagcttgga tcggcgtgaa tccactgagc gggaaatgcc agctcatctg 12540
   gctcattgat ccggtgtatg ccgcagcagg catgagcagc ccgaatatgc gcctgctggc 12600
   tgcaacgacc gaggaaatga cccgcgtttt cggcgctgac caggcttttt cacataggct 12660
   gagccgtggc cactgcactc tccgacgatc ccagccgtac cgctggcatg cccagcacaa 12720
   tcgcgtggat cgcctagctg atcttatgga ggttgctcgc atgatctcag gcacagaaaa 12780
   acctaaaaaa cgctatgagc aggagttttc tagcggacgg gcacgtatcg aagcggcaag 12840
   aaaagccact gcggaagcaa aagcacttgc cacgcttgaa gcaagcctgc cgagcgccgc 12900
   tgaagcgtct ggagagctga tcgacggcgt ccgtgtcctc tggactgctc cagggcgtgc 12960
   cgcccgtgat gagacggctt ttcgccacgc tttgactgtg ggataccagt taaaagcggc 13020
   tggtgagcgc ctaaaagaca ccaagggtca tcgagcctac gagcgtgcct acaccgtcgc 13080
   tcaggcggtc ggaggaggcc gtgagcctga tctgccgccg gactgtgacc gccagacgga 13140
   ttggccgcga cgtgtgcgcg gctacgtcgc taaaggccag ccagtcgtcc ctgctcgtca 13200
   gacagagacg cagagccagc cgaggcgaaa agctctggcc actatgggaa gacgtggcgg 13260
   taaaaaggcc gcagaacgct ggaaagaccc aaacagtgag tacgcccgag cacagcgaga 13320
   aaaactagct aagtccagtc aacgacaagc taggaaagct aaaggaaatc gcttgaccat 13380
   tgcaggttgg tttatgactg ttgagggaga gactggctcg tggccgacaa tcaatgaagc 13440
   tatgtctgaa tttagcgtgt cacgtcagac cgtgaataga gcacttaagg tctgcgggca 13500
   ttgaacttcc acgaggacgc cgaaagcttc ccagtaaatg tgccatctcg taggcagaaa 13560
   acggttcccc cgtagggtct ctctcttggc ctcctttcta ggtcgggctg attgctcttg 13620
   aagctctcta ggggggctca caccataggc agataacgtt ccccaccggc tcgcctcgta 13680
   agcgcacaag gactgctccc aaagatcttc aaagccactg ccgcgactgc cttcgcgaag 13740
   ccttgccccg cggaaatttc ctccaccgag ttcgtgcaca cccctatgcc aagcttcttt 13800
   caccctaaat tcgagagatt ggattcttac cgtggaaatt cttcgcaaaa atcgtcccct 13860
   gatcgccctt gcgacgttgg cgtcggtgcc gctggttgcg cttggcttga ccgac      13915
<210> 37
   <211> 14063
   <212> DNA
   <213> artificial
<220>
   <223> Plasmid pOM621AR
<400> 37
   ttgatcagcg gccgcttcgc gaagcttgtc gaccgaaaca gcagttataa ggcatgaagc 60
   tgtccggttt ttgcaaaagt ggctgtgact gtaaaaagaa atcgaaaaag accgttttgt 120
   gtgaaaacgg tctttttgtt tccttttaac caactgccat aactcgaggc tattgacgac 180
   agctatggtt cactgtccac caaccaaaac tgtgctcagt accgccaata tttctccctt 240
   gaggggtaca aagaggtgtc cctagaagag atccacgctg tgtaaaaatt ttacaaaaag 300
   gtattgactt tccctacagg gtgtgtaata atttaattac aggcgggggc aaccccgcct 360
   gttctagaag gaggtgatag tatgtcttct gcagctaccc gccgtaattc agcccccttc 420
   gttcagcgcc acatcggccc aaaccaggcc gatacccagg agatcctcga ttacctgggc 480
   tatgaatctt ccgccgcgct ggccgacgat gccctcccga agtcgatccg ccaggcaggc 540
   ccgatcggcc tgccggaggc actggatgag acggacaccc tggccgccct gcgtgcttac 600
   gctgacaaga acgtgcagaa gcagcagctg atcggcaacg gttacttcga cacgatcact 660
   ccggccgtga ttcgccgcaa cgtggtggag aacccgggct ggtacaccgc ctacaccccc 720
   taccagccgg aaatctccca ggggcgcctc gaggccctgc tgaacttcca gacgatggtg 780
   caggacctga ccggcctgcc agtggccggt gcttcgctgt tagacgaagc caccgcagtg 840
   gccgaggccg tgcagctgat ggctcgcggc aatgcgaagg ctgccaagaa gggcggcgtg 900
   gtgctgctgg attcctccct gcaccagcag tccatcaccg taaccctggc gcgcgctgag 960
   gctgcgggta tcccggtgga ggtcgtggac ctggacggcg aagatgctac cgccgcgttt 1020
   gagggccgcg agaacctcgt cggcgtggtg ctttccaacc ccggctccac cggccgcgtc 1080
   cgcgacctgt ccggtctgat ctctgcggcg aaggagaccg gcgctctggt gacggtcgct 1140
   tgtgacctgc tggctcaggt tctggtgacc tccccgggct cccaaggtgc cgacattgct 1200
   gtcggctccg cccagcgctt cggcgtgccg ctattcttcg gtggcccgca cgcgggcttc 1260
   atctcctgca ccgaggctct gcagcgtaag ctgccgggcc gcatcgtggg cgtgtccgtg 1320
   gatgccgagg gcaccccggc ctaccgcttg gctctgcaga cccgtgagca gcacattcgc 1380
   cgcgacaagg ccaccagtaa catctgtacc gctcaggctc tgctggccgt cgttgccggt 1440
   ttctacgcgg tctggcacgg cccagccggc ctgcgcgcca tcgccgaggg ggtgcacgcc 1500
   cgcgcgaccg ccctggccgt tgccctgtcc gaggctggcc tgacgctggc gcacgatacc 1560
   ttcttcgaca ccgtcaccgt tgacgtgtct ggttcttctc ttggtgacgc ccccacggct 1620
   ctgcgcgccg cagccgaggc aggctacaac ctgcgccagg ttaacgattc cttcgtcggc 1680
   atctctgtcg gcgagtccac cacggacgag gacattgcca agctgatcga ggtgctgggc 1740
   tcccgcaccg gcgaggtcaa ctccgcgagc ttcgacgtca ccgccggccc gctgggcgag 1800
   gccggcgtgc tgcgcgcgga ggacgaggag attctgaccc acccgatctt caccgccatc 1860
   acttccgaga cccagatgat gcgctacatg cgcaagctgg ccgaccgcga cctggcgctg 1920
   gatcgtacga tgatcccgct gggctcctgc accatgaagc tgaacgcggc cgtctccatg 1980
   gagccgatca cctggcctgg cttcgcaggc atccacccgc acgtcccggc cgagcaggcg 2040
   cagggctggc tggagctcat cgaggacctg gaggagcgcc tggcgaagat caccggctac 2100
   gccaaggttt ccgtccagcc gaacgcgggc tcccagggcg agttcgccgg cctgctggcg 2160
   atccaccgct accaccagtc ccgcggcgac gatcagcgtg acatcgttct gatcccggcc 2220
   tccgcgcacg gcaccaacgc tgcctctgca gcgctggcgg gcctgaaggt cgtggctgtg 2280
   aagaatgccg aagacggctc catcgacgtg ccggacctgg aggccaagct ggagaagtac 2340
   ggcgagcaga ccgccgccat catgctgacc tacccctcca cccacggcgt gttcgaggag 2400
   caggtgcgcg acgtctgcca gaaggttcac gacgctggcg gccaggtgta cgtcgacggc 2460
   gctaacctga acgccctggt cggcctggcc cagccgggcg agttcggcgg cgacgtatcc 2520
   cacctgaacc tgcacaagac cttcaccatc ccgcacggtg gtggcggccc gggcgttggc 2580
   ccggtgtgcg tggcagagca cctgatcccg ttcctgccca ccgacccgaa cgccgacgtt 2640
   atcgagggcg atgctgccct gcagtccggc cagccggtct ccggcgcgca gtacggctcc 2700
   gctggcgtgc tgccgatcac ctggtcctac atcgcacaga tgggcgacga aggcctgact 2760
   gaggcctccc gcatggccct ggtgaacgcc aactacgttt cccgcaagct ggaggactac 2820
   tacccgacgc tgtacaaggg cgacaccggc ctggtcgccc acgagtgcat cctggacctg 2880
   cgtgaactca cgaaggcctc cggcatcacc gccgaggacg tatccaagcg cctgatggac 2940
   ttcggcttcc acgccccgac cttggccttc ccggtcgccg gcaccctgat gatggagccc 3000
   accgagtcgg aggacaagga agagctggac cgcttcatcg aagcgatgat caccatccac 3060
   ggggagatcc aggaagttat cgacggcaag gtcaccgccg aacagtccgt tctgcgccac 3120
   gcgccgttta ccgcctattc tgttgtgcgg gacgacttcg aggaagctgt atccggtggt    3180
   cacttcagcc gtgccaaggc cgcatacccg gtggctagcc tgcgacacac caagtacttc    3240
   acccccgtgc gtcgtatcga caacgcctat ggcgaccgca atctcgtgtg cacctgcccg 3300
   cccctggaag acttcgcaat taacgaggac tagggtccgc tggagctaag ggcgtcacaa 3360
   agagaagaag gagaacaaga catgaccgaa cttaagaaga ccgcgctgca cctggtgcac 3420
   gagaagttgg gcgcgcgatt taccgacttc ggcggctggg acatgcctct gaagtacagc 3480
   agtgagctgg acgagcacca cgctgtacgc aatgccgtgg gcgtattcga cctctcccac 3540
   atgggtgagg ttcgcgtgac cggcccgcag gcagcggagt tcctggacca cgcgctgatt 3600
   tcgaagctgt cggcagtgaa ggtcggcaag gcgaagtact cgatgatctg caccgaatcc 3660
   ggtggcatca tcgacgacct gatcacctac cgcctgggcg acaacgagtt cctgatcgtg 3720
   ccgaacgcgg gcaacgtgga caacgtggtc tccgcactgc agggccgcac cgagggcttt 3780
   gacgtggagg ttaacaacga gtccgatgcg acctccatga tcgccgtaca ggggcccaag 3840
   gccgcgcagg cgatgctgga gatcgtggag aacgtcgtgg atgcacccga ggcatccggc 3900
   gcgggcgaga ccgttgccga ggctatcgag gggctgggtt actacgcggc attcagcggt 3960
   gttgccgcag gtcagcccgt gctggtggcc cgcacaggct ataccggcga ggacggtttc 4020
   gagctgatcg tggctaacga tggtgcggag accgtgtgga ccaaggctat ggaccaggct 4080
   gcgcagctgg gtggcctgcc gtgtggcctg gcctgccgcg acaccctgcg cctggaggct 4140
   ggcatgccgc tgtacggcaa cgagctatcg ctgaagctca ccccggtcga tgctgggctg 4200
   ggcattcttg cggcgacgaa gtctaaggac tctttcgttg gtcgtgacgc catcgtttcc 4260
   gccaaggaaa agggtaccca gcaggtactt atcgggctgg cgggcgaggg tcgccgcgct 4320
   gcccgtgggg gatacgaggt gtttgccggt gacggcgaga aggccatcgg tgccgtgacc 4380
   tccggtgcac tgtcgccgac gctgggccac ccggtggcat tggcatacgt cgcgaagtcc 4440
   gcagtgtcct ccggcgcggc cgctgagggt gcgaccgtgg aggtagacat ccgcggcaag 4500
   cgctttgaat acaaggttgt ggcgctgccg ttctactccc gcgagaagta acgcaagaag 4560
   taacgaaaag ggttgtctgc ccgctggtgt aaggtttggg cagacaacca cagaagtaac 4620
   cacacaaggt ttcacaagag aggcttaaaa caatgactgc actgccaact gacttcctgt 4680
   actccgaaga gcacgagtgg gttaacacct ccgctgttgt tgagggcgag accgtgcgcg 4740
   tgggcattac ccacatcgcc gctgaggcgc tgggtgacat cgtgttcgtc gagctgccgg 4800
   aggttggctc cgaggttgag gccggcgagg ctttcggcga ggttgagtcc accaagtccg 4860
   tttccgacat ctacgcaccg gtttctggcg aggttgtggc tgtcaacgag gcgctggaag 4920
   acaacgctgg cctgatcaac gaagatccat acggtgaggg ctggctgtac gaggtcaagg 4980
   tgaccgaggc cggcgagctg atggaggctg aggcttacca ggcggctaac gagtaaaaca 5040
   agaccctatg gtcggttcca tgttcgcatt aatcctcggc ttggccctgt tgggccttgc 5100
   cgtgtggtta ctcctgcgag caaaagatag caccggatcc gccctcccgc acgctttgcg 5160
   ggagggcttt tcttttcccg gtatttcagt agctcggggg cgtctcattc agcaaaccta 5220
   gctccatatc cgcctctggg ctctcccacc ccgcaccaaa atctctcgcg agaggccttc 5280
   agggccctta aatgtcggaa aagttgcaat ttgttaatgc tactggtttt caaatatgca 5340
   aatgcgcagg tcaaggggct tttgggaaag tggaaaccaa tcccgatttg aagaattgca 5400
   acttttccga cattaagcgg ccttcaggaa gtgccaaaca ccggaggcac cagcatcaac 5460
   gtcaccacct ggcatatcta gcaccatcag aagcttcagc ctcactacag gcggaacctg 5520
   acgccagcgg gacctcaccc agcgggacag aacccagcgg aacttggcgc cagcggaacc 5580
   gcagccacca gctcccacca gcacccagca gcgccaaggc accactggag tatcatcgcc 5640
   accatgggat tccagcaagg cagcatccgc aaagcaaaca tgggcacaac cggcactaac 5700
   gacggggcaa ccaccccacc agccaacacc agcaccccag ccgtagacat cgacgtccgc 5760
   gacctgggca ccgtggacta cgaggacact tggcacctcc aggcaaatct cgccgcccag 5820
   cgcgccgaag aaaaaatccc agacaccatc ctcctactcc agcatccgcc gacgtacacc 5880
   gccggcaagc gcacccagga ttccgaccgc cctaccaacg gcctgccagt agtcgacgtc 5940
   gaccgcggag gccgcatcac ctggcatgga cccggccagt tggtcgcata tcccatcatc 6000
   aagctggccg acccggtgga cgtggtcgat tatgtccgcc gcctggagca ggcgctaatc 6060
   cagacttgtg aagatcttgg cctgcacggc accggccgcg tagaggggcg ttcgggcgtg 6120
   tggctgcctg cgggcgtcat taatggcgag ctcaagcccg cacgtaagat agccgcgatc 6180
   ggcatccgcg tgacgcgcgg cgtgaccatg cacggagtgg ccctcaactg cgataacacc 6240
   atggagtatt acgaccacat tgtgccttgt gggctggcgg atgcgggtgt cacgacgctc 6300
   accgaggagc tggggcgcga tgttagtgtt tctgacgcct actcatccct cgcccacaac 6360
   ctcgttgatg ctttgaacgg cgacttgccg gtgcattcct aggggaaaac ccgaggtgga 6420
   ttcctatacg gcacccagcg taggctagtc ggcatgagtg tgaccgcaga tggacgccgg 6480
   atgctacgca tcgaggcgaa gaatgccgaa acccccatcg agtcgaagcc tcggtggatc 6540
   cgcacgaccg cgaaggtcgg cccggagtat cgggatatta agaatcgcgt gaagggcgct 6600
   ggcctgcaca ccgtgtgcca agaggctggc tgcccgaata tcaacgagtg ctgggaggac 6660
   cgcgaggcga cgttcctcat cggcggtgat acgtgttccc gccgttgcga cttctgccag 6720
   attaagtccg gccgcccgtc cccgctggat atggacgagc cacgccgcgt ggcggagaat 6780
   gtccgcgaga tgggtctgcg ctacgccacc atcaccggcg tgacgcgcga tgacctggat 6840
   gatgagggcg cgtggctgta tgccgaggtt gtgaagaaga ttcacgagct gaacccgaac 6900
   acgggtgtgg agaatctgac gccagatttt tccaaccgcc cggagctgct gaaggtcgtc 6960
   ttcgattccc agccggaggt gtttgcccac aacctggaga cagttccgcg catctttaag 7020
   cgcatccgcc cggcctttaa gtacgaccgt tccctggagg tcattcaggc agctcacgat 7080
   tacggcctgg tgacgaagtc caacctgatc ctgggcatgg gtgagaagaa ggaagaggtc 7140
   cgcgcggcta tcaaggacct ggcagacgcc ggcaccgaca ttctgacgat tacccagtac 7200
   ctgcgcccgt cttccatgca ccacccgatt gagcgttggg tgaagccgga ggagttcatg 7260
   gagcactccg acgcagccta cgagctgggc atcaaggctg ttatgtccgg cccgttggtg 7320
   cgttcctctt accgcgccgg ccgcctgtac gcgcaggcca agcaggcgcg cggcgaggct 7380
   attccggaga acctgaagca cttggaggag actctcgatt ccaccacgtc gcaggaggcc 7440
   tctacactgc tggagcgcta cggcgcttcg gaggacacgc cggtcactgc gtcgcgccgc 7500
   tagctcggcg gggcactttc gccccgcctt tattcatgcc ttattgttga atccatggcg 7560
   aaggataaaa aggacgtaag ggtcaaggaa gagaagaagg cagctaaggc aaatcgctag 7620
   cgggctgcta aaggaagcgg aacacgtaga aagccagtcc gcagaaacgg tgctgacccc 7680
   ggatgaatgt cagctactgg gctatctgga caagggaaaa cgcaagcgca aagagaaagc 7740
   aggtagcttg cagtgggctt acatggcgat agctagactg ggcggtttta tggacagcaa 7800
   gcgaaccgga attgccagct ggggcgccct ctggtaaggt tgggaagccc tgcaaagtaa 7860
   actggatggc tttcttgccg ccaaggatct gatggcgcag gggatcaaga tctgatcaag 7920
   agacaggatg aggatcgttt cgcatgattg aacaagatgg attgcacgca ggttctccgg 7980
   ccgcttgggt ggagaggcta ttcggctatg actgggcaca acagacaatc ggctgctctg 8040
   atgccgccgt gttccggctg tcagcgcagg ggcgcccggt tctttttgtc aagaccgacc 8100
   tgtccggtgc cctgaatgaa ctgcaggacg aggcagcgcg gctatcgtgg ctggccacga 8160
   cgggcgttcc ttgcgcagct gtgctcgacg ttgtcactga agcgggaagg gactggctgc 8220
   tattgggcga agtgccgggg caggatctcc tgtcatctca ccttgctcct gccgagaaag 8280
   tatccatcat ggctgatgca atgcggcggc tgcatacgct tgatccggct acctgcccat 8340
   tcgaccacca agcgaaacat cgcatcgagc gagcacgtac tcggatggaa gccggtcttg 8400
   tcgatcagga tgatctggac gaagagcatc aggggctcgc gccagccgaa ctgttcgcca 8460
   ggctcaaggc gcgcatgccc gacggcgagg atctcgtcgt gacccatggc gatgcctgct 8520
   tgccgaatat catggtggaa aatggccgct tttctggatt catcgactgt ggccggctgg 8580
   gtgtggcgga ccgctatcag gacatagcgt tggctacccg tgatattgct gaagagcttg 8640
   gcggcgaatg ggctgaccgc ttcctcgtgc tttacggtat cgccgctccc gattcgcagc 8700
   gcatcgcctt ctatcgcctt cttgacgagt tcttctgagc gggactctgg ggttcgaaat 8760
   gaccgaccaa gcgacgccca acctgccatc acgagatttc gattccaccg ccgccttcta 8820
   tgaaaggttg ggcttcggaa tcgttttccg ggacgccggc tggatgatcc tccagcgcgg 8880
   ggatctcatg ctggagttct tcgcccacgc tagtttaaac tgcggatcag tgagggtttg 8940
   taactgcggg tcaaggatct ggatttcgat cacggcacga tcatcgtgcg ggagggcaag 9000
   ggctccaagg atcgggcctt gatgttaccc gagagcttgg cacccagcct gcgcgagcag 9060
   gggaattgat ccggtggatg accttttgaa tgacctttaa tagattatat tactaattaa 9120
   ttggggaccc tagaggtccc cttttttatt ttaaaaattt tttcacaaaa cggtttacaa 9180
   gcataacggg ttttgctgcc cgcaaacggg ctgttctggt gttgctagtt tgttatcaga 9240
   atcgcagatc cggcttcagg tttgccggct gaaagcgcta tttcttccag aattgccatg 9300
   attttttccc cacgggaggc gtcactggct cccgtgttgt cggcagcttt gattcgataa 9360
   gcagcatcgc ctgtttcagg ctgtctatgt gtgactgttg agctgtaaca agttgtctca 9420
   ggtgttcaat ttcatgttct agttgctttg ttttactggt ttcacctgtt ctattaggtg 9480
   ttacatgctg ttcatctgtt acattgtcga tctgttcatg gtgaacagct ttaaatgcac 9540
   caaaaactcg taaaagctct gatgtatcta tcttttttac accgttttca tctgtgcata 9600
   tggacagttt tccctttgat atctaacggt gaacagttgt tctacttttg tttgttagtc 9660
   ttgatgcttc actgatagat acaagagcca taagaacctc agatccttcc gtatttagcc 9720
   agtatgttct ctagtgtggt tcgttgtttt tgcgtgagcc atgagaacga accattgaga 9780
   tcatgcttac tttgcatgtc actcaaaaat tttgcctcaa aactggtgag ctgaattttt 9840
   gcagttaaag catcgtgtag tgtttttctt agtccgttac gtaggtagga atctgatgta 9900
   atggttgttg gtattttgtc accattcatt tttatctggt tgttctcaag ttcggttacg 9960
   agatccattt gtctatctag ttcaacttgg aaaatcaacg tatcagtcgg gcggcctcgc 10020
   ttatcaacca ccaatttcat attgctgtaa gtgtttaaat ctttacttat tggtttcaaa 10080
   acccattggt taagcctttt aaactcatgg tagttatttt caagcattaa catgaactta 10140
   aattcatcaa ggctaatctc tatatttgcc ttgtgagttt tcttttgtgt tagttctttt 10200
   aataaccact cataaatcct catagagtat ttgttttcaa aagacttaac atgttccaga 10260
   ttatatttta tgaatttttt taactggaaa agataaggca atatctcttc actaaaaact 10320
   aattctaatt tttcgcttga gaacttggca tagtttgtcc actggaaaat ctcaaagcct 10380
   ttaaccaaag gattcctgat ttccacagtt ctcgtcatca gctctctggt tgctttagct 10440
   aatacaccat aagcattttc cctactgatg ttcatcatct gagcgtattg gttataagtg 10500
   aacgataccg tccgttcttt ccttgtaggg ttttcaatcg tggggttgag tagtgccaca 10560
   cagcataaaa ttagcttggt ttcatgctcc gttaagtcat agcgactaat cgctagttca 10620
   tttgctttga aaacaactaa ttcagacata catctcaatt ggtctaggtg attttaatca 10680
   ctataccaat tgagatgggc tagtcaatga taattactag tccttttcct ttgagttgtg 10740
   ggtatctgta aattctgcta gacctttgct ggaaaacttg taaattctgc tagaccctct 10800
   gtaaattccg ctagaccttt gtgtgttttt tttgtttata ttcaagtggt tataatttat 10860
   agaataaaga aagaataaaa aaagataaaa agaatagatc ccagccctgt gtataactca 10920
   ctactttagt cagttccgca gtattacaaa aggatgtcgc aaacgctgtt tgctcctcta 10980
   caaaacagac cttaaaaccc taaaggctta agtagcaccc tcgcaagctc gggcaaatcg 11040
   ctgaatattc cttttgtctc cgaccatcag gcacctgagt cgctgtcttt ttcgtgacat 11100
   tcagttcgct gcgctcacgg ctctggcagt gaatgggggt aaatggcact acaggcgcct 11160
   tttatggatt catgcaagga aactacccat aatacaagaa aagcccgtca cgggcttctc 11220
   agggcgtttt atggcgggtc tgctatgtgg tgctatctga ctttttgctg ttcagcagtt 11280
   cctgccctct gattttccag tctgaccact tcggattatc ccgtgacagg tcattcagac 11340
   tggctaatgc acccagtaag gcagcggtat catcaacagg cttagtttaa accgcaaagt 11400
   cccgcttcgt gaaaattttc gtgccgcgtg attttccgcc aaaaacttta acgaacgttc 11460
   gttataatgg tgtcatgacc ttcacgacga agtactaaaa ttggcccgaa tcatcagcta 11520
   tggatctctc tgatgtcgcg ctggagtccg acgcgctcga tgctgccgtc gatttaaaaa 11580
   cggtgatcgg atttttccga gctctcgata cgacggacgc gccagcatca cgagactggg 11640
   ccagtgccgc gagcgaccta gaaactctcg tggcggatct tgaggagctg gctgacgagc 11700
   tgcgtgctcg gccagcgcca ggaggacgca cagtagtgga ggatgcaatc agttgcgcct 11760
   actgcggtgg cctgattcct ccccggcctg acccgcgagg acggcgcgca aaatattgct 11820
   cagatgcgtg tcgtgccgca gccagccgcg agcgcgccaa caaacgccac gccgaggagc 11880
   tggaggcggc taggtcgcaa atggcgctgg aagtgcgtcc cccgagcgaa attttggcca 11940
   tggtcgtcac agagctggaa gcggcagcga gaattatcgc gatcgtggcg gtgcccgcag 12000
   gcatgacaaa catcgtaaat gccgcgtttc gtgtgccgtg gccgcccagg acgtgtcagc 12060
   gccgccacca cctgcaccga atcggcagca gcgtcgcgcg tcgaaaaagc gcacaggcgg 12120
   caagaagcga taagctgcac gaatacctga aaaatgttga acgccccgtg agcggtaact 12180
   cacagggcgt cggctaaccc ccagtccaaa cctgggagaa agcgctcaaa aatgactcta 12240
   gcggattcac gagacattga cacaccggcc tggaaatttt ccgctgatct gttcgacacc 12300
   catcccgagc tcgcgctgcg atcacgtggc tggacgagcg aagaccgccg cgaattcctc 12360
   gctcacctgg gcagagaaaa tttccagggc agcaagaccc gcgacttcgc cagcgcttgg 12420
   atcaaagacc cggacacgga gaaacacagc cgaagttata ccgagttggt tcaaaatcgc 12480
   ttgcccggtg ccagtatgtt gctctgacgc acgcgcagca cgcagccgtg cttgtcctgg 12540
   acattgatgt gccgagccac caggccggcg ggaaaatcga gcacgtaaac cccgaggtct 12600
   acgcgatttt ggagcgctgg gcacgcctgg aaaaagcgcc agcttggatc ggcgtgaatc 12660
   cactgagcgg gaaatgccag ctcatctggc tcattgatcc ggtgtatgcc gcagcaggca 12720
   tgagcagccc gaatatgcgc ctgctggctg caacgaccga ggaaatgacc cgcgttttcg 12780
   gcgctgacca ggctttttca cataggctga gccgtggcca ctgcactctc cgacgatccc 12840
   agccgtaccg ctggcatgcc cagcacaatc gcgtggatcg cctagctgat cttatggagg 12900
   ttgctcgcat gatctcaggc acagaaaaac ctaaaaaacg ctatgagcag gagttttcta 12960
   gcggacgggc acgtatcgaa gcggcaagaa aagccactgc ggaagcaaaa gcacttgcca 13020
   cgcttgaagc aagcctgccg agcgccgctg aagcgtctgg agagctgatc gacggcgtcc 13080
   gtgtcctctg gactgctcca gggcgtgccg cccgtgatga gacggctttt cgccacgctt 13140
   tgactgtggg ataccagtta aaagcggctg gtgagcgcct aaaagacacc aagggtcatc 13200
   gagcctacga gcgtgcctac accgtcgctc aggcggtcgg aggaggccgt gagcctgatc 13260
   tgccgccgga ctgtgaccgc cagacggatt ggccgcgacg tgtgcgcggc tacgtcgcta 13320
   aaggccagcc agtcgtccct gctcgtcaga cagagacgca gagccagccg aggcgaaaag 13380
   ctctggccac tatgggaaga cgtggcggta aaaaggccgc agaacgctgg aaagacccaa 13440
   acagtgagta cgcccgagca cagcgagaaa aactagctaa gtccagtcaa cgacaagcta 13500
   ggaaagctaa aggaaatcgc ttgaccattg caggttggtt tatgactgtt gagggagaga 13560
   ctggctcgtg gccgacaatc aatgaagcta tgtctgaatt tagcgtgtca cgtcagaccg 13620
   tgaatagagc acttaaggtc tgcgggcatt gaacttccac gaggacgccg aaagcttccc 13680
   agtaaatgtg ccatctcgta ggcagaaaac ggttcccccg tagggtctct ctcttggcct 13740
   cctttctagg tcgggctgat tgctcttgaa gctctctagg ggggctcaca ccataggcag 13800
   ataacgttcc ccaccggctc gcctcgtaag cgcacaagga ctgctcccaa agatcttcaa 13860
   agccactgcc gcgactgcct tcgcgaagcc ttgccccgcg gaaatttcct ccaccgagtt 13920
   cgtgcacacc cctatgccaa gcttctttca ccctaaattc gagagattgg attcttaccg 13980
   tggaaattct tcgcaaaaat cgtcccctga tcgcccttgc gacgttggcg tcggtgccgc 14040
   tggttgcgct tggcttgacc gac 14063
<210> 38
   <211> 11872
   <212> DNA
   <213> artificial
<220>
   <223> Plasmid pOM627
<400> 38
   ggatcggcgg ccagggccct catggatgac catccccggc accatccttg acaccgcccg 60
   cacccaagtt ctggaacagg gaattggcct taatcagcag cagttgatgg aggttctcac 120
   cttgcctgaa gagcaaatcc cagacttgat ggaattagcc caccaggttc ggttgaagtg 180
   gtgtggggaa gaaatcgagg tcgagggcat tatttccctc aaaactggcg gttgccctga 240
   agattgtcat ttctgctcac agtctgggtt gtttgaatcg ccggtgcgtt cggtgtggct 300
   ggatattccg aatctggttg aagccgctaa acagaccgca aaaactggcg ctaccgaatt 360
   ctgtatcgtc gccgcagtca aggggcctga tgagaggctc atgacccagc tggaggaagc 420
   agtcctcgcg attcactctg aagttgaaat tgaagtcgca gcatcgatcg gaacgttaaa 480
   taaggaacag gtggatcgcc tcgctgctgc cggcgtgcac cgctagcggc cgcttcgcga 540
   agcttgtcga ccgaaacagc agttataagg catgaagctg tccggttttt gcaaaagtgg 600
   ctgtgactgt aaaaagaaat cgaaaaagac cgttttgtgt gaaaacggtc tttttgtttc 660
   cttttaacca actgccataa ctcgaggcta ttgacgacag ctatggttca ctgtccacca 720
   accaaaactg tgctcagtac cgccaatatt tctcccttga ggggtacaaa gaggtgtccc 780
   tagaagagat ccacgctgtg taaaaatttt acaaaaaggt attgactttc cctacagggt 840
   gtgtaataat ttaattacag gcgggggcaa ccccgcctgt tctagaagga ggtgatagta 900
   tgtcttctgc agctacccgc cgtaattcag cccccttcgt tcagcgccac atcggcccaa 960
   accaggccga tacccaggag atcctcgatt acctgggcta tgaatcttcc gccgcgctgg 1020
   ccgacgatgc cctcccgaag tcgatccgcc aggcaggccc gatcggcctg ccggaggcac 1080
   tggatgagac ggacaccctg gccgccctgc gtgcttacgc tgacaagaac gtgcagaagc 1140
   agcagctgat cggcaacggt tacttcgaca cgatcactcc ggccgtgatt cgccgcaacg 1200
   tggtggagaa cccgggctgg tacaccgcct acacccccta ccagccggaa atctcccagg 1260
   ggcgcctcga ggccctgctg aacttccaga cgatggtgca ggacctgacc ggcctgccag 1320
   tggccggtgc ttcgctgtta gacgaagcca ccgcagtggc cgaggccgtg cagctgatgg 1380
   ctcgcggcaa tgcgaaggct gccaagaagg gcggcgtggt gctgctggat tcctccctgc 1440
   accagcagtc catcaccgta accctggcgc gcgctgaggc tgcgggtatc ccggtggagg 1500
   tcgtggacct ggacggcgaa gatgctaccg ccgcgtttga gggccgcgag aacctcgtcg 1560
   gcgtggtgct ttccaacccc ggctccaccg gccgcgtccg cgacctgtcc ggtctgatct 1620
   ctgcggcgaa ggagaccggc gctctggtga cggtcgcttg tgacctgctg gctcaggttc 1680
   tggtgacctc cccgggctcc caaggtgccg acattgctgt cggctccgcc cagcgcttcg 1740
   gcgtgccgct attcttcggt ggcccgcacg cgggcttcat ctcctgcacc gaggctctgc 1800
   agcgtaagct gccgggccgc atcgtgggcg tgtccgtgga tgccgagggc accccggcct 1860
   accgcttggc tctgcagacc cgtgagcagc acattcgccg cgacaaggcc accagtaaca 1920
   tctgtaccgc tcaggctctg ctggccgtcg ttgccggttt ctacgcggtc tggcacggcc 1980
   cagccggcct gcgcgccatc gccgaggggg tgcacgcccg cgcgaccgcc ctggccgttg 2040
   ccctgtccga ggctggcctg acgctggcgc acgatacctt cttcgacacc gtcaccgttg 2100
   acgtgtctgg ttcttctctt ggtgacgccc ccacggctct gcgcgccgca gccgaggcag 2160
   gctacaacct gcgccaggtt aacgattcct tcgtcggcat ctctgtcggc gagtccacca 2220
   cggacgagga cattgccaag ctgatcgagg tgctgggctc ccgcaccggc gaggtcaact 2280
   ccgcgagctt cgacgtcacc gccggcccgc tgggcgaggc cggcgtgctg cgcgcggagg 2340
   acgaggagat tctgacccac ccgatcttca ccgccatcac ttccgagacc cagatgatgc 2400
   gctacatgcg caagctggcc gaccgcgacc tggcgctgga tcgtacgatg atcccgctgg 2460
   gctcctgcac catgaagctg aacgcggccg tctccatgga gccgatcacc tggcctggct 2520
   tcgcaggcat ccacccgcac gtcccggccg agcaggcgca gggctggctg gagctcatcg 2580
   aggacctgga ggagcgcctg gcgaagatca ccggctacgc caaggtttcc gtccagccga 2640
   acgcgggctc ccagggcgag ttcgccggcc tgctggcgat ccaccgctac caccagtccc 2700
   gcggcgacga tcagcgtgac atcgttctga tcccggcctc cgcgcacggc accaacgctg 2760
   cctctgcagc gctggcgggc ctgaaggtcg tggctgtgaa gaatgccgaa gacggctcca 2820
   tcgacgtgcc ggacctggag gccaagctgg agaagtacgg cgagcagacc gccgccatca 2880
   tgctgaccta cccctccacc cacggcgtgt tcgaggagca ggtgcgcgac gtctgccaga 2940
   aggttcacga cgctggcggc caggtgtacg tcgacggcgc taacctgaac gccctggtcg 3000
   gcctggccca gccgggcgag ttcggcggcg acgtatccca cctgaacctg cacaagacct 3060
   tcaccatccc gcacggtggt ggcggcccgg gcgttggccc ggtgtgcgtg gcagagcacc 3120
   tgatcccgtt cctgcccacc gacccgaacg ccgacgttat cgagggcgat gctgccctgc 3180
   agtccggcca gccggtctcc ggcgcgcagt acggctccgc tggcgtgctg ccgatcacct 3240
   ggtcctacat cgcacagatg ggcgacgaag gcctgactga ggcctcccgc atggccctgg 3300
   tgaacgccaa ctacgtttcc cgcaagctgg aggactacta cccgacgctg tacaagggcg 3360
   acaccggcct ggtcgcccac gagtgcatcc tggacctgcg tgaactcacg aaggcctccg 3420
   gcatcaccgc cgaggacgta tccaagcgcc tgatggactt cggcttccac gccccgacct 3480
   tggccttccc ggtcgccggc accctgatga tggagcccac cgagtcggag gacaaggaag 3540
   agctggaccg cttcatcgaa gcgatgatca ccatccacgg ggagatccag gaagttatcg 3600
   acggcaaggt caccgccgaa cagtccgttc tgcgccacgc gccgtttacc gcctattctg 3660
   ttgtgcggga cgacttcgag gaagctgtat ccggtggtca cttcagccgt gccaaggccg 3720
   catacccggt ggctagcctg cgacacacca agtacttcac ccccgtgcgt cgtatcgaca 3780
   acgcctatgg cgaccgcaat ctcgtgtgca cctgcccgcc cctggaagac ttcgcaatta 3840
   acgaggacta gggtccgctg gagctaaggg cgtcacaaag agaagaagga gaacaagaca 3900
   tgaccgaact taagaagacc gcgctgcacc tggtgcacga gaagttgggc gcgcgattta 3960
   ccgacttcgg cggctgggac atgcctctga agtacagcag tgagctggac gagcaccacg 4020
   ctgtacgcaa tgccgtgggc gtattcgacc tctcccacat gggtgaggtt cgcgtgaccg 4080
   gcccgcaggc agcggagttc ctggaccacg cgctgatttc gaagctgtcg gcagtgaagg 4140
   tcggcaaggc gaagtactcg atgatctgca ccgaatccgg tggcatcatc gacgacctga 4200
   tcacctaccg cctgggcgac aacgagttcc tgatcgtgcc gaacgcgggc aacgtggaca 4260
   acgtggtctc cgcactgcag ggccgcaccg agggctttga cgtggaggtt aacaacgagt 4320
   ccgatgcgac ctccatgatc gccgtacagg ggcccaaggc cgcgcaggcg atgctggaga 4380
   tcgtggagaa cgtcgtggat gcacccgagg catccggcgc gggcgagacc gttgccgagg 4440
   ctatcgaggg gctgggttac tacgcggcat tcagcggtgt tgccgcaggt cagcccgtgc 4500
   tggtggcccg cacaggctat accggcgagg acggtttcga gctgatcgtg gctaacgatg 4560
   gtgcggagac cgtgtggacc aaggctatgg accaggctgc gcagctgggt ggcctgccgt 4620
   gtggcctggc ctgccgcgac accctgcgcc tggaggctgg catgccgctg tacggcaacg 4680
   agctatcgct gaagctcacc ccggtcgatg ctgggctggg cattcttgcg gcgacgaagt 4740
   ctaaggactc tttcgttggt cgtgacgcca tcgtttccgc caaggaaaag ggtacccagc 4800
   aggtacttat cgggctggcg ggcgagggtc gccgcgctgc ccgtggggga tacgaggtgt 4860
   ttgccggtga cggcgagaag gccatcggtg ccgtgacctc cggtgcactg tcgccgacgc 4920
   tgggccaccc ggtggcattg gcatacgtcg cgaagtccgc agtgtcctcc ggcgcggccg 4980
   ctgagggtgc gaccgtggag gtagacatcc gcggcaagcg ctttgaatac aaggttgtgg 5040
   cgctgccgtt ctactcccgc gagaagtaac gcaagaagta acgaaaaggg ttgtctgccc 5100
   gctggtgtaa ggtttgggca gacaaccaca gaagtaacca cacaaggttt cacaagagag 5160
   gcttaaaaca atgactgcac tgccaactga cttcctgtac tccgaagagc acgagtgggt 5220
   taacacctcc gctgttgttg agggcgagac cgtgcgcgtg ggcattaccc acatcgccgc 5280
   tgaggcgctg ggtgacatcg tgttcgtcga gctgccggag gttggctccg aggttgaggc 5340
   cggcgaggct ttcggcgagg ttgagtccac caagtccgtt tccgacatct acgcaccggt 5400
   ttctggcgag gttgtggctg tcaacgaggc gctggaagac aacgctggcc tgatcaacga 5460
   agatccatac ggtgagggct ggctgtacga ggtcaaggtg accgaggccg gcgagctgat 5520
   ggaggctgag gcttaccagg cggctaacga gtaaaacaag accctatggt cggttccatg 5580
   ttcgcattaa tcctcggctt ggccctgttg ggccttgccg tgtggttact cctgcgagca 5640
   aaagatagca ccggatccgc cctcccgcaa agcgtgcggg agggcggtac ctgcgcgttc 5700
   ctatttccct gaagttgtca ccactcatac atgggaagag cgccgcgaaa ctttgcgcct 5760
   ggtggcagaa gctggaatgg aagtctgttc cggcggaatc ttaggaatgg gcgaaacttt 5820
   agagcagcgc gccgagtttg ccgtgcagct ggcggagctt gatccgcacg aagtccccat 5880
   gaacttcctt gatcctcgcc cgggcacccc atttgccgat agggaattga tggacagccg 5940
   tgacgctctg cgctctattg gtgcgttccg ccttgcgatg cctcacacca tgcttcgttt 6000
   tgctggcggt cgcgagctga ctttgggcga caagggttcc gagcaagccc tcctgggagg 6060
   catcaatgcg atgatcgtcg gaaactacct gactacgctc ggccgcccaa tggaagatga 6120
   cctcgacatg atggatcgtc tccagctgcc catcaaagtc cttaataagg acgcgtcccg 6180
   ggatttaaat cgctagcggg ctgctaaagg aagcggaaca cgtagaaagc cagtccgcag 6240
   aaacggtgct gaccccggat gaatgtcagc tactgggcta tctggacaag ggaaaacgca 6300
   agcgcaaaga gaaagcaggt agcttgcagt gggcttacat ggcgatagct agactgggcg 6360
   gttttatgga cagcaagcga accggaattg ccagctgggg cgccctctgg taaggttggg 6420
   aagccctgca aagtaaactg gatggctttc ttgccgccaa ggatctgatg gcgcagggga 6480
   tcaagatctg atcaagagac aggatgagga tcgtttcgca tgattgaaca agatggattg 6540
   cacgcaggtt ctccggccgc ttgggtggag aggctattcg gctatgactg ggcacaacag 6600
   acaatcggct gctctgatgc cgccgtgttc cggctgtcag cgcaggggcg cccggttctt 6660
   tttgtcaaga ccgacctgtc cggtgccctg aatgaactgc aggacgaggc agcgcggcta 6720
   tcgtggctgg ccacgacggg cgttccttgc gcagctgtgc tcgacgttgt cactgaagcg 6780
   ggaagggact ggctgctatt gggcgaagtg ccggggcagg atctcctgtc atctcacctt 6840
   gctcctgccg agaaagtatc catcatggct gatgcaatgc ggcggctgca tacgcttgat 6900
   ccggctacct gcccattcga ccaccaagcg aaacatcgca tcgagcgagc acgtactcgg 6960
   atggaagccg gtcttgtcga tcaggatgat ctggacgaag agcatcaggg gctcgcgcca 7020
   gccgaactgt tcgccaggct caaggcgcgc atgcccgacg gcgaggatct cgtcgtgacc 7080
   catggcgatg cctgcttgcc gaatatcatg gtggaaaatg gccgcttttc tggattcatc 7140
   gactgtggcc ggctgggtgt ggcggaccgc tatcaggaca tagcgttggc tacccgtgat 7200
   attgctgaag agcttggcgg cgaatgggct gaccgcttcc tcgtgcttta cggtatcgcc 7260
   gctcccgatt cgcagcgcat cgccttctat cgccttcttg acgagttctt ctgagcggga 7320
   ctctggggtt cgaaatgacc gaccaagcga cgcccaacct gccatcacga gatttcgatt 7380
   ccaccgccgc cttctatgaa aggttgggct tcggaatcgt tttccgggac gccggctgga 7440
   tgatcctcca gcgcggggat ctcatgctgg agttcttcgc ccacgctagt ttaaactgcg 7500
   gatcagtgag ggtttgtaac tgcgggtcaa ggatctggat ttcgatcacg gcacgatcat 7560
   cgtgcgggag ggcaagggct ccaaggatcg ggccttgatg ttacccgaga gcttggcacc 7620
   cagcctgcgc gagcagggga attgatccgg tggatgacct tttgaatgac ctttaataga 7680
   ttatattact aattaattgg ggaccctaga ggtccccttt tttattttaa aaattttttc 7740
   acaaaacggt ttacaagcat aacgggtttt gctgcccgca aacgggctgt tctggtgttg 7800
   ctagtttgtt atcagaatcg cagatccggc ttcaggtttg ccggctgaaa gcgctatttc 7860
   ttccagaatt gccatgattt tttccccacg ggaggcgtca ctggctcccg tgttgtcggc 7920
   agctttgatt cgataagcag catcgcctgt ttcaggctgt ctatgtgtga ctgttgagct 7980
   gtaacaagtt gtctcaggtg ttcaatttca tgttctagtt gctttgtttt actggtttca 8040
   cctgttctat taggtgttac atgctgttca tctgttacat tgtcgatctg ttcatggtga 8100
   acagctttaa atgcaccaaa aactcgtaaa agctctgatg tatctatctt ttttacaccg 8160
   ttttcatctg tgcatatgga cagttttccc tttgatatct aacggtgaac agttgttcta 8220
   cttttgtttg ttagtcttga tgcttcactg atagatacaa gagccataag aacctcagat 8280
   ccttccgtat ttagccagta tgttctctag tgtggttcgt tgtttttgcg tgagccatga 8340
   gaacgaacca ttgagatcat gcttactttg catgtcactc aaaaattttg cctcaaaact 8400
   ggtgagctga atttttgcag ttaaagcatc gtgtagtgtt tttcttagtc cgttacgtag 8460
   gtaggaatct gatgtaatgg ttgttggtat tttgtcacca ttcattttta tctggttgtt 8520
   ctcaagttcg gttacgagat ccatttgtct atctagttca acttggaaaa tcaacgtatc 8580
   agtcgggcgg cctcgcttat caaccaccaa tttcatattg ctgtaagtgt ttaaatcttt 8640
   acttattggt ttcaaaaccc attggttaag ccttttaaac tcatggtagt tattttcaag 8700
   cattaacatg aacttaaatt catcaaggct aatctctata tttgccttgt gagttttctt 8760
   ttgtgttagt tcttttaata accactcata aatcctcata gagtatttgt tttcaaaaga 8820
   cttaacatgt tccagattat attttatgaa tttttttaac tggaaaagat aaggcaatat 8880
   ctcttcacta aaaactaatt ctaatttttc gcttgagaac ttggcatagt ttgtccactg 8940
   gaaaatctca aagcctttaa ccaaaggatt cctgatttcc acagttctcg tcatcagctc 9000
   tctggttgct ttagctaata caccataagc attttcccta ctgatgttca tcatctgagc 9060
   gtattggtta taagtgaacg ataccgtccg ttctttcctt gtagggtttt caatcgtggg 9120
   gttgagtagt gccacacagc ataaaattag cttggtttca tgctccgtta agtcatagcg 9180
   actaatcgct agttcatttg ctttgaaaac aactaattca gacatacatc tcaattggtc 9240
   taggtgattt taatcactat accaattgag atgggctagt caatgataat tactagtcct 9300
   tttcctttga gttgtgggta tctgtaaatt ctgctagacc tttgctggaa aacttgtaaa 9360
   ttctgctaga ccctctgtaa attccgctag acctttgtgt gttttttttg tttatattca 9420
   agtggttata atttatagaa taaagaaaga ataaaaaaag ataaaaagaa tagatcccag 9480
   ccctgtgtat aactcactac tttagtcagt tccgcagtat tacaaaagga tgtcgcaaac 9540
   gctgtttgct cctctacaaa acagacctta aaaccctaaa ggcttaagta gcaccctcgc 9600
   aagctcgggc aaatcgctga atattccttt tgtctccgac catcaggcac ctgagtcgct 9660
   gtctttttcg tgacattcag ttcgctgcgc tcacggctct ggcagtgaat gggggtaaat 9720
   ggcactacag gcgcctttta tggattcatg caaggaaact acccataata caagaaaagc 9780
   ccgtcacggg cttctcaggg cgttttatgg cgggtctgct atgtggtgct atctgacttt 9840
   ttgctgttca gcagttcctg ccctctgatt ttccagtctg accacttcgg attatcccgt 9900
   gacaggtcat tcagactggc taatgcaccc agtaaggcag cggtatcatc aacaggctta 9960
   gtttaaaccc atcggcattt tcttttgcgt ttttatttgt taactgttaa ttgtccttgt 10020
   tcaaggatgc tgtctttgac aacagatgtt ttcttgcctt tgatgttcag caggaagctc 10080
   ggcgcaaacg ttgattgttt gtctgcgtag aatcctctgt ttgtcatata gcttgtaatc 10140
   acgacattgt ttcctttcgc ttgaggtaca gcgaagtgtg agtaagtaaa ggttacatcg 10200
   ttaggatcaa gatccatttt taacacaagg ccagttttgt tcagcggctt gtatgggcca 10260
   gttaaagaat tagaaacata accaagcatg taaatatcgt tagacgtaat gccgtcaatc 10320
   gtcatttttg atccgcggga gtcagtgaac aggtaccatt tgccgttcat tttaaagacg 10380
   ttcgcgcgtt caatttcatc tgttactgtg ttagatgcaa tcagcggttt catcactttt 10440
   ttcagtgtgt aatcatcgtt tagctcaatc ataccgagag cgccgtttgc taactcagcc 10500
   gtgcgttttt tatcgctttg cagaagtttt tgactttctt gacggaagaa tgatgtgctt 10560
   ttgccatagt atgctttgtt aaataaagat tcttcgcctt ggtagccatc ttcagttcca 10620
   gtgtttgctt caaatactaa gtatttgtgg cctttatctt ctacgtagtg aggatctctc 10680
   agcgtatggt tgtcgcctga gctgtagttg ccttcatcga tgaactgctg tacattttga 10740
   tacgtttttc cgtcaccgtc aaagattgat ttataatcct ctacaccgtt gatgttcaaa 10800
   gagctgtctg atgctgatac gttaacttgt gcagttgtca gtgtttgttt gccgtaatgt 10860
   ttaccggaga aatcagtgta gaataaacgg atttttccgt cagatgtaaa tgtggctgaa 10920
   cctgaccatt cttgtgtttg gtcttttagg atagaatcat ttgcatcgaa tttgtcgctg 10980
   tctttaaaga cgcggccagc gtttttccag ctgtcaatag aagtttcgcc gactttttga 11040
   tagaacatgt aaatcgatgt gtcatccgca tttttaggat ctccggctaa tgcaaagacg 11100
   atgtggtagc cgtgatagtt tgcgacagtg ccgtcagcgt tttgtaatgg ccagctgtcc 11160
   caaacgtcca ggccttttgc agaagagata tttttaattg tggacgaatc aaattcagaa 11220
   acttgatatt tttcattttt ttgctgttca gggatttgca gcatatcatg gcgtgtaata 11280
   tgggaaatgc cgtatgtttc cttatatggc ttttggttcg tttctttcgc aaacgcttga 11340
   gttgcgcctc ctgccagcag tgcggtagta aaggttaata ctgttgcttg ttttgcaaac 11400
   tttttgatgt tcatcgttca tgtctccttt tttatgtact gtgttagcgg tctgcttctt 11460
   ccagccctcc tgtttgaaga tggcaagtta gttacgcaca ataaaaaaag acctaaaata 11520
   tgtaaggggt gacgccaaag tatacacttt gccctttaca cattttaggt cttgcctgct  11580
   ttatcagtaa caaacccgcg cgatttactt ttcgacctca ttctattaga ctctcgtttg  11640
   gattgcaact ggtctatttt cctcttttgt ttgatagaaa atcataaaag gatttgcaga  11700
   ctacgggcct aaagaactaa aaaatctatc tgtttctttt cattctctgt attttttata  11760
   gtttctgttg catgggcata aagttgcctt tttaatcaca attcagaaaa tatcataata  11820
   tctcatttca ctaaataata gtgaacggca ggtatatgtg atgggttaaa aa          11872
<210> 39
   <211> 9206
   <212> DNA
   <213> artificial
<220>
   <223> Plasmid pOM180
<400> 39
   ggatcggcgg ccagggccct catgggccgg gcgtgcgcaa tagactcgtc accaaaaccg     60
   atggagtgtt tttgacgctg gaagatggca gcaccgtgat tgacgcgatg agctcctggt    120
   ggtcggcaat tcatggacac ggacaccccc gactgaaagc tgccgcccaa aaacaaatcg    180
   acaccatgag tcacgtcatg tttggcggac taacccacga gcccgccatt aagctcaccc    240
   acaaactcct caatctcact ggaaattcct ttgaccacgt cttttattcc gattcgggct    300
   cggtctcagt ggaggtcgcc atcaaaatgg cactgcaggc ctccaaagga caaggccacc    360
   cggaacgaac aaaactcctc acctggcggt ccggctacca cggagacaca ttcaccgcga    420
   tgagcgtgtg cgacccagaa aatggcatgc atagcctctg gaaaggcaca ctccccgagc    480
   agattttcgc ccccgcccca ccagttcggg ggtcatcgcc gcaggcgatt tccgagtacc    540
   tgcgcagcat ggaattgctt atcgacgaga ccgtctccgc aatcatcatc gaaccgatcg    600
   tccaaggcgc tggaggcatg cgcgcggccg cacagcgatc ccagaggaaa tatcctctgg    660
   ggtcgctgtg tcgaccttaa agtttggctg ccatgtgaat ttttagcacc ctcaacagtt    720
   gagtgctggc actctcgggg gtagagtgcc aaataggttg tttgacacac agttgttcac    780
   ccgcgacgac ggctgtgctg gaaacccaca accggcacac acaaaatttt tctagaagga    840
   ggtgatagta tgggtttcca gcaaggcagc atccgcaaag caaacatggg cacaaccggc    900
   actaacgacg gggcaaccac cccaccagcc aacaccagca ccccagccgt agacatcgac    960
   gtccgcgacc tgggcaccgt ggactacgag gacacttggc acctccaggc aaatctcgcc   1020
   gcccagcgcg ccgaagaaaa aatcccagac accatcctcc tactccagca tccgccgacg   1080
   tacaccgccg gcaagcgcac ccaggattcc gaccgcccta ccaacggcct gccagtagtc   1140
   gacgtcgacc gcggaggccg catcacctgg catggacccg gccagttggt cgcatatccc   1200
   atcatcaagc tggccgaccc ggtggacgtg gtcgattatg tccgccgcct ggagcaggcg   1260
   ctaatccaga cttgtgaaga tcttggcctg cacggcaccg gccgcgtaga ggggcgttcg   1320
   ggcgtgtggc tgcctgcggg cgtcattaat ggcgagctca agcccgcacg taagatagcc 1380
   gcgatcggca tccgcgtgac gcgcggcgtg accatgcacg gagtggccct caactgcgat 1440
   aacaccatgg agtattacga ccacattgtg ccttgtgggc tggcggatgc gggtgtcacg 1500
   acgctcaccg aggagctggg gcgcgatgtt agtgtttctg acgcctactc atccctcgcc 1560
   cacaacctcg ttgatgcttt gaacggcgac ttgccggtgc attcctaggg gaaaacccga 1620
   ggtggattcc tatacggcac ccagcgtagg ctagtcggca tgagtgtgac cgcagatgga 1680
   cgccggatgc tacgcatcga ggcgaagaat gccgaaaccc ccatcgagtc gaagcctcgg 1740
   tggatccgca cgaccgcgaa ggtcggcccg gagtatcggg atattaagaa tcgcgtgaag 1800
   ggcgctggcc tgcacaccgt gtgccaagag gctggctgcc cgaatatcaa cgagtgctgg 1860
   gaggaccgcg aggcgacgtt cctcatcggc ggtgatacgt gttcccgccg ttgcgacttc 1920
   tgccagatta agtccggccg cccgtccccg ctggatatgg acgagccacg ccgcgtggcg 1980
   gagaatgtcc gcgagatggg tctgcgctac gccaccatca ccggcgtgac gcgcgatgac 2040
   ctggatgatg agggcgcgtg gctgtatgcc gaggttgtga agaagattca cgagctgaac 2100
   ccgaacacgg gtgtggagaa tctgacgcca gatttttcca accgcccgga gctgctgaag 2160
   gtcgtcttcg attcccagcc ggaggtgttt gcccacaacc tggagacagt tccgcgcatc 2220
   tttaagcgca tccgcccggc ctttaagtac gaccgttccc tggaggtcat tcaggcagct 2280
   cacgattacg gcctggtgac gaagtccaac ctgatcctgg gcatgggtga gaagaaggaa 2340
   gaggtccgcg cggctatcaa ggacctggca gacgccggca ccgacattct gacgattacc 2400
   cagtacctgc gcccgtcttc catgcaccac ccgattgagc gttgggtgaa gccggaggag 2460
   ttcatggagc actccgacgc agcctacgag ctgggcatca aggctgttat gtccggcccg 2520
   ttggtgcgtt cctcttaccg cgccggccgc ctgtacgcgc aggccaagca ggcgcgcggc 2580
   gaggctattc cggagaacct gaagcacttg gaggagactc tcgattccac cacgtcgcag 2640
   gaggcctcta cactgctgga gcgctacggc gcttcggagg acacgccggt cactgcgtcg 2700
   cgccgctagc tcggcggggc actttcgccc cgcctttatt catgccttat tgttgaatcc 2760
   atggcgaagg ataaaaagga cgtaagggtc aaggaagaga agtgatccgc cctcccgcac 2820
   gctttgcggg agggcttttc ttttaccggt accggaactg gggttgggaa aaccttctcc 2880
   acagccgttt tggttcgata cttagccgat caaggacacg atgttctgcc cgtaaagcta 2940
   gtccaaaccg gtgaacttcc aggcgaggga gacatcttta acattgaacg cttgactgga 3000
   attgctggag aggaatttgc tcgtttcaaa gaccctcttg cgccaaatct ggcagcccga 3060
   cgagaggggg tcgagccaat acagtttgat cagattatct cgtggcttcg tggttttgac 3120
   gacccagatc gcatcattgt ggtggagggc gctggtggcc tgctggtcag attaggggaa 3180
   gatttcaccc tggcagatgt tgcctccgct ttgaatgcac ccttagtgat tgtgacaagc 3240
   accggattgg gaagcctcaa cgctgctgaa ttaagcgttg aggcagcaaa ccgccgagga 3300
   ctcacagtgt tgggagtcct cggcggttcg atccctcaaa atcctgatct agctacgatg 3360
   cttaatctcg aagaatttga gagagtcacc ggcgtgccct tttggggagc tttgccggaa 3420
   gggttgtcac gggtggaggg gttcgtcgaa aagcaatctt ttccggccct tgatgccttt 3480
   aagaaaccgc cggcaaggct cccaacgcgt cccgggattt aaatcgctag cgggctgcta 3540
   aaggaagcgg aacacgtaga aagccagtcc gcagaaacgg tgctgacccc ggatgaatgt 3600
   cagctactgg gctatctgga caagggaaaa cgcaagcgca aagagaaagc aggtagcttg 3660
   cagtgggctt acatggcgat agctagactg ggcggtttta tggacagcaa gcgaaccgga 3720
   attgccagct ggggcgccct ctggtaaggt tgggaagccc tgcaaagtaa actggatggc 3780
   tttcttgccg ccaaggatct gatggcgcag gggatcaaga tctgatcaag agacaggatg 3840
   aggatcgttt cgcatgattg aacaagatgg attgcacgca ggttctccgg ccgcttgggt 3900
   ggagaggcta ttcggctatg actgggcaca acagacaatc ggctgctctg atgccgccgt 3960
   gttccggctg tcagcgcagg ggcgcccggt tctttttgtc aagaccgacc tgtccggtgc 4020
   cctgaatgaa ctgcaggacg aggcagcgcg gctatcgtgg ctggccacga cgggcgttcc 4080
   ttgcgcagct gtgctcgacg ttgtcactga agcgggaagg gactggctgc tattgggcga 4140
   agtgccgggg caggatctcc tgtcatctca ccttgctcct gccgagaaag tatccatcat 4200
   ggctgatgca atgcggcggc tgcatacgct tgatccggct acctgcccat tcgaccacca 4260
   agcgaaacat cgcatcgagc gagcacgtac tcggatggaa gccggtcttg tcgatcagga 4320
   tgatctggac gaagagcatc aggggctcgc gccagccgaa ctgttcgcca ggctcaaggc 4380
   gcgcatgccc gacggcgagg atctcgtcgt gacccatggc gatgcctgct tgccgaatat 4440
   catggtggaa aatggccgct tttctggatt catcgactgt ggccggctgg gtgtggcgga 4500
   ccgctatcag gacatagcgt tggctacccg tgatattgct gaagagcttg gcggcgaatg 4560
   ggctgaccgc ttcctcgtgc tttacggtat cgccgctccc gattcgcagc gcatcgcctt 4620
   ctatcgcctt cttgacgagt tcttctgagc gggactctgg ggttcgaaat gaccgaccaa 4680
   gcgacgccca acctgccatc acgagatttc gattccaccg ccgccttcta tgaaaggttg 4740
   ggcttcggaa tcgttttccg ggacgccggc tggatgatcc tccagcgcgg ggatctcatg 4800
   ctggagttct tcgcccacgc tagtttaaac tgcggatcag tgagggtttg taactgcggg 4860
   tcaaggatct ggatttcgat cacggcacga tcatcgtgcg ggagggcaag ggctccaagg 4920
   atcgggcctt gatgttaccc gagagcttgg cacccagcct gcgcgagcag gggaattgat 4980
   ccggtggatg accttttgaa tgacctttaa tagattatat tactaattaa ttggggaccc 5040
   tagaggtccc cttttttatt ttaaaaattt tttcacaaaa cggtttacaa gcataacggg 5100
   ttttgctgcc cgcaaacggg ctgttctggt gttgctagtt tgttatcaga atcgcagatc 5160
   cggcttcagg tttgccggct gaaagcgcta tttcttccag aattgccatg attttttccc 5220
   cacgggaggc gtcactggct cccgtgttgt cggcagcttt gattcgataa gcagcatcgc 5280
   ctgtttcagg ctgtctatgt gtgactgttg agctgtaaca agttgtctca ggtgttcaat 5340
   ttcatgttct agttgctttg ttttactggt ttcacctgtt ctattaggtg ttacatgctg 5400
   ttcatctgtt acattgtcga tctgttcatg gtgaacagct ttaaatgcac caaaaactcg 5460
   taaaagctct gatgtatcta tcttttttac accgttttca tctgtgcata tggacagttt 5520
   tccctttgat atctaacggt gaacagttgt tctacttttg tttgttagtc ttgatgcttc 5580
   actgatagat acaagagcca taagaacctc agatccttcc gtatttagcc agtatgttct 5640
   ctagtgtggt tcgttgtttt tgcgtgagcc atgagaacga accattgaga tcatgcttac 5700
   tttgcatgtc actcaaaaat tttgcctcaa aactggtgag ctgaattttt gcagttaaag 5760
   catcgtgtag tgtttttctt agtccgttac gtaggtagga atctgatgta atggttgttg 5820
   gtattttgtc accattcatt tttatctggt tgttctcaag ttcggttacg agatccattt 5880
   gtctatctag ttcaacttgg aaaatcaacg tatcagtcgg gcggcctcgc ttatcaacca 5940
   ccaatttcat attgctgtaa gtgtttaaat ctttacttat tggtttcaaa acccattggt 6000
   taagcctttt aaactcatgg tagttatttt caagcattaa catgaactta aattcatcaa 6060
   ggctaatctc tatatttgcc ttgtgagttt tcttttgtgt tagttctttt aataaccact 6120
   cataaatcct catagagtat ttgttttcaa aagacttaac atgttccaga ttatatttta 6180
   tgaatttttt taactggaaa agataaggca atatctcttc actaaaaact aattctaatt 6240
   tttcgcttga gaacttggca tagtttgtcc actggaaaat ctcaaagcct ttaaccaaag 6300
   gattcctgat ttccacagtt ctcgtcatca gctctctggt tgctttagct aatacaccat 6360
   aagcattttc cctactgatg ttcatcatct gagcgtattg gttataagtg aacgataccg 6420
   tccgttcttt ccttgtaggg ttttcaatcg tggggttgag tagtgccaca cagcataaaa 6480
   ttagcttggt ttcatgctcc gttaagtcat agcgactaat cgctagttca tttgctttga 6540
   aaacaactaa ttcagacata catctcaatt ggtctaggtg attttaatca ctataccaat 6600
   tgagatgggc tagtcaatga taattactag tccttttcct ttgagttgtg ggtatctgta 6660
   aattctgcta gacctttgct ggaaaacttg taaattctgc tagaccctct gtaaattccg 6720
   ctagaccttt gtgtgttttt tttgtttata ttcaagtggt tataatttat agaataaaga 6780
   aagaataaaa aaagataaaa agaatagatc ccagccctgt gtataactca ctactttagt 6840
   cagttccgca gtattacaaa aggatgtcgc aaacgctgtt tgctcctcta caaaacagac 6900
   cttaaaaccc taaaggctta agtagcaccc tcgcaagctc gggcaaatcg ctgaatattc 6960
   cttttgtctc cgaccatcag gcacctgagt cgctgtcttt ttcgtgacat tcagttcgct 7020
   gcgctcacgg ctctggcagt gaatgggggt aaatggcact acaggcgcct tttatggatt 7080
   catgcaagga aactacccat aatacaagaa aagcccgtca cgggcttctc agggcgtttt 7140
   atggcgggtc tgctatgtgg tgctatctga ctttttgctg ttcagcagtt cctgccctct 7200
   gattttccag tctgaccact tcggattatc ccgtgacagg tcattcagac tggctaatgc 7260
   acccagtaag gcagcggtat catcaacagg cttagtttaa acccatcggc attttctttt 7320
   gcgtttttat ttgttaactg ttaattgtcc ttgttcaagg atgctgtctt tgacaacaga 7380
   tgttttcttg cctttgatgt tcagcaggaa gctcggcgca aacgttgatt gtttgtctgc 7440
   gtagaatcct ctgtttgtca tatagcttgt aatcacgaca ttgtttcctt tcgcttgagg 7500
   tacagcgaag tgtgagtaag taaaggttac atcgttagga tcaagatcca tttttaacac 7560
   aaggccagtt ttgttcagcg gcttgtatgg gccagttaaa gaattagaaa cataaccaag 7620
   catgtaaata tcgttagacg taatgccgtc aatcgtcatt tttgatccgc gggagtcagt 7680
   gaacaggtac catttgccgt tcattttaaa gacgttcgcg cgttcaattt catctgttac 7740
   tgtgttagat gcaatcagcg gtttcatcac ttttttcagt gtgtaatcat cgtttagctc 7800
   aatcataccg agagcgccgt ttgctaactc agccgtgcgt tttttatcgc tttgcagaag 7860
   tttttgactt tcttgacgga agaatgatgt gcttttgcca tagtatgctt tgttaaataa 7920
   agattcttcg ccttggtagc catcttcagt tccagtgttt gcttcaaata ctaagtattt 7980
   gtggccttta tcttctacgt agtgaggatc tctcagcgta tggttgtcgc ctgagctgta 8040
   gttgccttca tcgatgaact gctgtacatt ttgatacgtt tttccgtcac cgtcaaagat 8100
   tgatttataa tcctctacac cgttgatgtt caaagagctg tctgatgctg atacgttaac 8160
   ttgtgcagtt gtcagtgttt gtttgccgta atgtttaccg gagaaatcag tgtagaataa 8220
   acggattttt ccgtcagatg taaatgtggc tgaacctgac cattcttgtg tttggtcttt 8280
   taggatagaa tcatttgcat cgaatttgtc gctgtcttta aagacgcggc cagcgttttt 8340
   ccagctgtca atagaagttt cgccgacttt ttgatagaac atgtaaatcg atgtgtcatc 8400
   cgcattttta ggatctccgg ctaatgcaaa gacgatgtgg tagccgtgat agtttgcgac 8460
   agtgccgtca gcgttttgta atggccagct gtcccaaacg tccaggcctt ttgcagaaga 8520
   gatattttta attgtggacg aatcaaattc agaaacttga tatttttcat ttttttgctg 8580
   ttcagggatt tgcagcatat catggcgtgt aatatgggaa atgccgtatg tttccttata 8640
   tggcttttgg ttcgtttctt tcgcaaacgc ttgagttgcg cctcctgcca gcagtgcggt 8700
   agtaaaggtt aatactgttg cttgttttgc aaactttttg atgttcatcg ttcatgtctc 8760
   cttttttatg tactgtgtta gcggtctgct tcttccagcc ctcctgtttg aagatggcaa 8820
   gttagttacg cacaataaaa aaagacctaa aatatgtaag gggtgacgcc aaagtataca 8880
   ctttgccctt tacacatttt aggtcttgcc tgctttatca gtaacaaacc cgcgcgattt 8940
   acttttcgac ctcattctat tagactctcg tttggattgc aactggtcta ttttcctctt 9000
   ttgtttgata gaaaatcata aaaggatttg cagactacgg gcctaaagaa ctaaaaaatc 9060
   tatctgtttc ttttcattct ctgtattttt tatagtttct gttgcatggg cataaagttg 9120
   cctttttaat cacaattcag aaaatatcat aatatctcat ttcactaaat aatagtgaac 9180
   ggcaggtata tgtgatgggt taaaaa 9206
<210> 40
   <211> 10003
   <212> DNA
   <213> artificial
<220>
   <223> Plasmid pOM331
<400> 40
   ggatcggcgg ccagggccct catgagatga gtctttgatc aagggtggcg cttttgactc 60
   ccttggacac gcacgaaaag gcctcatgct ggtcttcgaa gatgccgttg attccgtcat 120
   cgctaccaaa aaagctgctg acaagggaca atttgatctc tttgcagctt tcgactcgga 180
   taacaacgac gatgtggcaa gtttcttcca gatcaccgtt cctgatgacg aatgggaccg 240
   taagcatgag ctcgcactcg agcgagaaat gctgggtctg tatgtttctg gacacccact 300
   cgatggctat gaagatgcca ttgctgccca ggttgataca gcactgacca ccattgttgc 360
   cggtgaactc aagcacggcg cagaagtgac cgtgggtggc attatctctg gtgtggatcg 420
   acggttctcc aagaaggacg gttccccttg ggcgattgtc accattgaag atcacaacgg 480
   cgcgtccgtt gaattgttgg tcttcaacaa ggtgtattcc atcgttggat ccatgattgt 540
   ggaagacaac atcatcttgg ccaaggcaca catctccatt cgagatgatc gtatgagcct 600
   tttctgtgat gatctccgcg ttccagagct tgggccagga aacgggcaag gacttccgct 660
   tcgtttgtcc atgcgtactg atcagtgcac catgtccaac attgccaagc tcaagcaggt 720
   gctggtggac aacaagggtg aatctgatgt gtacctcaat ttgatcgatg gggataactc 780
   cacggtcatg attttgggtg atcacttaag agtcaaccga tccgcaagtt tgatgggcga 840
   cctcaaggca acgatggggc caggcatcct cggttaatca catcacactg ggattacccc 900
   gtgtaggggt gaaaacccga atgatgaata aaattccggg tgcagtgacc gtaggtgagg 960
   taaacgcggt tagagtcgaa tgagagtttg atactttctt tcgactttta gattggattt 1020
   tcaatgagcc agaaccgcat caggaccact cacgttggtt ccttgccccg taccccagag 1080
   ctacttgatg caaacatcaa gcgttctaac ggtgagattg gggaggagga attcttccag 1140
   attctgcagt cttctgtaga tgacctgcgg ccgcacagcg atcccagagg aaatatcctc 1200
   tggggtcgct gtgtcgacct taaagtttgg ctgccatgtg aatttttagc accctcaaca 1260
   gttgagtgct ggcactctcg ggggtagagt gccaaatagg ttgtttgaca cacagttgtt 1320
   cacccgcgac gacggctgtg ctggaaaccc acaaccggca cacacaaaat ttttctagaa 1380
   ggaggagaaa acatgtccac tgaaatcaaa actcaggtcg tggtacttgg ggcaggcccc 1440
   gcaggttact ccgctgcctt ccgttgcgct gatttaggtc tggaaaccgt aatcgtagaa 1500
   cgttacaaca cccttggcgg tgtttgcctg aacgtcggct gtatcccttc taaagcactg 1560
   ctgcacgtag caaaagttat cgaagaagcc aaagcgctgg ctgaacacgg tatcgtcttc 1620
   ggcgaaccga aaaccgatat cgacaagatt cgtacctgga aagagaaagt gatcaatcag 1680
   ctgaccggtg gtctggctgg tatggcgaaa ggccgcaaag tcaaagtggt caacggtctg 1740
   ggtaaattca ccggggctaa caccctggaa gttgaaggtg agaacggcaa aaccgtgatc 1800
   aacttcgaca acgcgatcat tgcagcgggt tctcgcccga tccaactgcc gtttattccg 1860
   catgaagatc cgcgtatctg ggactccact gacgcgctgg aactgaaaga agtaccagaa 1920
   cgcctgctgg taatgggtgg cggtatcatc ggtctggaaa tgggcaccgt ttaccacgcg 1980
   ctgggttcac agattgacgt ggttgaaatg ttcgaccagg ttatcccggc agctgacaaa 2040
   gacatcgtta aagtcttcac caagcgtatc agcaagaaat tcaacctgat gctggaaacc 2100
   aaagttaccg ccgttgaagc gaaagaagac ggcatttatg tgacgatgga aggcaaaaaa 2160
   gcacccgctg aaccgcagcg ttacgacgcc gtgctggtag cgattggtcg tgtgccgaac 2220
   ggtaaaaacc tcgacgcagg caaagcaggc gtggaagttg acgaccgtgg tttcatccgc 2280
   gttgacaaac agctgcgtac caacgtaccg cacatctttg ctatcggcga tatcgtcggt 2340
   caaccgatgc tggcacacaa aggtgttcac gaaggtcacg ttgccgctga agttatcgcc 2400
   ggtaagaaac actacttcga tccgaaagtt atcccgtcca tcgcctatac cgaaccagaa 2460
   gttgcatggg tgggtctgac tgagaaagaa gcgaaagaga aaggcatcag ctatgaaacc 2520
   gccaccttcc cgtgggctgc ttctggtcgt gctatcgctt ccgactgcgc agacggtatg 2580
   accaagctga ttttcgacaa agaatctcac cgtgtgatcg gtggtgcgat tgtcggtact 2640
   aacggcggcg agctgctggg tgaaatcggc ctggcaatcg aaatgggttg tgatgctgaa 2700
   gacatcgcac tgaccatcca cgcgcacccg actctgcacg agtctgtggg cctggcggca 2760
   gaagtgttcg aaggtagcat taccgacctg ccgaacccga aagcgaagaa gaagtaattt 2820
   ttcgtttgcc ggaacatccg gcaattaaaa aagcggctaa ccacgccgct ttttttacgt 2880
   ctgcaattta cctttccagt cttcttgctc cacggatccg ccctcccgca cgctttgcgg 2940
   gagggcggta ccaggtcact gacatcccat tcggtgacat cattggtgag atcctgcgcg 3000
   cagaggtcgg tggcttctcc ttcgaaggcg catctcctcg tcacgcacac gagtggcgtg 3060
   tatgggaaga aaacaagctt cctgaaggct ctgttatcta ccctggtgtt gtgtctcact 3120
   ccatcaacgc tgtggagcac ccacgcctgg ttgctgatcg tatcgttcag ttcgccaagc 3180
   ttgttggccc tgagaacgtc attgcgtcca ctgactgtgg tctgggcgga cgtctgcatt 3240
   cccagatcgc atgggcaaag ctggagtccc tagtagaggg cgctcgcatt gcatcaaagg 3300
   aactgttcta agctagacaa cgagggttgc tagtctaagc agcaaaatga gcggctgttg 3360
   ttccttcagg aaaattatct gaaggaacaa tagccgctca ttttatgtca gtgtgctttt 3420
   aagcgtcgac gttgatgcca aactgggtga gcatgtcacg cagagtctgc ttgaacgatg 3480
   ggtcgacggt caccatgaag gatgctgcgg tggaagcaag agtggtgatt gcgagcaatc 3540
   cctgcagtac cgcaaaagga atggcaagcc attctggtgg ggagacaatg gaaccaatca 3600
   ctggcatttc ggacaaccga tcgatcagtg cttgctgctc ttcgctgatt tcatcgtcat 3660
   catcgctggt gtctttgccg gaggagctca agcttggggc cgcaggaatg tcgccattgc 3720
   tgagcattga gctgccttca gagctgcctg gccaggtttc gtttccatcg actggatttc 3780
   catcatcatc aaggatctgt gatgaggtga tgttgtctga gagctgtgtc agtgcgtcag 3840
   aggactgagc ctgggcaact ggagtgaaca cggacaatgc cacagcgctt gctgtaacaa 3900
   gggtcaaagt acttcgacgc aaagacaaaa cttttctcct ggcaataaat atgcggattt 3960
   actatggaaa caagatagaa gattggatag cgaaagctat cctcaactcg tggaaagtgt 4020
   agtgccacaa ccacagtatt ggctagaaaa caatctatag cattgttcta caaagagctt 4080
   gttggaaata aaacctatgc caaagtaggt gcaattctag gagaagatta cactagtcaa 4140
   ccatgagtga aacatacgtg tctgagaaaa gtccaggagt gatggctagc ggagcggagc 4200
   tgattcgtgc cgccgacatt caaacggcgc aggcacgaat ttcctccgtc attgcaccaa 4260
   ctccattgca gtattgccct cgtctttctg agatcgagct cacgcgtccc gggatttaaa 4320
   tcgctagcgg gctgctaaag gaagcggaac acgtagaaag ccagtccgca gaaacggtgc 4380
   tgaccccgga tgaatgtcag ctactgggct atctggacaa gggaaaacgc aagcgcaaag 4440
   agaaagcagg tagcttgcag tgggcttaca tggcgatagc tagactgggc ggttttatgg 4500
   acagcaagcg aaccggaatt gccagctggg gcgccctctg gtaaggttgg gaagccctgc 4560
   aaagtaaact ggatggcttt cttgccgcca aggatctgat ggcgcagggg atcaagatct 4620
   gatcaagaga caggatgagg atcgtttcgc atgattgaac aagatggatt gcacgcaggt 4680
   tctccggccg cttgggtgga gaggctattc ggctatgact gggcacaaca gacaatcggc 4740
   tgctctgatg ccgccgtgtt ccggctgtca gcgcaggggc gcccggttct ttttgtcaag 4800
   accgacctgt ccggtgccct gaatgaactg caggacgagg cagcgcggct atcgtggctg 4860
   gccacgacgg gcgttccttg cgcagctgtg ctcgacgttg tcactgaagc gggaagggac 4920
   tggctgctat tgggcgaagt gccggggcag gatctcctgt catctcacct tgctcctgcc 4980
   gagaaagtat ccatcatggc tgatgcaatg cggcggctgc atacgcttga tccggctacc 5040
   tgcccattcg accaccaagc gaaacatcgc atcgagcgag cacgtactcg gatggaagcc 5100
   ggtcttgtcg atcaggatga tctggacgaa gagcatcagg ggctcgcgcc agccgaactg 5160
   ttcgccaggc tcaaggcgcg catgcccgac ggcgaggatc tcgtcgtgac ccatggcgat 5220
   gcctgcttgc cgaatatcat ggtggaaaat ggccgctttt ctggattcat cgactgtggc 5280
   cggctgggtg tggcggaccg ctatcaggac atagcgttgg ctacccgtga tattgctgaa 5340
   gagcttggcg gcgaatgggc tgaccgcttc ctcgtgcttt acggtatcgc cgctcccgat 5400
   tcgcagcgca tcgccttcta tcgccttctt gacgagttct tctgagcggg actctggggt 5460
   tcgaaatgac cgaccaagcg acgcccaacc tgccatcacg agatttcgat tccaccgccg 5520
   ccttctatga aaggttgggc ttcggaatcg ttttccggga cgccggctgg atgatcctcc 5580
   agcgcgggga tctcatgctg gagttcttcg cccacgctag tttaaactgc ggatcagtga 5640
   gggtttgtaa ctgcgggtca aggatctgga tttcgatcac ggcacgatca tcgtgcggga 5700
   gggcaagggc tccaaggatc gggccttgat gttacccgag agcttggcac ccagcctgcg 5760
   cgagcagggg aattgatccg gtggatgacc ttttgaatga cctttaatag attatattac 5820
   taattaattg gggaccctag aggtcccctt ttttatttta aaaatttttt cacaaaacgg 5880
   tttacaagca taacgggttt tgctgcccgc aaacgggctg ttctggtgtt gctagtttgt 5940
   tatcagaatc gcagatccgg cttcaggttt gccggctgaa agcgctattt cttccagaat 6000
   tgccatgatt ttttccccac gggaggcgtc actggctccc gtgttgtcgg cagctttgat 6060
   tcgataagca gcatcgcctg tttcaggctg tctatgtgtg actgttgagc tgtaacaagt 6120
   tgtctcaggt gttcaatttc atgttctagt tgctttgttt tactggtttc acctgttcta 6180
   ttaggtgtta catgctgttc atctgttaca ttgtcgatct gttcatggtg aacagcttta 6240
   aatgcaccaa aaactcgtaa aagctctgat gtatctatct tttttacacc gttttcatct 6300
   gtgcatatgg acagttttcc ctttgatatc taacggtgaa cagttgttct acttttgttt 6360
   gttagtcttg atgcttcact gatagataca agagccataa gaacctcaga tccttccgta 6420
   tttagccagt atgttctcta gtgtggttcg ttgtttttgc gtgagccatg agaacgaacc 6480
   attgagatca tgcttacttt gcatgtcact caaaaatttt gcctcaaaac tggtgagctg 6540
   aatttttgca gttaaagcat cgtgtagtgt ttttcttagt ccgttacgta ggtaggaatc 6600
   tgatgtaatg gttgttggta ttttgtcacc attcattttt atctggttgt tctcaagttc 6660
   ggttacgaga tccatttgtc tatctagttc aacttggaaa atcaacgtat cagtcgggcg 6720
   gcctcgctta tcaaccacca atttcatatt gctgtaagtg tttaaatctt tacttattgg 6780
   tttcaaaacc cattggttaa gccttttaaa ctcatggtag ttattttcaa gcattaacat 6840
   gaacttaaat tcatcaaggc taatctctat atttgccttg tgagttttct tttgtgttag 6900
   ttcttttaat aaccactcat aaatcctcat agagtatttg ttttcaaaag acttaacatg 6960
   ttccagatta tattttatga atttttttaa ctggaaaaga taaggcaata tctcttcact 7020
   aaaaactaat tctaattttt cgcttgagaa cttggcatag tttgtccact ggaaaatctc 7080
   aaagccttta accaaaggat tcctgatttc cacagttctc gtcatcagct ctctggttgc 7140
   tttagctaat acaccataag cattttccct actgatgttc atcatctgag cgtattggtt 7200
   ataagtgaac gataccgtcc gttctttcct tgtagggttt tcaatcgtgg ggttgagtag 7260
   tgccacacag cataaaatta gcttggtttc atgctccgtt aagtcatagc gactaatcgc 7320
   tagttcattt gctttgaaaa caactaattc agacatacat ctcaattggt ctaggtgatt 7380
   ttaatcacta taccaattga gatgggctag tcaatgataa ttactagtcc ttttcctttg 7440
   agttgtgggt atctgtaaat tctgctagac ctttgctgga aaacttgtaa attctgctag 7500
   accctctgta aattccgcta gacctttgtg tgtttttttt gtttatattc aagtggttat 7560
   aatttataga ataaagaaag aataaaaaaa gataaaaaga atagatccca gccctgtgta 7620
   taactcacta ctttagtcag ttccgcagta ttacaaaagg atgtcgcaaa cgctgtttgc 7680
   tcctctacaa aacagacctt aaaaccctaa aggcttaagt agcaccctcg caagctcggg 7740
   caaatcgctg aatattcctt ttgtctccga ccatcaggca cctgagtcgc tgtctttttc 7800
   gtgacattca gttcgctgcg ctcacggctc tggcagtgaa tgggggtaaa tggcactaca 7860
   ggcgcctttt atggattcat gcaaggaaac tacccataat acaagaaaag cccgtcacgg 7920
   gcttctcagg gcgttttatg gcgggtctgc tatgtggtgc tatctgactt tttgctgttc 7980
   agcagttcct gccctctgat tttccagtct gaccacttcg gattatcccg tgacaggtca 8040
   ttcagactgg ctaatgcacc cagtaaggca gcggtatcat caacaggctt agtttaaacc 8100
   catcggcatt ttcttttgcg tttttatttg ttaactgtta attgtccttg ttcaaggatg 8160
   ctgtctttga caacagatgt tttcttgcct ttgatgttca gcaggaagct cggcgcaaac 8220
   gttgattgtt tgtctgcgta gaatcctctg tttgtcatat agcttgtaat cacgacattg 8280
   tttcctttcg cttgaggtac agcgaagtgt gagtaagtaa aggttacatc gttaggatca 8340
   agatccattt ttaacacaag gccagttttg ttcagcggct tgtatgggcc agttaaagaa 8400
   ttagaaacat aaccaagcat gtaaatatcg ttagacgtaa tgccgtcaat cgtcattttt 8460
   gatccgcggg agtcagtgaa caggtaccat ttgccgttca ttttaaagac gttcgcgcgt 8520
   tcaatttcat ctgttactgt gttagatgca atcagcggtt tcatcacttt tttcagtgtg 8580
   taatcatcgt ttagctcaat cataccgaga gcgccgtttg ctaactcagc cgtgcgtttt 8640
   ttatcgcttt gcagaagttt ttgactttct tgacggaaga atgatgtgct tttgccatag 8700
   tatgctttgt taaataaaga ttcttcgcct tggtagccat cttcagttcc agtgtttgct 8760
   tcaaatacta agtatttgtg gcctttatct tctacgtagt gaggatctct cagcgtatgg 8820
   ttgtcgcctg agctgtagtt gccttcatcg atgaactgct gtacattttg atacgttttt 8880
   ccgtcaccgt caaagattga tttataatcc tctacaccgt tgatgttcaa agagctgtct 8940
   gatgctgata cgttaacttg tgcagttgtc agtgtttgtt tgccgtaatg tttaccggag 9000
   aaatcagtgt agaataaacg gatttttccg tcagatgtaa atgtggctga acctgaccat 9060
   tcttgtgttt ggtcttttag gatagaatca tttgcatcga atttgtcgct gtctttaaag 9120
   acgcggccag cgtttttcca gctgtcaata gaagtttcgc cgactttttg atagaacatg 9180
   taaatcgatg tgtcatccgc atttttagga tctccggcta atgcaaagac gatgtggtag 9240
   ccgtgatagt ttgcgacagt gccgtcagcg ttttgtaatg gccagctgtc ccaaacgtcc 9300
   aggccttttg cagaagagat atttttaatt gtggacgaat caaattcaga aacttgatat 9360
   ttttcatttt tttgctgttc agggatttgc agcatatcat ggcgtgtaat atgggaaatg 9420
   ccgtatgttt ccttatatgg cttttggttc gtttctttcg caaacgcttg agttgcgcct 9480
   cctgccagca gtgcggtagt aaaggttaat actgttgctt gttttgcaaa ctttttgatg 9540
   ttcatcgttc atgtctcctt ttttatgtac tgtgttagcg gtctgcttct tccagccctc 9600
   ctgtttgaag atggcaagtt agttacgcac aataaaaaaa gacctaaaat atgtaagggg 9660
   tgacgccaaa gtatacactt tgccctttac acattttagg tcttgcctgc tttatcagta 9720
   acaaacccgc gcgatttact tttcgacctc attctattag actctcgttt ggattgcaac 9780
   tggtctattt tcctcttttg tttgatagaa aatcataaaa ggatttgcag actacgggcc 9840
   taaagaacta aaaaatctat ctgtttcttt tcattctctg tattttttat agtttctgtt 9900
   gcatgggcat aaagttgcct ttttaatcac aattcagaaa atatcataat atctcatttc 9960
   actaaataat agtgaacggc aggtatatgt gatgggttaa aaa 10003
<210> 41
   <211> 11325
   <212> DNA
   <213> artificial
<220>
   <223> Plasmid pOM344
<400> 41
   ttgatcagcg gccgcacagc gatcccagag gaaatatcct ctggggtcgc tgtgtcgacc      60
   ttaaagtttg gctgccatgt gaatttttag caccctcaac agttgagtgc tggcactctc     120
   gggggtagag tgccaaatag gttgtttgac acacagttgt tcacccgcga cgacggctgt     180
   gctggaaacc cacaaccggc acacacaaaa tttttctaga ggaggtgaaa gtatggcaca     240
   gcagacccct ttgtacgaac aacacacgct ttgcggcgct cgcatggtgg atttccacgg     300
   ctggatgatg ccgctgcatt acggttcgca aatcgacgaa catcatgcgg tacgtaccga     360
   tgccggaatg tttgatgtgt cacatatgac catcgtcgat cttcgcggca gccgcacccg     420
   ggagtttctg cgttatctgc tggcgaacga tgtggcgaag ctcaccaaaa gcggcaaagc     480
   cctttactcg gggatgttga atgcctctgg cggtgtgata gatgacctca tcgtctacta     540
   ctttactgaa gatttcttcc gcctcgttgt taactccgcc acccgcgaaa aagacctctc     600
   ctggattacc caacacgctg aacctttcgg catcgaaatt accgttcgtg atgacctttc     660
   catgattgcc gtgcaagggc cgaatgcgca ggcaaaagct gccacactgt ttaatgacgc     720
   ccagcgtcag gcggtggaag ggatgaaacc gttctttggc gtgcaggcgg gcgatctgtt     780
   tattgccacc actggttata ccggtgaagc gggctatgaa attgcgctgc ccaatgaaaa     840
   agcggccgat ttctggcgtg cgctggtgga agcgggtgtt aagccatgtg gcttgggcgc     900
   gcgtgacacg ctgcgtctgg aagcgggcat gaatctttat ggtcaggaga tggacgaaac     960
   catctctcct ttagccgcca acatgggctg gaccatcgcc tgggaaccgg cagatcgtga 1020
   ctttatcggt cgtgaagccc tggaagtgca gcgtgagcat ggtacagaaa aactggttgg 1080
   tctggtgatg accgaaaaag gcgtgctgcg taatgaactg ccggtacgct ttaccgatgc 1140
   gcagggcaac cagcatgaag gcattatcac cagcggtact ttctccccga cgctgggtta 1200
   cagcattgcg ctggcgcgcg tgccggaagg tattggcgaa acggcgattg tgcaaattcg 1260
   caaccgtgaa atgccggtta aagtgacaaa acctgttttt gtgcgtaacg gcaaagccgt 1320
   cgcgtgattt acttttttgg agattgattg atgagcaacg taccagcaga actgaaatac 1380
   agcaaagaac acgaatggct gcgtaaagaa gccgacggca cttacaccgt tggtattacc 1440
   gaacatgctc aggagctgtt aggcgatatg gtgtttgttg acctgccgga agtgggcgca 1500
   acggttagcg cgggcgatga ctgcgcggtt gccgaatcgg taaaagcggc gtcagacatt 1560
   tatgcgccag taagcggtga aatcgtggcg gtaaacgacg cactgagcga ttccccggaa 1620
   ctggtgaaca gcgaaccgta tgcaggcggc tggatcttta aaatcaaagc cagcgatgaa 1680
   agcgaactgg aatcactgct ggatgcgacc gcatacgaag cattgttaga agacgagtaa 1740
   cggctttatt cctcttctgc gggagaggat cagggtgagg aaaatttatg cctcaccctc 1800
   actctcttcg taaggagaga ggttcacaat tcactgcacg tttcaggaac catcgctcat 1860
   gacacagacg ttaagccagc ttgaaaacag cggcgctttt attgaacgcc atatcggacc 1920
   ggacgccgcg caacagcaag aaatgctgaa tgccgttggt gcacaatcgt taaacgcgct 1980
   gaccggccag attgtgccga aagatattca acttgcgaca ccaccgcagg ttggcgcacc 2040
   ggcgaccgaa tacgccgcac tggcagaact caaggctatt gccagtcgca ataaacgctt 2100
   cacgtcttac atcggcatgg gttacaccgc cgtgcagcta ccgccggtta tcctgcgtaa 2160
   catgctggaa aatccgggct ggtataccgc gtacactccg tatcaacctg aagtctccca 2220
   gggccgcctt gaagcactgc tcaacttcca gcaggtaacg ctggatttga ctggactgga 2280
   tatggcctct gcttctcttc tggacgaggc caccgctgcc gccgaagcaa tggcgatggc 2340
   gaaacgcgtc agcaaactga aaaatgccaa ccgcttcttc gtggcttccg atgtgcatcc 2400
   gcaaacgctg gatgtggtcc gtactcgtgc cgaaaccttt ggttttgaag tgattgtcga 2460
   tgacgcgcaa aaagtgctcg accatcagga cgtcttcggc gtgctgttac agcaggtagg 2520
   cactaccggt gaaattcacg actacactgc gcttattagc gaactgaaat cacgcaaaat 2580
   tgtggtcagc gttgccgccg atattatggc gctggtgctg ttaactgcgc cgggtaaaca 2640
   gggcgcggat attgtttttg gttcggcgca acgcttcggc gtgccgatgg gctacggtgg 2700
   cccacacgcg gcattctttg cggcgaaaga tgaatacaaa cgctcaatgc cgggccgtat 2760
   tatcggtgta tcgaaagatg cagctggcaa taccgcgctg cgcatggcga tgcagactcg 2820
   cgagcaacat atccgccgtg agaaagcgaa ctccaacatt tgtacttccc aggtactgct 2880
   ggcaaacatc gccagcctgt atgccgttta tcacggcccg gttggcctga aacgtatcgc 2940
   taaccgcatt caccgtctga ccgatatcct ggcggcgggc ctgcaacaaa aaggtctgaa 3000
   actgcgccat gcgcactatt tcgacacctt gtgtgtggaa gtggccgaca aagcgggcgt 3060
   actgacgcgt gccgaagcgg ctgaaatcaa cctgcgtagc gatattctga acgcggttgg 3120
   gatcaccctt gatgaaacaa ccacgcgtga aaacgtaatg cagcttttca acgtgctgct 3180
   gggcgataac cacggcctgg acatcgacac gctggacaaa gacgtggctc acgacagccg 3240
   ctctatccag cctgcgatgc tgcgcgacga cgaaatcctc acccatccgg tgtttaatcg 3300
   ctaccacagc gaaaccgaaa tgatgcgcta tatgcactcg ctggagcgta aagatctggc 3360
   gctgaatcag gcgatgatcc cgctgggttc ctgcaccatg aaactgaacg ccgccgccga 3420
   gatgatccca atcacctggc cggaatttgc cgaactgcac ccgttctgcc cgccggagca 3480
   ggccgaaggt tatcagcaga tgattgcgca gctggctgac tggctggtga aactgaccgg 3540
   ttacgacgcc gtttgtatgc agccgaactc tggcgcacag ggcgaatacg cgggcctgct 3600
   ggcgattcgt cattatcatg aaagccgcaa cgaagggcat cgcgatatct gcctgatccc 3660
   ggcttctgcg cacggaacta accccgcttc tgcacatatg gcaggaatgc aggtggtggt 3720
   tgtggcgtgt gataaaaacg gcaacatcga tctgactgat ctgcgcgcga aagcggaaca 3780
   ggcgggcgat aacctctcct gtatcatggt gacttatcct tctacccacg gcgtgtatga 3840
   agaaacgatc cgtgaagtgt gtgaagtcgt gcatcagttc ggcggtcagg tttaccttga 3900
   tggcgcgaac atgaacgccc aggttggcat cacctcgccg ggctttattg gtgcggacgt 3960
   ttcacacctt aacctacata aaactttctg cattccgcac ggcggtggtg gtccgggtat 4020
   gggaccgatc ggcgtgaaag cgcatttggc accgtttgta ccgggtcata gcgtggtgca 4080
   aatcgaaggc atgttaaccc gtcagggcgc ggtttctgcg gcaccgttcg gtagcgcctc 4140
   tatcctgcca atcagctgga tgtacatccg catgatgggc gcagaagggc tgaaaaaagc 4200
   aagccaggtg gcaatcctca acgccaacta tattgccagc cgcctgcagg atgccttccc 4260
   ggtgctgtat accggtcgcg acggtcgcgt ggcgcacgaa tgtattctcg atattcgccc 4320
   gctgaaagaa gaaaccggca tcagcgagct ggatattgcc aagcgcctga tcgactacgg 4380
   tttccacgcg ccgacgatgt cgttcccggt ggcgggtacg ctgatggttg aaccgactga 4440
   atctgaaagc aaagtggaac tggatcgctt tatcgacgcg atgctggcta tccgcgcaga 4500
   aattgaccag gtgaaagccg gtgtctggcc gctggaagat aacccgctgg tgaacgcgcc 4560
   gcacattcag agcgaactgg tcgccgagtg ggcgcatccg tacagccgtg aagttgcggt 4620
   attcccggca ggtgtggcag acaaatactg gccgacagtg aaacgtctgg atgatgttta 4680
   cggcgaccgt aacctgttct gctcctgcgt accgattagc gaataccagt aattcactga 4740
   ttcgactatt ttctaaaggc gcttcggcgc ctttttagtc agatgacaaa gtacaaaagt 4800
   gctcagacag tcccctcgcc ggatccgccc tcccgcacgc tttgcgggag ggcttttctt 4860
   ttcccggtat ttaaatcgct agcgggctgc taaaggaagc ggaacacgta gaaagccagt 4920
   ccgcagaaac ggtgctgacc ccggatgaat gtcagctact gggctatctg gacaagggaa 4980
   aacgcaagcg caaagagaaa gcaggtagct tgcagtgggc ttacatggcg atagctagac 5040
   tgggcggttt tatggacagc aagcgaaccg gaattgccag ctggggcgcc ctctggtaag 5100
   gttgggaagc cctgcaaagt aaactggatg gctttcttgc cgccaaggat ctgatggcgc 5160
   aggggatcaa gatctgatca agagacagga tgaggatcgt ttcgcatgat tgaacaagat 5220
   ggattgcacg caggttctcc ggccgcttgg gtggagaggc tattcggcta tgactgggca 5280
   caacagacaa tcggctgctc tgatgccgcc gtgttccggc tgtcagcgca ggggcgcccg 5340
   gttctttttg tcaagaccga cctgtccggt gccctgaatg aactgcagga cgaggcagcg 5400
   cggctatcgt ggctggccac gacgggcgtt ccttgcgcag ctgtgctcga cgttgtcact 5460
   gaagcgggaa gggactggct gctattgggc gaagtgccgg ggcaggatct cctgtcatct 5520
   caccttgctc ctgccgagaa agtatccatc atggctgatg caatgcggcg gctgcatacg 5580
   cttgatccgg ctacctgccc attcgaccac caagcgaaac atcgcatcga gcgagcacgt 5640
   actcggatgg aagccggtct tgtcgatcag gatgatctgg acgaagagca tcaggggctc 5700
   gcgccagccg aactgttcgc caggctcaag gcgcgcatgc ccgacggcga ggatctcgtc 5760
   gtgacccatg gcgatgcctg cttgccgaat atcatggtgg aaaatggccg cttttctgga 5820
   ttcatcgact gtggccggct gggtgtggcg gaccgctatc aggacatagc gttggctacc 5880
   cgtgatattg ctgaagagct tggcggcgaa tgggctgacc gcttcctcgt gctttacggt 5940
   atcgccgctc ccgattcgca gcgcatcgcc ttctatcgcc ttcttgacga gttcttctga 6000
   gcgggactct ggggttcgaa atgaccgacc aagcgacgcc caacctgcca tcacgagatt 6060
   tcgattccac cgccgccttc tatgaaaggt tgggcttcgg aatcgttttc cgggacgccg 6120
   gctggatgat cctccagcgc ggggatctca tgctggagtt cttcgcccac gctagtttaa 6180
   actgcggatc agtgagggtt tgtaactgcg ggtcaaggat ctggatttcg atcacggcac 6240
   gatcatcgtg cgggagggca agggctccaa ggatcgggcc ttgatgttac ccgagagctt 6300
   ggcacccagc ctgcgcgagc aggggaattg atccggtgga tgaccttttg aatgaccttt 6360
   aatagattat attactaatt aattggggac cctagaggtc ccctttttta ttttaaaaat 6420
   tttttcacaa aacggtttac aagcataacg ggttttgctg cccgcaaacg ggctgttctg 6480
   gtgttgctag tttgttatca gaatcgcaga tccggcttca ggtttgccgg ctgaaagcgc 6540
   tatttcttcc agaattgcca tgattttttc cccacgggag gcgtcactgg ctcccgtgtt 6600
   gtcggcagct ttgattcgat aagcagcatc gcctgtttca ggctgtctat gtgtgactgt 6660
   tgagctgtaa caagttgtct caggtgttca atttcatgtt ctagttgctt tgttttactg 6720
   gtttcacctg ttctattagg tgttacatgc tgttcatctg ttacattgtc gatctgttca 6780
   tggtgaacag ctttaaatgc accaaaaact cgtaaaagct ctgatgtatc tatctttttt 6840
   acaccgtttt catctgtgca tatggacagt tttccctttg atatctaacg gtgaacagtt 6900
   gttctacttt tgtttgttag tcttgatgct tcactgatag atacaagagc cataagaacc 6960
   tcagatcctt ccgtatttag ccagtatgtt ctctagtgtg gttcgttgtt tttgcgtgag 7020
   ccatgagaac gaaccattga gatcatgctt actttgcatg tcactcaaaa attttgcctc 7080
   aaaactggtg agctgaattt ttgcagttaa agcatcgtgt agtgtttttc ttagtccgtt 7140
   acgtaggtag gaatctgatg taatggttgt tggtattttg tcaccattca tttttatctg 7200
   gttgttctca agttcggtta cgagatccat ttgtctatct agttcaactt ggaaaatcaa 7260
   cgtatcagtc gggcggcctc gcttatcaac caccaatttc atattgctgt aagtgtttaa 7320
   atctttactt attggtttca aaacccattg gttaagcctt ttaaactcat ggtagttatt 7380
   ttcaagcatt aacatgaact taaattcatc aaggctaatc tctatatttg ccttgtgagt 7440
   tttcttttgt gttagttctt ttaataacca ctcataaatc ctcatagagt atttgttttc 7500
   aaaagactta acatgttcca gattatattt tatgaatttt tttaactgga aaagataagg 7560
   caatatctct tcactaaaaa ctaattctaa tttttcgctt gagaacttgg catagtttgt 7620
   ccactggaaa atctcaaagc ctttaaccaa aggattcctg atttccacag ttctcgtcat 7680
   cagctctctg gttgctttag ctaatacacc ataagcattt tccctactga tgttcatcat 7740
   ctgagcgtat tggttataag tgaacgatac cgtccgttct ttccttgtag ggttttcaat 7800
   cgtggggttg agtagtgcca cacagcataa aattagcttg gtttcatgct ccgttaagtc 7860
   atagcgacta atcgctagtt catttgcttt gaaaacaact aattcagaca tacatctcaa 7920
   ttggtctagg tgattttaat cactatacca attgagatgg gctagtcaat gataattact 7980
   agtccttttc ctttgagttg tgggtatctg taaattctgc tagacctttg ctggaaaact 8040
   tgtaaattct gctagaccct ctgtaaattc cgctagacct ttgtgtgttt tttttgttta 8100
   tattcaagtg gttataattt atagaataaa gaaagaataa aaaaagataa aaagaataga 8160
   tcccagccct gtgtataact cactacttta gtcagttccg cagtattaca aaaggatgtc 8220
   gcaaacgctg tttgctcctc tacaaaacag accttaaaac cctaaaggct taagtagcac 8280
   cctcgcaagc tcgggcaaat cgctgaatat tccttttgtc tccgaccatc aggcacctga 8340
   gtcgctgtct ttttcgtgac attcagttcg ctgcgctcac ggctctggca gtgaatgggg 8400
   gtaaatggca ctacaggcgc cttttatgga ttcatgcaag gaaactaccc ataatacaag 8460
   aaaagcccgt cacgggcttc tcagggcgtt ttatggcggg tctgctatgt ggtgctatct 8520
   gactttttgc tgttcagcag ttcctgccct ctgattttcc agtctgacca cttcggatta 8580
   tcccgtgaca ggtcattcag actggctaat gcacccagta aggcagcggt atcatcaaca 8640
   ggcttagttt aaaccgcaaa gtcccgcttc gtgaaaattt tcgtgccgcg tgattttccg 8700
   ccaaaaactt taacgaacgt tcgttataat ggtgtcatga ccttcacgac gaagtactaa 8760
   aattggcccg aatcatcagc tatggatctc tctgatgtcg cgctggagtc cgacgcgctc 8820
   gatgctgccg tcgatttaaa aacggtgatc ggatttttcc gagctctcga tacgacggac 8880
   gcgccagcat cacgagactg ggccagtgcc gcgagcgacc tagaaactct cgtggcggat 8940
   cttgaggagc tggctgacga gctgcgtgct cggccagcgc caggaggacg cacagtagtg 9000
   gaggatgcaa tcagttgcgc ctactgcggt ggcctgattc ctccccggcc tgacccgcga 9060
   ggacggcgcg caaaatattg ctcagatgcg tgtcgtgccg cagccagccg cgagcgcgcc 9120
   aacaaacgcc acgccgagga gctggaggcg gctaggtcgc aaatggcgct ggaagtgcgt 9180
   cccccgagcg aaattttggc catggtcgtc acagagctgg aagcggcagc gagaattatc 9240
   gcgatcgtgg cggtgcccgc aggcatgaca aacatcgtaa atgccgcgtt tcgtgtgccg 9300
   tggccgccca ggacgtgtca gcgccgccac cacctgcacc gaatcggcag cagcgtcgcg 9360
   cgtcgaaaaa gcgcacaggc ggcaagaagc gataagctgc acgaatacct gaaaaatgtt 9420
   gaacgccccg tgagcggtaa ctcacagggc gtcggctaac ccccagtcca aacctgggag 9480
   aaagcgctca aaaatgactc tagcggattc acgagacatt gacacaccgg cctggaaatt 9540
   ttccgctgat ctgttcgaca cccatcccga gctcgcgctg cgatcacgtg gctggacgag 9600
   cgaagaccgc cgcgaattcc tcgctcacct gggcagagaa aatttccagg gcagcaagac 9660
   ccgcgacttc gccagcgctt ggatcaaaga cccggacacg gagaaacaca gccgaagtta 9720
   taccgagttg gttcaaaatc gcttgcccgg tgccagtatg ttgctctgac gcacgcgcag 9780
   cacgcagccg tgcttgtcct ggacattgat gtgccgagcc accaggccgg cgggaaaatc 9840
   gagcacgtaa accccgaggt ctacgcgatt ttggagcgct gggcacgcct ggaaaaagcg 9900
   ccagcttgga tcggcgtgaa tccactgagc gggaaatgcc agctcatctg gctcattgat 9960
   ccggtgtatg ccgcagcagg catgagcagc ccgaatatgc gcctgctggc tgcaacgacc 10020
   gaggaaatga cccgcgtttt cggcgctgac caggcttttt cacataggct gagccgtggc 10080
   cactgcactc tccgacgatc ccagccgtac cgctggcatg cccagcacaa tcgcgtggat 10140
   cgcctagctg atcttatgga ggttgctcgc atgatctcag gcacagaaaa acctaaaaaa 10200
   cgctatgagc aggagttttc tagcggacgg gcacgtatcg aagcggcaag aaaagccact 10260
   gcggaagcaa aagcacttgc cacgcttgaa gcaagcctgc cgagcgccgc tgaagcgtct 10320
   ggagagctga tcgacggcgt ccgtgtcctc tggactgctc cagggcgtgc cgcccgtgat 10380
   gagacggctt ttcgccacgc tttgactgtg ggataccagt taaaagcggc tggtgagcgc 10440
   ctaaaagaca ccaagggtca tcgagcctac gagcgtgcct acaccgtcgc tcaggcggtc 10500
   ggaggaggcc gtgagcctga tctgccgccg gactgtgacc gccagacgga ttggccgcga 10560
   cgtgtgcgcg gctacgtcgc taaaggccag ccagtcgtcc ctgctcgtca gacagagacg 10620
   cagagccagc cgaggcgaaa agctctggcc actatgggaa gacgtggcgg taaaaaggcc 10680
   gcagaacgct ggaaagaccc aaacagtgag tacgcccgag cacagcgaga aaaactagct 10740
   aagtccagtc aacgacaagc taggaaagct aaaggaaatc gcttgaccat tgcaggttgg 10800
   tttatgactg ttgagggaga gactggctcg tggccgacaa tcaatgaagc tatgtctgaa 10860
   tttagcgtgt cacgtcagac cgtgaataga gcacttaagg tctgcgggca ttgaacttcc 10920
   acgaggacgc cgaaagcttc ccagtaaatg tgccatctcg taggcagaaa acggttcccc 10980
   cgtagggtct ctctcttggc ctcctttcta ggtcgggctg attgctcttg aagctctcta 11040
   ggggggctca caccataggc agataacgtt ccccaccggc tcgcctcgta agcgcacaag 11100
   gactgctccc aaagatcttc aaagccactg ccgcgactgc cttcgcgaag ccttgccccg 11160
   cggaaatttc ctccaccgag ttcgtgcaca cccctatgcc aagcttcttt caccctaaat 11220
   tcgagagatt ggattcttac cgtggaaatt cttcgcaaaa atcgtcccct gatcgccctt 11280
   gcgacgttgg cgtcggtgcc gctggttgcg cttggcttga ccgac 11325
<210> 42
   <211> 200
   <212> DNA
   <213> artificial
<220>
   <223> P15 Promoter
<400> 42
   tcgaggctat tgacgacagc tatggttcac tgtccaccaa ccaaaactgt gctcagtacc 60
   gccaatattt ctcccttgag gggtacaaag aggtgtccct agaagagatc cacgctgtgt 120
   aaaaatttta caaaaaggta ttgactttcc ctacagggtg tgtaataatt taattacagg 180
   cgggggcaac cccgcctgtt 200
<210> 43
   <211> 5666
   <212> DNA
   <213> artificial
<220>
   <223> Plasmid pHF96
<400> 43
   tcgagaggcc tgacgtcggg cccggtacca cgcgtctttc gaccaaacca cactgcccac 60
   gaatcctaga aatgggatcc gtgggcattc gtgtctggtt taacggcctt gaaaccggac 120
   aggattgccc aaagtagtcg aatttccagt tcgtgggcat gtgtgtttgg tcaggtttag 180
   tcgggtcgaa aaatgaactc ggaatcggta aaaagtgacc tcacagaatc gcttctaagg 240
   gccgttcaaa gtgttgccta tacaaacaca tgttctgact gcccgacctc ttaaaatggc 300
   gttaaatggc gcgaaatgga aaacgcctcg tggcctggat ggagcgttag tacgagacca 360
   gtacgagacc agacacacgt gacaaaaaat cctgaaaagt ggaatcattg tcacgcctgt 420
   ctggtttagc tctggttcgg gacgggcgtg gaatggaggc agtgcaccga gaccttgacc 480
   cgcggcccga caagccaaaa gtccccaaaa caaacccacc ccgccggaga cgtgaataaa 540
   attcgcagct cattccatca gcgtaaacgc agctttttgc atggtgagac acctttgggg 600
   gtaaatctca cagcatgaat ctctgggtta gatgactttc tgggtggggg agggtttaga 660
   atgtttctag tcgcacgcca aaacccggcg tggacacgtc tgcagccgac gcggtcgtgc 720
   ctgttgtaga cggacattcc tagtttttcc aggagtaact tgatatctta gagatccatt 780
   tgcttgaacc gccttcccat ctttgaattc attcaaggtg gtaaggcggt tttcgctctt 840
   ttaatacagt tttaaaggta gatttgggag agaagatttc ccttaagaaa ggttcttaac 900
   aaccatgccg cctgcgacgc tgttcaatgt tttgacttca gctggacttg accctcacca 960
   gtcaggtgat gccattgttg tcgagtctgc ccatttcaca ttgacgttca cgtgggatga 1020
   gtggctgcga gctcaagcga cgtgggtggg ggagttgagt gcgtcggatt atgtgcgttc 1080
   tattgtggcg attaactctg cccatgatgc acgggcaacg ccgaagatga tgttggatgc 1140
   cccgactggt ctgacaacgg tgcttaaggc ggataagggt cagttgcagg cgtttgccgt 1200
   ggaggcgctg ccgattggcg atggcctcag cgaggctcag ttggcggggt ttgtggctgc 1260
   cgcgtttgat ggcgccatcg acctcactcg tgagtttcat gcactttacc cggagcgctc 1320
   gccgcaggag cgcggcgcaa tgctcaacat taagcttgtc gacgcctccc cctctcaaac 1380
   agttacgccc gtgcgagtag ctaactggtt catggatcag ggggtggagg aagttcctta 1440
   tgatgcagct tctagacccg ggatttaaat cgctagcggg ctgctaaagg aagcggaaca 1500
   cgtagaaagc cagtccgcag aaacggtgct gaccccggat gaatgtcagc tactgggcta 1560
   tctggacaag ggaaaacgca agcgcaaaga gaaagcaggt agcttgcagt gggcttacat 1620
   ggcgatagct agactgggcg gttttatgga cagcaagcga accggaattg ccagctgggg 1680
   cgccctctgg taaggttggg aagccctgca aagtaaactg gatggctttc ttgccgccaa 1740
   ggatctgatg gcgcagggga tcaagatctg atcaagagac aggatgagga tcgtttcgca 1800
   tgattgaaca agatggattg cacgcaggtt ctccggccgc ttgggtggag aggctattcg 1860
   gctatgactg ggcacaacag acaatcggct gctctgatgc cgccgtgttc cggctgtcag 1920
   cgcaggggcg cccggttctt tttgtcaaga ccgacctgtc cggtgccctg aatgaactgc 1980
   aggacgaggc agcgcggcta tcgtggctgg ccacgacggg cgttccttgc gcagctgtgc 2040
   tcgacgttgt cactgaagcg ggaagggact ggctgctatt gggcgaagtg ccggggcagg 2100
   atctcctgtc atctcacctt gctcctgccg agaaagtatc catcatggct gatgcaatgc 2160
   ggcggctgca tacgcttgat ccggctacct gcccattcga ccaccaagcg aaacatcgca 2220
   tcgagcgagc acgtactcgg atggaagccg gtcttgtcga tcaggatgat ctggacgaag 2280
   agcatcaggg gctcgcgcca gccgaactgt tcgccaggct caaggcgcgc atgcccgacg 2340
   gcgaggatct cgtcgtgacc catggcgatg cctgcttgcc gaatatcatg gtggaaaatg 2400
   gccgcttttc tggattcatc gactgtggcc ggctgggtgt ggcggaccgc tatcaggaca 2460
   tagcgttggc tacccgtgat attgctgaag agcttggcgg cgaatgggct gaccgcttcc 2520
   tcgtgcttta cggtatcgcc gctcccgatt cgcagcgcat cgccttctat cgccttcttg 2580
   acgagttctt ctgagcggga ctctggggtt cgaaatgacc gaccaagcga cgcccaacct 2640
   gccatcacga gatttcgatt ccaccgccgc cttctatgaa aggttgggct tcggaatcgt 2700
   tttccgggac gccggctgga tgatcctcca gcgcggggat ctcatgctgg agttcttcgc 2760
   ccacgctagc ggcgcgccgg ccggcccggt gtgaaatacc gcacagatgc gtaaggagaa 2820
   aataccgcat caggcgctct tccgcttcct cgctcactga ctcgctgcgc tcggtcgttc 2880
   ggctgcggcg agcggtatca gctcactcaa aggcggtaat acggttatcc acagaatcag 2940
   gggataacgc aggaaagaac atgtgagcaa aaggccagca aaaggccagg aaccgtaaaa 3000
   aggccgcgtt gctggcgttt ttccataggc tccgcccccc tgacgagcat cacaaaaatc 3060
   gacgctcaag tcagaggtgg cgaaacccga caggactata aagataccag gcgtttcccc 3120
   ctggaagctc cctcgtgcgc tctcctgttc cgaccctgcc gcttaccgga tacctgtccg 3180
   cctttctccc ttcgggaagc gtggcgcttt ctcatagctc acgctgtagg tatctcagtt 3240
   cggtgtaggt cgttcgctcc aagctgggct gtgtgcacga accccccgtt cagcccgacc 3300
   gctgcgcctt atccggtaac tatcgtcttg agtccaaccc ggtaagacac gacttatcgc 3360
   cactggcagc agccactggt aacaggatta gcagagcgag gtatgtaggc ggtgctacag 3420
   agttcttgaa gtggtggcct aactacggct acactagaag gacagtattt ggtatctgcg 3480
   ctctgctgaa gccagttacc ttcggaaaaa gagttggtag ctcttgatcc ggcaaacaaa 3540
   ccaccgctgg tagcggtggt ttttttgttt gcaagcagca gattacgcgc agaaaaaaag 3600
   gatctcaaga agatcctttg atcttttcta cggggtctga cgctcagtgg aacgaaaact 3660
   cacgttaagg gattttggtc atgagattat caaaaaggat cttcacctag atccttttaa 3720
   aggccggccg cggccgccat cggcattttc ttttgcgttt ttatttgtta actgttaatt 3780
   gtccttgttc aaggatgctg tctttgacaa cagatgtttt cttgcctttg atgttcagca 3840
   ggaagctcgg cgcaaacgtt gattgtttgt ctgcgtagaa tcctctgttt gtcatatagc 3900
   ttgtaatcac gacattgttt cctttcgctt gaggtacagc gaagtgtgag taagtaaagg 3960
   ttacatcgtt aggatcaaga tccattttta acacaaggcc agttttgttc agcggcttgt 4020
   atgggccagt taaagaatta gaaacataac caagcatgta aatatcgtta gacgtaatgc 4080
   cgtcaatcgt catttttgat ccgcgggagt cagtgaacag gtaccatttg ccgttcattt 4140
   taaagacgtt cgcgcgttca atttcatctg ttactgtgtt agatgcaatc agcggtttca 4200
   tcactttttt cagtgtgtaa tcatcgttta gctcaatcat accgagagcg ccgtttgcta 4260
   actcagccgt gcgtttttta tcgctttgca gaagtttttg actttcttga cggaagaatg 4320
   atgtgctttt gccatagtat gctttgttaa ataaagattc ttcgccttgg tagccatctt 4380
   cagttccagt gtttgcttca aatactaagt atttgtggcc tttatcttct acgtagtgag 4440
   gatctctcag cgtatggttg tcgcctgagc tgtagttgcc ttcatcgatg aactgctgta 4500
   cattttgata cgtttttccg tcaccgtcaa agattgattt ataatcctct acaccgttga 4560
   tgttcaaaga gctgtctgat gctgatacgt taacttgtgc agttgtcagt gtttgtttgc 4620
   cgtaatgttt accggagaaa tcagtgtaga ataaacggat ttttccgtca gatgtaaatg 4680
   tggctgaacc tgaccattct tgtgtttggt cttttaggat agaatcattt gcatcgaatt 4740
   tgtcgctgtc tttaaagacg cggccagcgt ttttccagct gtcaatagaa gtttcgccga 4800
   ctttttgata gaacatgtaa atcgatgtgt catccgcatt tttaggatct ccggctaatg 4860
   caaagacgat gtggtagccg tgatagtttg cgacagtgcc gtcagcgttt tgtaatggcc 4920
   agctgtccca aacgtccagg ccttttgcag aagagatatt tttaattgtg gacgaatcaa 4980
   attcagaaac ttgatatttt tcattttttt gctgttcagg gatttgcagc atatcatggc 5040
   gtgtaatatg ggaaatgccg tatgtttcct tatatggctt ttggttcgtt tctttcgcaa 5100
   acgcttgagt tgcgcctcct gccagcagtg cggtagtaaa ggttaatact gttgcttgtt 5160
   ttgcaaactt tttgatgttc atcgttcatg tctccttttt tatgtactgt gttagcggtc 5220
   tgcttcttcc agccctcctg tttgaagatg gcaagttagt tacgcacaat aaaaaaagac 5280
   ctaaaatatg taaggggtga cgccaaagta tacactttgc cctttacaca ttttaggtct 5340
   tgcctgcttt atcagtaaca aacccgcgcg atttactttt cgacctcatt ctattagact 5400
   ctcgtttgga ttgcaactgg tctattttcc tcttttgttt gatagaaaat cataaaagga 5460
   tttgcagact acgggcctaa agaactaaaa aatctatctg tttcttttca ttctctgtat 5520
   tttttatagt ttctgttgca tgggcataaa gttgcctttt taatcacaat tcagaaaata 5580
   tcataatatc tcatttcact aaataatagt gaacggcagg tatatgtgat gggttaaaaa 5640
   ggatcggcgg ccgctcgatt taaatc 5666
<210> 44
   <211> 40
   <212> DNA
   <213> artificial
<220>
   <223> Plasmid HS1304
<400> 44
   tacgaaagga ttttttaccc atgactaact cttctgcaac 40
<210> 45
   <211> 32
   <212> DNA
   <213> artificial
<220>
   <223> HS1305
<400> 45
   agcgcgtcta gattagaaca gccccgagat aa 32
<210> 46
   <211> 32
   <212> DNA
   <213> artificial
<220>
   <223> HS1302
<400> 46
   gctagaacgc gtgagctgcc aattattccg gg 32
<210> 47
   <211> 40
   <212> DNA
   <213> artificial
<220>
   <223> HS1303
<400> 47
   gttgcagaag agttagtcat gggtaaaaaa tcctttcgta 40
<210> 48
   <211> 6965
   <212> DNA
   <213> artificial
<220>
   <223> Plasmid pH655
<400> 48
   ctagagttct gtgaaaaaca ccgtggggca gtttctgctt cgcggtgttt tttatttgtg      60
   gggcactaga cccgggattt aaatcgctag cgggctgcta aaggaagcgg aacacgtaga     120
   aagccagtcc gcagaaacgg tgctgacccc ggatgaatgt cagctactgg gctatctgga     180
   caagggaaaa cgcaagcgca aagagaaagc aggtagcttg cagtgggctt acatggcgat     240
   agctagactg ggcggtttta tggacagcaa gcgaaccgga attgccagct ggggcgccct     300
   ctggtaaggt tgggaagccc tgcaaagtaa actggatggc tttcttgccg ccaaggatct     360
   gatggcgcag gggatcaaga tctgatcaag agacaggatg aggatcgttt cgcatgattg     420
   aacaagatgg attgcacgca ggttctccgg ccgcttgggt ggagaggcta ttcggctatg     480
   actgggcaca acagacaatc ggctgctctg atgccgccgt gttccggctg tcagcgcagg     540
   ggcgcccggt tctttttgtc aagaccgacc tgtccggtgc cctgaatgaa ctgcaggacg     600
   aggcagcgcg gctatcgtgg ctggccacga cgggcgttcc ttgcgcagct gtgctcgacg     660
   ttgtcactga agcgggaagg gactggctgc tattgggcga agtgccgggg caggatctcc     720
   tgtcatctca ccttgctcct gccgagaaag tatccatcat ggctgatgca atgcggcggc     780
   tgcatacgct tgatccggct acctgcccat tcgaccacca agcgaaacat cgcatcgagc     840
   gagcacgtac tcggatggaa gccggtcttg tcgatcagga tgatctggac gaagagcatc 900
   aggggctcgc gccagccgaa ctgttcgcca ggctcaaggc gcgcatgccc gacggcgagg 960
   atctcgtcgt gacccatggc gatgcctgct tgccgaatat catggtggaa aatggccgct 1020
   tttctggatt catcgactgt ggccggctgg gtgtggcgga ccgctatcag gacatagcgt 1080
   tggctacccg tgatattgct gaagagcttg gcggcgaatg ggctgaccgc ttcctcgtgc 1140
   tttacggtat cgccgctccc gattcgcagc gcatcgcctt ctatcgcctt cttgacgagt 1200
   tcttctgagc gggactctgg ggttcgaaat gaccgaccaa gcgacgccca acctgccatc 1260
   acgagatttc gattccaccg ccgccttcta tgaaaggttg ggcttcggaa tcgttttccg 1320
   ggacgccggc tggatgatcc tccagcgcgg ggatctcatg ctggagttct tcgcccacgc 1380
   tagcggcgcg ccggccggcc cggtgtgaaa taccgcacag atgcgtaagg agaaaatacc 1440
   gcatcaggcg ctcttccgct tcctcgctca ctgactcgct gcgctcggtc gttcggctgc 1500
   ggcgagcggt atcagctcac tcaaaggcgg taatacggtt atccacagaa tcaggggata 1560
   acgcaggaaa gaacatgtga gcaaaaggcc agcaaaaggc caggaaccgt aaaaaggccg 1620
   cgttgctggc gtttttccat aggctccgcc cccctgacga gcatcacaaa aatcgacgct 1680
   caagtcagag gtggcgaaac ccgacaggac tataaagata ccaggcgttt ccccctggaa 1740
   gctccctcgt gcgctctcct gttccgaccc tgccgcttac cggatacctg tccgcctttc 1800
   tcccttcggg aagcgtggcg ctttctcata gctcacgctg taggtatctc agttcggtgt 1860
   aggtcgttcg ctccaagctg ggctgtgtgc acgaaccccc cgttcagccc gaccgctgcg 1920
   ccttatccgg taactatcgt cttgagtcca acccggtaag acacgactta tcgccactgg 1980
   cagcagccac tggtaacagg attagcagag cgaggtatgt aggcggtgct acagagttct 2040
   tgaagtggtg gcctaactac ggctacacta gaaggacagt atttggtatc tgcgctctgc 2100
   tgaagccagt taccttcgga aaaagagttg gtagctcttg atccggcaaa caaaccaccg 2160
   ctggtagcgg tggttttttt gtttgcaagc agcagattac gcgcagaaaa aaaggatctc 2220
   aagaagatcc tttgatcttt tctacggggt ctgacgctca gtggaacgaa aactcacgtt 2280
   aagggatttt ggtcatgaga ttatcaaaaa ggatcttcac ctagatcctt ttaaaggccg 2340
   gccgcggccg cgcaaagtcc cgcttcgtga aaattttcgt gccgcgtgat tttccgccaa 2400
   aaactttaac gaacgttcgt tataatggtg tcatgacctt cacgacgaag tactaaaatt 2460
   ggcccgaatc atcagctatg gatctctctg atgtcgcgct ggagtccgac gcgctcgatg 2520
   ctgccgtcga tttaaaaacg gtgatcggat ttttccgagc tctcgatacg acggacgcgc 2580
   cagcatcacg agactgggcc agtgccgcga gcgacctaga aactctcgtg gcggatcttg 2640
   aggagctggc tgacgagctg cgtgctcggc cagcgccagg aggacgcaca gtagtggagg 2700
   atgcaatcag ttgcgcctac tgcggtggcc tgattcctcc ccggcctgac ccgcgaggac 2760
   ggcgcgcaaa atattgctca gatgcgtgtc gtgccgcagc cagccgcgag cgcgccaaca 2820
   aacgccacgc cgaggagctg gaggcggcta ggtcgcaaat ggcgctggaa gtgcgtcccc 2880
   cgagcgaaat tttggccatg gtcgtcacag agctggaagc ggcagcgaga attatcgcga 2940
   tcgtggcggt gcccgcaggc atgacaaaca tcgtaaatgc cgcgtttcgt gtgccgtggc 3000
   cgcccaggac gtgtcagcgc cgccaccacc tgcaccgaat cggcagcagc gtcgcgcgtc 3060
   gaaaaagcgc acaggcggca agaagcgata agctgcacga atacctgaaa aatgttgaac 3120
   gccccgtgag cggtaactca cagggcgtcg gctaaccccc agtccaaacc tgggagaaag 3180
   cgctcaaaaa tgactctagc ggattcacga gacattgaca caccggcctg gaaattttcc 3240
   gctgatctgt tcgacaccca tcccgagctc gcgctgcgat cacgtggctg gacgagcgaa 3300
   gaccgccgcg aattcctcgc tcacctgggc agagaaaatt tccagggcag caagacccgc 3360
   gacttcgcca gcgcttggat caaagacccg gacacggaga aacacagccg aagttatacc 3420
   gagttggttc aaaatcgctt gcccggtgcc agtatgttgc tctgacgcac gcgcagcacg 3480
   cagccgtgct tgtcctggac attgatgtgc cgagccacca ggccggcggg aaaatcgagc 3540
   acgtaaaccc cgaggtctac gcgattttgg agcgctgggc acgcctggaa aaagcgccag 3600
   cttggatcgg cgtgaatcca ctgagcggga aatgccagct catctggctc attgatccgg 3660
   tgtatgccgc agcaggcatg agcagcccga atatgcgcct gctggctgca acgaccgagg 3720
   aaatgacccg cgttttcggc gctgaccagg ctttttcaca taggctgagc cgtggccact 3780
   gcactctccg acgatcccag ccgtaccgct ggcatgccca gcacaatcgc gtggatcgcc 3840
   tagctgatct tatggaggtt gctcgcatga tctcaggcac agaaaaacct aaaaaacgct 3900
   atgagcagga gttttctagc ggacgggcac gtatcgaagc ggcaagaaaa gccactgcgg 3960
   aagcaaaagc acttgccacg cttgaagcaa gcctgccgag cgccgctgaa gcgtctggag 4020
   agctgatcga cggcgtccgt gtcctctgga ctgctccagg gcgtgccgcc cgtgatgaga 4080
   cggcttttcg ccacgctttg actgtgggat accagttaaa agcggctggt gagcgcctaa 4140
   aagacaccaa gggtcatcga gcctacgagc gtgcctacac cgtcgctcag gcggtcggag 4200
   gaggccgtga gcctgatctg ccgccggact gtgaccgcca gacggattgg ccgcgacgtg 4260
   tgcgcggcta cgtcgctaaa ggccagccag tcgtccctgc tcgtcagaca gagacgcaga 4320
   gccagccgag gcgaaaagct ctggccacta tgggaagacg tggcggtaaa aaggccgcag 4380
   aacgctggaa agacccaaac agtgagtacg cccgagcaca gcgagaaaaa ctagctaagt 4440
   ccagtcaacg acaagctagg aaagctaaag gaaatcgctt gaccattgca ggttggttta 4500
   tgactgttga gggagagact ggctcgtggc cgacaatcaa tgaagctatg tctgaattta 4560
   gcgtgtcacg tcagaccgtg aatagagcac ttaaggtctg cgggcattga acttccacga 4620
   ggacgccgaa agcttcccag taaatgtgcc atctcgtagg cagaaaacgg ttcccccgta 4680
   gggtctctct cttggcctcc tttctaggtc gggctgattg ctcttgaagc tctctagggg 4740
   ggctcacacc ataggcagat aacgttcccc accggctcgc ctcgtaagcg cacaaggact 4800
   gctcccaaag atcttcaaag ccactgccgc gactgccttc gcgaagcctt gccccgcgga 4860
   aatttcctcc accgagttcg tgcacacccc tatgccaagc ttctttcacc ctaaattcga 4920
   gagattggat tcttaccgtg gaaattcttc gcaaaaatcg tcccctgatc gcccttgcga 4980
   cgttggcgtc ggtgccgctg gttgcgcttg gcttgaccga cttgatcagc ggccgctcga 5040
   tttaaatctc gagaggcctg acgtcgggcc cggtaccacg cgtgagctgc caattattcc 5100
   gggcttgtga cccgctaccc gataaatagg tcggctgaaa aatttcgttg caatatcaac 5160
   aaaaaggcct atcattggga ggtgtcgcac caagtacttt tgcgaagcgc catctgacgg 5220
   attttcaaaa gatgtatatg ctcggtgcgg aaacctacga aaggattttt tacccatgac 5280
   taactcttct gcaaccagca atccccaacc cagcgacgtc gaaatcgcac aggcccacac 5340
   cctggagccc atcaccacga tcgcggagcg cgccggcatc ccggaggccg ctttgattcc 5400
   ctacggccgt acaaaggcca agatcgacgt tcccgctctg cgggcggagc gtgagggcgt 5460
   caataaaaag ggcaagctcg tgctcgtgac cgcaatgagc ccgacccccg cgggtgaagg 5520
   caagtccacg gtgctgatcg gcttggcgga cgcggtgcgc acggcagggc gccagacgat 5580
   ggtggcgatc cgcgagccgt cccaggggcc agtcatgggc atcaagggcg gcgctgctgg 5640
   cggcggttat gcgcagatcg tgccgatgga agacatcaac ctgcacttca caggcgacat 5700
   gcacgccatc accgccgcga cgaacactct ggcggcgatg gtggacaacc acgtgcagca 5760
   cggcaatgct ctgggcatcg acccgcggcg ggtgacgtgg cgacgctgcc ttgacgtcaa 5820
   cgaccgctcg ctgcgccatg tagtcacagg cctaggcggt cctggtcagg gcacgccccg 5880
   cgagggcgga ttcgatatca cggcggccag cgagatcatg gctatcctct gtctggccac 5940
   cgacctggag gatctaaaga agcgcatcgg gcgcatcgtg gtgggccaga cctacgaccg 6000
   tcgaccagtc accgcgggcg acctgaagtg cgcgggcgct atcaccgccc tgctgcgcga 6060
   cgcgatcaac ccgaacctgg tgcagaccct gggcggcact cccgccctgg tgcacggcgg 6120
   gcctttcgcc aacatcgcgc acggctgtaa ctctctgatc gcaaccacca cggcgctgga 6180
   tctatcagaa gttgtgctga ccgaggctgg tttcggcagc gacctgggtg cggagaagtt 6240
   cttcgacatc aagtcccgcg ctggcgacct cgacgtcgcc gcaacggttg tggtggccac 6300
   catccgttcg ctgaagcaca acggcgattc cgtgttgaag gccggtctgg ccaacctgga 6360
   gcgccacgtc agtaacatcc gcaagttcgg cgtggaaccc gtggtggcgc tgaacctgtt 6420
   tagctccgat accgccgcgg agcgctctat ggtcgccgat tggggcgagc agttcggtgt 6480
   gcgcgtggtg gagtgcagcg tgtgggctga gggcggtgcc ggcgcggcgg atctggccac 6540
   tgcggtgctg gaggtcgttg atggtgtttc tgatgaggac gcctccagct ccagccacca 6600
   gatctatcag cccgtcgatg gcgtggaagc caccctgcac acacttgcca cggaaatcta 6660
   cggcgcggcg gatgtgcagt tcggcccgca ggctctgaag gacctggcgt ttctgaagga   6720
   caacggctgg gacaagctgc cagtgtgcgt gtcgaagaca cagtactcct tcagcgatga   6780
   ccccagcgcg ctgggcgcgc cgagcggcca caccctgcat gtccgcgagc tggtgccacg   6840
   catcggcgcc ggattcgtgg tggctctcac cggcgacgtg atgactctgc cgggtctgcc   6900
   gaagaaaccc gcagccgagc gtattgatgt gaacgcgcag ggggttatct cggggctgtt    6960
   ctaat                                                               6965
<210> 49
   <211> 6132
   <212> DNA
   <213> artificial
<220>
   <223> Plasmid pH657
<400> 49
   cgcgtgagct gccaattatt ccgggcttgt gacccgctac ccgataaata ggtcggctga 60
   aaaatttcgt tgcaatatca acaaaaaggc ctatcattgg gaggtgtcgc accaagtact 120
   tttgcgaagc gccatctgac ggattttcaa aagatgtata tgctcggtgc ggaaacctac 180
   gaaaggattt tttacccatg actaactctt ctgcaaccag caatccccaa cccagcgacg 240
   tcgaaatcgc acaggcccac tccctggagc ccatcaccac gatcgcggag cgcgccggca 300
   tcccggaggc cgctttgatt ccctacggcc gcacaaaggc caagatcgac gttcccgctc 360
   tgcgggcgga gcgtgagggc gtcaatagaa agggcaagct cgtgctcgtg accgcaatga 420
   gcccgacccc cgcgggtgaa ggcaagtcca cggtgctgat cggcttggcg gacgcggtgc 480
   gcacggcagg gcgccagacg atggtggcga tccgcgagcc gtcccagggg ccggtcatgg 540
   gcatcaaggg cggcgctgct ggcggcggtt atgcgcagat cgtgccgatg gaagacatta 600
   acctgcactt cacaggcgac atgcacgcca tcaccgccgc gacgaacact ctggcggcga 660
   tggtggacaa ccacgtgcag cacggcaatg ctctgggcat cgacccgcgg cgggtgacgt 720
   ggcggcgctg cctggacgtc aacgaccgct cgctgcgcca tgtggtcaca ggcctaggcg 780
   gccctggtca gggcacgccc cgcgagggcg gattcgatat cacggcggcc agcgagatca 840
   tggctatcct ctgtctggcc accgacctgg aagatctaaa gaagcgcatc gggcgcatcg 900
   tggtgggcca aacttatgat cgcaagccag tcaccgcggg cgacctgaag tgcgcgggcg 960
   ctatcaccgc cctgctgcgc gatgcgatca acccgaacct agtgcagacc ctgggtggca 1020
   cccccgcact ggtgcacggt gggcctttcg ccaacatcgc gcacggatgc aactcgctga 1080
   tcgcaaccac cacggcgctg gacctgtcgg aggttgtgct gaccgaggcc ggcttcggca 1140
   gcgacctggg tgcggagaag ttcttcgaca tcaagtcccg cgccggtggc ctcgacgtcg 1200
   ccgcaacggt cgtggtggcc accatccgct cgctaaagca caacggcgat tccgtactaa 1260
   aggccggttt ggccaacctg gagcgccacg tcagcaacat tcgcaagttc ggcgtggaac 1320
   ccgtggtcgc gctaaacctg tttaactctg acaccgccac cgagcgcgct caggtcgccg 1380
   actggggcga gcagttcggc gtccgcgtgg tggagtgcaa cgtgtgggcc gagggcggtg 1440
   ccggcgcgac cgatctggcc tctgcggtgc tggaggtcgt tgatggtgtt tctgatgagg 1500
   acgcccccag ctccagccac cagatctatc agccggtcga tggtgtggaa gccaccttgc 1560
   gcacacttgc cacggagatc tacggcgcgg cggacgtaca gttcggcccg caggctctga 1620
   aggacctggc gtttctgaag gataacggct gggacaagct gccggtgtgc gtgtcgaaga 1680
   cgcagtactc cttcagcgat gaccctagcg cgctgggcgc gccaagcggc cacaccctgc 1740
   acgtccgcga gctggtacca cgtatcggtg ccggattcgt ggtggcactc accggcgatg 1800
   tgatgactct gccgggcctg ccgaagaaac ccgcggccga gcgcattgat gtgaacgcgc 1860
   agggggttat ctcggggctg ttctaatcta gacccgggat ttaaatcgct agcgggctgc 1920
   taaaggaagc ggaacacgta gaaagccagt ccgcagaaac ggtgctgacc ccggatgaat 1980
   gtcagctact gggctatctg gacaagggaa aacgcaagcg caaagagaaa gcaggtagct 2040
   tgcagtgggc ttacatggcg atagctagac tgggcggttt tatggacagc aagcgaaccg 2100
   gaattgccag ctggggcgcc ctctggtaag gttgggaagc cctgcaaagt aaactggatg 2160
   gctttcttgc cgccaaggat ctgatggcgc aggggatcaa gatctgatca agagacagga 2220
   tgaggatcgt ttcgcatgat tgaacaagat ggattgcacg caggttctcc ggccgcttgg 2280
   gtggagaggc tattcggcta tgactgggca caacagacaa tcggctgctc tgatgccgcc 2340
   gtgttccggc tgtcagcgca ggggcgcccg gttctttttg tcaagaccga cctgtccggt 2400
   gccctgaatg aactgcagga cgaggcagcg cggctatcgt ggctggccac gacgggcgtt 2460
   ccttgcgcag ctgtgctcga cgttgtcact gaagcgggaa gggactggct gctattgggc 2520
   gaagtgccgg ggcaggatct cctgtcatct caccttgctc ctgccgagaa agtatccatc 2580
   atggctgatg caatgcggcg gctgcatacg cttgatccgg ctacctgccc attcgaccac 2640
   caagcgaaac atcgcatcga gcgagcacgt actcggatgg aagccggtct tgtcgatcag 2700
   gatgatctgg acgaagagca tcaggggctc gcgccagccg aactgttcgc caggctcaag 2760
   gcgcgcatgc ccgacggcga ggatctcgtc gtgacccatg gcgatgcctg cttgccgaat 2820
   atcatggtgg aaaatggccg cttttctgga ttcatcgact gtggccggct gggtgtggcg 2880
   gaccgctatc aggacatagc gttggctacc cgtgatattg ctgaagagct tggcggcgaa 2940
   tgggctgacc gcttcctcgt gctttacggt atcgccgctc ccgattcgca gcgcatcgcc 3000
   ttctatcgcc ttcttgacga gttcttctga gcgggactct ggggttcgaa atgaccgacc 3060
   aagcgacgcc caacctgcca tcacgagatt tcgattccac cgccgccttc tatgaaaggt 3120
   tgggcttcgg aatcgttttc cgggacgccg gctggatgat cctccagcgc ggggatctca 3180
   tgctggagtt cttcgcccac gctagcggcg cgccggccgg cccggtgtga aataccgcac 3240
   agatgcgtaa ggagaaaata ccgcatcagg cgctcttccg cttcctcgct cactgactcg 3300
   ctgcgctcgg tcgttcggct gcggcgagcg gtatcagctc actcaaaggc ggtaatacgg 3360
   ttatccacag aatcagggga taacgcagga aagaacatgt gagcaaaagg ccagcaaaag 3420
   gccaggaacc gtaaaaaggc cgcgttgctg gcgtttttcc ataggctccg cccccctgac 3480
   gagcatcaca aaaatcgacg ctcaagtcag aggtggcgaa acccgacagg actataaaga 3540
   taccaggcgt ttccccctgg aagctccctc gtgcgctctc ctgttccgac cctgccgctt 3600
   accggatacc tgtccgcctt tctcccttcg ggaagcgtgg cgctttctca tagctcacgc 3660
   tgtaggtatc tcagttcggt gtaggtcgtt cgctccaagc tgggctgtgt gcacgaaccc 3720
   cccgttcagc ccgaccgctg cgccttatcc ggtaactatc gtcttgagtc caacccggta 3780
   agacacgact tatcgccact ggcagcagcc actggtaaca ggattagcag agcgaggtat 3840
   gtaggcggtg ctacagagtt cttgaagtgg tggcctaact acggctacac tagaaggaca 3900
   gtatttggta tctgcgctct gctgaagcca gttaccttcg gaaaaagagt tggtagctct 3960
   tgatccggca aacaaaccac cgctggtagc ggtggttttt ttgtttgcaa gcagcagatt 4020
   acgcgcagaa aaaaaggatc tcaagaagat cctttgatct tttctacggg gtctgacgct 4080
   cagtggaacg aaaactcacg ttaagggatt ttggtcatga gattatcaaa aaggatcttc 4140
   acctagatcc ttttaaaggc cggccgcggc cgccatcggc attttctttt gcgtttttat 4200
   ttgttaactg ttaattgtcc ttgttcaagg atgctgtctt tgacaacaga tgttttcttg 4260
   cctttgatgt tcagcaggaa gctcggcgca aacgttgatt gtttgtctgc gtagaatcct 4320
   ctgtttgtca tatagcttgt aatcacgaca ttgtttcctt tcgcttgagg tacagcgaag 4380
   tgtgagtaag taaaggttac atcgttagga tcaagatcca tttttaacac aaggccagtt 4440
   ttgttcagcg gcttgtatgg gccagttaaa gaattagaaa cataaccaag catgtaaata 4500
   tcgttagacg taatgccgtc aatcgtcatt tttgatccgc gggagtcagt gaacaggtac 4560
   catttgccgt tcattttaaa gacgttcgcg cgttcaattt catctgttac tgtgttagat 4620
   gcaatcagcg gtttcatcac ttttttcagt gtgtaatcat cgtttagctc aatcataccg 4680
   agagcgccgt ttgctaactc agccgtgcgt tttttatcgc tttgcagaag tttttgactt 4740
   tcttgacgga agaatgatgt gcttttgcca tagtatgctt tgttaaataa agattcttcg 4800
   ccttggtagc catcttcagt tccagtgttt gcttcaaata ctaagtattt gtggccttta 4860
   tcttctacgt agtgaggatc tctcagcgta tggttgtcgc ctgagctgta gttgccttca 4920
   tcgatgaact gctgtacatt ttgatacgtt tttccgtcac cgtcaaagat tgatttataa 4980
   tcctctacac cgttgatgtt caaagagctg tctgatgctg atacgttaac ttgtgcagtt 5040
   gtcagtgttt gtttgccgta atgtttaccg gagaaatcag tgtagaataa acggattttt 5100
   ccgtcagatg taaatgtggc tgaacctgac cattcttgtg tttggtcttt taggatagaa 5160
   tcatttgcat cgaatttgtc gctgtcttta aagacgcggc cagcgttttt ccagctgtca 5220
   atagaagttt cgccgacttt ttgatagaac atgtaaatcg atgtgtcatc cgcattttta 5280
   ggatctccgg ctaatgcaaa gacgatgtgg tagccgtgat agtttgcgac agtgccgtca 5340
   gcgttttgta atggccagct gtcccaaacg tccaggcctt ttgcagaaga gatattttta 5400
   attgtggacg aatcaaattc agaaacttga tatttttcat ttttttgctg ttcagggatt 5460
   tgcagcatat catggcgtgt aatatgggaa atgccgtatg tttccttata tggcttttgg 5520
   ttcgtttctt tcgcaaacgc ttgagttgcg cctcctgcca gcagtgcggt agtaaaggtt 5580
   aatactgttg cttgttttgc aaactttttg atgttcatcg ttcatgtctc cttttttatg 5640
   tactgtgtta gcggtctgct tcttccagcc ctcctgtttg aagatggcaa gttagttacg 5700
   cacaataaaa aaagacctaa aatatgtaag gggtgacgcc aaagtataca ctttgccctt 5760
   tacacatttt aggtcttgcc tgctttatca gtaacaaacc cgcgcgattt acttttcgac 5820
   ctcattctat tagactctcg tttggattgc aactggtcta ttttcctctt ttgtttgata 5880
   gaaaatcata aaaggatttg cagactacgg gcctaaagaa ctaaaaaatc tatctgtttc 5940
   ttttcattct ctgtattttt tatagtttct gttgcatggg cataaagttg cctttttaat 6000
   cacaattcag aaaatatcat aatatctcat ttcactaaat aatagtgaac ggcaggtata 6060
   tgtgatgggt taaaaaggat cggcggccgc tcgatttaaa tctcgagagg cctgacgtcg 6120
   ggcccggtac ca 6132
<210> 50
   <211> 5288
   <212> DNA
   <213> artificial
<220>
   <223> Plasmid pH670 int sacB delta deformylase
<400> 50
   atcgtcttga gtccaacccg gtaagacacg acttatcgcc actggcagca gccactggta 60
   acaggattag cagagcgagg tatgtaggcg gtgctacaga gttcttgaag tggtggccta 120
   actacggcta cactagaagg acagtatttg gtatctgcgc tctgctgaag ccagttacct 180
   tcggaaaaag agttggtagc tcttgatccg gcaaacaaac caccgctggt agcggtggtt 240
   tttttgtttg caagcagcag attacgcgca gaaaaaaagg atctcaagaa gatcctttga 300
   tcttttctac ggggtctgac gctcagtgga acgaaaactc acgttaaggg attttggtca 360
   tgagattatc aaaaaggatc ttcacctaga tccttttaaa ggccggccgc ggccgccatc 420
   ggcattttct tttgcgtttt tatttgttaa ctgttaattg tccttgttca aggatgctgt 480
   ctttgacaac agatgttttc ttgcctttga tgttcagcag gaagctcggc gcaaacgttg 540
   attgtttgtc tgcgtagaat cctctgtttg tcatatagct tgtaatcacg acattgtttc 600
   ctttcgcttg aggtacagcg aagtgtgagt aagtaaaggt tacatcgtta ggatcaagat 660
   ccatttttaa cacaaggcca gttttgttca gcggcttgta tgggccagtt aaagaattag 720
   aaacataacc aagcatgtaa atatcgttag acgtaatgcc gtcaatcgtc atttttgatc 780
   cgcgggagtc agtgaacagg taccatttgc cgttcatttt aaagacgttc gcgcgttcaa 840
   tttcatctgt tactgtgtta gatgcaatca gcggtttcat cacttttttc agtgtgtaat 900
   catcgtttag ctcaatcata ccgagagcgc cgtttgctaa ctcagccgtg cgttttttat 960
   cgctttgcag aagtttttga ctttcttgac ggaagaatga tgtgcttttg ccatagtatg 1020
   ctttgttaaa taaagattct tcgccttggt agccatcttc agttccagtg tttgcttcaa 1080
   atactaagta tttgtggcct ttatcttcta cgtagtgagg atctctcagc gtatggttgt 1140
   cgcctgagct gtagttgcct tcatcgatga actgctgtac attttgatac gtttttccgt 1200
   caccgtcaaa gattgattta taatcctcta caccgttgat gttcaaagag ctgtctgatg 1260
   ctgatacgtt aacttgtgca gttgtcagtg tttgtttgcc gtaatgttta ccggagaaat 1320
   cagtgtagaa taaacggatt tttccgtcag atgtaaatgt ggctgaacct gaccattctt 1380
   gtgtttggtc ttttaggata gaatcatttg catcgaattt gtcgctgtct ttaaagacgc 1440
   ggccagcgtt tttccagctg tcaatagaag tttcgccgac tttttgatag aacatgtaaa 1500
   tcgatgtgtc atccgcattt ttaggatctc cggctaatgc aaagacgatg tggtagccgt 1560
   gatagtttgc gacagtgccg tcagcgtttt gtaatggcca gctgtcccaa acgtccaggc 1620
   cttttgcaga agagatattt ttaattgtgg acgaatcaaa ttcagaaact tgatattttt 1680
   catttttttg ctgttcaggg atttgcagca tatcatggcg tgtaatatgg gaaatgccgt 1740
   atgtttcctt atatggcttt tggttcgttt ctttcgcaaa cgcttgagtt gcgcctcctg 1800
   ccagcagtgc ggtagtaaag gttaatactg ttgcttgttt tgcaaacttt ttgatgttca 1860
   tcgttcatgt ctcctttttt atgtactgtg ttagcggtct gcttcttcca gccctcctgt 1920
   ttgaagatgg caagttagtt acgcacaata aaaaaagacc taaaatatgt aaggggtgac 1980
   gccaaagtat acactttgcc ctttacacat tttaggtctt gcctgcttta tcagtaacaa 2040
   acccgcgcga tttacttttc gacctcattc tattagactc tcgtttggat tgcaactggt 2100
   ctattttcct cttttgtttg atagaaaatc ataaaaggat ttgcagacta cgggcctaaa 2160
   gaactaaaaa atctatctgt ttcttttcat tctctgtatt ttttatagtt tctgttgcat 2220
   gggcataaag ttgccttttt aatcacaatt cagaaaatat cataatatct catttcacta 2280
   aataatagtg aacggcaggt atatgtgatg ggttaaaaag gatcggcggc cgctcgattt 2340
   aaatctcgag aggcctgacg tcgggcccgg taccacgcgt aacgcattca ctgcagtcac 2400
   aattgcttca tcactcacaa cagctgtttc gaggatgaat ttcagcacca caggagatgg 2460
   aacagcctcc ctgattgcca caatttcctg cagcaaccta ttggcatctc cctctttcac 2520
   taccgcaata tccaaaacaa catctacttc ggaagctccg gactgtacgg caaggcgcgc 2580
   ttcggcggct ttcaccaacg cgggggtttt gccgtgcggg aatcctgcga cggtggccac 2640
   tcgaattcca gcttcttggg cttttgcagt taggttgacc atgctgttgg ggacacagat 2700
   cgtgccgact cccagctcaa ttgcggaatc tataaatgcg gccagttcgg agttggttac 2760
   ttctggtccg aggagggtgt agtcaaggat ttgggccatg gtggagcggg aaatcgtcat 2820
   atccataccc tacttagacc tgacttagtg tgggaaaatt tccagggtag aatgcaacga 2880
   ggctttttgc ttttcgacgc gaccctaaac aaatgagacc tacccgaggg taggtctcat 2940
   ttgttttgtg tttagtctgt ggtggtttcg cgtagttttt cttgcttaat aaacagcaag 3000
   agaagcagcg cgatgccgat cagtggcatc atcacgtaga acactgggat gagtgcgtcg 3060
   ttgtaggaac cggcgaacgc atcgtggagc gcggttggca attgattgac gattgccggc 3120
   gtcaattcgt tggagtccag tccgccttgg gctgccatgg cggcttgttc ttctggtgaa 3180
   agttgtgcca tggctgctgg cattctttct tccatgaggg ttcccaagtt gccaacgaac 3240
   atgccaccga ccagcgcgga tccgagtgag gaaccgattt gacggaagaa gttgttcaca 3300
   gcggttgcgg atccgaccac cgcggttggc agggtgttct gaacgatcag gaccagaacc 3360
   tgcatggcta gaccgtcgac atcgatgctc ttctgcgtta attaacaatt gggatcctct 3420
   agacccggga tttaaatcgc tagcgggctg ctaaaggaag cggaacacgt agaaagccag 3480
   tccgcagaaa cggtgctgac cccggatgaa tgtcagctac tgggctatct ggacaaggga 3540
   aaacgcaagc gcaaagagaa agcaggtagc ttgcagtggg cttacatggc gatagctaga 3600
   ctgggcggtt ttatggacag caagcgaacc ggaattgcca gctggggcgc cctctggtaa 3660
   ggttgggaag ccctgcaaag taaactggat ggctttcttg ccgccaagga tctgatggcg 3720
   caggggatca agatctgatc aagagacagg atgaggatcg tttcgcatga ttgaacaaga 3780
   tggattgcac gcaggttctc cggccgcttg ggtggagagg ctattcggct atgactgggc 3840
   acaacagaca atcggctgct ctgatgccgc cgtgttccgg ctgtcagcgc aggggcgccc 3900
   ggttcttttt gtcaagaccg acctgtccgg tgccctgaat gaactgcagg acgaggcagc 3960
   gcggctatcg tggctggcca cgacgggcgt tccttgcgca gctgtgctcg acgttgtcac 4020
   tgaagcggga agggactggc tgctattggg cgaagtgccg gggcaggatc tcctgtcatc 4080
   tcaccttgct cctgccgaga aagtatccat catggctgat gcaatgcggc ggctgcatac 4140
   gcttgatccg gctacctgcc cattcgacca ccaagcgaaa catcgcatcg agcgagcacg 4200
   tactcggatg gaagccggtc ttgtcgatca ggatgatctg gacgaagagc atcaggggct 4260
   cgcgccagcc gaactgttcg ccaggctcaa ggcgcgcatg cccgacggcg aggatctcgt 4320
   cgtgacccat ggcgatgcct gcttgccgaa tatcatggtg gaaaatggcc gcttttctgg 4380
   attcatcgac tgtggccggc tgggtgtggc ggaccgctat caggacatag cgttggctac 4440
   ccgtgatatt gctgaagagc ttggcggcga atgggctgac cgcttcctcg tgctttacgg 4500
   tatcgccgct cccgattcgc agcgcatcgc cttctatcgc cttcttgacg agttcttctg 4560
   agcgggactc tggggttcga aatgaccgac caagcgacgc ccaacctgcc atcacgagat 4620
   ttcgattcca ccgccgcctt ctatgaaagg ttgggcttcg gaatcgtttt ccgggacgcc 4680
   ggctggatga tcctccagcg cggggatctc atgctggagt tcttcgccca cgctagcggc 4740
   gcgccggccg gcccggtgtg aaataccgca cagatgcgta aggagaaaat accgcatcag 4800
   gcgctcttcc gcttcctcgc tcactgactc gctgcgctcg gtcgttcggc tgcggcgagc 4860
   ggtatcagct cactcaaagg cggtaatacg gttatccaca gaatcagggg ataacgcagg 4920
   aaagaacatg tgagcaaaag gccagcaaaa ggccaggaac cgtaaaaagg ccgcgttgct 4980
   ggcgtttttc cataggctcc gcccccctga cgagcatcac aaaaatcgac gctcaagtca 5040
   gaggtggcga aacccgacag gactataaag ataccaggcg tttccccctg gaagctccct 5100
   cgtgcgctct cctgttccga ccctgccgct taccggatac ctgtccgcct ttctcccttc 5160
   gggaagcgtg gcgctttctc atagctcacg ctgtaggtat ctcagttcgg tgtaggtcgt 5220
   tcgctccaag ctgggctgtg tgcacgaacc ccccgttcag cccgaccgct gcgccttatc 5280
   cggtaact 5288
<210> 51
   <211> 1689
   <212> DNA
   <213> Corynebacterium jeikeium
<400> 51
   atgactaact cttctgcaac cagcaatccc caacccagcg acgtcgaaat cgcacaggcc     60
   cactccctgg agcccatcac cacgatcgcg gagcgcgccg gcatcccgga ggccgctttg    120
   attccctacg gccgcacaaa ggccaagatc gacgttcccg ctctgcgggc ggagcgtgag    180
   ggcgtcaata gaaagggcaa gctcgtgctc gtgaccgcaa tgagcccgac ccccgcgggt     240
   gaaggcaagt ccacggtgct gatcggcttg gcggacgcgg tgcgcacggc agggcgccag    300
   acgatggtgg cgatccgcga gccgtcccag gggccggtca tgggcatcaa gggcggcgct    360
   gctggcggcg gttatgcgca gatcgtgccg atggaagaca ttaacctgca cttcacaggc    420
   gacatgcacg ccatcaccgc cgcgacgaac actctggcgg cgatggtgga caaccacgtg    480
   cagcacggca atgctctggg catcgacccg cggcgggtga cgtggcggcg ctgcctggac    540
   gtcaacgacc gctcgctgcg ccatgtggtc acaggcctag gcggccctgg tcagggcacg    600
   ccccgcgagg gcggattcga tatcacggcg gccagcgaga tcatggctat cctctgtctg    660
   gccaccgacc tggaagatct aaagaagcgc atcgggcgca tcgtggtggg ccaaacttat    720
   gatcgcaagc cagtcaccgc gggcgacctg aagtgcgcgg gcgctatcac cgccctgctg    780
   cgcgatgcga tcaacccgaa cctagtgcag accctgggtg gcacccccgc actggtgcac    840
   ggtgggcctt tcgccaacat cgcgcacgga tgcaactcgc tgatcgcaac caccacggcg    900
   ctggacctgt cggaggttgt gctgaccgag gccggcttcg gcagcgacct gggtgcggag    960
   aagttcttcg acatcaagtc ccgcgccggt ggcctcgacg tcgccgcaac ggtcgtggtg   1020
   gccaccatcc gctcgctaaa gcacaacggc gattccgtac taaaggccgg tttggccaac   1080
   ctggagcgcc acgtcagcaa cattcgcaag ttcggcgtgg aacccgtggt cgcgctaaac   1140
   ctgtttaact ctgacaccgc caccgagcgc gctcaggtcg ccgactgggg cgagcagttc   1200
   ggcgtccgcg tggtggagtg caacgtgtgg gccgagggcg gtgccggcgc gaccgatctg   1260
   gcctctgcgg tgctggaggt cgttgatggt gtttctgatg aggacgcccc cagctccagc   1320
   caccagatct atcagccggt cgatggtgtg gaagccacct tgcgcacact tgccacggag   1380
   atctacggcg cggcggacgt acagttcggc ccgcaggctc tgaaggacct ggcgtttctg   1440
   aaggataacg gctgggacaa gctgccggtg tgcgtgtcga agacgcagta ctccttcagc   1500
   gatgacccta gcgcgctggg cgcgccaagc ggccacaccc tgcacgtccg cgagctggta   1560
   ccacgtatcg gtgccggatt cgtggtggca ctcaccggcg atgtgatgac tctgccgggc   1620
   ctgccgaaga aacccgcggc cgagcgcatt gatgtgaacg cgcagggggt tatctcgggg   1680
   ctgttctaa                                                           1689
<210> 52
   <211> 562
   <212> PRT
   <213> Corynebacterium jeikeium
<400> 52
   Met Thr Asn Ser Ser Ala Thr Ser Asn Pro Gln Pro Ser Asp Val Glu
   1               5                   10                  15
   Ile Ala Gln Ala His Ser Leu Glu Pro Ile Thr Thr Ile Ala Glu Arg
               20                  25                  30
   Ala Gly Ile Pro Glu Ala Ala Leu Ile Pro Tyr Gly Arg Thr Lys Ala
           35                  40                  45
   Lys Ile Asp Val Pro Ala Leu Arg Ala Glu Arg Glu Gly Val Asn Arg
       50                  55                  60
   Lys Gly Lys Leu Val Leu Val Thr Ala Met Ser Pro Thr Pro Ala Gly
   65                  70                  75                  80
   Glu Gly Lys Ser Thr Val Leu Ile Gly Leu Ala Asp Ala Val Arg Thr
                   85                  90                  95
   Ala Gly Arg Gln Thr Met Val Ala Ile Arg Glu Pro Ser Gln Gly Pro
               100                 105                 110
   Val Met Gly Ile Lys Gly Gly Ala Ala Gly Gly Gly Tyr Ala Gln Ile
           115                 120                 125
   Val Pro Met Glu Asp Ile Asn Leu His Phe Thr Gly Asp Met His Ala
   130 135 140
   Ile Thr Ala Ala Thr Asn Thr Leu Ala Ala Met Val Asp Asn His Val
   145 150 155 160
   Gln His Gly Asn Ala Leu Gly Ile Asp Pro Arg Arg Val Thr Trp Arg
   165 170 175
   Arg Cys Leu Asp Val Asn Asp Arg Ser Leu Arg His Val Val Thr Gly
   180 185 190
   Leu Gly Gly Pro Gly Gln Gly Thr Pro Arg Glu Gly Gly Phe Asp Ile
   195 200 205
   Thr Ala Ala Ser Glu Ile Met Ala Ile Leu Cys Leu Ala Thr Asp Leu
   210 215 220
   Glu Asp Leu Lys Lys Arg Ile Gly Arg Ile Val Val Gly Gln Thr Tyr
   225 230 235 240
   Asp Arg Lys Pro Val Thr Ala Gly Asp Leu Lys Cys Ala Gly Ala Ile
   245 250 255
   Thr Ala Leu Leu Arg Asp Ala Ile Asn Pro Asn Leu Val Gln Thr Leu
   260 265 270
   Gly Gly Thr Pro Ala Leu Val His Gly Gly Pro Phe Ala Asn Ile Ala
   275 280 285
   His Gly Cys Asn Ser Leu Ile Ala Thr Thr Thr Ala Leu Asp Leu Ser
   290 295 300
   Glu Val Val Leu Thr Glu Ala Gly Phe Gly Ser Asp Leu Gly Ala Glu
   305 310 315 320
   Lys Phe Phe Asp Ile Lys Ser Arg Ala Gly Gly Leu Asp Val Ala Ala
   325 330 335
   Thr Val Val Val Ala Thr Ile Arg Ser Leu Lys His Asn Gly Asp Ser
   340 345 350
   Val Leu Lys Ala Gly Leu Ala Asn Leu Glu Arg His Val Ser Asn Ile
   355 360 365
   Arg Lys Phe Gly Val Glu Pro Val Val Ala Leu Asn Leu Phe Asn Ser
   370 375 380
   Asp Thr Ala Thr Glu Arg Ala Gln Val Ala Asp Trp Gly Glu Gln Phe
   385                 390                 395                 400
   Gly Val Arg Val Val Glu Cys Asn Val Trp Ala Glu Gly Gly Ala Gly
                   405                 410                 415
   Ala Thr Asp Leu Ala Ser Ala Val Leu Glu Val Val Asp Gly Val Ser
               420                 425                 430
   Asp Glu Asp Ala Pro Ser Ser Ser His Gln Ile Tyr Gln Pro Val Asp
           435                 440                 445
   Gly Val Glu Ala Thr Leu Arg Thr Leu Ala Thr Glu Ile Tyr Gly Ala
       450                 455                 460
   Ala Asp Val Gln Phe Gly Pro Gln Ala Leu Lys Asp Leu Ala Phe Leu
   465                 470                 475                 480
   Lys Asp Asn Gly Trp Asp Lys Leu Pro Val Cys Val Ser Lys Thr Gln
                   485                 490                 495
   Tyr Ser Phe Ser Asp Asp Pro Ser Ala Leu Gly Ala Pro Ser Gly His
               500                 505                 510
   Thr Leu His Val Arg Glu Leu Val Pro Arg Ile Gly Ala Gly Phe Val
           515                 520                 525
   Val Ala Leu Thr Gly Asp Val Met Thr Leu Pro Gly Leu Pro Lys Lys
       530                 535                 540
   Pro Ala Ala Glu Arg Ile Asp Val Asn Ala Gln Gly Val Ile Ser Gly
   545                 550                 555                 560
   Leu Phe